# EUROPEAN PATENT APPLICATION

(11) **EP 1 096 012 A1**
(43) Date of publication of application: **02.05.2001**
(21) Application number: 99402668.0
(22) Date of filing: 26.10.1999
(51) Int. Cl.: C12N 15/52, C07K 14/705, C12Q 1/68, C07K 16/18

(54) **Nucleics acids of the human ABC1 gene and their therapeutic and diagnostic application**

(71) Applicant: Aventis Pharma S.A., 92160 Antony (FR)
(72) Inventor: Denefle, Patrice, 94100 Saint Maur (FR); Rosier-Montus, Marie-Françoise, 92160 Antony (FR); Arnould-Reguigne, Isabelle, 94430 Chennevieres Sur Marne (FR); Prades, Catherine, 94320 Thais (FR); Naudin, Laurent, 91150 Etampes (FR); Lemoine, Cendrine, 93310 Montlhery (FR); Duverger, Nicolas, 75005 Paris (FR); Jaye, Michael, Glennside, Pennsylvania 19038 (US); Searfoss III, George H., Birdsboro, Pennsylvania 19508 (US); Remaley, Alan, Bethesda, Maryland 20892 (US); Brewer, H. Bryan, Potomac, Maryland 20854 (US); Dean, Michael, Frederick, Maryland 21703 (US)
(74) Representative: Bouvet, Philippe

(57) **Abstract**

The present invention relates to nucleic acids corresponding to the various exons and introns of the ABC1 gene, which is a causal gene for pathologies linked to a cholesterol metabolism dysfunction inducing diseases such as atherosclerosis, more particularly disruption in the reverse transport of cholesterol, and more particularly familial HDL deficiencies (FHD), such as Tangier disease. The present invention also relates to ABC1 cDNAs encoding the novel full length ABC1 protein. The invention also relates to means for the detection of polymorphisms in general, and of mutations in particular, in the ABC1 gene or in the corresponding protein produced by the allelic form of the ABC1 gene.

## Description

### FIELD OF THE INVENTION

The present invention relates to nucleic acids corresponding to the various exons and introns of the ABC1 gene, which is a causal gene for pathologies linked to a cholesterol metabolism dysfunction inducing diseases such as atherosclerosis, more particularly disruption in the reverse transport of cholesterol, and more particularly familial HDL deficiencies (FHD), such as Tangier disease. The present invention also relates to ABC1 cDNAs encoding the novel full length ABC1 protein. The invention also relates to means for the detection of polymorphisms in general, and of mutations in particular, in the ABC1 gene or in the corresponding protein produced by the allelic form of the ABC1 gene.

### BACKGROUND OF THE INVENTION

Lipids are water-insoluble organic biomolecules, which are essential components of diverse biological functions, including the storage, transport, and metabolism of energy, and membrane structure and fluidity. Lipids are derived from two sources in humans and other animals: some lipids are ingested as dietary fats and oils and other lipids are biosynthesized by the human or animal. In mammals at least 10% of the body weight is lipid, the bulk of which is in the form of triacylglycerols.

Triacylglycerols, also known as triglycerides and triacylglycerides, are made up of three fatty acids esterified to glycerol. Dietary triacylglycerols are stored in adipose tissues as a source of energy, or hydrolyzed in the digestive tract by triacylglycerol lipases, the most important of which is pancreatic lipase. Triacylglycerols are transported between tissues in the form of lipoproteins.

Lipoproteins are micelle-like assemblies found in plasma and contain varying proportions of different types of lipids and proteins (called apoproteins). There are five main classes of plasma lipoproteins, the major function of which is lipid transport. These classes are, in order of increasing density, chylomicrons, very low density lipoproteins (VLDL), intermediate-density lipoproteins (IDL), low density lipoproteins (LDL), and high density lipoproteins (HDL). Although many types of lipid are found associated with each lipoprotein class, each class transports predominantly one type of lipid: triacylglycerols are transported in chylomicrons, VLDL, and IDL; while phospholipids and cholesterol esters are transported in HDL and LDL respectively.

Phospholipids are di-fatty acid esters of glycerol phosphate, also containing a polar group coupled to the phosphate. Phospholipids are important structural components of cellular membranes. Phospholipids are hydrolyzed by enzymes called phospholipases. Phosphatidylcholine, an exemplary phospholipid, is a major component of most eukaryotic cell membranes.

Cholesterol is the metabolic precursor of steroid hormones and bile acids as well as an essential constituent of cell membranes. In humans and other animals, cholesterol is ingested in the diet and also synthesized by the liver and other tissues. Cholesterol is transported between tissues in the form of cholesteryl esters in LDLs and other lipoproteins.

Membranes surround every living cell, and serve as a barrier between the intracellular and extracellular compartments. Membranes also enclose the eukaryotic nucleus, make up the endoplasmic reticulum, and serve specialized functions such as in the myelin sheath that surrounds axons. A typical membrane contains about 40% lipid and 60% protein, but there is considerable variation. The major lipid components are phospholipids, specifically phosphatidylcholine and phosphatidylethanolamine, and cholesterol. The physicochemical properties of membranes, such as fluidity, can be changed by modification of either the fatty acid profiles of the phospholipids or the cholesterol content. Modulating the composition and organization of membrane lipids also modulates membrane-dependent cellular functions, such as receptor activity, endocytosis, and cholesterol flux.

High-density lipoproteins (HDL) are one of the four major classes of lipoproteins circulating in blood plasma. These lipoproteins are involved in various metabolic pathways such as lipid transport, the formation of bile acids, steroidogenesis, cell proliferation and, in addition, interfere with the plasma proteinase systems.

HDLs are perfect free cholesterol acceptors and, in combination with the cholesterol ester transfer proteins (CETP), lipoprotein lipase (LPL), hepatic lipase (HL) and lecithin:cholesterol acyltransferase (LCAT), play a major role in the reverse transport of cholesterol, that is to say the transport of excess cholesterol in the peripheral cells to the liver for its elimination from the body in the form of bile acid. It has been demonstrated that the HDLs play a central role in the transport of cholesterol from the peripheral tissues to the liver.

Various diseases linked to an HDL deficiency have been described, including Tangier and/or FHD disease, HDL deficiency, and LCAT deficiency. In addition, HDL-cholesterol deficiencies have been observed in patients suffering from malaria and diabetes (Kittl et al., 1992; Nilsson et al., 1990; Djoumessi, 1989; Mohanty et al., 1992; Maurois et al., 1985; Grellier et al., 1997; Agbedana et al., 1990; Erel et al., 1998; Cuisinier et al., 1990; Chander et al., 1998; Efthimiou et al., 1992; Baptista et al., 1996; Davis et al., 1993; Davis et al., 1995; Pirich et al., 1993; Tomlinson and Raper, 1996; Hager and Hajduk, 1997, Kwiterovich, 1995, Syvanne et al., 1995a, Syvanne et al., 1995b, and French et al., 1993). The deficiency involved in Tangier and/or FHD disease is linked to a cellular defect in the translocation of cellular cholesterol which causes a degradation of the HDLs and leads to a disruption in the lipoprotein metabolism. Nevertheless, for Tangier and/or FHD disease, the exact nature of the defect has not yet been precisely defined.

Tangier disease is an autosomal co-dominant condition characterized in the homozygous state by the absence of HDL-cholesterol (HDL-C) from plasma, hepatosplenomegaly, peripheral neuropathy, and frequently premature coronary artery disease (CAD). In heterozygotes, HDL-C levels are about one-half those of normal individuals. Impaired cholesterol efflux from macrophages leads to the presence of foam cells throughout the body, which may explain the increased risk of CAD in some Tangier disease families.

In Tangier disease patients, the HDL particles do not incorporate cholesterol from the peripheral cells, are not metabolized correctly, and are rapidly eliminated from the body. The plasma HDL concentration in these patients is therefore, extremely reduced and the HDLs no longer ensure the return of cholesterol to the liver. Cholesterol accumulates in these peripheral cells and causes characteristic clinical manifestations such as the formation of orange-colored tonsils. Furthermore, other lipoprotein disruptions, such as overproduction of triglycerides as well as increased synthesis and intracellular catabolism of phospholipids are also observed in Tangier disease patients.

Tangier disease, whose symptoms have been described above, is classified among the familial conditions linked to the metabolism of HDLs, which are the ones most commonly detected in patients affected by coronary diseases. Numerous studies have shown that a reduced level of HDL cholesterol is an excellent indicator of an individual's risk of developing or already having a cardiovascular condition. In this context, syndromes linked to HDL deficiencies have been of increasing interest for the past decade because they make it possible to increase understanding of the role of HDLs in atherogenesis.

Atherosclerosis is defined in histological terms by deposits (lipid or fibrolipid plaques) of lipids and of other blood derivatives in blood vessel walls, especially the large arteries (aorta, coronary arteries, carotid). These plaques, which are more or less calcified according to the degree of progression of the atherosclerotic process, may be coupled with lesions and are associated with the accumulation in the vessels of fatty deposits consisting essentially of cholesterol esters. These plaques are accompanied by a thickening of the vessel wall, hypertrophy of the smooth muscle, appearance of foam cells (lipid-laden cells resulting from uncontrolled uptake of cholesterol by recruited macrophages) and accumulation of fibrous tissue. The atheromatous plaque protrudes markedly from the wall, endowing it with a stenosing character responsible for vascular occlusions by atheroma, thrombosis or embolism, which occur in those patients who are most affected. These lesions can lead to serious cardiovascular pathologies such as infarction, sudden death, cardiac insufficiency, and stroke.

Mutations within genes that play a role in lipoprotein metabolism have been identified. Specifically, several mutations in the apolipoprotein apo A-I gene have been characterized. These mutations are rare and may lead to a lack of production of apo A-I. Mutations in the genes encoding LPL or its activator apo C-II are associated with severe hypertriglyceridemias and substantially reduced HDL-C levels. Mutations in the gene encoding the enzyme LCAT are also associated with a severe HDL deficiency.

In addition, dysfunctions in the reverse transport of cholesterol may be induced by physiological deficiencies affecting one or more of the steps in the transport of stored cholesterol, from the intracellular vesicles to the membrane surface where it is accepted by the HDLs.

Therefore, an increasing need exists in the state of the art to identify genes involved in any of the steps in the metabolism of cholesterol and/or lipoproteins, and in particular, genes associated with dysfunctions in the reverse transport of cholesterol from the peripheral cells to the liver.

Recently, a study was conducted on the segregation of different allelic forms of 343 microsatellite markers distributed over the entire genome and distant from each other by 10.3 cM on average (Rust et al., 1998). This linkage study was carried out on a family comprising five consanguineous lines and which has been well characterized over eleven generations, in which many members are affected by Tangier disease. This study identified a region located in the 9q31 locus of human chromosome 9 that is statistically associated with Tangier disease.

However, the study by Rust et al. (1998) only defined a wide region of the genome whose impairments are likely to be associated with Tangier disease and that the relevant 9q31-34 region contains ESTs but no known gene. It has since been shown that a region of about 1cM situated in the 9q31 locus in humans was generally associated with familial HDL deficiencies (Rust et al., 1999 and Brooks-Wilson et al., 1999). More precisely, it has been shown that a gene encoding a protein of the family of ABC transporters, which is located precisely in the region of 1 cM of the 9q31 locus, was involved in pathologies linked to a deficiency in the reverse transport of cholesterol. More particularly, mutations have been described within the gene encoding the ABC-1 transporter in patients impaired in the reverse transport of cholesterol, and most particularly in patients suffering from Tangier and FHD diseases (Rust et al., 1999, Brooks-Wilson et al., 1999, Bodzioch et al., 1999, Marcil et al., 1999b, and Lawn et al., 1999).

The ABC (ATP-binding cassette) transport proteins constitute a family of proteins that are extremely well conserved during evolution, from bacteria to humans. The ABC transport proteins are involved in membrane transport of various substrates, for example ions, amino acids, peptides, sugars, vitamins or steroid hormones.

The characterization of the complete amino acid sequence of some ABC transporters has made it possible to determine that these proteins had a common general structure, in particular two nucleotide binding folds (NBF) with Walker A and B type units as well as two transmembrane domains, each of the transmembrane domains consisting of six helices. The specificity of the ABC transporters for the various transported molecules appears to be determined by the structure of the transmembrane domains, whereas the energy necessary for the transport activity is provided by the degradation of ATP at the level of the NBF fold.

Several ABC transport proteins that have been identified in humans are associated with various diseases. For example, cystic fibrosis is caused by mutations in the CFTR (cystic fibrosis transmembrane conductance regulator) gene. Moreover, some multiple drug resistance phenotypes in tumor cells have been associated with mutations in the gene encoding the MDR (multi-drug resistance) protein, which also has an ABC transporter structure. Other ABC transporters have been associated with neuronal and tumor conditions (US Patent No. 5,858,719) or potentially involved in diseases caused by impairment of the homeostasis of metals, such as the ABC-3 protein. Likewise, another transport ABC, designated PFIC2, appears to be involved in a progressive familial intrahepatic cholestasia form, this protein being potentially responsible, in humans, for the export of bile salts.

In 1994, a cDNA encoding a new mouse ABC transporter was identified and designated ABC1 (Luciani et al., 1994). This protein is characteristic of the ABC transporters in that it has a symmetrical structure comprising two transmembrane domains linked to a highly hydrophobic segment and two NBF units.

In humans, a partial cDNA comprising a 6603 base pair (bp) open reading frame (ORF) of the human ABC1 transporter has been identified (Langmann et al., 1999). Based upon this partial cDNA, the human ABC1 protein is predicted to comprise 2201 amino acids (aa), which results in a protein of approximately 220 kDa in size (*Ibid*.). This study also showed that the gene encoding the human ABC1 protein is expressed in various tissues, and more particularly at high levels in the placenta, the liver, the lungs, the adrenal glands, as well as several fetal tissues. These authors demonstrated that the expression of the gene encoding the human ABC1 protein was induced during the differentiation of monocytes into macrophages *in vitro.* Furthermore, this study reported that the expression of the gene encoding the ABC1 protein is increased when human macrophages are incubated in the presence of acetylated low-density lipoproteins (AcLDLs).

Recently, partial exonic and intronic ABC1 genomic DNA and an additional portion of the 3' untranslated region (UTR) of the human ABC1 cDNA have been isolated and identified (Rust et al., 1999).

While it has been shown that patients suffering from Tangier and FHD diseases have a mutated ABC1 gene (Rust et al., 1999, Brooks-Wilson et al., 1999, Bodzioch et al., 1999, Marcil et al., 1999b, and Lawn et al., 1999), the exact role of the human ABC1 protein in the lipid transport system is unknown. It is simply assumed that the ABC1protein has a translocase activity for membrane lipid transport.

### SUMMARY OF THE INVENTION

The present invention relates to nucleic acids corresponding to the various exons and introns of the human ABC1 gene, which is a causal gene for pathologies linked to a cholesterol metabolism dysfunction inducing diseases such as atherosclerosis, more particularly disruption in the reverse transport of cholesterol, and familial HDL deficiencies, such as Tangier and/or FHD disease.

Thus, a first subject of the invention is a nucleic acid comprising a polynucleotide sequence of any one of SEQ ID NOs: 1, 4-65, 68, 72-88, 103, 109-118, and 137, or of a complementary polynucleotide sequence.

The invention also relates to a nucleic acid comprising at least 8 consecutive nucleotides of a polynucleotide sequence of a) any one of SEQ ID NOs: 1, 4-8, 11-16, 18-26, 28-35, 37-52, 54-65, 68, and 137, or of a complementary polynucleotide sequence; b) nucleotides 3154-3200 of SEQ ID NO: 9 or of a complementary polynucleotide sequence; c) nucleotides 1-242 of SEQ ID NO: 10 or of a complementary polynucleotide sequence; d) nucleotides 1-1851 of SEQ ID NO: 17; or of a complementary polynucleotide sequence; e) nucleotides 152-198 of SEQ ID NO: 27, or of a complementary polynucleotide sequence; f) nucleotides 1-1657 of SEQ ID NO: 36, or of a complementary polynucleotide sequence; or g) nucleotides 1-242 of SEQ ID NO: 53, or of a complementary polynucleotide sequence.

The invention also relates to a nucleic acid having at least 80% nucleotide identity with a nucleic acid comprising a polynucleotide sequence of a) any one of SEQ ID NOs: 1, 4-8, 11-16, 18-26, 28-35, 37-52, 54-65, 68, and 137, or of a complementary polynucleotide sequence; b) nucleotides 3154-3200 of SEQ ID NO: 9 or of a complementary polynucleotide sequence; c) nucleotides 1-242 of SEQ ID NO: 10 or of a complementary polynucleotide sequence; d) nucleotides 1-1851 of SEQ ID NO: 17; or of a complementary polynucleotide sequence; e) nucleotides 152-198 of SEQ ID NO: 27, or of a complementary polynucleotide sequence; f) nucleotides 1-1657 of SEQ ID NO: 36, or of a complementary polynucleotide sequence; or g) nucleotides 1-242 of SEQ ID NO: 53, or of a complementary polynucleotide sequence.

The invention also relates to a nucleic acid having at least 85%, preferably 90%, more preferably 95% and still more preferably 98% nucleotide identity with a nucleic acid comprising a polynucleotide sequence of a) any one of SEQ ID NOs: 1, 4-8, 11-16, 18-26, 28-35, 37-52, 54-65, 68, and 137, or of a complementary polynucleotide sequence; b) nucleotides 3154-3200 of SEQ ID NO: 9 or of a complementary polynucleotide sequence; c) nucleotides 1-242 of SEQ ID NO: 10 or of a complementary polynucleotide sequence; d) nucleotides 1-1851 of SEQ ID NO: 17; or of a complementary polynucleotide sequence; e) nucleotides 152-198 of SEQ ID NO: 27, or of a complementary polynucleotide sequence; f) nucleotides 1-1657 of SEQ ID NO: 36, or of a complementary polynucleotide sequence; or g) nucleotides 1-242 of SEQ ID NO: 53, or of a complementary polynucleotide sequence.

The invention also relates to a nucleic acid hybridizing, under high stringency conditions, with a polynucleotide of a) any one of SEQ ID NOs: 1, 4-8, 11-16, 18-26, 28-35, 37-52, 54-65, 68, and 137, or of a complementary polynucleotide sequence; b) nucleotides 3154-3200 of SEQ ID NO: 9 or of a complementary polynucleotide sequence; c) nucleotides 1-242 of SEQ ID NO: 10 or of a complementary polynucleotide sequence; d) nucleotides 1-1851 of SEQ ID NO: 17; or of a complementary polynucleotide sequence; e) nucleotides 152-198 of SEQ ID NO: 27, or of a complementary polynucleotide sequence; f) nucleotides 1-1657 of SEQ ID NO: 36, or of a complementary polynucleotide sequence; or g) nucleotides 1-242 of SEQ ID NO: 53, or of a complementary polynucleotide sequence.

The invention also relates to nucleic acids, particularly cDNA molecules, which encode the full length human ABC1 protein. Thus, the invention relates to a nucleic acid comprising a polynucleotide sequence of either SEQ ID NO: 69 or SEQ ID NO: 70, or of a complementary polynucleotide sequence.

The invention also relates to a nucleic acid comprising a polynucleotide sequence as depicted in either SEQ ID NO: 69 or SEQ ID NO: 70, or a complementary polynucleotide sequence.

The invention also relates to a nucleic acid comprising nucleotides 1-244 of SEQ ID NO: 69 or nucleotides 1-357 of SEQ ID NO: 70, or a complementary polynucleotide sequence.

The invention also relates to a nucleic acid comprising at least eight consecutive nucleotides of nucleotides 1-244 of SEQ ID NO: 69 or nucleotides 1-357 of SEQ ID NO: 70, or a complementary polynucleotide sequence.

The subject of the invention is also a nucleic acid having at least 80% nucleotide identity with a nucleic acid comprising nucleotides 1-244 of SEQ ID NO: 69 or nucleotides 1-357 of SEQ ID NO: 70, or a complementary polynucleotide sequence.

The invention also relates to a nucleic acid having at least 85%, preferably 90%, more preferably 95% and still more preferably 98% nucleotide identity with a nucleic acid comprising nucleotides 1-244 of SEQ ID NO: 69 or nucleotides 1-357 of SEQ ID NO: 70, or a complementary polynucleotide sequence.

Another subject of the invention is a nucleic acid hybridizing, under high stringency conditions, with a nucleic acid comprising nucleotides 1-244 of SEQ ID NO: 69 or nucleotides 1-357 of SEQ ID NO: 70, or a complementary polynucleotide sequence.

According to the invention, a nucleic acid comprising a polynucleotide sequence of either SEQ ID NO: 69 or SEQ ID NO: 70 encodes a full length ABC1 polypeptide of 2261 amino acids comprising the amino acid sequence of SEQ ID NO: 71.

Thus, the invention also relates to a nucleic acid encoding a polypeptide comprising an amino acid sequence of SEQ ID NO: 71.

In a specific embodiment, the invention also relates to a nucleic acid encoding a polypeptide comprising amino acids 1-60 of SEQ ID NO: 71.

Thus, the invention also relates to a polypeptide comprising an amino acid sequence of SEQ ID NO: 71.

The invention also relates to a polypeptide comprising an amino acid sequence as depicted in SEQ ID NO: 71.

The invention also relates to a polypeptide comprising amino acids 1-60 of SEQ ID NO: 71.

The invention also relates to a polypeptide comprising an amino acid sequence having at least 80% amino acid identity with a polypeptide comprising amino acids 1-60 of SEQ ID NO: 71.

The invention also relates to a polypeptide having at least 85%, preferably 90%, more preferably 95% and still more preferably 98% amino acid identity with a polypeptide comprising amino acids 1-60 of SEQ ID NO: 71.

Preferably, a polypeptide according to the invention will have a length of 15, 18 or 20 to 25, 35, 40, 50, 70, 80, 100 or 200 consecutive amino acids of a polypeptide according to the invention, in particular a polypeptide comprising an amino acid sequence comprising amino acids 1-60 of SEQ ID NO: 71.

Alternatively, a polypeptide according to the invention will comprise a fragment having a length of 15, 18, 20, 25, 35, 40, 50, 100 or 200 consecutive amino acids of a polypeptide according to the invention, more particularly of a polypeptide comprising an amino acid sequence comprising amino acids 1-60 of SEQ ID NO: 71.

The invention also relates to a means for detecting polymorphisms in general, and mutations in particular, in the ABC1 gene or in the corresponding protein produced by the allelic form of the ABC1 gene. It has been recently shown that patients suffering from Tangier and FHD diseases have a mutated ABC1 gene (Rust et al., 1999, Brooks-Wilson et al., 1999, Bodzioch et al., 1999, and Marcil et al., 1999b). According to the present invention, several additional mutations distributed in different exons of the ABC1 gene have been identified in the genome of various patients, in particular patients suffering from a severe form of the disease associated with cardiovascular disorders. Moreover, various polymorphisms have been found in the introns of the ABC1 gene in patients suffering from the mildest forms of the disease, indicating that these patients carry particular alleles of the gene, distinct from the "wild-type" allele(s). Such alleles, partly characterized by these polymorphisms, are likely to contain substitutions, additions or deletions of nucleotides in the noncoding regions located respectively on the 5' side of the first exon or alternatively on the 3' side of the last exon of the gene, in particular in the regulatory regions, for example, in the promoter sequences or alternatively in the enhancer sequences, of the type which induces defects. These defects may result in either an increase or a decrease in the synthesis of the ABC1 polypeptide.

Thus, the invention also relates to a polypeptide encoded by a mutated ABC1gene, and more particularly a mutated ABC1 gene in patients suffering from a deficiency in the reverse transport of cholesterol, most particularly in patients suffering from Tangier disease.

The invention therefore relates to a polypeptide comprising an amino acid sequence of any one of SEQ ID NOs: 89-102.

The invention also relates to a polypeptide comprising an amino acid sequence as depicted in any one of SEQ ID NOs: 89-102.

Thus, another subject of the invention is a nucleic acid encoding a mutated ABC1 polypeptide, wherein the nucleic acid comprises a polynucleotide sequence of any one of SEQ ID NOs: 72-88, or of a complementary polynucleotide sequence.

The invention also relates to a nucleic acid encoding a mutated ABC1 polypeptide, wherein the nucleic acid comprises a polynucleotide sequence as depicted in any one of SEQ ID NOs: 72-88, or of a complementary polynucleotide sequence.

The invention also relates to a nucleic acid encoding a polypeptide comprising any one of SEQ ID NOs: 89-102.

According to another aspect, the invention also relates to the nucleotide sequences of the ABC1 gene comprising at least one biallelic polymorphism. Thus, another subject of the invention is a nucleic acid comprising a polynucleotide sequence of any one of SEQ ID NOs: 103 and 109-118, or of a complementary polynucleotide sequence.

The invention also relates to a nucleic acid comprising a polynucleotide sequence comprising any one of SEQ ID NOs: 103 and 109-118, or of a complementary polynucleotide sequence.

The invention also relates to a nucleic acid comprising a polynucleotide sequence as depicted in any one of SEQ ID NOs: 103 and 109-118, or of a complementary polynucleotide sequence.

Nucleotide probes and primers hybridizing with a nucleic acid sequence located in the region of an ABC1 nucleic acid (genomic DNA, messenger RNA, cDNA), in particular, a nucleic acid sequence comprising any one of the mutations or polymorphisms described above, also form part of the invention.

According to the invention, nucleic acid fragments derived from a nucleic acid comprising a polynucleotide sequence of a) any one of SEQ ID NOs: 1, 4-8, 11-16, 18-26, 28-35, 37-52, 54-65, 68, and 137, or of a complementary polynucleotide sequence; b) nucleotides 3154-3200 of SEQ ID NO: 9 or of a complementary polynucleotide sequence; c) nucleotides 1-242 of SEQ ID NO: 10 or of a complementary polynucleotide sequence; d) nucleotides 1-1851 of SEQ ID NO: 17; or of a complementary polynucleotide sequence; e) nucleotides 152-198 of SEQ ID NO: 27, or of a complementary polynucleotide sequence; f) nucleotides 1-1657 of SEQ ID NO: 36, or of a complementary polynucleotide sequence; g) nucleotides 1-242 of SEQ ID NO: 53, or of a complementary polynucleotide sequence, or h) nucleotides 1-244 of SEQ ID NO: 69 or nucleotides 1-357 of SEQ ID NO: 70, or a complementary polynucleotide sequence, are useful for the detection of the presence of at least one copy of a nucleotide sequence of the ABC1 gene or of a fragment or of a variant (containing a mutation or a polymorphism) thereof in a sample.

The nucleotide probes or primers according to the invention comprise at least 8 consecutive nucleotides of a nucleic acid comprising a) any one of SEQ ID NOs: 1, 4-8, 11-16, 18-26, 28-35, 37-52, 54-65, 68, and 137, or of a complementary polynucleotide sequence; b) nucleotides 3154-3200 of SEQ ID NO: 9 or of a complementary polynucleotide sequence; c) nucleotides 1-242 of SEQ ID NO: 10 or of a complementary polynucleotide sequence; d) nucleotides 1-1851 of SEQ ID NO: 17; or of a complementary polynucleotide sequence; e) nucleotides 152-198 of SEQ ID NO: 27, or of a complementary polynucleotide sequence; f) nucleotides 1-1657 of SEQ ID NO: 36, or of a complementary polynucleotide sequence; g) nucleotides 1-242 of SEQ ID NO: 53, or of a complementary polynucleotide sequence, or h) nucleotides 1-244 of SEQ ID NO: 69 or nucleotides 1-357 of SEQ ID NO: 70, or a complementary polynucleotide sequence.

Preferably, nucleotide probes or primers according to the invention will have a length of 10, 12, 15, 18 or 20 to 25, 35, 40, 50, 70, 80, 100, 200, 500, 1000, 1500 consecutive nucleotides of a nucleic acid according to the invention, in particular of a nucleic acid comprising a) any one of SEQ ID NOs: 1,4-8, 11-16, 18-26, 28-35, 37-52, 54-65, 68, and 137, or of a complementary polynucleotide sequence; b) nucleotides 3154-3200 of SEQ ID NO: 9 or of a complementary polynucleotide sequence; c) nucleotides 1-242 of SEQ ID NO: 10 or of a complementary polynucleotide sequence; d) nucleotides 1-1851 of SEQ ID NO: 17; or of a complementary polynucleotide sequence; e) nucleotides 152-198 of SEQ ID NO: 27, or of a complementary polynucleotide sequence; f) nucleotides 1-1657 of SEQ ID NO: 36, or of a complementary polynucleotide sequence; g) nucleotides 1-242 of SEQ ID NO: 53, or of a complementary polynucleotide sequence, or h) nucleotides 1-244 of SEQ ID NO: 69 or nucleotides 1-357 of SEQ ID NO: 70, or a complementary polynucleotide sequence.

Alternatively, a nucleotide probe or primer according to the invention will consist of and/or comprise the fragments having a length of 12, 15, 18, 20, 25, 35, 40, 50, 100, 200, 500, 1000, 1500 consecutive nucleotides of a nucleic acid according to the invention, more particularly of a nucleic acid comprising a) any one of SEQ ID NOs: 1, 4-8, 11-16, 18-26, 28-35, 37-52, 54-65, 68, and 137, or of a complementary polynucleotide sequence; b) nucleotides 3154-3200 of SEQ ID NO: 9 or of a complementary polynucleotide sequence; c) nucleotides 1-242 of SEQ ID NO: 10 or of a complementary polynucleotide sequence; d) nucleotides 1-1851 of SEQ ID NO: 17; or of a complementary polynucleotide sequence; e) nucleotides 152-198 of SEQ ID NO: 27, or of a complementary polynucleotide sequence; f) nucleotides 1-1657 of SEQ ID NO: 36, or of a complementary polynucleotide sequence; g) nucleotides 1-242 of SEQ ID NO: 53, or of a complementary polynucleotide sequence, or h) nucleotides 1-244 of SEQ ID NO: 69 or nucleotides 1-357 of SEQ ID NO: 70, or a complementary polynucleotide sequence.

The definition of a nucleotide probe or primer according to the invention therefore covers oligonucleotides which hybridize, under the high stringency hybridization conditions defined above, with a nucleic acid comprising a) any one of SEQ ID NOs: 1,4-8, 11-16, 18-26, 28-35, 37-52, 54-65, 68, and 137, or of a complementary polynucleotide sequence; b) nucleotides 3154-3200 of SEQ ID NO: 9 or of a complementary polynucleotide sequence; c) nucleotides 1-242 of SEQ ID NO: 10 or of a complementary polynucleotide sequence; d) nucleotides 1-1851 of SEQ ID NO: 17; or of a complementary polynucleotide sequence; e) nucleotides 152-198 of SEQ ID NO: 27, or of a complementary polynucleotide sequence; f) nucleotides 1-1657 of SEQ ID NO: 36, or of a complementary polynucleotide sequence; g) nucleotides 1-242 of SEQ ID NO: 53, or of a complementary polynucleotide sequence, or h) nucleotides 1-244 of SEQ ID NO: 69 or nucleotides 1-357 of SEQ ID NO: 70, or a complementary polynucleotide sequence.

The preferred probes and primers according to the invention comprise all or part of a polynucleotide sequence comprising any one of SEQ ID NOs: 119-136, 138, and 141-152, or of a complementary polynucleotide sequence.

The nucleotide primers according to the invention may be used to amplify any one of the nucleic acids according to the invention, and more particularly a nucleic acid comprising a polynucleotide sequence of any one of SEQ ID NOs: 1, 4-65, 68-70, 72-88, 103, 109-118, and 137, or of a complementary polynucleotide sequence. Alternatively, the nucleotide primers according to the invention may be used to amplify a nucleic acid fragment or variant of any one of SEQ ID NOs: 1, 4-65, 68-70, 72-88, 103, 109-118, and 137, or of a complementary polynucleotide sequence.

In a particular embodiment, the nucleotide primers according to the invention may be used to amplify a nucleic acid comprising a) any one of SEQ ID NOs: 1, 4-8, 11-16, 18-26, 28-35, 37-52, 54-65, 68, and 137, or of a complementary polynucleotide sequence; b) nucleotides 3154-3200 of SEQ ID NO: 9 or of a complementary polynucleotide sequence; c) nucleotides 1-242 of SEQ ID NO: 10 or of a complementary polynucleotide sequence; d) nucleotides 1-1851 of SEQ ID NO: 17; or of a complementary polynucleotide sequence; e) nucleotides 152-198 of SEQ ID NO: 27, or of a complementary polynucleotide sequence; f) nucleotides 1-1657 of SEQ ID NO: 36, or of a complementary polynucleotide sequence; g) nucleotides 1-242 of SEQ ID NO: 53, or of a complementary polynucleotide sequence, h) nucleotides 1-244 of SEQ ID NO: 69 or nucleotides 1-357 of SEQ ID NO: 70, or a complementary polynucleotide sequence, or i) as depicted in any one of SEQ ID NOs: 1, 4-65, 68-70, 72-88, 103, 109-118, and 137, or of a complementary polynucleotide sequence.

Another subject of the invention relates to a method of amplifying a nucleic acid according to the invention, and more particularly a nucleic acid comprising a polynucleotide sequence of any one of SEQ ID NOs: 1, 4-65, 68-70, 72-88, 103, 109-118, and 137, or of a complementary polynucleotide sequence, or a nucleic acid fragment or variant thereof contained in a sample, said method comprising the steps of:
a) bringing the sample in which the presence of the target nucleic acid is suspected into contact with a pair of nucleotide primers whose hybridization position is located respectively on the 5' side and on the 3' side of the region of the target nucleic acid whose amplification is sought, in the presence of the reagents necessary for the amplification reaction; and
b) detecting the amplified nucleic acids.

Another subject of the invention relates to a method of amplifying a nucleic acid according to the invention, and more particularly a nucleic acid comprising a) any one of SEQ ID NOs: 1, 4-8, 11-16, 18-26, 28-35, 37-52, 54-65, 68, and 137, or of a complementary polynucleotide sequence; b) nucleotides 3154-3200 of SEQ ID NO: 9 or of a complementary polynucleotide sequence; c) nucleotides 1-242 of SEQ ID NO: 10 or of a complementary polynucleotide sequence; d) nucleotides 1-1851 of SEQ ID NO: 17; or of a complementary polynucleotide sequence; e) nucleotides 152-198 of SEQ ID NO: 27, or of a complementary polynucleotide sequence; f) nucleotides 1-1657 of SEQ ID NO: 36, or of a complementary polynucleotide sequence; g) nucleotides 1-242 of SEQ ID NO: 53, or of a complementary polynucleotide sequence, h) nucleotides 1-244 of SEQ ID NO: 69 or nucleotides 1-357 of SEQ ID NO: 70, or a complementary polynucleotide sequence, or i) as depicted in any one of SEQ ID NOs: 1, 4-65, 68-70, 72-88, 103, 109-118, and 137, or of a complementary polynucleotide sequence, contained in a sample, said method comprising the steps of:
a) bringing the sample in which the presence of the target nucleic acid is suspected into contact with a pair of nucleotide primers whose hybridization position is located respectively on the 5' side and on the 3' side of the region of the target nucleic acid whose amplification is sought, in the presence of the reagents necessary for the amplification reaction; and
b) detecting the amplified nucleic acids.

The present invention also relates to a method of detecting the presence of a nucleic acid comprising a polynucleotide sequence of any one of SEQ ID NOs: 1, 4-65, 68-70, 72-88, 103, 109-118, and 137, or of a complementary polynucleotide sequence, or a nucleic acid fragment or variant of any one of SEQ ID NOs: 1, 4-65, 68-70, 72-88, 103, 109-118, and 137, or of a complementary polynucleotide sequence in a sample, said method comprising the steps of:
1) bringing one or more nucleotide probes according to the invention into contact with the sample to be tested;
2) detecting the complex which may have formed between the probe(s) and the nucleic acid present in the sample.

According to a specific embodiment of the method of detection according to the invention, the oligonucleotide probes are immobilized on a support.

According to another aspect, the oligonucleotide probes comprise a detectable marker.

Another subject of the invention is a box or kit for amplifying a nucleic acid comprising a) any one of SEQ ID NOs: 1, 4-8, 11-16, 18-26, 28-35, 37-52, 54-65, 68-70, and 137, or of a complementary polynucleotide sequence; b) nucleotides 3154-3200 of SEQ ID NO: 9 or of a complementary polynucleotide sequence; c) nucleotides 1-242 of SEQ ID NO: 10 or of a complementary polynucleotide sequence; d) nucleotides 1-1851 of SEQ ID NO: 17; or of a complementary polynucleotide sequence; e) nucleotides 152-198 of SEQ ID NO: 27, or of a complementary polynucleotide sequence; f) nucleotides 1-1657 of SEQ ID NO: 36, or of a complementary polynucleotide sequence; g) nucleotides 1-242 of SEQ ID NO: 53, or of a complementary polynucleotide sequence, or h) as depicted in any one of SEQ ID NOs: 1, 4-65, 68-70, 72-88, 103, 109-118, and 137, or of a complementary polynucleotide sequence, said box or kit comprising:
1) a pair of nucleotide primers in accordance with the invention, whose hybridization position is located respectively on the 5' side and 3' side of the target nucleic acid whose amplification is sought; and optionally,
2) reagents necessary for an amplification reaction.

Such an amplification box or kit will preferably comprise at least one pair of nucleotide primers as described above.

The subject of the invention is, in addition, a box or kit for amplifying all or part of a nucleic acid comprising a) any one of SEQ ID NOs: 1, 4-8, 11-16, 18-26, 28-35, 37-52, 54-65, 68, and 137, or of a complementary polynucleotide sequence; b) nucleotides 3154-3200 of SEQ ID NO: 9 or of a complementary polynucleotide sequence; c) nucleotides 1-242 of SEQ ID NO: 10 or of a complementary polynucleotide sequence; d) nucleotides 1-1851 of SEQ ID NO: 17; or of a complementary polynucleotide sequence; e) nucleotides 152-198 of SEQ ID NO: 27, or of a complementary polynucleotide sequence; f) nucleotides 1-1657 of SEQ ID NO: 36, or of a complementary polynucleotide sequence; g) nucleotides 1-242 of SEQ ID NO: 53, or of a complementary polynucleotide sequence, or h) nucleotides 1-244 of SEQ ID NO: 69 or nucleotides 1-357 of SEQ ID NO: 70, or a complementary polynucleotide sequence, said box or kit comprising:
1) a pair of nucleotide primers in accordance with the invention, whose hybridization position is located respectively on the 5' side and 3' side of the target nucleic acid whose amplification is sought; and optionally,
2) reagents necessary for an amplification reaction.

Such an amplification box or kit will preferably comprise at least one pair of nucleotide primers as described above.

The invention also relates to a box or kit for detecting the presence of a nucleic acid according to the invention in a sample, said box or kit comprising:
a) one or more nucleotide probes according to the invention;
b) where appropriate, reagents necessary for a hybridization reaction.

According to a first aspect, the detection box or kit is characterized in that the nucleotide probe(s) and primer(s)are immobilized on a support.

According to a second aspect, the detection box or kit is characterized in that the nucleotide probe(s) and primer(s) comprise a detectable marker.

According to a specific embodiment of the detection kit described above, such a kit will comprise a plurality of oligonucleotide probes and/or primers in accordance with the invention which may be used to detect target nucleic acids of interest or alternatively to detect mutations in the coding regions or the non-coding regions of the nucleic acids according to the invention. According to preferred embodiment of the invention, the target nucleic acid comprises a polynucleotide sequence of any one of SEQ ID NOs: 1, 4-65, 68-70, 72-88, 103, 109-118, and 137, or of a complementary nucleic acid sequence. Alternatively, the target nucleic acid is a nucleic acid fragment or variant of a nucleic acid comprising any one of SEQ ID NOs: 1, 4-65, 68-70, 72-88, 103, 109-118, and 137, or of a complementary polynucleotide sequence.

According to a preferred embodiment, two primers according to the invention comprise all or part of SEQ ID NOs: 125 and 126, making it possible to amplify the region of exon 5 of the ABC1 gene carrying the mutation as depicted in SEQ ID NO: 72 described above, or nucleic acids having a complementary polynucleotide sequence.

According to a second preferred embodiment, two primers according to the invention comprise all or part of SEQ ID NOs: 127 and 128, making it possible to amplify the region of exon 6 of the ABC1 gene carrying the mutations as depicted in SEQ ID NOs: 73 or 74 described above, or nucleic acids having a complementary polynucleotide sequence.

According to a third preferred embodiment, two primers according to the invention comprise all or part of SEQ ID NOs: 131 and 132, making it possible to amplify the region of exon 8 of the ABC1 gene carrying the mutations as depicted in SEQ ID NOs: 75-77 described above, or nucleic acids having a complementary polynucleotide sequence.

According to a fourth preferred embodiment, two primers according to the invention comprise all or part of SEQ ID NOs: 155 and 156, making it possible to amplify the region of exon 27 of the ABC1 gene carrying the mutation as depicted in SEQ ID NO: 84 described above, or nucleic acids having a complementary polynucleotide sequence.

According to a fifth preferred embodiment, two primers according to the invention comprise all or part of SEQ ID NOs: 159 and 160, making it possible to amplify the region of exon 32 of the ABC1 gene carrying the mutation as depicted in SEQ ID NO: 85 described above, or nucleic acids having a complementary polynucleotide sequence.

According to a sixth preferred embodiment, two primers according to the invention comprise all or part of SEQ ID NOs: 175 and 176, making it possible to amplify the region of exon 47 of the ABC1 gene carrying the mutations as depicted in SEQ ID NOs: 87 or 88 described above, or nucleic acids having a complementary polynucleotide sequence.

According to another preferred embodiment, a primer according to the invention comprises, generally, all or part of any one of SEQ ID NOs: 119-136, 138, and 141-152, or a complementary sequence.

The invention also relates to a recombinant vector comprising a nucleic acid according to the invention. Preferably, such a recombinant vector will comprise a nucleic acid selected from the group consisting of
a) a nucleic acid comprising a polynucleotide sequence of any one of SEQ ID NOS: 1, 4-65, 68-70, 72-88, 103, 109-118, and 137, or of a complementary polynucleotide sequence,
b) a nucleic acid comprising a polynucleotide sequence as depicted in any one of SEQ ID NOs: 1, 4-65, 68-70, 72-88, 103, 109-118, and 137, or of a complementary polynucleotide sequence,
c) a nucleic acid comprising nucleotides 1-244 of SEQ ID NO: 69 or nucleotides 1-357 of SEQ ID NO: 70, or of a complementary polynucleotide sequence,
d) a nucleic acid having at least eight consecutive nucleotides of a nucleic acid comprising a polynucleotide sequence of 1) any one of SEQ ID NOs: 1, 4-8, 11-16, 18-26, 28-35, 37-52, 54-65, 68, and 137, or of a complementary polynucleotide sequence; 2) nucleotides 3154-3200 of SEQ ID NO: 9 or of a complementary polynucleotide sequence; 3) nucleotides 1-242 of SEQ ID NO: 10 or of a complementary polynucleotide sequence; 4) nucleotides 1-1851 of SEQ ID NO: 17; or of a complementary polynucleotide sequence; 5) nucleotides 152-198 of SEQ ID NO: 27, or of a complementary polynucleotide sequence; 6) nucleotides 1-1657 of SEQ ID NO: 36, or of a complementary polynucleotide sequence; 7) nucleotides 1-242 of SEQ ID NO: 53, or of a complementary polynucleotide sequence, or 8) nucleotides 1-244 of SEQ ID NO: 69 or nucleotides 1-357 of SEQ ID NO: 70, or a complementary polynucleotide sequence;
e) a nucleic acid having at least 80% nucleotide identity with a nucleic acid comprising a polynucleotide sequence of 1) any one of SEQ ID NOs: 1, 4-8, 11-16, 18-26, 28-35, 37-52, 54-65, 68, and 137, or of a complementary polynucleotide sequence; 2) nucleotides 3154-3200 of SEQ ID NO: 9 or of a complementary polynucleotide sequence; 3) nucleotides 1-242 of SEQ ID NO: 10 or of a complementary polynucleotide sequence; 4) nucleotides 1-1851 of SEQ ID NO: 17; or of a complementary polynucleotide sequence; 5) nucleotides 152-198 of SEQ ID NO: 27, or of a complementary polynucleotide sequence; 6) nucleotides 1-1657 of SEQ ID NO: 36, or of a complementary polynucleotide sequence; 7) nucleotides 1-242 of SEQ ID NO: 53, or of a complementary polynucleotide sequence, or 8) nucleotides 1-244 of SEQ ID NO: 69 or nucleotides 1-357 of SEQ ID NO: 70, or a complementary polynucleotide sequence;
f) a nucleic acid having 85%, 90%, 95%, or 98% nucleotide identity with a nucleic acid comprising a polynucleotide sequence of 1) any one of SEQ ID NOs: 1, 4-8, 11-16, 18-26, 28-35, 37-52, 54-65, 68, and 137, or of a complementary polynucleotide sequence; 2) nucleotides 3154-3200 of SEQ ID NO: 9 or of a complementary polynucleotide sequence; 3) nucleotides 1-242 of SEQ ID NO: 10 or of a complementary polynucleotide sequence; 4) nucleotides 1-1851 of SEQ ID NO: 17; or of a complementary polynucleotide sequence; 5) nucleotides 152-198 of SEQ ID NO: 27, or of a complementary polynucleotide sequence; 6) nucleotides 1-1657 of SEQ ID NO: 36, or of a complementary polynucleotide sequence; 7) nucleotides 1-242 of SEQ ID NO: 53, or of a complementary polynucleotide sequence, or 8) nucleotides 1-244 of SEQ ID NO: 69 or nucleotides 1-357 of SEQ ID NO: 70, or a complementary polynucleotide sequence;
g) a nucleic acid hybridizing, under high stringency hybridization conditions, with a nucleic acid comprising a polynucleotide sequence of 1) any one of SEQ ID NOs: 1, 4-8, 11-16, 18-26, 28-35, 37-52, 54-65, 68, and 137, or of a complementary polynucleotide sequence; 2) nucleotides 3154-3200 of SEQ ID NO: 9 or of a complementary polynucleotide sequence; 3) nucleotides 1-242 of SEQ ID NO: 10 or of a complementary polynucleotide sequence; 4) nucleotides 1-1851 of SEQ ID NO: 17; or of a complementary polynucleotide sequence; 5) nucleotides 152-198 of SEQ ID NO: 27, or of a complementary polynucleotide sequence; 6) nucleotides 1-1657 of SEQ ID NO: 36, or of a complementary polynucleotide sequence; 7) nucleotides 1-242 of SEQ ID NO: 53, or of a complementary polynucleotide sequence, or 8) nucleotides 1-244 of SEQ ID NO: 69 or nucleotides 1-357 of SEQ ID NO: 70, or a complementary polynucleotide sequence;
h) a nucleic acid encoding a polypeptide comprising an amino acid sequence of SEQ ID NO: 71; and
i) a nucleic acid encoding a polypeptide comprising amino acids 1-60 of SEQ ID NO: 71.

According to a first embodiment, a recombinant vector according to the invention is used to amplify a nucleic acid inserted therein, following transformation or transfection of a desired cellular host.

According to a second embodiment, a recombinant vector according to the invention corresponds to an expression vector comprising, in addition to a nucleic acid in accordance with the invention, a regulatory signal or nucleotide sequence that directs or controls transcription and/or translation of the nucleic acid and its encoded mRNA.

According to an preferred embodiment, a recombinant vector according to the invention will comprise in particular the following components:
(1) an element or signal for regulating the expression of the nucleic acid to be inserted, such as a promoter and/or enhancer sequence;
(2) a nucleotide coding region comprised within the nucleic acid in accordance with the invention to be inserted into such a vector, said coding region being placed in phase with the regulatory element or signal described in (1); and
(3) an appropriate nucleic acid for initiation and termination of transcription of the nucleotide coding region of the nucleic acid described in (2).

The present invention also relates to a defective recombinant virus comprising a cDNA nucleic acid encoding an ABC1 polypeptide involved in the transport and metabolism of cholesterol. In another preferred embodiment of the invention, the defective recombinant virus comprises a gDNA nucleic acid encoding an ABC1 polypeptide involved in the transport and metabolism of cholesterol. Preferably, the ABC1 polypeptide comprises an amino acid sequence of SEQ ID NO: 71. More preferably, the ABC1 polypeptide comprises amino acids 1-60 of SEQ ID NO: 71.

In another preferred embodiment, the invention relates to a defective recombinant virus comprising a nucleic acid encoding an ABC1 protein involved in the transport and metabolism of cholesterol under the control of a promoter chosen from RSV-LTR or the CMV early promoter.

According to a specific embodiment, a method of introducing a nucleic acid according to the invention into a host cell, in particular a host cell obtained from a mammal, *in vivo,* comprises a step during which a preparation comprising a pharmaceutically compatible vector and a "naked" nucleic acid according to the invention, placed under the control of appropriate regulatory sequences, is introduced by local injection at the level of the chosen tissue, for example a smooth muscle tissue, the "naked" nucleic acid being absorbed by the cells of this tissue.

According to a specific embodiment of the invention, a composition is provided for the *in vivo* production of the ABC1 protein. This composition comprises a nucleic acid encoding the ABC1 polypeptide placed under the control of appropriate regulatory sequences, in solution in a physiologically acceptable vehicle and/or excipient.

Therefore, the present invention also relates to a composition comprising a nucleic acid encoding a polypeptide comprising an amino acid sequence of SEQ ID NO: 71, wherein the nucleic acid is placed under the control of appropriate regulatory elements.

The present invention also relates to a composition comprising a nucleic acid encoding a polypeptide comprising amino acids 1-60 of SEQ ID NO: 71, wherein the nucleic acid is placed under the control of appropriate regulatory elements.

Consequently, the invention also relates to a pharmaceutical composition intended for the prevention of or treatment of a patient or subject affected by a dysfunction in the reverse transport of cholesterol comprising a nucleic acid encoding the ABC1 protein, in combination with one or more physiologically compatible excipients.

Preferably, such a composition will comprise a nucleic acid comprising a polynucleotide sequence of either SEQ ID NO: 69 or SEQ ID NO: 70, wherein the nucleic acid is placed under the control of an appropriate regulatory element or signal.

The subject of the invention is, in addition, a pharmaceutical composition intended for the prevention of or treatment of a patient or a subject affected by a dysfunction in the reverse transport of cholesterol comprising a recombinant vector according to the invention, in combination with one or more physiologically compatible excipients.

The invention also relates to the use of a nucleic acid according to the invention encoding an ABC1 protein for the manufacture of a medicament intended for the prevention of atherosclerosis in various forms or more particularly for the treatment of subjects affected by a dysfunction in the reverse transport of cholesterol.

The invention also relates to the use of a recombinant vector according to the invention comprising a nucleic acid encoding an ABC1 protein for the manufacture of a medicament intended for the prevention of atherosclerosis in various forms or more particularly for the treatment of subjects affected by a dysfunction in the reverse transport of cholesterol.

The subject of the invention is therefore also a recombinant vector comprising a nucleic acid according to the invention that encodes an ABC1 protein or polypeptide involved in the metabolism of cholesterol.

The invention also relates to the use of such a recombinant vector for the preparation of a pharmaceutical composition intended for the treatment and/or for the prevention of cardiovascular diseases or conditions associated with HDL deficiency, such as the HDL deficiency associated with Tangier and/or FHD disease, HDL deficiency, LCAT deficiency, malaria, and diabetes.

The present invention also relates to the use of cells genetically modified *ex vivo* with such a recombinant vector according to the invention, or of cells producing a recombinant vector, wherein the cells are implanted in the body, to allow a prolonged and effective expression *in vivo* of a biologically active ABC1 polypeptide.

The invention also relates to the use of a nucleic acid according to the invention encoding an ABC1 protein for the manufacture of a medicament intended for the prevention or treatment of atherosclerosis in various forms or more particularly for the treatment of subjects affected by a dysfunction in the reverse transport of cholesterol.

The invention also relates to the use of a recombinant vector according to the invention comprising a nucleic acid encoding an ABC1 polypeptide according to the invention for the manufacture of a medicament intended for the prevention of atherosclerosis in various forms or more particularly for the treatment of subjects affected by a dysfunction in the reverse transport of cholesterol.

The invention also relates to the use of a recombinant host cell according to the invention, comprising a nucleic acid encoding an ABC1 polypeptide according to the invention for the manufacture of a medicament intended for the prevention of atherosclerosis in various forms or more particularly for the treatment of subjects affected by a dysfunction in the reverse transport of cholesterol.

The present invention also relates to the use of a recombinant vector according to the invention, preferably a defective recombinant virus, for the preparation of a pharmaceutical composition for the treatment and/or prevention of pathologies linked to the transport of cholesterol.

The invention relates to the use of such a recombinant vector or defective recombinant virus for the preparation of a pharmaceutical composition intended for the treatment and/or for the prevention of cardiovascular disease linked to a deficiency in the reverse transport of cholesterol. Thus, the present invention also relates to a pharmaceutical composition comprising one or more recombinant vectors or defective recombinant viruses according to the invention.

The present invention also relates to the use of cells genetically modified ex vivo with a virus according to the invention, or of cells producing such viruses, implanted in the body, allowing a prolonged and effective expression *in vivo* of a biologically active ABC1 protein.

The present invention shows that it is possible to incorporate a nucleic acid encoding an ABC1 polypeptide according to the invention into a viral vector, and that these vectors make it possible to effectively express a biologically active, mature polypeptide. More particularly, the invention shows that the *in vivo* expression of ABC1 may be obtained by direct administration of an adenovirus or by implantation of a producing cell or of a cell genetically modified by an adenovirus or by a retrovirus incorporating such a nucleic acid.

In this regard, another subject of the invention relates to any mammalian cell infected with one or more defective recombinant viruses according to the invention. More particularly, the invention relates to any population of human cells infected with these viruses. These may be in particular cells of blood origin (totipotent stem cells or precursors), fibroblasts, myoblasts, hepatocytes, keratinocytes, smooth muscle and endothelial cells, glial cells and the like.

Another subject of the invention relates to an implant comprising mammalian cells infected with one or more defective recombinant viruses according to the invention or cells producing recombinant viruses, and an extracellular matrix. Preferably, the implants according to the invention comprise 10⁵ to 10¹⁰ cells. More preferably, they comprise 10⁶ to 10⁸ cells.

More particularly, in the implants of the invention, the extracellular matrix comprises a gelling compound and optionally, a support allowing the anchorage of the cells.

The invention also relates to a recombinant host cell comprising a nucleic acid of the invention, and more particularly, a nucleic acid comprising either SEQ ID NO: 69 or SEQ ID NO: 70, or of a complementary polynucleotide sequence.

The invention also relates to a recombinant host cell comprising a nucleic acid of the invention, and more particularly a nucleic acid comprising a nucleotide sequence as depicted in SEQ ID NO: 69 or SEQ ID NO: 70, or of a complementary polynucleotide sequence.

Specifically, the invention relates to a recombinant host cell comprising nucleic acid comprising any one of SEQ ID NOs: 1, 4-65, 68-70, 72-88, 103, 109-118, and 137, or of a complementary polynucleotide sequence.

The invention also relates to a recombinant host cell comprising a nucleic acid comprising a polynucleotide sequence as depicted in any one of SEQ ID NOs: 1, 4-65, 68-70, 72-88, 103, 109-118, and 137, or of a complementary polynucleotide sequence.

The invention also relates to a recombinant host cell comprising a nucleic acid comprising nucleotides 1-244 of SEQ ID NO: 69 or nucleotides 1-357 of SEQ ID NO: 70, or a complementary polynucleotide sequence.

The invention also relates to a recombinant host cell comprising a nucleic acid encoding a polypeptide comprising an amino acid sequence of SEQ ID NO: 71.
The invention also relates to a recombinant host cell comprising a nucleic acid encoding a polypeptide comprising amino acids 1-60 of SEQ ID NO: 71.

According to another aspect, the invention also relates to a recombinant host cell comprising a recombinant vector according to the invention. Therefore, the invention also relates to a recombinant host cell comprising a recombinant vector comprising any of the nucleic acids of the invention, and more particularly a nucleic acid comprising a nucleotide sequence of either SEQ ID NO: 69 or SEQ ID NO: 70, or of a complementary polynucleotide sequence.

Specifically, the invention relates to a recombinant host cell comprising a recombinant vector comprising a nucleic acid comprising any one of SEQ ID NOs: 1, 4-65, 68-70, 72-88, 103, 109-118, and 137, or of a complementary polynucleotide sequence.

The invention also relates to a recombinant host cell comprising a recombinant vector comprising a nucleic acid comprising a polynucleotide sequence as depicted in any one of SEQ ID NOs: 1, 4-65, 68-70, 72-88, 103, 109-118, and 137, or of a complementary polynucleotide sequence.

The invention also relates to a recombinant host cell comprising a recombinant vector comprising a nucleic acid comprising nucleotides 1-244 of SEQ ID NO: 69 or nucleotides 1-357 of SEQ ID NO: 70, or of a complementary polynucleotide sequence.

The invention also relates to a recombinant host cell comprising a recombinant vector comprising a nucleic acid encoding a polypeptide comprising an amino acid sequence of SEQ ID NO: 71.

The invention also relates to a recombinant host cell comprising a recombinant vector comprising a nucleic acid encoding a polypeptide comprising amino acids 1-60 of SEQ ID NO: 71.

The invention also relates to a method for the production of a polypeptide comprising an amino acid sequence of any one of SEQ ID NOs: 71 and 89-102, or of a peptide fragment or a variant thereof, wherein the peptide fragment or variant comprises amino acids 1-60 of SEQ ID NO: 71, said method comprising the steps of:
a) inserting a nucleic acid encoding said polypeptide into an appropriate vector;
b) culturing, in an appropriate culture medium, a previously transformed host cell or transfecting a host cell with the recombinant vector of step a);
c) recovering the conditioned culture medium or lysing the host cell, for example by sonication or by osmotic shock;
d) separating and purifying said polypeptide from said culture medium or alternatively from the cell lysates obtained in step c); and
e) where appropriate, characterizing the recombinant polypeptide produced.

A specific embodiment of the invention relates to a method for producing a polypeptide comprising an amino acid sequence of amino acids 1-60 of SEQ ID NO: 71.

A polypeptide termed "homologous" to a polypeptide having an amino acid sequence comprising amino acids 1-60 of SEQ ID NO: 71 also forms part of the invention. Such a homologous polypeptide comprises an amino acid sequence possessing one or more substitutions of an amino acid by an equivalent amino acid, relative to amino acids 1-60 of SEQ ID NO: 71.

The ABC1 polypeptides according to the invention, in particular 1) a polypeptide comprising an amino acid sequence of any one of SEQ ID NOs: 71 and 89-102, 2) a polypeptide comprising amino acids 1-60 of SEQ ID NO: 71, 3) a polypeptide fragment or variant of a polypeptide comprising an amino acid sequence of any one of SEQ ID NOs: 71 and 89-102, wherein the polypeptide fragment or variant comprises amino acids 1-60 of SEQ ID NO: 71, or 4) a polypeptide termed "homologous" to a polypeptide comprising amino acids 1-60 of SEQ ID NO: 71.

In a specific embodiment, an antibody according to the invention is directed against 1) a polypeptide comprising an amino acid sequence of any one of SEQ ID NOs: 71 and 89-102, 2) a polypeptide comprising amino acids 1-60 of SEQ ID NO: 71, 3) a polypeptide fragment or variant of a polypeptide comprising an amino acid sequence of any one of SEQ ID NOs: 71 and 89-102, wherein the polypeptide fragment or variant comprises amino acids 1-60 of SEQ ID NO: 71, or 4) a polypeptide termed "homologous" to a polypeptide comprising amino acids 1-60 of SEQ ID NO: 71.

The present invention relates to an antibody directed against 1) a polypeptide comprising an amino acid sequence of any one of SEQ ID NOs: 71 and 89-102, 2) a polypeptide comprising amino acids 1-60 of SEQ ID NO: 71, 3) a polypeptide fragment or variant of a polypeptide comprising an amino acid sequence of any one of SEQ ID NOs: 71 and 89-102, wherein the polypeptide fragment or variant comprises amino acids 1-60 of SEQ ID NO: 71, or 4) a polypeptide termed "homologous" to a polypeptide comprising amino acids 1-60 of SEQ ID NO: 71 also forms part of the invention, as produced in the trioma technique or the hybridoma technique described by Kozbor et al. (1983b).

Thus, the subject of the invention is, in addition, a method of detecting the presence of a polypeptide according to the invention in a sample, said method comprising the steps of:
a) bringing the sample to be tested into contact with an antibody directed against 1) a polypeptide comprising an amino acid sequence of any one of SEQ ID NOs: 71 and 89-102, 2) a polypeptide comprising amino acids 1-60 of SEQ ID NO: 71, 3) a polypeptide fragment or variant of a polypeptide comprising an amino acid sequence of any one of SEQ ID NOs: 71 and 89-102, wherein the polypeptide fragment or variant comprises amino acids 1-60 of SEQ ID NO: 71, or 4) a polypeptide termed "homologous" to a polypeptide comprising amino acids 1-60 of SEQ ID NO: 71, and
b) detecting the antigen/antibody complex formed.

The invention also relates to a box or kit for diagnosis or for detecting the presence of a polypeptide in accordance with the invention in a sample, said box comprising:
a) an antibody directed against 1) a polypeptide comprising an amino acid sequence of any one of SEQ ID NOs: 71 and 89-102, 2) a polypeptide comprising amino acids 1-60 of SEQ ID NO: 71, 3) a polypeptide fragment or variant of a polypeptide comprising an amino acid sequence of any one of SEQ ID NOs: 71 and 89-102, wherein the polypeptide fragment or variant comprises amino acids 1-60 of SEQ ID NO: 71, or 4) a polypeptide "homologous" to a polypeptide comprising amino acids 1-60 of SEQ ID NO: 71, and
b) a reagent allowing the detection of the antigen/antibody complexes formed.

The invention also relates to a pharmaceutical composition comprising a nucleic acid according to the invention.

The invention also provides pharmaceutical compositions comprising a nucleic acid encoding an ABC1 polypeptide according to the invention and pharmaceutical compositions comprising an ABC1 polypeptide according to the invention intended for the treatment of diseases linked to a deficiency in the reverse transport of cholesterol, such as Tangier and/or FHD disease.

The present invention also relates to a new therapeutic approach for the treatment of pathologies linked to the transport of cholesterol, comprising transferring and expressing *in vivo* nucleic acids encoding an ABC1 protein according to the invention. Specifically, the present invention provides a new therapeutic approach for the treatment and/or prevention of HDL deficiency, such as the HDL deficiency associated with Tangier and/or FHD disease, HDL deficiency, LCAT deficiency, malaria, and diabetes.

Thus, the present invention offers a new approach for the treatment and prevention of cardiovascular and neurological pathologies linked to the abnormalities of the transport and metabolism of cholesterol. Specifically, the present invention provides methods to restore or promote improved reverse transport of cholesterol within a patient or subject.

Consequently, the invention also relates to a pharmaceutical composition intended for the prevention of or treatment of subjects affected by, a dysfunction in the reverse transport of cholesterol, comprising a nucleic acid encoding the ABC1 protein, in combination with one or more physiologically compatible vehicle and/or excipient.

According to a specific embodiment of the invention, a composition is provided for the *in vivo* production of the ABC1 protein. This composition comprises a nucleic acid encoding the ABC1 polypeptide placed under the control of appropriate regulatory sequences, in solution in a physiologically compatible vehicle and/or excipient.

Therefore, the present invention also relates to a composition comprising a nucleic acid encoding a polypeptide comprising an amino acid sequence of SEQ ID NO: 71, wherein the nucleic acid is placed under the control of appropriate regulatory elements.

The present invention also relates to a composition comprising a nucleic acid encoding a polypeptide comprising amino acids 1-60 of SEQ ID NO: 71, wherein the nucleic acid is placed under the control of appropriate regulatory elements.

Preferably, such a composition will comprise a nucleic acid comprising a polynucleotide sequence of SEQ ID NO: 69 or SEQ ID NO: 70, placed under the control of appropriate regulatory elements.

The invention also relates to a pharmaceutical composition intended for the prevention of or treatment of subjects affected by, a dysfunction in the reverse transport of cholesterol, comprising a recombinant vector according to the invention, in combination with one or more physiologically compatible vehicle and/or excipient.

According to another aspect, the subject of the invention is also a preventive or curative therapeutic method of treating diseases caused by a deficiency in the metabolism of cholesterol, more particularly in the transport of cholesterol and still more particularly in the reverse transport of cholesterol, such a method comprising a step in which there is administered to a patient a nucleic acid encoding an ABC1 polypeptide according to the invention in said patient, said nucleic acid being, where appropriate, combined with one or more physiologically compatible vehicles and/or excipients.

The invention relates to a pharmaceutical composition for the prevention or treatment of a patient or subject affected by a dysfunction in the reverse transport of cholesterol, comprising a therapeutically effective quantity of a polypeptide having an amino acid sequence of SEQ ID NO: 71 or a polypeptide comprising amino acids 1-60 of SEQ ID NO: 71, combined with one or more physiologically compatible vehicles and/or excipients.

According to a specific embodiment, a method of introducing a nucleic acid according to the invention into a host cell, in particular a host cell obtained from a mammal, *in vivo,* comprises a step during which a preparation comprising a pharmaceutically compatible vector and a "naked" nucleic acid according to the invention, placed under the control of appropriate regulatory sequences, is introduced by local injection at the level of the chosen tissue, for example a smooth muscle tissue, the "naked" nucleic acid being absorbed by the cells of this tissue.

According to yet another aspect, the subject of the invention is also a preventive or curative therapeutic method of treating diseases caused by a deficiency in the metabolism of cholesterol, more particularly in the transport of cholesterol and still more particularly in the reverse transport of cholesterol, such a method comprising a step in which there is administered to a patient a therapeutically effective quantity of an ABC1 polypeptide according to the invention in said patient, said polypeptide being, where appropriate, combined with one or more physiologically compatible vehicles and/or excipients.

Preferably, a pharmaceutical composition comprising an ABC1 polypeptide according to the invention will be administered to the patient. Thus, the invention also relates to pharmaceutical compositions intended for the prevention or treatment of a deficiency in the metabolism of cholesterol such as atherosclerosis, particularly in the transport of cholesterol, and still more particularly in the reverse transport of cholesterol, characterized in that they comprise a therapeutically effective quantity of a nucleic acid encoding a normal ABC1 polypeptide, in particular an ABC1 polypeptide having an amino acid sequence of SEQ ID NO: 71. In a specific embodiment, the ABC1 polypeptide comprises amino acids 1-60 of SEQ ID NO:71.

The subject of the invention is, in addition, pharmaceutical compositions intended for the prevention or treatment of a deficiency in the metabolism of cholesterol such as atherosclerosis, particularly in the transport of cholesterol, and still more particularly in the reverse transport of cholesterol, characterized in that they comprise a therapeutically effective quantity of a normal ABC1 polypeptide, in particular of a polypeptide comprising an amino acid sequence of SEQ ID NO: 71. In a specific embodiment, the ABC1 polypeptide comprises amino acids 1-60 of SEQ ID NO: 71.

The invention also provides methods for screening small molecules and compounds that act on the ABC1 protein to identify agonists and antagonists of ABC1 polypeptide that can restore or promote improved reverse transport of cholesterol to effectively combat atherosclerosis from a therapeutic point of view. These methods are useful to identify small molecules and compounds for therapeutic use in the treatment of diseases due to a deficiency in the metabolism of cholesterol, particularly in the transport of cholesterol, still more particularly in the reverse transport of cholesterol, such as Tangier disease, or more generally, FHD-type conditions.

Therefore, the invention also relates to the use of an ABC1 polypeptide or a cell expressing an ABC1 polypeptide according to the invention, for screening active ingredients for the prevention or treatment of diseases resulting from a dysfunction in the reverse transport of cholesterol.

The invention also relates to a method of screening a compound or small molecule active on the metabolism of cholesterol, an agonist or antagonist of an ABC1 polypeptide, said method comprising the following steps:
a) preparing a membrane vesicle comprising an ABC1 polypeptide and a lipid substrate comprising a detectable marker;
b) incubating the vesicle obtained in step a) with an agonist or antagonist candidate compound;
c) qualitatively and/or quantitatively measuring release of the lipid substrate comprising a detectable marker; and
d) comparing the release measurement obtained in step b) with a measurement of release of a labeled lipid substrate by a vesicle that has not been previously incubated with the agonist or antagonist candidate compound.

In a first specific embodiment, the ABC1 polypeptide comprises SEQ ID NO: 71.

In a second specific embodiment, the ABC1 polypeptide comprises amino acids 1-60 of SEQ ID NO: 71.

The invention also relates to a method of screening a compound or small molecule active on the metabolism of cholesterol, an agonist or antagonist of an ABC1 polypeptide, said method comprising the following steps:
a) obtaining a cell, for example a cell line, that, either naturally or after transfecting the cell with an ABC1 encoding nucleic acid, expresses an ABC1 polypeptide;
b) incubating the cell of step a) in the presence of an anion labeled with a detectable marker;
c) washing the cell of step b) in order to remove the excess of the labeled anion which has not penetrated into these cells;
d) incubating the cell obtained in step c) with an agonist or antagonist candidate compound for the ABC1 polypeptide;
e) measuring efflux of the labeled anion; and
f) comparing the value of efflux of the labeled anion determined in step e) with a value of efflux of a labeled anion measured with cell which have not been previously incubated in the presence of the agonist or antagonist candidate compound for the ABC1 polypeptide.

In a first specific embodiment, the ABC1 polypeptide comprises SEQ ID NO: 71.

In a second specific embodiment, the ABC1 polypeptide comprises amino acids 1-60 of SEQ ID NO: 71.

The subject of the invention is also a method of screening a compound or small molecule active on the metabolism of cholesterol, an agonist or antagonist of an ABC1 polypeptide, said method comprising the following steps:
a) culturing cells of a human monocytic line in an appropriate culture medium, in the presence of purified human albumin;
b) incubating the cells of step a) simultaneously in the presence of a compound stimulating the production of IL-1 beta and of the agonist or antagonist candidate compound;
c) incubating the cells obtained in step b) in the presence of an appropriate concentration of ATP;
d) measuring IL-1 beta released into the cell culture supernatant; and
e) comparing the value of the release of the IL-1 beta obtained in step d) with the value of the IL-1 beta released into the culture supernatant of cells which have not been previously incubated in the presence of the agonist or antagonist candidate compound.

### BRIEF DESCRIPTION OF THE DRAWINGS

- Figure 1:: ABC1 5'cDNA Extension Strategy
- Figure 2:: Agarose Gel Analysis of Primary and Secondary Human ABC1 5' cDNA Extension PCR Reactions
- Figure 3:: Agarose Gel Analysis of Nested Secondary Human ABC1 5' cDNA Extension Nested PCR Reactions

### DETAILED DESCRIPTION OF THE INVENTION

### GENERAL DEFINITIONS

The present invention contemplates isolation of a gene encoding an ABC1 polypeptide of the invention, including a full length, or naturally occurring form of ABC1, and any antigenic fragments thereof from any animal, particularly mammalian or avian, and more particularly human, source.

In accordance with the present invention there may be employed conventional molecular biology, microbiology, and recombinant DNA techniques within the skill of the art. Such techniques are explained fully in the literature. See, *e.g.*, Sambrook et al., 1989; Glover, 1985; Gait, 1984; Hames and Higgins, 1985; Hames and Higgins, 1984; Freshney, 1986; Perbal, 1984; and F. Ausubel et al., 1994.

Therefore, if appearing herein, the following terms shall have the definitions set out below.

As used herein, the term "gene" refers to an assembly of nucleotides that encode a polypeptide, and includes cDNA and genomic DNA nucleic acids.

The term "isolated" for the purposes of the present invention designates a biological material (nucleic acid or protein) which has been removed from its original environment (the environment in which it is naturally present).

For example, a polynucleotide present in the natural state in a plant or an animal is not isolated. The same polynucleotide separated from the adjacent nucleic acids in which it is naturally inserted in the genome of the plant or animal is considered as being "isolated".

Such a polynucleotide may be included in a vector and/or such a polynucleotide may be included in a composition and remains nevertheless in the isolated state because of the fact that the vector or the composition does not constitute its natural environment.

The term "purified" does not require the material to be present in a form exhibiting absolute purity, exclusive of the presence of other compounds. It is rather a relative definition.

A polynucleotide is in the "purified" state after purification of the starting material or of the natural material by at least one order of magnitude, preferably 2 or 3 and preferably 4 or 5 orders of magnitude.

For the purposes of the present description, the expression "nucleotide sequence" may be used to designate either a polynucleotide or a nucleic acid. The expression "nucleotide sequence" covers the genetic material itself and is therefore not restricted to the information relating to its sequence.

The terms "nucleic acid", "polynucleotide", "oligonucleotide" or "nucleotide sequence" cover RNA, DNA, gDNA or cDNA sequences or alternatively RNA/DNA hybrid sequences of more than one nucleotide, either in the single-stranded form or in the duplex, double-stranded form.

A "nucleic acid" is a polymeric compound comprised of covalently linked subunits called nucleotides. Nucleic acid includes polyribonucleic acid (RNA) and polydeoxyribonucleic acid (DNA), both of which may be single-stranded or double-stranded. DNA includes cDNA, genomic DNA, synthetic DNA, and semi-synthetic DNA. The sequence of nucleotides that encodes a protein is called the sense sequence or coding sequence.

The term "nucleotide" designates both the natural nucleotides (A, T, G, C) as well as the modified nucleotides that comprise at least one modification such as (1) an analog of a purine, (2) an analog of a pyrimidine, or (3) an analogous sugar, examples of such modified nucleotides being described, for example, in the PCT application No. WO 95/04 064.

For the purposes of the present invention, a first polynucleotide is considered as being "complementary" to a second polynucleotide when each base of the first nucleotide is paired with the complementary base of the second polynucleotide whose orientation is reversed. The complementary bases are A and T (or A and U), or C and G.

"Heterologous" DNA refers to DNA not naturally located in the cell, or in a chromosomal site of the cell. Preferably, the heterologous DNA includes a gene foreign to the cell.

As used herein, the term "homologous" in all its grammatical forms and spelling variations refers to the relationship between proteins that possess a "common evolutionary origin," including proteins from superfamilies (*e.g.,* the immunoglobulin superfamily) and homologous proteins from different species (*e.g.,* myosin light chain, etc.) (Reeck et al., 1987). Such proteins (and their encoding genes) have sequence homology, as reflected by their high degree of sequence similarity.

Accordingly, the term "sequence similarity" in all its grammatical forms refers to the degree of identity or correspondence between nucleic acid or amino acid sequences of proteins that may or may not share a common evolutionary origin (*see* Reeck et al., *supra*). However, in common usage and in the instant application, the term "homologous," when modified with an adverb such as "highly," may refer to sequence similarity and not a common evolutionary origin.

In a specific embodiment, two DNA sequences are "substantially homologous" or "substantially similar" when at least about 50% (preferably at least about 75%, and more preferably at least about 90 or 95%) of the nucleotides match over the defined length of the DNA sequences. Sequences that are substantially homologous can be identified by comparing the sequences using standard software available in sequence data banks, or in a Southern hybridization experiment under, for example, stringent conditions as defined for that particular system. Defining appropriate hybridization conditions is within the skill of the art. See, e.g., Maniatis et al., *supra;* Glover et al., 1985; Hames and Higgins, 1985.

Similarly, in a particular embodiment, two amino acid sequences are "substantially homologous" or "substantially similar" when greater than 30% of the amino acids are identical, or greater than about 60% are similar (functionally identical). Preferably, the similar or homologous sequences are identified by alignment using, for example, the GCG (Genetics Computer Group, Program Manual for the GCG Package, *Version* 7, Madison, Wisconsin) pileup program.

The "percentage identity" between two nucleotide or amino acid sequences, for the purposes of the present invention, may be determined by comparing two sequences aligned optimally, through a window for comparison.

The portion of the nucleotide or polypeptide sequence in the window for comparison may thus comprise additions or deletions (for example "gaps") relative to the reference sequence (which does not comprise these additions or these deletions) so as to obtain an optimum alignment of the two sequences.

The percentage is calculated by determining the number of positions at which an identical nucleic base or an identical amino acid residue is observed for the two sequences (nucleic or peptide) compared, and then by dividing the number of positions at which there is identity between the two bases or amino acid residues by the total number of positions in the window for comparison, and then multiplying the result by 100 in order to obtain the percentage sequence identity.

The optimum sequence alignment for the comparison may be achieved using a computer with the aid of known algorithms contained in the package from the company WISCONSIN GENETICS SOFTWARE PACKAGE, GENETICS COMPUTER GROUP (GCG), 575 Science Doctor, Madison, WISCONSIN.

By way of illustration, it will be possible to produce the percentage sequence identity with the aid of the BLAST software (versions BLAST 1.4.9 of March 1996, BLAST 2.0.4 of February 1998 and BLAST 2.0.6 of September 1998), using exclusively the default parameters (Altschul et al, 1990; Altschul et al, 1997). Blast searches for sequences similar/homologous to a reference "request" sequence, with the aid of the Altschul et al. algorithm. The request sequence and the databases used may be of the peptide or nucleic types, any combination being possible.

The term "corresponding to" is used herein to refer to similar or homologous sequences, whether the exact position is identical or different from the molecule to which the similarity or homology is measured. A nucleic acid or amino acid sequence alignment may include spaces. Thus, the term "corresponding to" refers to the sequence similarity, and not the numbering of the amino acid residues or nucleotide bases.

A gene encoding an ABC1 polypeptide of the invention, whether genomic DNA or cDNA, can be isolated from any source, particularly from a human cDNA or genomic library. Methods for obtaining genes are well known in the art, as described above *(see, e.g.,* Sambrook et al., 1989).

Accordingly, any animal cell potentially can serve as the nucleic acid source for the molecular cloning of an ABC1 gene. The DNA may be obtained by standard procedures known in the art from cloned DNA (*e.g.,* a DNA "library"), and preferably is obtained from a cDNA library prepared from tissues with high level expression of the protein, by chemical synthesis, by cDNA cloning, or by the cloning of genomic DNA, or fragments thereof, purified from the desired cell (See, for example, Sambrook et al., 1989; Glover, 1985). Clones derived from genomic DNA may contain regulatory and intron DNA regions in addition to coding regions; clones derived from cDNA will not contain intron sequences. Whatever the source, the gene should be molecularly cloned into a suitable vector for propagation of the gene.

In the molecular cloning of the gene from genomic DNA, DNA fragments are generated, some of which will encode the desired gene. The DNA may be cleaved at specific sites using various restriction enzymes. Alternatively, one may use DNAse in the presence of manganese to fragment the DNA, or the DNA can be physically sheared, as for example, by sonication. The linear DNA fragments can then be separated according to size by standard techniques, including but not limited to, agarose and polyacrylamide gel electrophoresis and column chromatography.

Once the DNA fragments are generated, identification of the specific DNA fragment containing the desired ABC1 gene may be accomplished in a number of ways. For example, if an amount of a portion of a ABC1 gene or its specific RNA, or a fragment thereof, is available and can be purified and labeled, the generated DNA fragments may be screened by nucleic acid hybridization to the labeled probe (Benton and Davis, 1977; Grunstein and Hogness, 1975). For example, a set of oligonucleotides corresponding to the partial amino acid sequence information obtained for the ABC1 protein can be prepared and used as probes for DNA encoding ABC1, as was done in a specific example, *infra,* or as primers for cDNA or mRNA (*e.g.,* in combination with a poly-T primer for RT-PCR). Preferably, a fragment is selected that is highly unique to an ABC1 nucleic acid or polypeptide of the invention. Those DNA fragments with substantial homology to the probe will hybridize. As noted above, the greater the degree of homology, the more stringent hybridization conditions can be used. In a specific embodiment, stringency hybridization conditions are used to identify a homologous ABC1 gene.

Further selection can be carried out on the basis of the properties of the gene, *e.g.,* if the gene encodes a protein product having the isoelectric, electrophoretic, amino acid composition, or partial amino acid sequence of a ABC1 protein as disclosed herein. Thus, the presence of the gene may be detected by assays based on the physical, chemical, or immunological properties of its expressed product. For example, cDNA clones, or DNA clones which hybrid-select the proper mRNAs, can be selected which produce a protein that, *e.g.,* has similar or identical electrophoretic migration, isoelectric focusing or non-equilibrium pH gel electrophoresis behavior, proteolytic digestion maps, or antigenic properties as known for ABC1.

An ABC1 gene of the invention can also be identified by mRNA selection, *i.e.,* by nucleic acid hybridization followed by *in vitro* translation. In this procedure, nucleotide fragments are used to isolate complementary mRNAs by hybridization. Such DNA fragments may represent available, purified ABC1 DNA, or may be synthetic oligonucleotides designed from the partial amino acid sequence information. Immunoprecipitation analysis or functional assays *(e.g.,* tyrosine phosphatase activity) of the *in vitro* translation products of the products of the isolated mRNAs identifies the mRNA and, therefore, the complementary DNA fragments, that contain the desired sequences. In addition, specific mRNAs may be selected by adsorption of polysomes isolated from cells to immobilized antibodies specifically directed against an ABC1 polypeptide of the invention.

A radiolabeled ABC1 cDNA can be synthesized using the selected mRNA (from the adsorbed polysomes) as a template. The radiolabeled mRNA or cDNA may then be used as a probe to identify homologous ABC1 DNA fragments from among other genomic DNA fragments.

"Variant" of a nucleic acid according to the invention will be understood to mean a nucleic acid which differs by one or more bases relative to the reference polynucleotide. A variant nucleic acid may be of natural origin, such as an allelic variant which exists naturally, or it may also be a nonnatural variant obtained, for example, by mutagenic techniques.

In general, the differences between the reference (generally, wild-type) nucleic acid and the variant nucleic acid are small such that the nucleotide sequences of the reference nucleic acid and of the variant nucleic acid are very similar and, in many regions, identical. The nucleotide modifications present in a variant nucleic acid may be silent, which means that they do not alter the amino acid sequences encoded by said variant nucleic acid.

However, the changes in nucleotides in a variant nucleic acid may also result in substitutions, additions or deletions in the polypeptide encoded by the variant nucleic acid in relation to the polypeptides encoded by the reference nucleic acid. In addition, nucleotide modifications in the coding regions may produce conservative or non-conservative substitutions in the amino acid sequence of the polypeptide.

Preferably, the variant nucleic acids according to the invention encode polypeptides which substantially conserve the same function or biological activity as the polypeptide of the reference nucleic acid or alternatively the capacity to be recognized by antibodies directed against the polypeptides encoded by the initial reference nucleic acid.

Some variant nucleic acids will thus encode mutated forms of the polypeptides whose systematic study will make it possible to deduce structure-activity relationships of the proteins in question. Knowledge of these variants in relation to the disease studied is essential since it makes it possible to understand the molecular cause of the pathology.

"Fragment" will be understood to mean a nucleotide sequence of reduced length relative to the reference nucleic acid and comprising, over the common portion, a nucleotide sequence identical to the reference nucleic acid. Such a nucleic acid "fragment" according to the invention may be, where appropriate, included in a larger polynucleotide of which it is a constituent. Such fragments comprise, or alternatively consist of, oligonucleotides ranging in length from 8, 10, 12, 15, 18, 20 to 25, 30, 40, 50, 70, 80, 100, 200, 500, 1000 or 1500 consecutive nucleotides of a nucleic acid according to the invention.

A "nucleic acid molecule" refers to the phosphate ester polymeric form of ribonucleosides (adenosine, guanosine, uridine or cytidine; "RNA molecules") or deoxyribonucleosides (deoxyadenosine, deoxyguanosine, deoxythymidine, or deoxycytidine; "DNA molecules"), or any phosphoester anologs thereof, such as phosphorothioates and thioesters, in either single stranded form, or a double-stranded helix. Double stranded DNA-DNA, DNA-RNA and RNA-RNA helices are possible. The term nucleic acid molecule, and in particular DNA or RNA molecule, refers only to the primary and secondary structure of the molecule, and does not limit it to any particular tertiary forms. Thus, this term includes double-stranded DNA found, *inter alia,* in linear or circular DNA molecules (*e.g.,* restriction fragments), plasmids, and chromosomes. In discussing the structure of particular double-stranded DNA molecules, sequences may be described herein according to the normal convention of giving only the sequence in the 5' to 3' direction along the nontranscribed strand of DNA (*i.e.,* the strand having a sequence homologous to the mRNA). A "recombinant DNA molecule" is a DNA molecule that has undergone a molecular biological manipulation.

A nucleic acid molecule is "hybridizable" to another nucleic acid molecule, such as a cDNA, genomic DNA, or RNA, when a single stranded form of the nucleic acid molecule can anneal to the other nucleic acid molecule under the appropriate conditions of temperature and solution ionic strength (*see* Sambrook et al., *supra*). The conditions of temperature and ionic strength determine the "stringency" of the hybridization. For preliminary screening for homologous nucleic acids, low stringency hybridization conditions, corresponding to a Tₘ of 55°, can be used, *e.g.,* 5x SSC, 0.1% SDS, 0.25% milk, and no formamide; or 30% formamide, 5x SSC, 0.5% SDS). Moderate stringency hybridization conditions correspond to a higher Tₘ, *e.g.,* 40% formamide, with 5x or 6x SCC. High stringency hybridization conditions correspond to the highest Tₘ, *e.g.,* 50% formamide, 5x or 6x SCC. Hybridization requires that the two nucleic acids contain complementary sequences, although depending on the stringency of the hybridization, mismatches between bases are possible. The appropriate stringency for hybridizing nucleic acids depends on the length of the nucleic acids and the degree of complementation, variables well known in the art. The greater the degree of similarity or homology between two nucleotide sequences, the greater the value of Tₘ for hybrids of nucleic acids having those sequences. The relative stability (corresponding to higher Tₘ) of nucleic acid hybridizations decreases in the following order: RNA:RNA, DNA:RNA, DNA:DNA. For hybrids of greater than 100 nucleotides in length, equations for calculating Tₘ have been derived (*see* Sambrook et al., *supra,* 9.50-0.51). For hybridization with shorter nucleic acids, *i.e.,* oligonucleotides, the position of mismatches becomes more important, and the length of the oligonucleotide determines its specificity (*see* Sambrook et al., *supra,* 11.7-11.8).
Preferably a minimum length for a hybridizable nucleic acid is at least about 10 nucleotides; preferably at least about 15 nucleotides; and more preferably the length is at least about 20 nucleotides.

In a specific embodiment, the term "standard hybridization conditions" refers to a Tₘ of 55°C, and utilizes conditions as set forth above. In a preferred embodiment, the Tₘ is 60°C; in a more preferred embodiment, the Tₘ is 65°C.

"High stringency hybridization conditions" for the purposes of the present invention will be understood to mean the following conditions:
1- Membrane competition and PREHYBRIDIZATION:
   - Mix: 40 µl salmon sperm DNA (10 mg/ml) + 40 µl human placental DNA (10 mg/ml)
   - Denature for 5 minutes at 96°C, then immerse the mixture in ice.
   - Remove the 2X SSC and pour 4 ml of formamide mix in the hybridization tube containing the membranes.
   - Add the mixture of the two denatured DNAs.
   - Incubation at 42°C for 5 to 6 hours, with rotation.
2- Labeled probe competition:
   - Add to the labeled and purified probe 10 to 50 µl Cot I DNA, depending on the quantity of repeats.
   - Denature for 7 to 10 minutes at 95°C.
   - Incubate at 65°C for 2 to 5 hours.
3- HYBRIDIZATION:
   - Remove the prehybridization mix.
   - Mix 40 µl salmon sperm DNA + 40 µl human placental DNA; denature for 5 min at 96°C, then immerse in ice.
   - Add to the hybridization tube 4 ml of formamide mix, the mixture of the two DNAs and the denatured labeled probe/Cot I DNA .
   - Incubate 15 to 20 hours at 42°C, with rotation.
4- Washes and Exposure:
   - One wash at room temperature in 2X SSC, to rinse.
   - Twice 5 minutes at room temperature 2X SSC and 0.1% SDS at 65°C.
   - Twice 15 minutes at 65°C IX SSC and 0.1% SDS at 65°C.
   - Envelope the membranes in clear plastic wrap and expose.

The hybridization conditions described above are adapted to hybridization, under high stringency conditions, of a molecule of nucleic acid of varying length from 20 nucleotides to several hundreds of nucleotides. It goes without saying that the hybridization conditions described above may be adjusted as a function of the length of the nucleic acid whose hybridization is sought or of the type of labeling chosen, according to techniques known to one skilled in the art. Suitable hybridization conditions may, for example, be adjusted according to the teaching contained in the manual by Hames and Higgins (1985) or in the manual by F. Ausubel et al. (1999).

As used herein, the term "oligonucleotide" refers to a nucleic acid, generally of at least 15 nucleotides, that is hybridizable to a nucleic acid according to the invention.
Oligonucleotides can be labeled, *e.g.,* with ³²P-nucleotides or nucleotides to which a label, such as biotin, has been covalently conjugated. In one embodiment, a labeled oligonucleotide can be used as a probe to detect the presence of a nucleic acid encoding an ABC1 polypeptide of the invention. In another embodiment, oligonucleotides (one or both of which may be labeled) can be used as PCR primers, either for cloning full length or a fragment of an ABC1 nucleic acid, or to detect the presence of nucleic acids encoding ABC1. In a further embodiment, an oligonucleotide of the invention can form a triple helix with an ABC1 DNA molecule. Generally, oligonucleotides are prepared synthetically, preferably on a nucleic acid synthesizer. Accordingly, oligonucleotides can be prepared with non-naturally occurring phosphoester analog bonds, such as thioester bonds, etc.

"Homologous recombination" refers to the insertion of a foreign DNA sequence of a vector in a chromosome. Preferably, the vector targets a specific chromosomal site for homologous recombination. For specific homologous recombination, the vector will contain sufficiently long regions of homology to sequences of the chromosome to allow complementary binding and incorporation of the vector into the chromosome. Longer regions of homology, and greater degrees of sequence similarity, may increase the efficiency of homologous recombination.

A DNA "coding sequence" is a double-stranded DNA sequence which is transcribed and translated into a polypeptide in a cell *in vitro* or *in vivo* when placed under the control of appropriate regulatory sequences. The boundaries of the coding sequence are determined by a start codon at the 5' (amino) terminus and a translation stop codon at the 3' (carboxyl) terminus. A coding sequence can include, but is not limited to, prokaryotic sequences, cDNA from eukaryotic mRNA, genomic DNA sequences from eukaryotic (*e.g*., mammalian) DNA, and even synthetic DNA sequences. If the coding sequence is intended for expression in a eukaryotic cell, a polyadenylation signal and transcription termination sequence will usually be located 3' to the coding sequence.

Transcriptional and translational control sequences are DNA regulatory sequences, such as promoters, enhancers, terminators, and the like, that provide for the expression of a coding sequence in a host cell. In eukaryotic cells, polyadenylation signals are control sequences.

"Regulatory region" means a nucleic acid sequence which regulates the expression of a nucleic acid. A regulatory region may include sequences which are naturally responsible for expressing a particular nucleic acid (a homologous region) or may include sequences of a different origin (responsible for expressing different proteins or even synthetic proteins). In particular, the sequences can be sequences of eukaryotic or viral genes or derived sequences which stimulate or repress transcription of a gene in a specific or non-specific manner and in an inducible or non-inducible manner. Regulatory regions include origins of replication, RNA splice sites, enhancers, transcriptional termination sequences, signal sequences which direct the polypeptide into the secretory pathways of the target cell, and promoters.

A regulatory region from a "heterologous source" is a regulatory region which is not naturally associated with the expressed nucleic acid. Included among the heterologous regulatory regions are regulatory regions from a different species, regulatory regions from a different gene, hybrid regulatory sequences, and regulatory sequences which do not occur in nature, but which are designed by one having ordinary skill in the art.

A "cassette" refers to a segment of DNA that can be inserted into a vector at specific restriction sites. The segment of DNA encodes a polypeptide of interest, and the cassette and restriction sites are designed to ensure insertion of the cassette in the proper reading frame for transcription and translation.

A "promoter sequence" is a DNA regulatory region capable of binding RNA polymerase in a cell and initiating transcription of a downstream (3' direction) coding sequence. For purposes of defining the present invention, the promoter sequence is bounded at its 3' terminus by the transcription initiation site and extends upstream (5' direction) to include the minimum number of bases or elements necessary to initiate transcription at levels detectable above background. Within the promoter sequence will be found a transcription initiation site (conveniently defined for example, by mapping with nuclease S1), as well as protein binding domains (consensus sequences) responsible for the binding of RNA polymerase.

A coding sequence is "under the control" of transcriptional and translational control sequences in a cell when RNA polymerase transcribes the coding sequence into mRNA, which is then trans-RNA spliced and translated into the protein encoded by the coding sequence.

A "signal sequence" is included at the beginning of the coding sequence of a protein to be expressed on the surface of a cell. This sequence encodes a signal peptide, N-terminal to the mature polypeptide, that directs the host cell to translocate the polypeptide. The term "translocation signal sequence" is used herein to refer to this sort of signal sequence.
Translocation signal sequences can be found associated with a variety of proteins native to eukaryotes and prokaryotes, and are often functional in both types of organisms.

A "polypeptide" is a polymeric compound comprised of covalently linked amino acid residues. Amino acids have the following general structure: 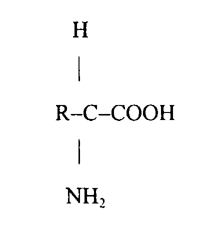 Amino acids are classified into seven groups on the basis of the side chain R: (1) aliphatic side chains, (2) side chains containing a hydroxylic (OH) group, (3) side chains containing sulfur atoms, (4) side chains containing an acidic or amide group, (5) side chains containing a basic group, (6) side chains containing an aromatic ring, and (7) proline, an imino acid in which the side chain is fused to the amino group.

A "protein" is a polypeptide which plays a structural or functional role in a living cell.

The polypeptides and proteins of the invention may be glycosylated or unglycosylated.

"Homology" means similarity of sequence reflecting a common evolutionary origin. Polypeptides or proteins are said to have homology, or similarity, if a substantial number of their amino acids are either (1) identical, or (2) have a chemically similar R side chain. Nucleic acids are said to have homology if a substantial number of their nucleotides are identical.

"Isolated polypeptide" or "isolated protein" is a polypeptide or protein which is substantially free of those compounds that are normally associated therewith in its natural state (e.g., other proteins or polypeptides, nucleic acids, carbohydrates, lipids). "Isolated" is not meant to exclude artificial or synthetic mixtures with other compounds, or the presence of impurities which do not interfere with biological activity, and which may be present, for example, due to incomplete purification, addition of stabilizers, or compounding into a pharmaceutically acceptable preparation.

"Fragment" of a polypeptide according to the invention will be understood to mean a polypeptide whose amino acid sequence is shorter than that of the reference polypeptide and which comprises, over the entire portion with these reference polypeptides, an identical amino acid sequence. Such fragments may, where appropriate, be included in a larger polypeptide of which they are a part. Such fragments of a polypeptide according to the invention may have a length of 10, 15, 20, 30 to 40, 50, 100, 200 or 300 amino acids.

"Variant" of a polypeptide according to the invention will be understood to mean mainly a polypeptide whose amino acid sequence contains one or more substitutions, additions or deletions of at least one amino acid residue, relative to the amino acid sequence of the reference polypeptide, it being understood that the amino acid substitutions may be either conservative or nonconservative.

A "variant" of a polypeptide or protein is any analogue, fragment, derivative, or mutant which is derived from a polypeptide or protein and which retains at least one biological property of the polypeptide or protein. Different variants of the polypeptide or protein may exist in nature. These variants may be allelic variations characterized by differences in the nucleotide sequences of the structural gene coding for the protein, or may involve differential splicing or post-translational modification. Variants also include a related protein having substantially the same biological activity, but obtained from a different species.

The skilled artisan can produce variants having single or multiple amino acid substitutions, deletions, additions, or replacements. These variants may include, *inter alia:* (a) variants in which one or more amino acid residues are substituted with conservative or non-conservative amino acids, (b) variants in which one or more amino acids are added to the polypeptide or protein, (c) variants in which one or more of the amino acids includes a substituent group, and (d) variants in which the polypeptide or protein is fused with another polypeptide such as serum albumin. The techniques for obtaining these variants, including genetic (suppressions, deletions, mutations, etc.), chemical, and enzymatic techniques, are known to persons having ordinary skill in the art.

If such allelic variations, analogues, fragments, derivatives, mutants, and modifications, including alternative mRNA splicing forms and alternative post-translational modification forms result in derivatives of the polypeptide which retain any of the biological properties of the polypeptide, they are intended to be included within the scope of this invention.

A "vector" is a replicon, such as plasmid, virus, phage or cosmid, to which another DNA segment may be attached so as to bring about the replication of the attached segment. A "replicon" is any genetic element (e.g., plasmid, chromosome, virus) that functions as an autonomous unit of DNA replication *in vivo, i.e.,* capable of replication under its own control.

The present invention also relates to cloning vectors containing genes encoding analogs and derivatives of a ABC1 polypeptide of the invention, that have the same or homologous functional activity as that ABC1 polypeptide, and homologs thereof from other species. The production and use of derivatives and analogs related to ABC1 are within the scope of the present invention. In a specific embodiment, the derivative or analog is functionally active, *i.e.,* capable of exhibiting one or more functional activities associated with a full-length, wild-type an ABC1 polypeptide of the invention.

ABC1 derivatives can be made by altering encoding nucleic acid sequences by substitutions, additions or deletions that provide for functionally equivalent molecules. Preferably, derivatives are made that have enhanced or increased functional activity relative to native ABC1. Alternatively, such derivatives may encode soluble fragments of the ABC1 extracellular domain that have the same or greater affinity for the natural ligand of an ABC1 polypeptide of the invention. Such soluble derivatives may be potent inhibitors of ligand binding to ABC 1.

Due to the degeneracy of nucleotide coding sequences, other DNA sequences which encode substantially the same amino acid sequence as an ABC1 gene may be used in the practice of the present invention. These include but are not limited to allelic genes, homologous genes from other species, and nucleotide sequences comprising all or portions of ABC1 genes which are altered by the substitution of different codons that encode the same amino acid residue within the sequence, thus producing a silent change. Likewise, the ABC1 derivatives of the invention include, but are not limited to, those containing, as a primary amino acid sequence, all or part of the amino acid sequence of an ABC1 protein including altered sequences in which functionally equivalent amino acid residues are substituted for residues within the sequence resulting in a conservative amino acid substitution. For example, one or more amino acid residues within the sequence can be substituted by another amino acid of a similar polarity, which acts as a functional equivalent, resulting in a silent alteration. Substitutes for an amino acid within the sequence may be selected from other members of the class to which the amino acid belongs. For example, the nonpolar (hydrophobic) amino acids include alanine, leucine, isoleucine, valine, proline, phenylalanine, tryptophan and methionine. Amino acids containing aromatic ring structures are phenylalanine, tryptophan, and tyrosine. The polar neutral amino acids include glycine, serine, threonine, cysteine, tyrosine, asparagine, and glutamine. The positively charged (basic) amino acids include arginine, lysine and histidine. The negatively charged (acidic) amino acids include aspartic acid and glutamic acid. Such alterations will not be expected to affect apparent molecular weight as determined by polyacrylamide gel electrophoresis, or isoelectric point.

Particularly preferred substitutions are:
- Lys for Arg and vice versa such that a positive charge may be maintained;
- Glu for Asp and vice versa such that a negative charge may be maintained;
- Ser for Thr such that a free -OH can be maintained; and
- Gln for Asn such that a free CONH₂ can be maintained.

Amino acid substitutions may also be introduced to substitute an amino acid with a particularly preferable property. For example, a Cys may be introduced a potential site for disulfide bridges with another Cys. A His may be introduced as a particularly "catalytic" site (i.e., His can act as an acid or base and is the most common amino acid in biochemical catalysis). Pro may be introduced because of its particularly planar structure, which induces b-turns in the protein's structure.

The genes encoding ABC1 derivatives and analogs of the invention can be produced by various methods known in the art. The manipulations which result in their production can occur at the gene or protein level. For example, the cloned ABC1 gene sequence can be modified by any of numerous strategies known in the art (Sambrook et al., 1989, *supra).* The sequence can be cleaved at appropriate sites with restriction endonuclease(s), followed by further enzymatic modification if desired, isolated, and ligated *in vitro.* In the production of the gene encoding a derivative or analog of ABC1, care should be taken to ensure that the modified gene remains within the same translational reading frame as the ABC1 gene, uninterrupted by translational stop signals, in the gene region where the desired activity is encoded.

Additionally, the ABC1-encoding nucleic acids can be mutated *in vitro* or *in vivo,* to create and/or destroy translation, initiation, and/or termination sequences, or to create variations in coding regions and/or form new restriction endonuclease sites or destroy preexisting ones, to facilitate further *in vitro* modification. Preferably, such mutations enhance the functional activity of the mutated ABC1 gene product. Any technique for mutagenesis known in the art can be used, including but not limited to, *in vitro* site-directed mutagenesis (Hutchinson, C., et al., 1978; Zoller and Smith, 1984; Oliphant et al., 1986; Hutchinson et al., 1986; Huygen et al., 1996) use of TAB® linkers (Pharmacia), etc. PCR techniques are preferred for site directed mutagenesis (see Higuchi, 1989, "Using PCR to Engineer DNA", in *PCR Technology: Principles and Applications for DNA Amplification,* H. Erlich, ed., Stockton Press, Chapter 6, pp. 61-70).

The identified and isolated gene can then be inserted into an appropriate cloning vector. A large number of vector-host systems known in the art may be used. Possible vectors include, but are not limited to, plasmids or modified viruses, but the vector system must be compatible with the host cell used. Examples of vectors include, but are not limited to, *Escherichia coli,* bacteriophages such as lambda derivatives, or plasmids such as pBR322 derivatives or pUC plasmid derivatives, *e.g.,* pGEX vectors, pmal-c, pFLAG, etc. The insertion into a cloning vector can, for example, be accomplished by ligating the DNA fragment into a cloning vector which has complementary cohesive termini. However, if the complementary restriction sites used to fragment the DNA are not present in the cloning vector, the ends of the DNA molecules may be enzymatically modified. Alternatively, any site desired may be produced by ligating nucleotide sequences (linkers) onto the DNA termini; these ligated linkers may comprise specific chemically synthesized oligonucleotides encoding restriction endonuclease recognition sequences. Recombinant molecules can be introduced into host cells via transformation, transfection, infection, electroporation, etc., so that many copies of the gene sequence are generated. Preferably, the cloned gene is contained on a shuttle vector plasmid, which provides for expansion in a cloning cell, *e.g., Escherichia coli,* and facile purification for subsequent insertion into an appropriate expression cell line, if such is desired. For example, a shuttle vector, which is a vector that can replicate in more than one type of organism, can be prepared for replication in both *Escherichia coli* and *Saccharomyces cerevisiae* by linking sequences from an *Escherichia coli* plasmid with sequences form the yeast 2m plasmid.

In an alternative method, the desired gene may be identified and isolated after insertion into a suitable cloning vector in a "shot gun" approach. Enrichment for the desired gene, for example, by size fractionation, can be done before insertion into the cloning vector.

The nucleotide sequence coding for an ABC1 polypeptide or antigenic fragment, derivative or analog thereof, or a functionally active derivative, including a chimeric protein, thereof, can be inserted into an appropriate expression vector, *i.e.,* a vector which contains the necessary elements for the transcription and translation of the inserted protein-coding sequence. Such elements are termed herein a "promoter." Thus, the nucleic acid encoding an ABC1 polypeptide of the invention is operationally associated with a promoter in an expression vector of the invention. Both cDNA and genomic sequences can be cloned and expressed under control of such regulatory sequences. An expression vector also preferably includes a replication origin.

The necessary transcriptional and translational signals can be provided on a recombinant expression vector, or they may be supplied by a native gene encoding ABC1 and/or its flanking regions.

Potential host-vector systems include but are not limited to mammalian cell systems infected with virus (*e.g.,* vaccinia virus, adenovirus, etc.); insect cell systems infected with virus (*e.g.,* baculovirus); microorganisms such as yeast containing yeast vectors; or bacteria transformed with bacteriophage, DNA, plasmid DNA, or cosmid DNA. The expression elements of vectors vary in their strengths and specificities. Depending on the host-vector system utilized, any one of a number of suitable transcription and translation elements may be used.

A recombinant ABC1 protein of the invention, or functional fragment, derivative, chimeric construct, or analog thereof, may be expressed chromosomally, after integration of the coding sequence by recombination. In this regard, any of a number of amplification systems may be used to achieve high levels of stable gene expression (*See* Sambrook et al., 1989, *supra).*

The cell into which the recombinant vector comprising the nucleic acid encoding an ABC1 polypeptide according to the invention is cultured in an appropriate cell culture medium under conditions that provide for expression of the ABC1 polypeptide by the cell.

Any of the methods previously described for the insertion of DNA fragments into a cloning vector may be used to construct expression vectors containing a gene consisting of appropriate transcriptional/translational control signals and the protein coding sequences. These methods may include *in vitro* recombinant DNA and synthetic techniques and *in vivo* recombination (genetic recombination).

Expression of an ABC1 polypeptide may be controlled by any promoter/enhancer element known in the art, but these regulatory elements must be functional in the host selected for expression. Promoters which may be used to control ABC1 gene expression include, but are not limited to, the SV40 early promoter region (Benoist and Chambon, 1981), the promoter contained in the 3' long terminal repeat of Rous sarcoma virus (Yamamoto, et al., 1980), the herpes thymidine kinase promoter (Wagner et al., 1981), the regulatory sequences of the metallothionein gene (Brinster et al., 1982); prokaryotic expression vectors such as the b-lactamase promoter (Villa-Kamaroff, et al., 1978), or the *tac* promoter (DeBoer, et al., 1983); see also "Useful proteins from recombinant bacteria" in Scientific American, 1980, 242:74-94; promoter elements from yeast or other fungi such as the Gal 4 promoter, the ADC (alcohol dehydrogenase) promoter, PGK (phosphoglycerol kinase) promoter, alkaline phosphatase promoter; and the animal transcriptional control regions, which exhibit tissue specificity and have been utilized in transgenic animals: elastase I gene control region which is active in pancreatic acinar cells (Swift et al., 1984; Ornitz et al., 1986; MacDonald, 1987); insulin gene control region which is active in pancreatic beta cells (Hanahan, 1985), immunoglobulin gene control region which is active in lymphoid cells (Grosschedl et al., 1984; Adames et al., 1985; Alexander et al., 1987), mouse mammary tumor virus control region which is active in testicular, breast, lymphoid and mast cells (Leder et al., 1986), albumin gene control region which is active in liver (Pinkert et al., 1987), alpha-fetoprotein gene control region which is active in liver (Krumlauf et al., 1985; Hammer et al., 1987), alpha 1-antitrypsin gene control region which is active in the liver (Kelsey et al., 1987) beta-globin gene control region which is active in myeloid cells (Mogram et al., 1985; Kollias et al., 1986), myelin basic protein gene control region which is active in oligodendrocyte cells in the brain (Readhead et al., 1987), myosin light chain-2 gene control region which is active in skeletal muscle (Sani, 1985), and gonadotropic releasing hormone gene control region which is active in the hypothalamus (Mason et al., 1986).

Expression vectors containing a nucleic acid encoding an ABC1 polypeptide of the invention can be identified by four general approaches: (a) polymerase chain reaction (PCR) amplification of the desired plasmid DNA or specific mRNA, (b) nucleic acid hybridization, (c) presence or absence of selection marker gene functions, (d) analyses with appropriate restriction endonucleases, and (e) expression of inserted sequences. In the first approach, the nucleic acids can be amplified by PCR to provide for detection of the amplified product. In the second approach, the presence of a foreign gene inserted in an expression vector can be detected by nucleic acid hybridization using probes comprising sequences that are homologous to an inserted marker gene. In the third approach, the recombinant vector/host system can be identified and selected based upon the presence or absence of certain "selection marker" gene functions (e.g., b-galactosidase activity, thymidine kinase activity, resistance to antibiotics, transformation phenotype, occlusion body formation in baculovirus, etc.) caused by the insertion of foreign genes in the vector. In another example, if the nucleic acid encoding an ABC1 polypeptide is inserted within the "selection marker" gene sequence of the vector, recombinants containing the ABC1 nucleic acid insert can be identified by the absence of the ABC1 gene function. In the fourth approach, recombinant expression vectors are identified by digestion with appropriate restriction enzymes. In the fifth approach, recombinant expression vectors can be identified by assaying for the activity, biochemical, or immunological characteristics of the gene product expressed by the recombinant, provided that the expressed protein assumes a functionally active conformation.

A wide variety of host/expression vector combinations may be employed in expressing the nucleic acids of this invention. Useful expression vectors, for example, may consist of segments of chromosomal, non-chromosomal and synthetic DNA sequences. Suitable vectors include derivatives of SV40 and known bacterial plasmids, e.g., *Escherichia coli* plasmids col El, pCR1, pBR322, pMal-C2, pET, pGEX (Smith *et al.,* 1988), pMB9 and their derivatives, plasmids such as RP4; phage DNAs, e.g., the numerous derivatives of phage 1, e.g., NM989, and other phage DNA, e.g., M13 and filamentous single stranded phage DNA; yeast plasmids such as the 2m plasmid or derivatives thereof; vectors useful in eukaryotic cells, such as vectors useful in insect or mammalian cells; vectors derived from combinations of plasmids and phage DNAs, such as plasmids that have been modified to employ phage DNA or other expression control sequences; and the like.

For example, in a baculovirus expression systems, both non-fusion transfer vectors, such as but not limited to pVL941 (*Bam*H1 cloning site; Summers), pVL1393 (*Bam*H1, *Sma*I*, Xba*I, *Eco*R1*, Not*I*, Xma*III, *BgI*II*,* and *Pst*I cloning site; Invitrogen), pVL1392 (*BgI*II*, Pst*I*, Not*I*, Xma*III, *EcoR*I, *Xba*I*, Sma*I, and *Bam*H1 cloning site; Summers and Invitrogen), and pBlue*Bac*III (*Bam*H1*, Bgl*II, *Pst*I*, Nco*I, and *Hind*III cloning site, with blue/white recombinant screening possible; Invitrogen), and fusion transfer vectors, such as but not limited to pAc700 (*Bam*H1 and *Kpn*I cloning site, in which the *Bam*H1 recognition site begins with the initiation codon; Summers), pAc701 and pAc702 (same as pAc700, with different reading frames), pAc360 (*Bam*H1 cloning site 36 base pairs downstream of a polyhedrin initiation codon; Invitrogen(195)), and pBlueBacHisA, B, C (three different reading frames, with *Bam*H1, *Bgl*II, *Pst*I*, Nco*I, and *Hind*III cloning site, an N-terminal peptide for ProBond purification, and blue/white recombinant screening of plaques; Invitrogen (220) can be used.

Mammalian expression vectors contemplated for use in the invention include vectors with inducible promoters, such as the dihydrofolate reductase (DHFR) promoter, e.g., any expression vector with a *DHFR* expression vector, or a *DHFR*/methotrexate co-amplification vector, such as pED *(Pst*I*, Sal*I*, Sba*I*, Sma*I*,* and *Eco*RI cloning site, with the vector expressing both the cloned gene and *DHFR; see* Kaufman, *Current Protocols in Molecular Biology,* 16.12 (1991). Alternatively, a glutamine synthetase/methionine sulfoximine co-amplification vector, such as pEE14 (*Hind*III*, Xba*I*, Sma*I, *Sba*I*, Eco*RI*,* and *Bcl*I cloning site, in which the vector expresses glutamine synthase and the cloned gene; Celltech). In another embodiment, a vector that directs episomal expression under control of Epstein Barr Virus (EBV) can be used, such as pREP4 *(BamH*I, *Sfi*I*, Xho*I*, Not*I*, Nhe*I*, Hind*III*, Nhe*I*, Pvu*II, and *Kpn*I cloning site, constitutive RSV-LTR promoter, hygromycin selectable marker; Invitrogen), pCEP4 (*Bam*HI, *Sfi*I, *Xho*I, *Not*I, *Nhe*I, *Hind*III, *Nhe*I, *Pvu*II, and *Kpn*I cloning site, constitutive hCMV immediate early gene, hygromycin selectable marker; Invitrogen), pMEP4 (*Kpn*I*, Pvu*I*, Nhe*I, *Hind*III*, Not*I*, Xho*I*, Sfi*I*, Bam*H1 cloning site, inducible methallothionein IIa gene promoter, hygromycin selectable marker: Invitrogen), pREP8 (*BamH*I*, Xho*I*, Not*I*, Hind*III*, Nhe*I*,* and *Kpn*I cloning site, RSV-LTR promoter, histidinol selectable marker; Invitrogen), pREP9 (*Kpn*I*, Nhe*I*, Hind*III*, Not*I*, Xho*I*, Sfi*I*,* and BamHI cloning site, RSV-LTR promoter, G418 selectable marker; Invitrogen), and pEBVHis (RSV-LTR promoter, hygromycin selectable marker, N-terminal peptide purifiable via ProBond resin and cleaved by enterokinase; Invitrogen). Selectable mammalian expression vectors for use in the invention include pRc/CMV (*Hind*III*, Bst*XI*, Not*I*, Sba*I*, and Apa*I cloning site, G418 selection; Invitrogen), pRc/RSV (*Hind*III*, Spel, Bst*XI*, Not*I*, Xba*I cloning site, G418 selection; Invitrogen), and others. Vaccinia virus mammalian expression vectors *(see,* Kaufman, 1991, *supra)* for use according to the invention include but are not limited to pSC11 (*Sma*I cloning site, TK- and b-gal selection), pMJ601 *(Sal*I*, Sma*I*, Afl*I, *Nar*I*, Bsp*MII*, Bam*HI*, Apa*I*, Nhe*I*, Sac*II*, Kpn*I*,* and *Hind*III cloning site; TK-and b-gal selection), and pTKgptF1S (*EcoR*I*, Pst*I*, Sal*I*, Acc*I*, Hind*III*, Sba*I*, Bam*HI*,* and Hpa cloning site, TK or XPRT selection).

Yeast expression systems can also be used according to the invention to express an ABC1 polypeptide. For example, the non-fusion pYES2 vector (*Xba*I*, Sph*I*, Sho*I*, Not*I*, Gst*XI*, Eco*RI*, Bst*XI, *Bam*H1*, Sac*I*, Kpn*1, and *Hind*III cloning sit; Invitrogen) or the fusion pYESHisA, B, C (*Xba*I, *Sph*I*, Sho*I*, Not*I*, Bst*XI, *Eco*RI*, Bam*H1*, Sac*I*, Kpn*I*,* and *Hind*III cloning site, N-terminal peptide purified with ProBond resin and cleaved with enterokinase; Invitrogen), to mention just two, can be employed according to the invention.

Once a particular recombinant DNA molecule is identified and isolated, several methods known in the art may be used to propagate it. Once a suitable host system and growth conditions are established, recombinant expression vectors can be propagated and prepared in quantity. As previously explained, the expression vectors which can be used include, but are not limited to, the following vectors or their derivatives: human or animal viruses such as vaccinia virus or adenovirus; insect viruses such as baculovirus; yeast vectors; bacteriophage vectors (*e.g.,* lambda), and plasmid and cosmid DNA vectors, to name but a few.

In addition, a host cell strain may be chosen which modulates the expression of the inserted sequences, or modifies and processes the gene product in the specific fashion desired. Different host cells have characteristic and specific mechanisms for the translational and post-translational processing and modification (*e.g.,* glycosylation, cleavage [*e.g.,* of signal sequence]) of proteins. Appropriate cell lines or host systems can be chosen to ensure the desired modification and processing of the foreign protein expressed. For example, expression in a bacterial system can be used to produce an nonglycosylated core protein product. However, the transmembrane ABC1 protein expressed in bacteria may not be properly folded. Expression in yeast can produce a glycosylated product. Expression in eukaryotic cells can increase the likelihood of "native" glycosylation and folding of a heterologous protein. Moreover, expression in mammalian cells can provide a tool for reconstituting, or constituting, ABC1 activity. Furthermore, different vector/host expression systems may affect processing reactions, such as proteolytic cleavages, to a different extent.

Vectors are introduced into the desired host cells by methods known in the art, *e.g.,* transfection, electroporation, microinjection, transduction, cell fusion, DEAE dextran, calcium phosphate precipitation, lipofection (lysosome fusion), use of a gene gun, or a DNA vector transporter (see, *e.g.,* Wu et al., 1992; Wu and Wu, 1988; Hartmut et al., Canadian Patent Application No.2,012,311, filed March 15, 1990).

A cell has been "transfected" by exogenous or heterologous DNA when such DNA has been introduced inside the cell. A cell has been "transformed" by exogenous or heterologous DNA when the transfected DNA effects a phenotypic change. Preferably, the transforming DNA should be integrated (covalently linked) into chromosomal DNA making up the genome of the cell.

A recombinant marker protein expressed as an integral membrane protein can be isolated and purified by standard methods. Generally, the integral membrane protein can be obtained by lysing the membrane with detergents, such as but not limited to, sodium dodecyl sulfate (SDS), Triton X-100 polyoxyethylene ester, Ipagel/nonidet P-40 (NP-40) (octylphenoxy)-polyethoxyethanol, digoxin, sodium deoxycholate, and the like, including mixtures thereof. Solubilization can be enhanced by sonication of the suspension. Soluble forms of the protein can be obtained by collecting culture fluid, or solubilizing inclusion bodies, *e.g.,* by treatment with detergent, and if desired sonication or other mechanical processes, as described above. The solubilized or soluble protein can be isolated using various techniques, such as polyacrylamide gel electrophoresis (PAGE), isoelectric focusing, 2-dimensional gel electrophoresis, chromatography *(e.g.,* ion exchange, affinity, immunoaffinity, and sizing column chromatography), centrifugation, differential solubility, immunoprecipitation, or by any other standard technique for the purification of proteins.

Alternatively, a nucleic acid or vector according to the invention can be introduced *in vivo* by lipofection. For the past decade, there has been increasing use of liposomes for encapsulation and transfection of nucleic acids *in vitro.* Synthetic cationic lipids designed to limit the difficulties and dangers encountered with liposome mediated transfection can be used to prepare liposomes for *in vivo* transfection of a gene encoding a marker (Felgner, et. al. 1987; Mackey, et al., 1988; Ulmer et al., 1993). The use of cationic lipids may promote encapsulation of negatively charged nucleic acids, and also promote fusion with negatively charged cell membranes (Felgner and Ringold, 1989). Particularly useful lipid compounds and compositions for transfer of nucleic acids are described in International Patent Publications WO95/18863 and WO96/17823, and in U.S. Patent No. 5,459,127. The use of lipofection to introduce exogenous genes into the specific organs *in vivo* has certain practical advantages. Molecular targeting of liposomes to specific cells represents one area of benefit. It is clear that directing transfection to particular cell types would be particularly preferred in a tissue with cellular heterogeneity, such as pancreas, liver, kidney, and the brain. Lipids may be chemically coupled to other molecules for the purpose of targeting [*see* Mackey, et. al., *supra*]*.* Targeted peptides, *e.g.,* hormones or neurotransmitters, and proteins such as antibodies, or non-peptide molecules could be coupled to liposomes chemically.

Other molecules are also useful for facilitating transfection of a nucleic acid *in vivo,* such as a cationic oligopeptide (*e.g.,* International Patent Publication WO95/21931), peptides derived from DNA binding proteins (*e.g.,* International Patent Publication WO96/25508), or a cationic polymer (*e.g.,* International Patent Publication WO95/21931).

It is also possible to introduce the vector *in vivo* as a naked DNA plasmid (see U.S. Patents 5,693,622, 5,589,466 and 5,580,859). Naked DNA vectors for gene therapy can be introduced into the desired host cells by methods known in the art, *e.g.,* transfection, electroporation, microinjection, transduction, cell fusion, DEAE dextran, calcium phosphate precipitation, use of a gene gun, or use of a DNA vector transporter (*see,* Wu et al., 1992; Wu and Wu, 1988; Hartmut et al., Canadian Patent Application No. 2,012,311, filed March 15, 1990; Williams et al., 1991). Receptor-mediated DNA delivery approaches can also be used (Curiel et al., 1992; Wu and Wu, 1987).

"Pharmaceutically acceptable vehicle or excipient " includes diluents and fillers which are pharmaceutically acceptable for method of administration, are sterile, and may be aqueous or oleaginous suspensions formulated using suitable dispersing or wetting agents and suspending agents. The particular pharmaceutically acceptable carrier and the ratio of active compound to carrier are determined by the solubility and chemical properties of the composition, the particular mode of administration, and standard pharmaceutical practice.

Any nucleic acid, polypeptide, vector, or host cell of the invention will preferably be introduced *in vivo* in a pharmaceutically acceptable vehicle or excipient. The phrase "pharmaceutically acceptable" refers to molecular entities and compositions that are physiologically tolerable and do not typically produce an allergic or similar untoward reaction, such as gastric upset, dizziness and the like, when administered to a human. Preferably, as used herein, the term "pharmaceutically acceptable" means approved by a regulatory agency of the Federal or a state government or listed in the U.S. Pharmacopeia or other generally recognized pharmacopeia for use in animals, and more particularly in humans. The term "excipient" refers to a diluent, adjuvant, excipient, or vehicle with which the compound is administered. Such pharmaceutical carriers can be sterile liquids, such as water and oils, including those of petroleum, animal, vegetable or synthetic origin, such as peanut oil, soybean oil, mineral oil, sesame oil and the like. Water or aqueous solution saline solutions and aqueous dextrose and glycerol solutions are preferably employed as excipients, particularly for injectable solutions. Suitable pharmaceutical excipients are described in "Remington's Pharmaceutical Sciences" by E.W. Martin.

Naturally, the invention contemplates delivery of a vector that will express a therapeutically effective amount of an ABC1 polypeptide for gene therapy applications. The phrase "therapeutically effective amount" is used herein to mean an amount sufficient to reduce by at least about 15 percent, preferably by at least 50 percent, more preferably by at least 90 percent, and still more preferably prevent, a clinically significant deficit in the activity, function and response of the host. Alternatively, a therapeutically effective amount is sufficient to cause an improvement in a clinically significant condition in the host.

"Lipid profile" means the set of concentrations of cholesterol, triglyceride, lipoprotein cholesterol and other lipids in the body of a human or other animal.

An "undesirable lipid profile" is the condition in which the concentrations of cholesterol, triglyceride, or lipoprotein cholesterol are outside of the age- and gender-adjusted reference ranges. Generally, a concentration of total cholesterol > 200 mg/dl, of plasma triglycerides > 200 mg/dl, of LDL cholesterol > 130 mg/dl, of HDL cholesterol < 39 mg/dl, or a ratio of total cholesterol to HDL cholesterol > 4.0 is considered to be an undesirable lipid profile. An undesirable lipid profile is associated with a variety of pathological conditions, including hyperlipidaemias, diabetes hypercholesterolaemia, atherosclerosis, and other forms of coronary artery disease.

### NUCLEIC ACIDS OF THE ABC1 GENE

### GENOMIC SEQUENCES

The human ABC1 gene is thought to comprise 48 exons and 47 introns, if reference is made in particular to the structure of the orthologous ABC1 gene in mice. Recently, partial exonic and intronic ABC1 genomic DNA has been isolated and identified (Rust et al., 1999). According to the invention, it has now been shown that the human ABC1 gene comprises 49 exons and 48 introns.

Several partial genomic nucleotide sequences of the ABC1 gene have been isolated and characterized according to the invention, these genomic sequences comprise both new exonic sequences and intronic sequences which may be used in particular for the production of various means of detection of the ABC1 gene or of its nucleotide expression products in a sample.
These partial genomic sequences are represented in Table I.

**Table I**

| **Partial (p) genomic DNA of the human ABC1 gene** | |
|---|---|
| SEQ ID NO: | ABC1 genomic DNA |
| 1 | Intron 1(p), exon 2, intron 2, exon 3, intron 3, exon 4, intron 4(p) |
| 4 | Intron 4(p), exon 5, intron 5(p) |
| 5 | Intron 5(p), exon 6, intron 6(p) |
| 6 | Intron 6(p), exon 7, intron 7(p) |
| 7 | Intron 7(p), exon 8, intron 8(p) |
| 8 | Intron 8(p), exon 9, intron 9, exon 10, intron 10(p) |
| 9 | Intron 10(p), exon 11, intron 11(p) |
| 10 | Intron 11(p), exon 12, intron 12, exon 13, intron 13, exon 14, intron 14, exon 15, intron 15, exon 16, intron 16, exon 17, intron 17(p) |
| 11 | Intron 17(p), exon 18, intron 18(p) |
| 12 | Intron 38(p), exon 39, intron 39(p) |
| 13 | Intron 42(p), exon 43, intron 43(p) |
| 14 | Intron 43(p), exon 44, intron 44(p) |
| 15 | Intron 44(p), exon 45, intron 45 (p) |
| 16 | Intron 45(p), exon 46, intron 46, exon 47, intron 47(p) |
| 17 | Intron 48(p), exon 49, 3'distal sequence |

Thus, a first subject of the invention consists in a nucleic acid comprising a polynucleotide sequence of any one of SEQ ID NOs: 1 and 4-17, or of a complementary polynucleotide sequence.

The invention also relates to a nucleic acid comprising at least 8 consecutive nucleotides of a polynucleotide sequence comprising a) any one of SEQ ID NOs: 1 and 4-8, and 11 - 16, or a complementary polynucleotide sequence, b) nucleotides 3154-3200 of SEQ ID NO: 9, or a complementary polynucleotide sequence, c) nucleotides 1-242 of SEQ ID NO: 10, or a complementary polynucleotide sequence, or d) nucleotides 1-1851 of SEQ ID NO: 17, or a complementary polynucleotide sequence.

The subject of the invention is, in addition, a nucleic acid having at least 80% nucleotide identity with a nucleic acid comprising a) any one of SEQ ID NOs: 1 and 4-8, and 11-16, or a complementary polynucleotide sequence, b) nucleotides 3154-3200 of SEQ ID NO: 9, or a complementary polynucleotide sequence, c) nucleotides 1-242 of SEQ ID NO: 10, or a complementary polynucleotide sequence, or d) nucleotides 1-1851 of SEQ ID NO: 17, or a complementary polynucleotide sequence.

The invention also relates to a nucleic acid having at least 85%, preferably 90%, more preferably 95% and still more preferably 98% nucleotide identity with a nucleic acid comprising a) any one of SEQ ID NOs: 1 and 4-8, and 11-16, or a complementary polynucleotide sequence, b) nucleotides 3154-3200 of SEQ ID NO: 9, or a complementary polynucleotide sequence, c) nucleotides 1-242 of SEQ ID NO: 10, or a complementary polynucleotide sequence, or d) nucleotides 1-1851 of SEQ ID NO: 17, or a complementary polynucleotide sequence.

The invention also relates to a nucleic acid hybridizing, under high stringency conditions, with a nucleic acid comprising a) any one of SEQ ID NOs: 1 and 4-8, and 11 - 16, or a complementary polynucleotide sequence, b) nucleotides 3154-3200 of SEQ ID NO: 9, or a complementary polynucleotide sequence, c) nucleotides 1-242 of SEQ ID NO: 10, or a complementary polynucleotide sequence, or d) nucleotides 1-1851 of SEQ ID NO: 17, or a complementary polynucleotide sequence.

Several exons of the ABC1 gene have been characterized, at least partially, by their nucleotide sequence and are indicated in Table II.

**Table II**

| **Human ABC1 Exon DNA** | | | | |
|---|---|---|---|---|
| Exon No. | SEQ ID NO: | Located in SEQ ID NO: | Position of the 5' nucleotide | Position of the 3' nucleotide |
| 2 | 18 | 1 | 4647 | 4740 |
| 3 | 19 | 1 | 9274 | 9415 |
| 4 | 20 | 1 | 10685 | 10803 |
| 5 | 21 | 4 | 254 | 375 |
| 6 | 22 | 5 | 161 | 337 |
| 7 | 23 | 6 | 107 | 199 |
| 8 | 24 | 7 | 604 | 844 |
| 9 | 25 | 8 | 615 | 754 |
| 10 | 26 | 8 | 1084 | 1200 |
| 11 | 27 | 9 | 3003 | 3200 |
| 17 | 28 | 10 | 7888 | 8001 |
| 18 | 29 | 11 | 388 | 559 |
| 39 | 30 | 12 | 4 | 127 |
| 43 | 31 | 13 | 266 | 372 |
| 44 | 32 | 14 | 16 | 157 |
| 45 | 33 | 15 | 177 | 311 |
| 46 | 34 | 16 | 377 | 480 |
| 47 | 35 | 16 | 963 | 1055 |
| 49 | 36 | 17 | 195 | 3088 |

Thus, the invention also relates to a nucleic acid comprising any one of SEQ ID NOs: 18-36, or a complementary polynucleotide sequence.

The invention also relates to a nucleic acid comprising a polynucleotide sequence as depicted in any one of SEQ ID NOs: 18-36, or a complementary polynucleotide sequence.

The invention also relates to a nucleic acid comprising at least 8 consecutive nucleotides of a) any one of SEQ ID NOs: 18-26 and 28-35, or a complementary polynucleotide sequence, b) nucleotides 152-198 of SEQ ID NO: 27, or a complementary polynucleotide sequence, or c) nucleotides 1-1657 of SEQ ID NO: 36, or a complementary polynucleotide sequence.

The subject of the invention is, in addition, a nucleic acid having at least 80% nucleotide identity with a nucleic acid comprising a) any one of SEQ ID NOs: 18-26 and 28-35, or a complementary polynucleotide sequence, b) nucleotides 152-198 of SEQ ID NO: 27, or a complementary polynucleotide sequence, or c) nucleotides 1-1657 of SEQ ID NO: 36, or a complementary polynucleotide sequence.

The invention also relates to a nucleic acid having at least 85%, preferably 90%, more preferably 95% and still more preferably 98% nucleotide identity with a nucleic acid comprising a) any one of SEQ ID NOs: 18-26 and 28-35, or a complementary polynucleotide sequence, b) nucleotides 152-198 of SEQ ID NO: 27, or a complementary polynucleotide sequence, or c) nucleotides 1-1657 of SEQ ID NO: 36, or a complementary polynucleotide sequence.

The invention also relates to a nucleic acid hybridizing, under high stringency conditions, with a nucleic acid comprising a) any one of SEQ ID NOs: 18-26 and 28-35, or a complementary polynucleotide sequence, b) nucleotides 152-198 of SEQ ID NO: 27, or a complementary polynucleotide sequence, or c) nucleotides 1-1657 of SEQ ID NO: 36, or a complementary polynucleotide sequence.

Moreover, several introns of the ABC1 gene have been isolated and characterized, at least partially. The intronic nucleic acids of the ABC1 gene, as well as their fragments and their variants may also be used as nucleotide probes or primers for detecting the presence of at least one copy of the ABC1 gene in a sample, or alternatively for amplifying a given target sequence in the ABC1 gene.

The intronic nucleic acid sequences of the ABC1 gene are indicated in Table III.

**Table III**

| **Human ABC1 Intron DNA** | | | | |
|---|---|---|---|---|
| Intron No. | SEQ ID NO: | Located in SEQ ID NO: | Position of the 5' nucleotide | Position of the 3' nucleotide |
| 1 (3' end) | 37 | 1 | 1 | 4646 |
| 2 | 38 | 1 | 4741 | 9273 |
| 3 | 39 | 1 | 9416 | 10684 |
| 4 (5' end) | 40 | 1 | 10804 | 11754 |
| 4 (3' end) | 41 | 4 | 1 | 253 |
| 5 (5' end) | 42 | 4 | 376 | 571 |
| 5 (3' end) | 43 | 5 | 1 | 160 |
| 6 (5' end) | 44 | 5 | 338 | 428 |
| 6 (3' end) | 45 | 6 | 1 | 106 |
| 7 (5' end) | 46 | 6 | 200 | 213 |
| 7 (3' end) | 47 | 7 | 1 | 603 |
| 8 (5' end) | 48 | 7 | 845 | 1402 |
| 8 (3' end) | 49 | 8 | 1 | 614 |
| 9 | 50 | 8 | 755 | 1083 |
| 10 (5' end) | 51 | 8 | 1201 | 1808 |
| 11 (5' end) | 52 | 9 | 3201 | 3215 |
| 11 (3' end) | 53 | 10 | 1 | 639 |
| 17 (3' end) | 54 | 11 | 1 | 387 |
| 18 (5' end) | 55 | 11 | 560 | 578 |
| 38 (3' end) | 56 | 12 | 1 | 3 |
| 39 (5' end) | 57 | 12 | 128 | 384 |
| 42 (3' end) | 58 | 13 | 1 | 265 |
| 43 (5' end) | 59 | 13 | 373 | 386 |
| 43 (3' end) | 60 | 14 | 1 | 15 |
| 44 (5' end) | 61 | 14 | 158 | 345 |
| 44 (3' end) | 62 | 15 | 1 | 176 |
| 45 (5' end) | 63 | 15 | 312 | 618 |
| 45 (3' end) | 64 | 16 | 1 | 376 |
| 46 | 65 | 16 | 481 | 962 |
| 47 (5' end) | 137 | 16 | 1056 | 1329 |
| 48 (3' end) | 68 | 17 | 1 | 194 |

Thus, the invention also relates to a nucleic acid comprising a polynucleotide sequence of any one of SEQ ID NOs: 37-65, 68, and 137, or a complementary polynucleotide sequence.

The invention also relates to a nucleic acid comprising a polynucleotide sequence as depicted in any one of SEQ ID NOs: 37-65, 68, and 137, or a complementary polynucleotide sequence.

The invention also relates to a nucleic acid comprising at least 8 consecutive nucleotides of a) any one of SEQ ID NOs: 37-52, 54-65, 68, and 137, or a complementary polynucleotide sequence, or b) nucleotides 1-242 of SEQ ID NO: 53, or a complementary polynucleotide sequence.

The subject of the invention is, in addition, a nucleic acid having at least 80% nucleotide identity with a nucleic acid comprising a) any one of SEQ ID NOs: 37-52, 54-65, 68, and 137, or a complementary polynucleotide sequence, or b) nucleotides 1-242 of SEQ ID NO: 53, or a complementary polynucleotide sequence.

The invention also relates to a nucleic acid having at least 85%, preferably 90%, more preferably 95% and still more preferably 98% nucleotide identity with a nucleic acid comprising a) any one of SEQ ID NOs: 37-52, 54-65, 68, and 137, or a complementary polynucleotide sequence, or b) nucleotides 1-242 of SEQ ID NO: 53, or a complementary polynucleotide sequence.

The invention also relates to a nucleic acid hybridizing, under high stringency conditions, with a nucleic acid comprising a) any one of SEQ ID NOs: 37-52, 54-65, 68, and 137, or a complementary polynucleotide sequence, or b) nucleotides 1-242 of SEQ ID NO: 53, or a complementary polynucleotide sequence.

### cDNA MOLECULES ENCODING A FULL LENGTH ABC1 PROTEIN

As already indicated above, a partial cDNA sequence corresponding to the human ABC1 gene has been identified by Langmann et al. (1999). This partial cDNA sequence of ABC1 consists of 6880 nucleotides and contains a 6603 nucleotide open reading frame corresponding to a partial 2201 amino acid (aa) ABC1 polypeptide produced in subjects not affected by disorders linked to the reverse transport of cholesterol. The cDNA sequence described by Langmann et al. (1999) contains, in addition, a portion of the 5'- untranslated region (UTR) (nucleotides 1 to 120) and a portion of the 3'-UTR (nucleotides 6727 to 6880).

Recently, an additional portion of the 3'-UTR of the human ABC1 cDNA has been isolated and identified (Rust et al., 1999).

A cDNA that encodes the full length human ABC1 protein has now been identified according to the invention (see Example 2). This novel, human ABC1 cDNA (SEQ ID NO: 69) includes a newly identified 5'-region comprising 244 additional 5' nucleotides and the true initiation ATG codon. According to the invention, the new human ABC1 cDNA comprises a larger open reading frame than that previously reported by Langmann et al. (1999) and Rust et al. (1999). In particular, this cDNA nucleic acid molecule of the invention encodes 60 additional amino acids at the N-terminus of the full length human ABC1 protein. In addition, this new cDNA molecule of the invention comprises a newly identified 5'UTR.

Specifically, a cDNA molecule of the human ABC1 gene having the polynucleotide sequence of SEQ ID NO: 69 comprises an open reading frame beginning from the nucleotide at position 185 (base A of the ATG codon for initiation of translation) to the nucleotide at position 6967. A polyadenylation signal (having the sequence ATTAAA) is present, starting from the nucleotide at position 9698 of the sequence SEQ ID NO: 69. According to the invention, the ABC1 cDNA comprising SEQ ID NO: 69 encodes a full length ABC1 polypeptide of 2261 amino acids comprising the amino acid sequence of SEQ ID NO: 71.

Consequently, the invention also relates to a nucleic acid comprising SEQ ID NO: 69, or a complementary polynucleotide sequence.

The invention also relates to a nucleic acid comprising a polynucleotide sequence as depicted in SEQ ID NO: 69, or a complementary polynucleotide sequence.

The invention also relates to a nucleic acid molecule comprising nucleotides 1-244 of SEQ ID NO: 69, or a complementary polynucleotide sequence.

The invention also relates to a nucleic acid comprising at least eight consecutive nucleotides of nucleotides 1-244 of SEQ ID NO: 69, or a complementary polynucleotide sequence.

The subject of the invention is also a nucleic acid having at least 80% nucleotide identity with a nucleic acid comprising nucleotides 1-244 of SEQ ID NO: 69, or a nucleic acid having a complementary polynucleotide sequence.

The invention also relates to a nucleic acid having at least 85%, preferably 90%, more preferably 95% and still more preferably 98% nucleotide identity with a nucleic acid comprising a nucleotides 1-244 of SEQ ID NOs: 69, or a nucleic acid having a complementary polynucleotide sequence.

Another subject of the invention is a nucleic acid hybridizing, under high stringency conditions, with a nucleic acid comprising nucleotides 1-244 of SEQ ID NO: 69, or a nucleic acid having a complementary polynucleotide sequence.

A search of the GenBank Database (www.ncbi.nlm.nih.gov) revealed an expressed sequence tag (EST), accession number Z44377, from normalized infant brain that overlapped with nucleotides 1-179 of SEQ ID NO: 69 at nucleotides 114-292 of the EST. Until the cDNA molecule according to the invention was identified, no connection between this EST and ABC1 was known. Therefore, this EST represents additional 5' UTR nucleotides of the human ABC1 cDNA.

Thus, the invention also relates to a cDNA molecule comprising nucleotides 1 - 113 of EST (GenBank Accession # Z44377) and nucleotides 1-9741 (SEQ ID NO: 69) of the ABC1 cDNA molecule. This second cDNA molecule comprises the nucleotide sequence of SEQ ID NO: 70. The ORF of this second cDNA molecule is located from nucleotide 298 to nucleotide 7080 of SEQ ID NO: 70 and the polyadenylation signal (having the sequence ATTAAA) is located beginning at nucleotide 9811 of SEQ ID NO: 70. The nucleic acid comprising SEQ ID NO: 70 also encodes the full length human ABC1 protein comprising an amino acid sequence of SEQ ID NO: 71.

Consequently, the invention also relates to a nucleic acid comprising SEQ ID NO: 70, or a complementary polynucleotide sequence.

The invention also relates to a nucleic acid comprising a polynucleotide sequence as depicted in SEQ ID NO: 70, or a complementary polynucleotide sequence.

The invention also relates to a nucleic acid comprising nucleotides 1-357 of SEQ ID NO: 70, or a complementary polynucleotide sequence.

The invention also relates to a nucleic acid comprising at least eight consecutive nucleotides of nucleotides 1-357 of SEQ ID NO: 70, or a complementary polynucleotide sequence.

The subject of the invention is also a nucleic acid having at least 80% nucleotide identity with a nucleic acid comprising nucleotides 1-357 of SEQ ID NO: 70, or a complementary polynucleotide sequence.

The invention also relates to a nucleic acid having at least 85%, preferably 90%, more preferably 95% and still more preferably 98% nucleotide identity with a nucleic acid comprising a nucleotides 1-357 of SEQ ID NO: 70, or a complementary polynucleotide sequence.

Another subject of the invention is a nucleic acid hybridizing, under high stringency conditions, with a nucleic acid comprising nucleotides 1-357 of SEQ ID NO: 70, or a nucleic acid having a complementary polynucleotide sequence.

According to the invention, a nucleic acid comprising a polynucleotide sequence of either SEQ ID NO: 69 or SEQ ID NO: 70 encodes a full length human ABC polypeptide of 2261 amino acids comprising the amino acid sequence of SEQ ID NO: 71.

Thus, the invention also relates to a nucleic acid encoding a polypeptide comprising an amino acid sequence of SEQ ID NO: 71.

The invention also relates to a nucleic acid encoding a polypeptide comprising an amino acid sequence as depicted in SEQ ID NO: 71.

In a specific embodiment, the invention also relates to a nucleic acid encoding a polypeptide comprising amino acids 1-60 of SEQ ID NO: 71.

The invention also relates to a polypeptide comprising amino acid sequence of SEQ ID NO: 71.

The invention also relates to a polypeptide comprising amino acid sequence as depicted in SEQ ID NO: 71.

The invention relates to a polypeptide comprising amino acids 1-60 of SEQ ID NO: 71.

The invention also relates to a polypeptide comprising an amino acid sequence having at least 80% amino acid identity with a polypeptide comprising an amino acid sequence comprising amino acids 1-60 of SEQ ID NO: 71, or a peptide fragment thereof.

The invention also relates to a polypeptide having at least 85%, preferably 90%, more preferably 95% and still more preferably 98% amino acid identity with a polypeptide comprising an amino acid sequence comprising amino acids 1-60 of SEQ ID NO: 71.

Preferably, a polypeptide according to the invention will have a length of 15, 18 or 20 to 25, 35, 40, 50, 70, 80, 100 or 200 consecutive amino acids of a polypeptide according to the invention, in particular a polypeptide comprising an amino acid sequence comprising amino acids 1-60 of SEQ ID NO: 71.

Alternatively, a polypeptide according to the invention will comprise a fragment having a length of 15, 18, 20, 25, 35, 40, 50, 100 or 200 consecutive amino acids of a polypeptide according to the invention, more particularly of a polypeptide comprising an amino acid sequence comprising amino acids 1-60 of SEQ ID NO: 71.

### POLYMORPHISMS WITHIN THE ABC1 GENE

### MUTATIONS

The analysis of mutations in the ABC1 gene may be carried out on genomic DNA from several individuals belonging to a family of which several members suffer from Tangier and/or FHD disease with premature coronary disorders. According to the invention, several mutations have been identified in regions of the ABC1 gene, which encode the ABC1 polypeptide. These mutations have been found particularly in patients suffering from severe forms of Tangier disease, associated with serious coronary disorders. Seventeen mutations are described in Table IV. The mutation of the wild-type (WT) sequence is indicated in Table IV for each mutant sequence.

**Table IV**

| **Mutations found in the ABC1 gene** | | | | |
|---|---|---|---|---|
| ABC1 Gene Mutation Location | Nucleotide SEQ ID NO: of Mutant Exon: | Mutant ABC1 Polypeptide SEQ ID NO: | Mutation (WT → Mutant) | Effect of Mutation on ABC1 protein |
| Exon 5, nt 53 | 72 | ---- | G → A | Silent at as 158 |
| Exon 6, nt 113 | 73 | 89 | G → A | R → K at aa219 |
| Exon 6, nt 136 | 74 | 90 | C → T | STOP at aa226 |
| Exon 8, nt 31 | 75 | 91 | C → T | STOP at aa281 |
| Exon 8, nt 123 | 76 | ---- | C → T | Silent at aa312 |
| Exon 8, nt 135 | 77 | ---- | G → A | Silent at aa316 |
| Exon 13, nt 109 | 78 | 92 | G → - | Frameshift at aa608 → STOP at aa634 |
| Exon 15, nt 205 | 79 | 93 | A → C | T → P at aa774 |
| Exon 17, nt 9 | 80 | 94 | G → A | G → R at aa851 |
| Exon 17, nt 107 | 81 | 95 | A → G | I → M at aa883 |
| Exon 22, nt 101-102 | 82 | 96 | CT → - | Frameshift at aa1114 → STOP at aal 144 |
| Exon 22, nt 102-103 | 83 | 97 | TC → - | Frameshift at aa1114 → STOP at as 1144 |
| Exon 27, nt 123 | 84 | 98 | C → T | R → W at aa1342 |
| Exon 32, nt 19 | 85 | 99 | G → A | W → STOP at aa1525 |
| Exon 34, nt 62 | 86 | 100 | G → A | R → K at aa1587 |
| Exon 47, nt 2 | 87 | 101 | A → G | M → V at aa2104 |
| Exon 47, nt 27 | 88 | 102 | C → - | Frameshift at aa2112 → STOP at aa2130 |

The structural characteristics which make it possible to differentiate the normal sequences from the mutated sequences of ABC1 (genomic sequences, messenger RNAs, cDNA) may be exploited in order to produce means of detection of the mutated sequences of ABC1 in a sample, in particular, probes specifically hybridizing with the mutated sequences of ABC1 or pairs of primers making it possible to selectively amplify the regions of the ABC1 gene carrying the mutations described above, it being possible to carry out the detection of the presence of these mutations in particular by distinguishing the length of the amplified nucleic acid fragments, by hybridization of the amplified fragments with the aid of the specific probes described above, or by direct sequencing of these amplified fragments.

Thus, the invention relates to a nucleic acid comprising any one of SEQ ID NOs: 72-88, or a complementary polynucleotide sequence.

The invention also relates to a nucleic acid comprising a polynucleotide sequence as depicted in any one of SEQ ID NOs: 72-88, or a complementary polynucleotide sequence.

A further subject of the invention is a nucleic acid encoding a polypeptide comprising an amino acid sequence of any one of SEQ ID NOs: 89-102.

The invention also relates to a polypeptide comprising an amino acid sequence of any one of SEQ ID NOs: 89-102.

### OTHER POLYMORPHISMS

Other polymorphisms have been found within the ABC1 gene, in particular nucleotide substitutions located in the noncoding regions (introns). Each of the eight polymorphisms identified in the ABC1 gene are represented in Table V. Table V depicts the wild type (WT) allele and the mutant allele, with the polymorphism itself being defined by the two nucleotide sequences corresponding respectively to each of the alleles.

**Table V**

| **Polymorphisms found in the ABC1 gene** | | | |
|---|---|---|---|
| ABC1 Gene Intron Location | Wild Type Allele SEQ ID NO: | Mutant Allele SEQ ID NO: | Polymorphic base Allele 1/Allele 2 |
| Intron 7, nt 590 | 103 | 111 | Insertion of an A |
| Intron 24, nt 23 | 104 | 112 | G → A |
| Intron 31, nt 30 | 105 | 113 | G → T |
| Intron 32, nt 982 | 106 | 114 | G → A |
| Intron 32, nt 1099 | 107 | 115 | G → C |
| Intron 40, nt 146 | 108 | 116 | C → T |
| Intron 47, nt 13 | 109 | 117 | A → G |
| Intron 47, nt 88 | 110 | 118 | A → C |

According to another aspect, the invention also relates to nucleic acids of the ABC1 gene comprising a nucleotide sequence comprising at least one biallelic polymorphism as described in Table V.

Thus, the invention relates to a nucleic acid comprising any one of SEQ ID NOs: 103 and 109-118, or a complementary polynucleotide sequence.

The invention also relates to a nucleic acid comprising a nucleotide sequence as depicted in SEQ ID NOs: 103 and 109-118, or a complementary polynucleotide sequence.

The detection of these ABC1 gene polymorphisms within a DNA sample obtained from a subject may, for example, be carried out by a specific amplification of the nucleotide region of ABC1 containing the polymorphic base, and then sequencing the amplified fragment in order to determine the nature of the allele or of the alleles carried by said subject.

The detection of these polymorphisms in a DNA sample obtained from a subject may also be carried out with the aid of nucleotide probes or primers specifically hybridizing with a given allele containing one of the polymorphic bases of a polymorphism of the ABC1 gene according to the invention.

By way of illustration, appropriate nucleotide primers are for example primers whose base at the 3' end hybridizes with the base located immediately on the 5' side of the polymorphic base of the fragment comprising said polymorphism. After the step of hybridization of the specific primer, a step of extension with a mixture of the two dideoxynucleotides complementary to the polymorphic base of said polymorphism, for example differentially labeled by fluorescence, and then a step detection of the fluorescence signal obtained makes it possible to determine which of the two differentially labeled fluorescent dideoxynucleotides has been incorporated and to directly deduce the nature of the polymorphic base present at the level of this polymorphism.

Various approaches may be used for the labeling and detection of the dideoxynucleotides. A method in homogeneous phase based on FRET ("Fluorescence resonance energy transfer") has been described by Chen and Kwok (1997). According to this method, the amplified fragments of genomic DNA containing polymorphisms are incubated with a primer labeled with fluorescein at the 5' end in the presence of labeled dideoxynucleotide triphosphate and a modified Taq polymerase. The labeled primer is extended by one base by incorporation of the labeled dideoxynucleotide specific for the allele present on the complementary genomic DNA sequence. At the end of this genotyping reaction, the fluorescence intensities for the two labeling compounds for the labeled dideoxynucleotides are directly analyzed without separation or purification. All these steps may be carried out in the same tube and the modifications of the fluorescence signal monitored in real time. According to another embodiment, the extended primer may be analyzed by MALDI-TOF type mass spectrometry. The base located at the level of the polymorphic site is identified by measuring the mass added to the microsequencing primer (Haff and Smirnov, 1997).

Such nucleotide primers may, for example, be immobilized on a support. Furthermore, it is possible to immobilize on a support, for example in an orderly manner, multiple specific primers as described above, each of the primers being suited to the detection of one of the polymorphisms of the ABC1 gene according to the invention.

The polymorphisms of the ABC1 gene according to the invention are useful in particular as genetic markers in studies of association between the presence of a given allele in a subject and the predisposition of this subject to a given pathology, in particular to one of the pathologies already associated with the chromosomal region 9q31 preferably with a pathology linked to a dysfunction in the reverse transport of cholesterol.

The methods for the genetic analysis of complex characters (phenotypes) are of various types (Lander and Schork, 1994). In general, the biallelic polymorphisms according to the invention are useful in any of the methods described in the state of the art intended to demonstrate a statistically significant correlation between a genotype and a phenotype. The biallelic polymorphisms may be used in linkage analyses and in allele sharing methods. Preferably, the biallelic polymorphisms according to the invention are used to identify genes associated with detectable characters (phenotypes) in use for studies of association, an approach which does not require the use of families affected by the character, and which allows, in addition, the identification of genes associated with complex and sporadic characters.

Other statistical methods using biallelic polymorphisms according to the invention are for example those described by Forsell et al. (1997), Xiong et al. (1999), Horvath et al. (1998), Sham et al. (1995) or Nickerson et al. (1992).

### NUCLEOTIDE PROBES AND PRIMERS

Nucleotide probes and primers hybridizing with a nucleic acid (genomic DNA, messenger RNA, cDNA) according to the invention also form part of the invention.

According to the invention, nucleic acid fragments derived from a polynucleotide comprising a) any one of SEQ ID NOs: 1, 4-8, 11-16, 18-26, 28-35, 37-52, 54-65, 68, and 137, or of a complementary polynucleotide sequence; b) nucleotides 3154-3200 of SEQ ID NO: 9 or of a complementary polynucleotide sequence; c) nucleotides 1-242 of SEQ ID NO: 10 or of a complementary polynucleotide sequence; d) nucleotides 1-1851 of SEQ ID NO: 17; or of a complementary polynucleotide sequence; e) nucleotides 152-198 of SEQ ID NO: 27, or of a complementary polynucleotide sequence; f) nucleotides 1-1657 of SEQ ID NO: 36, or of a complementary polynucleotide sequence; g) nucleotides 1-242 of SEQ ID NO: 53, or of a complementary polynucleotide sequence, or h) nucleotides 1-244 of SEQ ID NO: 69 or nucleotides 1-357 of SEQ ID NO: 70, or a complementary polynucleotide sequence, are useful for the detection of the presence of at least one copy of a nucleotide sequence of the ABC1 gene or of a fragment or of a variant (containing a mutation or a polymorphism) thereof in a sample.

The nucleotide probes or primers according to the invention comprise a nucleotide sequence comprising a) any one of SEQ ID NOs: 1, 4-8, 11-16, 18-26, 28-35, 37-52, 54-65, 68, and 137, or of a complementary polynucleotide sequence; b) nucleotides 3154-3200 of SEQ ID NO: 9 or of a complementary polynucleotide sequence; c) nucleotides 1-242 of SEQ ID NO: 10 or of a complementary polynucleotide sequence; d) nucleotides 1-1851 of SEQ ID NO: 17; or of a complementary polynucleotide sequence; e) nucleotides 152-198 of SEQ ID NO: 27, or of a complementary polynucleotide sequence; f) nucleotides 1-1657 of SEQ ID NO: 36, or of a complementary polynucleotide sequence; g) nucleotides 1-242 of SEQ ID NO: 53, or of a complementary polynucleotide sequence, or h) nucleotides 1-244 of SEQ ID NO: 69 or nucleotides 1-357 of SEQ ID NO: 70, or a complementary polynucleotide sequence.

The nucleotide probes or primers according to the invention comprise at least 8 consecutive nucleotides of a nucleic acid comprising a) any one of SEQ ID NOS: 1, 4-8, 11-16, 18-26, 28-35, 37-52, 54-65, 68, and 137, or of a complementary polynucleotide sequence; b) nucleotides 3154-3200 of SEQ ID NO: 9 or of a complementary polynucleotide sequence; c) nucleotides 1-242 of SEQ ID NO: 10 or of a complementary polynucleotide sequence; d) nucleotides 1-1851 of SEQ ID NO: 17; or of a complementary polynucleotide sequence; e) nucleotides 152-198 of SEQ ID NO: 27, or of a complementary polynucleotide sequence; f) nucleotides 1-1657 of SEQ ID NO: 36, or of a complementary polynucleotide sequence; g) nucleotides 1-242 of SEQ ID NO: 53, or of a complementary polynucleotide sequence, or h) nucleotides 1-244 of SEQ ID NO: 69 or nucleotides 1-357 of SEQ ID NO: 70, or a complementary polynucleotide sequence.

Preferably, nucleotide probes or primers according to the invention will have a length of 10, 12, 15, 18 or 20 to 25, 35, 40, 50, 70, 80, 100, 200, 500, 1000, 1500 consecutive nucleotides of a nucleic acid according to the invention, in particular of a nucleic acid comprising a) any one of SEQ ID NOs: 1, 4-8, 11-16, 18-26, 28-35, 37-52, 54-65, 68, and 137, or of a complementary polynucleotide sequence; b) nucleotides 3154-3200 of SEQ ID NO: 9 or of a complementary polynucleotide sequence; c) nucleotides 1-242 of SEQ ID NO: 10 or of a complementary polynucleotide sequence; d) nucleotides 1-1851 of SEQ ID NO: 17; or of a complementary polynucleotide sequence; e) nucleotides 152-198 of SEQ ID NO: 27, or of a complementary polynucleotide sequence; f) nucleotides 1-1657 of SEQ ID NO: 36, or of a complementary polynucleotide sequence; g) nucleotides 1-242 of SEQ ID NO: 53, or of a complementary polynucleotide sequence, or h) nucleotides 1-244 of SEQ ID NO: 69 or nucleotides 1-357 of SEQ ID NO: 70, or a complementary polynucleotide sequence.

Alternatively, a nucleotide probe or primer according to the invention will consist of and/or comprise the fragments having a length of 12, 15, 18, 20, 25, 35, 40, 50, 100, 200, 500, 1000, 1500 consecutive nucleotides of a nucleic acid according to the invention, more particularly of a nucleic acid comprising a) any one of SEQ ID NOs: 1, 4-8, 11-16, 18-26, 28-35, 37-52, 54-65, 68, and 137, or of a complementary polynucleotide sequence; b) nucleotides 3154-3200 of SEQ ID NO: 9 or of a complementary polynucleotide sequence; c) nucleotides 1-242 of SEQ ID NO: 10 or of a complementary polynucleotide sequence; d) nucleotides 1-1851 of SEQ ID NO: 17; or of a complementary polynucleotide sequence; e) nucleotides 152-198 of SEQ ID NO: 27, or of a complementary polynucleotide sequence; f) nucleotides 1-1657 of SEQ ID NO: 36, or of a complementary polynucleotide sequence; g) nucleotides 1-242 of SEQ ID NO: 53, or of a complementary polynucleotide sequence, or h) nucleotides 1-244 of SEQ ID NO: 69 or nucleotides 1-357 of SEQ ID NO: 70, or a complementary polynucleotide sequence.

The definition of a nucleotide probe or primer according to the invention therefore covers oligonucleotides which hybridize, under the high stringency hybridization conditions defined above, with a nucleic acid comprising a) any one of SEQ ID NOs: 1, 4-8, 11-16, 18-26, 28-35, 37-52, 54-65, 68, and 137, or of a complementary polynucleotide sequence; b) nucleotides 3154-3200 of SEQ ID NO: 9 or of a complementary polynucleotide sequence; c) nucleotides 1-242 of SEQ ID NO: 10 or of a complementary polynucleotide sequence; d) nucleotides 1-1851 of SEQ ID NO: 17; or of a complementary polynucleotide sequence; e) nucleotides 152-198 of SEQ ID NO: 27, or of a complementary polynucleotide sequence; f) nucleotides 1-1657 of SEQ ID NO: 36, or of a complementary polynucleotide sequence; g) nucleotides 1-242 of SEQ ID NO: 53, or of a complementary polynucleotide sequence, or h) nucleotides 1-244 of SEQ ID NO: 69 or nucleotides 1-357 of SEQ ID NO: 70, or a complementary polynucleotide sequence.

According to a preferred embodiment, a nucleotide primer according to the invention comprises a nucleotide sequence of any one of SEQ ID NOs: 119-136, 138, and 141-152, or of a complementary nucleic acid sequence.

Examples of primers and pairs of primers which make it possible to amplify various regions of the ABC1 gene are presented in Table VI below. The location of each primer of SEQ ID NOs: 119-136, 138, and 141-152 within SEQ ID NOs: 1, 4-9, 11, and 13-16, and its hybridizing region is indicated in Table VI. The abbreviation "Comp" refers to the complementary nucleic acid sequence.

**Table VI**

| **Primers for the amplification of nucleic fragments of the ABC1 gene** | | | |
|---|---|---|---|
| Primer SEQ ID NO: | Located in SEQ ID NO: | Position in the sequence | Region for hybridization |
| 119 | 1 | 4621-4642 | Intron 1 |
| 120 | 1 | Comp 4793-4814 | Intron 2 |
| 121 | 1 | 9207-9226 | Intron 2 |
| 122 | 1 | Comp 9583-9603 | Intron 3 |
| 123 | 1 | 10455-10475 | Intron 3 |
| 124 | 1 | Comp 10880-10897 | Intron 4 |
| 125 | 4 | 101-121 | Intron 4 |
| 126 | 4 | Comp 473-492 | Intron 5 |
| 127 | 5 | 83-102 | Intron 5 |
| 128 | 5 | Comp 378-395 | Intron 6 |
| 129 | 6 | 4-25 | Intron 6 |
| 130 | 6 | Comp 193-212 | Exon 7/Intron 7 |
| 131 | 7 | 449-470 | Intron 7 |
| 132 | 7 | Comp 881-899 | Intron 8 |
| 133 | 8 | 451-472 | Intron 8 |
| 134 | 8 | Comp 916-934 | Intron 9 |
| 135 | 8 | 870-891 | Intron 9 |
| 136 | 8 | Comp 1311-1330 | Intron 10 |
| 138 | 9 | Comp 3191-3212 | Exon 11/Intron 11 |
| 141 | 11 | 251-269 | Intron 17 |
| 142 | 11 | Comp 551-570 | Exon 18/Intron 18 |
| 143 | 13 | 189-209 | Intron 42 |
| 144 | 13 | Comp 364-385 | Exon 43/Intron 43 |
| 145 | 14 | 12-32 | Intron 43/Exon 44 |
| 146 | 14 | Comp 184-202 | Intron 44 |
| 147 | 15 | 23-40 | Intron 44 |
| 148 | 15 | Comp 437-455 | Intron 45 |
| 149 | 16 | 158-178 | Intron 45 |
| 150 | 16 | Comp 528-549 | Intron 46 |
| 151 | 16 | 817-836 | Intron 46 |
| 152 | 16 | Comp 1174-1195 | Intron 47 |

According to a specific embodiment of preferred probes and primers according to the invention, they comprise all or part of a polynucleotide sequence comprising any one of SEQ ID NOs: 119-136, 138, and 141-152, or a nucleic acid having a complementary nucleic acid sequence.

A nucleotide primer or probe according to the invention may be prepared by any suitable method well known to persons skilled in the art, including by cloning and action of restriction enzymes or by direct chemical synthesis according to techniques such as the phosphodiester method by Narang et al. (1979) or by Brown et al. (1979), the diethylphosphoramidite method by Beaucage et al. (1980) or the technique on a solid support described in EU patent No. EP 0,707,592.

Each of the nucleic acids according to the invention, including the oligonucleotide probes and primers described above, may be labeled, if desired, by incorporating a marker which can be detected by spectroscopic, photochemical, biochemical, immunochemical or chemical means. For example, such markers may consist of radioactive isotopes (³²P, ³³P, ³H, ³⁵S), fluorescent molecules (5-bromodeoxyuridine, fluorescein, acetylaminofluorene, digoxigenin) or ligands such as biotin. The labeling of the probes is preferably carried out by incorporating labeled molecules into the polynucleotides by primer extension, or alternatively by addition to the 5' or 3' ends. Examples of nonradioactive labeling of nucleic acid fragments are described in particular in French patent No. 78 109 75 or in the articles by Urdea et al. (1988) or Sanchez-pescador et al. (1988).

Preferably, the nucleotide probes and primers according to the invention may have structural characteristics of the type to allow amplification of the signal, such as the probes described by Urdea et al. (1991) or alternatively in European patent No. EP-0,225,807 (CHIRON).

The oligonucleotide probes according to the invention may be used in particular in Southern-type hybridizations with the genomic DNA or alternatively in hybridizations with the corresponding messenger RNA when the expression of the corresponding transcript is sought in a sample.

The probes and primers according to the invention may also be used for the detection of products of PCR amplification or alternatively for the detection of mismatches.

Nucleotide probes or primers according to the invention may be immobilized on a solid support. Such solid supports are well known to persons skilled in the art and comprise surfaces of wells of microtiter plates, polystyrene beds, magnetic beds, nitrocellulose bands or microparticles such as latex particles.

Consequently, the present invention also relates to a method of detecting the presence of a nucleic acid comprising a polynucleotide sequence of any one of SEQ ID NOs: 1, 4-65, 68-70, 72-88, 103, 109-118, and 137, or of a complementary polynucleotide sequence, or a nucleic acid fragment or variant of any one of SEQ ID NOs: 1, 4-65, 68-70, 72-88, 103, 109-118, and 137, or of a complementary polynucleotide sequence in a sample, said method comprising the steps of:
1) bringing one or more nucleotide probes or primers according to the invention into contact with the sample to be tested;
2) detecting the complex which may have formed between the probe(s) and the nucleic acid present in the sample.

According to a specific embodiment of the method of detection according to the invention, the oligonucleotide probes and primers are immobilized on a support.

According to another aspect, the oligonucleotide probes and primers comprise a detectable marker.

The invention relates, in addition, to a box or kit for detecting the presence of a nucleic acid according to the invention in a sample, said box or kit comprising:
a) one or more nucleotide probe(s) or primer(s) as described above;
b) where appropriate, the reagents necessary for the hybridization reaction.

According to a first aspect, the detection box or kit is characterized in that the probe(s) or primer(s) are immobilized on a support.

According to a second aspect, the detection box or kit is characterized in that the oligonucleotide probes comprise a detectable marker.

According to a specific embodiment of the detection kit described above, such a kit will comprise a plurality of oligonucleotide probes and/or primers in accordance with the invention which may be used to detect a target nucleic acid of interest or alternatively to detect mutations in the coding regions or the non-coding regions of the nucleic acids according to the invention, more particularly of nucleic acids comprising any one of SEQ ID NOs: 1, 4-65, 68-70, 72-88, 103, 109-118, and 137, or a complementary polynucleotide sequence.

Thus, the probes according to the invention, immobilized on a support, may be ordered into matrices such as "DNA chips". Such ordered matrices have in particular been described in US patent No. 5,143,854, in published PCT applications WO 90/15070 and WO 92/10092.

Support matrices on which oligonucleotide probes have been immobilized at a high density are for example described in US patent No. 5,412,087 and in published PCT application WO 95/1 1995.

The nucleotide primers according to the invention may be used to amplify any one of the nucleic acids according to the invention, and more particularly a nucleic acid comprising a polynucleotide sequence of any one of SEQ ID NOs: 1, 4-65, 68-70, 72-88, 103, 109-118, and 137, or of a complementary polynucleotide sequence. Alternatively, the nucleotide primers according to the invention may be used to amplify a nucleic acid fragment or variant of any one of SEQ ID NOs: 1, 4-65, 68-70, 72-88, 103, 109-118, and 137, or of a complementary polynucleotide sequence.

In a particular embodiment, the nucleotide primers according to the invention may be used to amplify a nucleic acid comprising a) any one of SEQ ID NOs: 1, 4-8, 11-16, 18-26, 28-35, 37-52, 54-65, 68, and 137, or of a complementary polynucleotide sequence; b) nucleotides 3154-3200 of SEQ ID NO: 9 or of a complementary polynucleotide sequence; c) nucleotides 1-242 of SEQ ID NO: 10 or of a complementary polynucleotide sequence; d) nucleotides 1-1851 of SEQ ID NO: 17; or of a complementary polynucleotide sequence; e) nucleotides 152-198 of SEQ ID NO: 27, or of a complementary polynucleotide sequence; f) nucleotides 1-1657 of SEQ ID NO: 36, or of a complementary polynucleotide sequence; g) nucleotides 1-242 of SEQ ID NO: 53, or of a complementary polynucleotide sequence, h) nucleotides 1-244 of SEQ ID NO: 69 or nucleotides 1-357 of SEQ ID NO: 70, or a complementary polynucleotide sequence, or i) as depicted in any one of SEQ ID NOs: 1, 4-65, 68-70, 72-88, 103, 109-118, and 137, or of a complementary polynucleotide sequence.

Another subject of the invention relates to a method of amplifying a nucleic acid according to the invention, and more particularly a nucleic acid comprising a) any one of SEQ ID NOs: 1, 4-8, 11-16, 18-26, 28-35, 37-52, 54-65, 68, and 137, or of a complementary polynucleotide sequence; b) nucleotides 3154-3200 of SEQ ID NO: 9 or of a complementary polynucleotide sequence; c) nucleotides 1-242 of SEQ ID NO: 10 or of a complementary polynucleotide sequence; d) nucleotides 1-1851 of SEQ ID NO: 17; or of a complementary polynucleotide sequence; e) nucleotides 152-198 of SEQ ID NO: 27, or of a complementary polynucleotide sequence; f) nucleotides 1-1657 of SEQ ID NO: 36, or of a complementary polynucleotide sequence; g) nucleotides 1-242 of SEQ ID NO: 53, or of a complementary polynucleotide sequence, h) nucleotides 1-244 of SEQ ID NO: 69 or nucleotides 1-357 of SEQ ID NO: 70, or a complementary polynucleotide sequence, or i) as depicted in any one of SEQ ID NOs: 1, 4-65, 68-70, 72-88, 103, 109-118, and 137, or of a complementary polynucleotide sequence, contained in a sample, said method comprising the steps of:
a) bringing the sample in which the presence of the target nucleic acid is suspected into contact with a pair of nucleotide primers whose hybridization position is located respectively on the 5' side and on the 3' side of the region of the target nucleic acid whose amplification is sought, in the presence of the reagents necessary for the amplification reaction; and
b) detecting the amplified nucleic acids.

To carry out the amplification method as defined above, use will be preferably made of any of the nucleotide primers described above.

The subject of the invention is, in addition, a box or kit for amplifying a nucleic acid according to the invention, and more particularly a nucleic acid comprising a) any one of SEQ ID NOs: 1, 4-8, 11-16, 18-26, 28-35, 37-52, 54-65, 68-70, and 137, or of a complementary polynucleotide sequence; b) nucleotides 3154-3200 of SEQ ID NO: 9 or of a complementary polynucleotide sequence; c) nucleotides 1-242 of SEQ ID NO: 10 or of a complementary polynucleotide sequence; d) nucleotides 1-1851 of SEQ ID NO: 17; or of a complementary polynucleotide sequence; e) nucleotides 152-198 of SEQ ID NO: 27, or of a complementary polynucleotide sequence; f) nucleotides 1-1657 of SEQ ID NO: 36, or of a complementary polynucleotide sequence; g) nucleotides 1-242 of SEQ ID NO: 53, or of a complementary polynucleotide sequence, h) nucleotides 1-244 of SEQ ID NO: 69 or nucleotides 1-357 of SEQ ID NO: 70, or a complementary polynucleotide sequence, or i) as depicted in any one of SEQ ID NOs: 1, 4-65, 68-70, 72-88, 103, 109-118, and 137, or of a complementary polynucleotide sequence, said box or kit comprising:
a) a pair of nucleotide primers in accordance with the invention, whose hybridization position is located respectively on the 5' side and 3' side of the target nucleic acid whose amplification is sought; and optionally,
b) reagents necessary for the amplification reaction.

Such an amplification box or kit will preferably comprise at least one pair of nucleotide primers as described above.

The subject of the invention is, in addition, a box or kit for amplifying all or part of a nucleic acid comprising a) any one of SEQ ID NOs: 1, 4-8, 11-16, 18-26, 28-35, 37-52, 54-65, 68, and 137, or of a complementary polynucleotide sequence; b) nucleotides 3154-3200 of SEQ ID NO: 9 or of a complementary polynucleotide sequence; c) nucleotides 1-242 of SEQ ID NO: 10 or of a complementary polynucleotide sequence; d) nucleotides 1-1851 of SEQ ID NO: 17; or of a complementary polynucleotide sequence; e) nucleotides 152-198 of SEQ ID NO: 27, or of a complementary polynucleotide sequence; f) nucleotides 1-1657 of SEQ ID NO: 36, or of a complementary polynucleotide sequence; g) nucleotides 1-242 of SEQ ID NO: 53, or of a complementary polynucleotide sequence, or h) nucleotides 1-244 of SEQ ID NO: 69 or nucleotides 1-357 of SEQ ID NO: 70, or a complementary polynucleotide sequence, said box or kit comprising:
1) a pair of nucleotide primers in accordance with the invention, whose hybridization position is located respectively on the 5' side and 3' side of the target nucleic acid whose amplification is sought; and optionally,
2) reagents necessary for an amplification reaction.

Such an amplification box or kit will preferably comprise at least one pair of nucleotide primers as described above.

The invention also relates to a box or kit for detecting the presence of a nucleic acid according to the invention in a sample, said box or kit comprising:
a) one or more nucleotide probes according to the invention;
b) where appropriate, reagents necessary for a hybridization reaction.

According to a first aspect, the detection box or kit is characterized in that the nucleotide probe(s) and primer(s)are immobilized on a support.

According to a second aspect, the detection box or kit is characterized in that the nucleotide probe(s) and primer(s) comprise a detectable marker.

According to a specific embodiment of the detection kit described above, such a kit will comprise a plurality of oligonucleotide probes and/or primers in accordance with the invention which may be used to detect target nucleic acids of interest or alternatively to detect mutations in the coding regions or the non-coding regions of the nucleic acids according to the invention. According to preferred embodiment of the invention, the target nucleic acid comprises a polynucleotide sequence of any one of SEQ ID NOs: 1, 4-65, 68-70, 72-88, 103, 109-118, and 137, or of a complementary nucleic acid sequence. Alternatively, the target nucleic acid is a nucleic acid fragment or variant of a nucleic acid comprising any one of SEQ ID NOs: 1, 4-65, 68-70, 72-88, 103, 109-118, and 137, or of a complementary polynucleotide sequence.

According to a preferred embodiment, two primers according to the invention comprise all or part of SEQ ID NOs: 125 and 126, making it possible to amplify the region of exon 5 of the ABC1 gene carrying the mutation as depicted in SEQ ID NO: 72 described above, or nucleic acids having a complementary polynucleotide sequence.

According to a second preferred embodiment, two primers according to the invention comprise all or part of SEQ ID NOs: 127 and 128, making it possible to amplify the region of exon 6 of the ABC1 gene carrying the mutations as depicted in SEQ ID NOs: 73 or 74 described above, or nucleic acids having a complementary polynucleotide sequence.

According to a third preferred embodiment, two primers according to the invention comprise all or part of SEQ ID NOs: 131 and 132, making it possible to amplify the region of exon 8 of the ABC1 gene carrying the mutations as depicted in SEQ ID NOs: 75-77 described above, or nucleic acids having a complementary polynucleotide sequence.

According to a fourth preferred embodiment, two primers according to the invention comprise all or part of SEQ ID NOs: 155 and 156, making it possible to amplify the region of exon 27 of the ABC1 gene carrying the mutation as depicted in SEQ ID NO: 84 described above, or nucleic acids having a complementary polynucleotide sequence.

According to a fifth preferred embodiment, two primers according to the invention comprise all or part of SEQ ID NOs: 159 and 160, making it possible to amplify the region of exon 32 of the ABC1 gene carrying the mutation as depicted in SEQ ID NO: 85 described above, or nucleic acids having a complementary polynucleotide sequence.

According to a sixth preferred embodiment, two primers according to the invention comprise all or part of SEQ ID NOs: 175 and 176, making it possible to amplify the region of exon 47 of the ABC1 gene carrying the mutations as depicted in SEQ ID NOs: 87 or 88 described above, or nucleic acids having a complementary polynucleotide sequence.

According to another preferred embodiment, a primer according to the invention comprise, generally, all or part of any one of SEQ ID NOs: 119-136, 138, and 141-152, or a complementary sequence.

The nucleotide primers according to the invention are particularly useful in methods of genotyping subjects and/or of genotyping populations, in particular in the context of studies of association between particular allele forms or particular forms of groups of alleles (haplotypes) in subjects and the existence of a particular phenotype (character) in these subjects, for example the predisposition of these subjects to develop diseases linked to a deficiency in the reverse transport of cholesterol, or alternatively the predisposition of these subjects to develop a pathology whose candidate chromosomal region is situated on chromosome 9, more precisely on the 9q arm and still more precisely in the 9q31 locus.

### RECOMBINANT VECTORS

The invention also relates to a recombinant vector comprising a nucleic acid according to the invention. "Vector" for the purposes of the present invention will be understood to mean a circular or linear DNA or RNA molecule which is either in single-stranded or double-stranded form.

Preferably, such a recombinant vector will comprise a nucleic acid chosen from the following nucleic acids:
a) a nucleic acid comprising a polynucleotide sequence of any one of SEQ ID NOs: 1, 4-65, 68-70, 72-88, 103, 109-118, and 137, or of a complementary polynucleotide sequence,
b) a nucleic acid comprising a polynucleotide sequence as depicted in any one of SEQ ID NOs: 1, 4-65, 68-70, 72-88, 103, 109-118, and 137, or of a complementary polynucleotide sequence,
c) a nucleic acid comprising nucleotides 1-244 of SEQ ID NO: 69 or nucleotides 1-357 of SEQ ID NO: 70, or of a complementary polynucleotide sequence,
d) a nucleic acid having at least eight consecutive nucleotides of a nucleic acid comprising a polynucleotide sequence of 1) any one of SEQ ID NOs: 1, 4-8, 11-16, 18-26, 28-35, 37-52, 54-65, 68, and 137, or of a complementary polynucleotide sequence; 2) nucleotides 3154-3200 of SEQ ID NO: 9 or of a complementary polynucleotide sequence; 3) nucleotides 1-242 of SEQ ID NO: 10 or of a complementary polynucleotide sequence; 4) nucleotides 1-1851 of SEQ ID NO: 17; or of a complementary polynucleotide sequence; 5) nucleotides 152-198 of SEQ ID NO: 27, or of a complementary polynucleotide sequence; 6) nucleotides 1-1657 of SEQ ID NO: 36, or of a complementary polynucleotide sequence; 7) nucleotides 1-242 of SEQ ID NO: 53, or of a complementary polynucleotide sequence, or 8) nucleotides 1-244 of SEQ ID NO: 69 or nucleotides 1-357 of SEQ ID NO: 70, or a complementary polynucleotide sequence;
e) a nucleic acid having at least 80% nucleotide identity with a nucleic acid comprising a polynucleotide sequence of 1) any one of SEQ ID NOs: 1,4-8, 11-16, 18-26, 28-35, 37-52, 54-65, 68, and 137, or of a complementary polynucleotide sequence; 2) nucleotides 3154-3200 of SEQ ID NO: 9 or of a complementary polynucleotide sequence; 3) nucleotides 1-242 of SEQ ID NO: 10 or of a complementary polynucleotide sequence; 4) nucleotides 1-1851 of SEQ ID NO: 17; or of a complementary polynucleotide sequence; 5) nucleotides 152-198 of SEQ ID NO: 27, or of a complementary polynucleotide sequence; 6) nucleotides 1-1657 of SEQ ID NO: 36, or of a complementary polynucleotide sequence; 7) nucleotides 1-242 of SEQ ID NO: 53, or of a complementary polynucleotide sequence, or 8) nucleotides 1-244 of SEQ ID NO: 69 or nucleotides 1-357 as SEQ ID NO: 70, or a complementary polynucleotide sequence;
f) a nucleic acid having 85%, 90%, 95%, or 98% nucleotide identity with a nucleic acid comprising a polynucleotide sequence of 1) any one of SEQ ID NOs: 1, 4-8, 11-16, 18-26, 28-35, 37-52, 54-65, 68, and 137, or of a complementary polynucleotide sequence; 2) nucleotides 3154-3200 of SEQ ID NO: 9 or of a complementary polynucleotide sequence; 3) nucleotides 1-242 of SEQ ID NO: 10 or of a complementary polynucleotide sequence; 4) nucleotides 1-1851 of SEQ ID NO: 17; or of a complementary polynucleotide sequence; 5) nucleotides 152-198 of SEQ ID NO: 27, or of a complementary polynucleotide sequence; 6) nucleotides 1-1657 of SEQ ID NO: 36, or of a complementary polynucleotide sequence; 7) nucleotides 1-242 of SEQ ID NO: 53, or of a complementary polynucleotide sequence, or 8) nucleotides 1-244 of SEQ ID NO: 69 or nucleotides 1-357 of SEQ ID NO: 70, or a complementary polynucleotide sequence;
g) a nucleic acid hybridizing, under high stringency hybridization conditions, with a nucleic acid comprising a polynucleotide sequence of 1) any one of SEQ ID NOs: 1, 4-8, 11-16, 18-26, 28-35, 37-52, 54-65, 68, and 137, or of a complementary polynucleotide sequence; 2) nucleotides 3154-3200 of SEQ ID NO: 9 or of a complementary polynucleotide sequence; 3) nucleotides 1-242 of SEQ ID NO: 10 or of a complementary polynucleotide sequence; 4) nucleotides 1-1851 of SEQ ID NO: 17; or of a complementary polynucleotide sequence; 5) nucleotides 152-198 of SEQ ID NO: 27, or of a complementary polynucleotide sequence; 6) nucleotides 1-1657 of SEQ ID NO: 36, or of a complementary polynucleotide sequence; 7) nucleotides 1-242 of SEQ ID NO: 53, or of a complementary polynucleotide sequence, or 8) nucleotides 1-244 of SEQ ID NO: 69 or nucleotides 1-357 of SEQ ID NO: 70, or a complementary polynucleotide sequence;
h) a nucleic acid encoding a polypeptide comprising an amino acid sequence of SEQ ID NO: 71; and
i) a nucleic acid encoding a polypeptide comprising amino acids 1-60 of SEQ ID NO: 71.

According to a first embodiment, a recombinant vector according to the invention is used to amplify a nucleic acid inserted therein, following transformation or transfection of a desired cellular host.

According to a second embodiment, a recombinant vector according to the invention corresponds to an expression vector comprising, in addition to a nucleic acid in accordance with the invention, a regulatory signal or nucleotide sequence that directs or controls transcription and/or translation of the nucleic acid and its encoded mRNA.

According to a preferred embodiment, a recombinant vector according to the invention will comprise in particular the following components:
(1) an element or signal for regulating the expression of the nucleic acid to be inserted, such as a promoter and/or enhancer sequence;
(2) a nucleotide coding region comprised within the nucleic acid in accordance with the invention to be inserted into such a vector, said coding region being placed in phase with the regulatory element or signal described in (1); and
(3) an appropriate nucleic acid for initiation and termination of transcription of the nucleotide coding region of the nucleic acid described in (2).

In addition, the recombinant vectors according to the invention may include one or more origins for replication in the cellular hosts in which their amplification or their expression is sought, markers or selectable markers.

By way of example, the bacterial promoters may be the LacI or LacZ promoters, the T3 or T7 bacteriophage RNA polymerase promoters, the lambda phage PR or PL promoters.

The promoters for eukaryotic cells will comprise the herpes simplex virus (HSV) virus thymidine kinase promoter or alternatively the mouse metallothionein-L promoter.

Generally, for the choice of a suitable promoter, persons skilled in the art can preferably refer to the book by Sambrook et al. (1989) cited above or to the techniques described by Fuller et al. (1996).

When the expression of the genomic sequence of the ABC1 gene will be sought, use will preferably be made of the vectors capable of containing large insertion sequences. In a particular embodiment, bacteriophage vectors such as the P1 bacteriophage vectors such as the vector p158 or the vector p158/neo8 described by Sternberg (1992, 1994) will be preferably used.

The preferred bacterial vectors according to the invention are for example the vectors pBR322(ATCC37017) or alternatively vectors such as pAA223-3 (Pharmacia, Uppsala, Sweden), and pGEM I (Promega Biotech, Madison, WI, UNITED STATES).

There may also be cited other commercially available vectors such as the vectors pQE70, pQE60, pQE9 (Qiagen), psiX174, pBluescript SA, pNH8A, pNH16A, pNH18A, pNH46A, pWLNEO, pSV2CAT, pOG44, pXTI, pSG (Stratagene).

They may also be vectors of the baculovirus type such as the vector pVL 1392/1393 (Pharmingen) used to transfect cells of the Sf9 line (ATCC No. CRL 1711) derived from *Spodoptera frugiperda.*

They may also be adenoviral vectors such as the human adenovirus of type 2 or 5.

A recombinant vector according to the invention may also be a retroviral vector or an adeno-associated vector (AAV). Such adeno-associated vectors are for example described by Flotte et al. (1992), Samulski et al. (1989), or McLaughlin BA et al. (1996).

To allow the expression of a polynucleotide according to the invention, the latter must be introduced into a host cell. The introduction of a polynucleotide according to the invention into a host cell may be carried out *in vitro,* according to the techniques well known to persons skilled in the art for transforming or transfecting cells, either in primer culture, or in the form of cell lines. It is also possible to carry out the introduction of a polynucleotide according to the invention *in vivo* or *ex vivo,* for the prevention or treatment of diseases linked to a deficiency in the reverse transport of cholesterol.

To introduce a polynucleotide or vector of the invention into a host cell, a person skilled in the art can preferably refer to various techniques, such as the calcium phosphate precipitation technique (Graham et al., 1973 ; Chen et al., 1987), DEAE Dextran (Gopal, 1985), electroporation (Tur-Kaspa, 1896 ; Potter et al., 1984), direct microinjection (Harland et al., 1985), liposomes charged with DNA (Nicolau et al., 1982, Fraley et al., 1979).

Once the polynucleotide has been introduced into the host cell, it may be stably integrated into the genome of the cell. The intregration may be achieved at a precise site of the genome, by homologous recombination, or it may be randomly integrated. In some embodiments, the polynucleotide may be stably maintained in the host cell in the form of an episome fragment, the episome comprising sequences allowing the retention and the replication of the latter, either independently, or in a synchronized manner with the cell cycle.

According to a specific embodiment, a method of introducing a polynucleotide according to the invention into a host cell, in particular a host cell obtained from a mammal, *in vivo,* comprises a step during which a preparation comprising a pharmaceutically compatible vector and a "naked" polynucleotide according to the invention, placed under the control of appropriate regulatory sequences, is introduced by local injection at the level of the chosen tissue, for example a smooth muscle tissue, the "naked" polynucleotide being absorbed by the cells of this tissue.

Compositions for use *in vitro* and *in vivo* comprising "naked" polynucleotides are for example described in PCT Application No. WO 95/11307 (Institut Pasteur, Inserm, University of Ottawa) as well as in the articles by Tacson et al. (1996) and Huygen et al. (1996).

According to a specific embodiment of the invention, a composition is provided for the *in vivo* production of the ABC1 protein. This composition comprises a polynucleotide encoding the ABC1 polypeptide placed under the control of appropriate regulatory sequences, in solution in a physiologically acceptable vector.

The quantity of vector which is injected into the host organism chosen varies according to the site of the injection. As a guide, there may be injected between about 0.1 and about 100 µg of polynucleotide encoding the ABC1 protein into the body of an animal, preferably into a patient likely to develop a disease linked to a deficiency in the reverse transport of cholesterol or who has already developed this disease, in particular a patient having a predisposition to Tangier disease or a patient who has already developed the disease.

Consequently, the invention also relates to a pharmaceutical composition intended for the prevention of or treatment of a patient or subject affected by a dysfunction in the reverse transport of cholesterol comprising a nucleic acid encoding the ABC1 protein, in combination with one or more physiologically compatible excipients.

Preferably, such a composition will comprise a nucleic acid comprising a polynucleotide sequence of either SEQ ID NO: 69 or SEQ ID NO: 70, wherein the nucleic acid is placed under the control of an appropriate regulatory element or signal.

The subject of the invention is, in addition, a pharmaceutical composition intended for the prevention of or treatment of a patient or a subject affected by a dysfunction in the reverse transport of cholesterol comprising a recombinant vector according to the invention, in combination with one or more physiologically compatible excipients.

The invention also relates to the use of a nucleic acid according to the invention, encoding the ABC1 protein, for the manufacture of a medicament intended for the prevention of atherosclerosis in various forms or more particularly for the treatment of subjects affected by a dysfunction in the reverse transport of cholesterol.

The invention also relates to the use of a recombinant vector according to the invention, comprising a nucleic acid encoding the ABC1 protein, for the manufacture of a medicament intended for the prevention of atherosclerosis in various forms or more particularly for the treatment of subjects affected by a dysfunction in the reverse transport of cholesterol.

The subject of the invention is therefore also a recombinant vector comprising a nucleic acid according to the invention that encodes an ABC1 protein or polypeptide involved in the metabolism of cholesterol.

The invention also relates to the use of such a recombinant vector for the preparation of a pharmaceutical composition intended for the treatment and/or for the prevention of cardiovascular diseases or conditions associated with HDL deficiency, such as the HDL deficiency associated with Tangier and/or FHD disease, HDL deficiency, LCAT deficiency, malaria, and diabetes.

The present invention also relates to the use of cells genetically modified ex *vivo* with such a recombinant vector according to the invention, or of cells producing a recombinant vector, wherein the cells are implanted in the body, to allow a prolonged and effective expression *in vivo* of a biologically active ABC1 polypeptide.

### Vectors useful in methods of somatic gene therapy and compositions containing such vectors

The present invention also relates to a new therapeutic approach for the treatment of pathologies linked to the transport of cholesterol. It provides an advantageous solution to the disadvantages of the prior art, by demonstrating the possibility of treating the pathologies linked to the transport of cholesterol by gene therapy, by the transfer and expression in vivo of a gene encoding an ABC1 protein involved in the transport and the metabolism of cholesterol. The invention thus offers a simple means allowing a specific and effective treatment of related pathologies such as, for example, atherosclerosis.

Gene therapy consists in correcting a deficiency or an abnormality (mutation, aberrant expression and the like) and in bringing about the expression of a protein of therapeutic interest by introducing genetic information into the affected cell or organ. This genetic information may be introduced either ex vivo into a cell extracted from the organ, the modified cell then being reintroduced into the body, or directly in vivo into the appropriate tissue. In this second case, various techniques exist, among which various transfection techniques involving complexes of DNA and DEAE-dextran (Pagano et al., 1967), of DNA and nuclear proteins (Kaneda et al., 1989), of DNA and lipids (Felgner et al., 1987), the use of liposomes (Fraley et al., 1980), and the like. More recently, the use of viruses as vectors for the transfer of genes has appeared as a promising alternative to these physical transfection techniques. In this regard, various viruses have been tested for their capacity to infect certain cell populations. In particular, the retroviruses (RSV, HMS, MMS, and the like), the HSV virus, the adeno-associated viruses and the adenoviruses.

The present invention therefore also relates to a new therapeutic approach for the treatment of pathologies linked to the transport of cholesterol, consisting in transferring and in expressing *in vivo* genes encoding ABC1. Specifically, the present invention provides a new therapeutic approach for the treatment and/or prevention of HDL deficiency, such as the HDL deficiency associated with Tangier and/or FHD disease, HDL deficiency, LCAT deficiency, malaria, and diabetes. In a particularly preferred manner, the applicant has now found that it is possible to construct recombinant vectors comprising a nucleic acid encoding an ABC1 protein involved in the metabolism of cholesterol, to administer these recombinant vectors *in vivo,* and that this administration allows a stable and effective expression of a biologically active ABC1 protein *in vivo,* with no cytopathological effect.

The present invention also results from the demonstration that adenoviruses constitute particularly efficient vectors for the transfer and the expression of the ABC1 gene. In particular, the present invention shows that the use of recombinant adenoviruses as vectors makes it possible to obtain sufficiently high levels of expression of this gene to produce the desired therapeutic effect. Other viral vectors such as retroviruses or adeno-associated viruses (AAV) allowing a stable expression of the gene are also claimed.

The present invention thus offers a new approach for the treatment and prevention of cardiovascular and neurological pathologies linked to the abnormalities of the transport of cholesterol.

The subject of the invention is therefore also a defective recombinant virus comprising a nucleic acid according to the invention that encodes an ABC1 protein or polypeptide involved in the metabolism of cholesterol.

The invention also relates to the use of such a defective recombinant virus for the preparation of a pharmaceutical composition intended for the treatment and/or for the prevention of cardiovascular diseases or conditions associated with HDL deficiency, such as the HDL deficiency associated with Tangier and/or FHD disease, HDL deficiency, LCAT deficiency, malaria, and diabetes.

The present invention also relates to the use of cells genetically modified *ex vivo* with such a defective recombinant virus according to the invention, or of cells producing a defective recombinant virus, wherein the cells are implanted in the body, to allow a prolonged and effective expression *in vivo* of a biologically active ABC1 polypeptide.

The present invention shows that it is possible to incorporate a DNA sequence encoding ABC1 into a viral vector, and that these vectors make it possible to effectively express a biologically active, mature form. More particularly, the invention shows that the in vivo expression of ABC1 may be obtained by direct administration of an adenovirus or by implantation of a producing cell or of a cell genetically modified by an adenovirus or by a retrovirus incorporating such a DNA.

The present invention is particularly advantageous because it makes it possible to induce a controlled expression, and with no harmful effect, of ABC1 in organs which are not normally involved in the expression of this protein. In particular, a significant release of the ABC1 protein is obtained by implantation of cells producing vectors of the invention, or infected ex vivo with vectors of the invention.

The activity of transport of cholesterol produced in the context of the present invention may be of the human or animal ABC1 type. The nucleic sequence used in the context of the present invention may be a cDNA, a genomic DNA (gDNA), an RNA (in the case of retroviruses) or a hybrid construct consisting, for example, of a cDNA into which one or more introns (gDNA) would be inserted. It may also involve synthetic or semisynthetic sequences. In a particularly advantageous manner, a cDNA or a gDNA is used. In particular, the use of a gDNA allows a better expression in human cells. To allow their incorporation into a viral vector according to the invention, these sequences are preferably modified, for example by site-directed mutagenesis, in particular for the insertion of appropriate restriction sites. The sequences described in the prior art are indeed not constructed for use according to the invention, and prior adaptations may prove necessary, in order to obtain substantial expressions. In the context of the present invention, the use of a nucleic sequence encoding a human ABC1 protein is preferred. Moreover, it is also possible to use a construct encoding a derivative of these ABC1 proteins. A derivative of these ABC1 proteins comprises, for example, any sequence obtained by mutation, deletion and/or addition relative to the native sequence, and encoding a product retaining the cholesterol transport activity. These modifications may be made by techniques known to a person skilled in the art (see general molecular biological techniques below). The biological activity of the derivatives thus obtained can then be easily determined, as indicated in particular in the examples of the measurement of the efflux of cholesterol from cells. The derivatives for the purposes of the invention may also be obtained by hybridization from nucleic acid libraries, using as probe the native sequence or a fragment thereof.

These derivatives are in particular molecules having a higher affinity for their binding sites, molecules exhibiting greater resistance to proteases, molecules having a higher therapeutic efficacy or fewer side effects, or optionally new biological properties. The derivatives also include the modified DNA sequences allowing improved expression *in vivo.*

In a first embodiment, the present invention relates to a defective recombinant virus comprising a cDNA encoding an ABC1 polypeptide involved in the transport and metabolism of cholesterol. In another preferred embodiment of the invention, a defective recombinant virus comprises a genomic DNA (gDNA) encoding an ABC1 polypeptide involved in the transport and metabolism of cholesterol. Preferably, the ABC1 polypeptide comprises an amino acid sequence of SEQ ID NO: 71. More preferably, the ABC1 polypeptide comprised amino acids 1-60 of SEQ ID NO:71.

The vectors of the invention may be prepared from various types of viruses. Preferably, vectors derived from adenoviruses, adeno-associated viruses (AAV), herpesviruses (HSV) or retroviruses are used. It is preferable to use an adenovirus, for direct administration or for the ex vivo modification of cells intended to be implanted, or a retrovirus, for the implantation of producing cells.

The viruses according to the invention are defective, that is to say that they are incapable of autonomously replicating in the target cell. Generally, the genome of the defective viruses used in the context of the present invention therefore lacks at least the sequences necessary for the replication of said virus in the infected cell. These regions may be either eliminated (completely or partially), or made nonfunctional, or substituted with other sequences and in particular with the nucleic sequence encoding the ABC1 protein. Preferably, the defective virus retains, nevertheless, the sequences of its genome which are necessary for the encapsidation of the viral particles.

As regards more particularly adenoviruses, various serotypes, whose structure and properties vary somewhat, have been characterized. Among these serotypes, human adenoviruses of type 2 or 5 (Ad 2 or Ad 5) or adenoviruses of animal origin (see Application WO 94/26914) are preferably used in the context of the present invention. Among the adenoviruses of animal origin which can be used in the context of the present invention, there may be mentioned adenoviruses of canine, bovine, murine (example: Mav1, Beard et al., Virology 75 (1990) 81), ovine, porcine, avian or simian (example: SAV) origin. Preferably, the adenovirus of animal origin is a canine adenovirus, more preferably a CAV2 adenovirus [Manhattan or A26/61 strain (ATCC VR-800) for example]. Preferably, adenoviruses of human or canine or mixed origin are used in the context of the invention. Preferably, the defective adenoviruses of the invention comprise the ITRs, a sequence allowing the encapsidation and the sequence encoding the ABC1 protein. Preferably, in the genome of the adenoviruses of the invention, the E1 region at least is made nonfunctional. Still more preferably, in the genome of the adenoviruses of the invention, the E1 gene and at least one of the E2, E4 and L1-L5 genes are nonfunctional. The viral gene considered may be made nonfunctional by any technique known to a person skilled in the art, and in particular by total suppression, by substitution, by partial deletion or by addition of one or more bases in the gene(s) considered. Such modifications may be obtained in vitro (on the isolated DNA) or in situ, for example, by means of genetic engineering techniques, or by treatment by means of mutagenic agents. Other regions may also be modified, and in particular the E3 (WO95/02697), E2 (WO94/28938), E4 (WO94/28152, WO94/12649, WO95/02697) and L5 (WO95/02697) region. According to a preferred embodiment, the adenovirus according to the invention comprises a deletion in the E1 and E4 regions and the sequence encoding ABC1 is inserted at the level of the inactivated E1 region. According to another preferred embodiment, it comprises a deletion in the E1 region at the level of which the E4 region and the sequence encoding ABC1 (French Patent Application FR94 13355) are inserted.

The defective recombinant adenoviruses according to the invention may be prepared by any technique known to persons skilled in the art (Levrero et al., 1991, EP 185 573; and Graham, 1984). In particular, they may be prepared by homologous recombination between an adenovirus and a plasmid carrying, *inter alia,* the nucleic acid encoding the ABC1 protein. The homologous recombination occurs after cotransfection of said adenoviruses and plasmid into an appropriate cell line. The cell line used must preferably (i) be transformable by said elements, and (ii), contain the sequences capable of complementing the part of the defective adenovirus genome, preferably in integrated form in order to avoid the risks of recombination. By way of example of a line, there may be mentioned the human embryonic kidney line 293 (Graham et al., 1977), which contains in particular, integrated into its genome, the left part of the genome of an Ad5 adenovirus (12%) or lines capable of complementing the E1 and E4 functions as described in particular in Applications No. WO 94/26914 and WO95/02697.

Next, the adenoviruses which have multiplied are recovered and purified according to conventional molecular biological techniques, as illustrated in the examples.

As regards the adeno-associated viruses (AAV), they are DNA viruses of a relatively small size, which integrate into the genome of the cells which they infect, in a stable and site-specific manner. They are capable of infecting a broad spectrum of cells, without inducing any effect on cellular growth, morphology or differentiation. Moreover, they do not appear to be involved in pathologies in humans. The genome of AAVs has been cloned, sequenced and characterized. It comprises about 4700 bases, and contains at each end an inverted repeat region (ITR) of about 145 bases, serving as replication origin for the virus. The remainder of the genome is divided into 2 essential regions carrying the encapsidation functions: the left hand part of the genome, which contains the rep gene, involved in the viral replication and the expression of the viral genes; the right hand part of the genome, which contains the cap gene encoding the virus capsid proteins.

The use of vectors derived from AAVs for the transfer of genes *in vitro* and *in vivo* has been described in the literature (see in particular WO 91/18088; WO 93/09239; US 4,797,368, US5,139,941, EP 488 528). These applications describe various constructs derived from AAVs, in which the rep and/or cap genes are deleted and replaced by a gene of interest, and their use for transferring *in vitro* (on cells in culture) or *in vivo* (directly into an organism) said gene of interest. However, none of these documents either describes or suggests the use of a recombinant AAV for the transfer and expression *in vivo* or ex *vivo* of an ABC1 protein, or the advantages of such a transfer. The defective recombinant AAVs according to the invention may be prepared by cotransfection, into a cell line infected with a human helper virus (for example an adenovirus), of a plasmid containing the sequence encoding the ABC1 protein bordered by two AAV inverted repeat regions (ITR), and of a plasmid carrying the AAV encapsidation genes (rep and cap genes). The recombinant AAVs produced are then purified by conventional techniques.

As regards the herpesviruses and the retroviruses, the construction of recombinant vectors has been widely described in the literature: see in particular Breakfield et al., (1991); EP 453242, EP178220, Bernstein et al. (1985); McCormick, (1985), and the like.

In particular, the retroviruses are integrating viruses, infecting dividing cells. The genome of the retroviruses essentially comprises two long terminal repeats (LTRs), an encapsidation sequence and three coding regions (gag, pol and env). In the recombinant vectors derived from retroviruses, the gag, pol and env genes are generally deleted, completely or partially, and replaced with a heterologous nucleic acid sequence of interest. These vectors may be produced from various types of retroviruses such as in particular MoMuLV ("murine moloney leukemia virus"; also called MoMLV), MSV ("murine moloney sarcoma virus"), HaSV ("harvey sarcoma virus"); SNV ("spleen necrosis virus"); RSV ("rous sarcoma virus") or Friend's virus.

To construct recombinant retroviruses containing a sequence encoding the ABC1 protein according to the invention, a plasmid containing in particular the LTRs, the encapsidation sequence and said coding sequence is generally constructed, and then used to transfect a so-called encapsidation cell line, capable of providing in trans the retroviral functions deficient in the plasmid. Generally, the encapsidation lines are therefore capable of expressing the gag, pol and env genes. Such encapsidation lines have been described in the prior art, and in particular the PA317 line (US 4,861,719), the PsiCRIP line (WO 90 /02806) and the GP+envAm-12 line (WO 89/07150). Moreover, the recombinant retroviruses may contain modifications at the level of the LTRs in order to suppress the transcriptional activity, as well as extended encapsidation sequences, containing a portion of the gag gene (Bender et al., 1987). The recombinant retroviruses produced are then purified by conventional techniques.

To carry out the present invention, it is preferable to use a defective recombinant adenovirus. The particularly advantageous properties of adenoviruses are preferred for the *in vivo* expression of a protein having a cholesterol transport activity. The adenoviral vectors according to the invention are particularly preferred for a direct administration *in vivo* of a purified suspension, or for the ex vivo transformation of cells, in particular autologous cells, in view of their implantation. Furthermore, the adenoviral vectors according to the invention exhibit, in addition, considerable advantages, such as in particular their very high infection efficiency, which makes it possible to carry out infections using small volumes of viral suspension.

According to another particularly preferred embodiment of the invention, a line producing retroviral vectors containing the sequence encoding the ABC1 protein is used for implantation in vivo. The lines which can be used to this end are in particular the PA317 (US 4,861,719), PsiCrip (WO 90/02806) and GP+envAm-12 (US 5,278,056) cells modified so as to allow the production of a retrovirus containing a nucleic sequence encoding an ABC1 protein according to the invention. For example, totipotent stem cells, precursors of blood cell lines, may be collected and isolated from a subject. These cells, when cultured, may then be transfected with the retroviral vector containing the sequence encoding the ABC1 protein under the control of viral, nonviral or nonviral promoters specific for macrophages or under the control of its own promoter. These cells are then reintroduced into the subject. The differentiation of these cells will be responsible for blood cells expressing the ABC1 protein, in particular for monocytes which, when transformed to macrophages, participate in the removal of cholesterol from the arterial wall. These macrophages expressing the ABC1 protein will have an increased capacity to metabolize cholesterol in excess and will make it available to the cell surface for its removal by the primary acceptors of membrane cholesterol.

Preferably, in the vectors of the invention, the sequence encoding the ABC1 protein is placed under the control of signals allowing its expression in the infected cells. These may be expression signals which are homologous or heterologous, that is to say signals different from those which are naturally responsible for the expression of the ABC1 protein. They may also be in particular sequences responsible for the expression of other proteins, or synthetic sequences. In particular, they may be sequences of eukaryotic or viral genes or derived sequences, stimulating or repressing the transcription of a gene in a specific manner or otherwise and in an inducible manner or otherwise. By way of example, they may be promoter sequences derived from the genome of the cell which it is desired to infect, or from the genome of a virus, and in particular the promoters of the E1A or major late promoter (MLP) genes of adenoviruses, the cytomegalovirus (CMV) promoter, the RSV-LTR and the like. Among the eukaryotic promoters, there may also be mentioned the ubiquitous promoters (HPRT, vimentin, α-actin, tubulin and the like), the promoters of the intermediate filaments (desmin, neurofilaments, keratin, GFAP, and the like), the promoters of therapeutic genes (of the MDR, CFTR or factor VIII type, and the like), tissue-specific promoters (pyruvate kinase, villin, promoter of the fatty acid binding intestinal protein, promoter of the smooth muscle cell α-actin, promoters specific for the liver; Apo AI, Apo AII, human albumin and the like) or promoters corresponding to a stimulus (steroid hormone receptor, retinoic acid receptor and the like). In addition, these expression sequences may be modified by addition of enhancer or regulatory sequences and the like. Moreover, when the inserted gene does not contain expression sequences, it may be inserted into the genome of the defective virus downstream of such a sequence.

In a specific embodiment, the invention relates to a defective recombinant virus comprising a nucleic acid encoding an ABC1 protein involved in the metabolism of cholesterol under the control of a promoter chosen from RSV-LTR or the CMV early promoter.

As indicated above, the present invention also relates to any use of a virus as described above for the preparation of a pharmaceutical composition for the treatment and/or prevention of pathologies linked to the transport of cholesterol.

The present invention also relates to a pharmaceutical composition comprising one or more defective recombinant viruses as described above. These pharmaceutical compositions may be formulated for administration by the topical, oral, parenteral, intranasal, intravenous, intramuscular, subcutaneous, intraocular or transdermal route and the like. Preferably, the pharmaceutical compositions of the invention comprises a pharmaceutically acceptable vehicle or physiologically compatible excipient for an injectable formulation, in particular for an intravenous injection, such as for example into the patient's portal vein. These may relate in particular to isotonic sterile solutions or dry, in particular, freeze-dried, compositions which, upon addition depending on the case of sterilized water or physiological saline, allow the preparation of injectable solutions. Direct injection into the patient's portal vein is preferred ecause it makes it possible to target the infection at the level of the liver and thus to concentrate the therapeutic effect at the level of this organ.

The doses of defective recombinant virus used for the injection may be adjusted as a function of various parameters, and in particular as a function of the viral vector, of the mode of administration used, of the relevant pathology or of the desired duration of treatment. In general, the recombinant adenoviruses according to the invention are formulated and administered in the form of doses of between 10⁴ and 10¹⁴ pfu/ml, and preferably 10⁶ to 10¹⁰ pfu/ml. The term "pfu" (plaque forming unit) corresponds to the infectivity of a virus solution, and is determined by infecting an appropriate cell culture and measuring, generally after 48 hours, the number of plaques that result from infected cell lysis. The techniques for determining the pfu titer of a viral solution are well documented in the literature.

As regards retroviruses, the compositions according to the invention may directly contain the producing cells, with a view to their implantation.

In this regard, another subject of the invention relates to any mammalian cell infected with one or more defective recombinant viruses according to the invention. More particularly, the invention relates to any population of human cells infected with such viruses. These may be in particular cells of blood origin (totipotent stem cells or precursors), fibroblasts, myoblasts, hepatocytes, keratinocytes, smooth muscle and endothelial cells, glial cells and the like.

The cells according to the invention may be derived from primary cultures. These may be collected by any technique known to persons skilled in the art and then cultured under conditions allowing their proliferation. As regards more particularly fibroblasts, these may be easily obtained from biopsies, for example according to the technique described by Ham (1980). These cells may be used directly for infection with the viruses, or stored, for example by freezing, for the establishment of autologous libraries, in view of a subsequent use. The cells according to the invention may be secondary cultures, obtained for example from preestablished libraries (see for example EP 228458, EP 289034, EP 400047, EP 456640).

The cells in culture are then infected with a recombinant virus according to the invention, in order to confer on them the capacity to produce a biologically active ABC1 protein. The infection is carried out in vitro according to techniques known to persons skilled in the art. In particular, depending on the type of cells used and the desired number of copies of virus per cell, persons skilled in the art can adjust the multiplicity of infection and optionally the number of infectious cycles produced. It is clearly understood that these steps must be carried out under appropriate conditions of sterility when the cells are intended for administration in vivo. The doses of recombinant virus used for the infection of the cells may be adjusted by persons skilled in the art according to the desired aim. The conditions described above for the administration in vivo may be applied to the infection in vitro. For the infection with a retrovirus, it is also possible to co-culture a cell to be infected with a cell producing the recombinant retrovirus according to the invention. This makes it possible to eliminate purification of the retrovirus.

Another subject of the invention relates to an implant comprising mammalian cells infected with one or more defective recombinant viruses according to the invention or cells producing recombinant viruses, and an extracellular matrix. Preferably, the implants according to the invention comprise 10⁵ to 10¹⁰ cells. More preferably, they comprise 10⁶ to 10⁸ cells.

More particularly, in the implants of the invention, the extracellular matrix comprises a gelling compound and optionally a support allowing the anchorage of the cells.

For the preparation of the implants according to the invention, various types of gelling agents may be used. The gelling agents are used for the inclusion of the cells in a matrix having the constitution of a gel, and for promoting the anchorage of the cells on the support, where appropriate. Various cell adhesion agents can therefore be used as gelling agents, such as in particular collagen, gelatin, glycosaminoglycans, fibronectin, lectins and the like. Preferably, collagen is used in the context of the present invention. This may be collagen of human, bovine or murine origin. More preferably, type I collagen is used.

As indicated above, the compositions according to the invention preferably comprise a support allowing the anchorage of the cells. The term anchorage designates any form of biological and/or chemical and/or physical interaction causing the adhesion and/or the attachment of the cells to the support. Moreover, the cells may either cover the support used, or penetrate inside this support, or both. It is preferable to use in the context of the invention a solid, nontoxic and/or biocompatible support. In particular, it is possible to use polytetrafluoroethylene (PTFE) fibers or a support of biological origin.

The present invention thus offers a very effective means for the treatment or prevention of pathologies linked to the transport of cholesterol, in particular obesity, HDL deficiency, hypertriglyceridemia, atherosclerosis, or, in the field of cardiovascular conditions, myocardial infarction, angina, sudden death, cardiac decompensation and cerebrovascular accidents.

In addition, this treatment may be applied to both humans and any animals such as ovines, bovines, domestic animals (dogs, cats and the like), horses, fish and the like.

### RECOMBINANT HOST CELLS

The invention relates to a recombinant host cell comprising a nucleic acid of the invention, and more particularly, a nucleic acid comprising a polynucleotide sequence of either SEQ ID NO: 69 or SEQ ID NO: 70, or of a complementary polynucleotide sequence.

The invention also relates to a recombinant host cell comprising a nucleic acid of the invention, and more particularly a nucleic acid comprising a nucleotide sequence as depicted in SEQ ID NO: 69 or SEQ ID NO: 70, or of a complementary polynucleotide sequence.

Specifically, the invention relates to a recombinant host cell comprising nucleic acid comprising any one of SEQ ID NOs: 1, 4-65, 68-70, 72-88, 103, 109-118, and 137, or of a complementary polynucleotide sequence.

The invention also relates to a recombinant host cell comprising a nucleic acid comprising a polynucleotide sequence as depicted in any one of SEQ ID NOs: 1, 4-65, 68-70, 72-88, 103, 109-118, and 137, or of a complementary polynucleotide sequence.

The invention also relates to a recombinant host cell comprising a nucleic acid comprising nucleotides 1-244 of SEQ ID NO: 69 or nucleotides 1-357 of SEQ ID NO: 70, or a complementary polynucleotide sequence.

The invention also relates to a recombinant host cell comprising a nucleic acid encoding a polypeptide comprising an amino acid sequence of SEQ ID NO: 71.

The invention also relates to a recombinant host cell comprising a nucleic acid encoding a polypeptide comprising amino acids 1-60 of SEQ ID NO: 71.

According to another aspect, the invention also relates to a recombinant host cell comprising a recombinant vector according to the invention. Therefore, the invention also relates to a recombinant host cell comprising a recombinant vector comprising any of the nucleic acids of the invention, and more particularly a nucleic acid comprising a nucleotide sequence of either SEQ ID NO: 69 or SEQ ID NO: 70, or of a complementary polynucleotide sequence.

Specifically, the invention relates to a recombinant host cell comprising a recombinant vector comprising a nucleic acid comprising any one of SEQ ID NOs: 1, 4-65, 68-70, 72-88, 103, 109-118, and 137, or of a complementary polynucleotide sequence.

The invention also relates to a recombinant host cell comprising a recombinant vector comprising a nucleic acid comprising a polynucleotide sequence as depicted in any one of SEQ ID NOs: 1, 4-65, 68-70, 72-88, 103, 109-118, and 137, or of a complementary polynucleotide sequence.

The invention also relates to a recombinant host cell comprising a recombinant vector comprising a nucleic acid comprising nucleotides 1-244 of SEQ ID NO: 69 or nucleotides 1-357 of SEQ ID NO: 70, or of a complementary polynucleotide sequence.

The invention also relates to a recombinant host cell comprising a recombinant vector comprising a nucleic acid encoding a polypeptide comprising an amino acid sequence of SEQ ID NO: 71.

The invention also relates to a recombinant host cell comprising a recombinant vector comprising a nucleic acid encoding a polypeptide comprising amino acids 1-60 of SEQ ID NO: 71.

The preferred host cells according to the invention are for example the following:
a) prokaryotic host cells: strains of *Escherichia coli* (strain DH5-α), of *Bacillus subtilis,* of *Salmonella typhimurium,* or strains of genera such as *Pseudomonas, Streptomyces* and *Staphylococus;*
b) eukaryotic host cells: HeLa cells (ATCC No. CCL2), Cv 1 cells (ATCC No. CCL70), COS cells (ATCC No. CRL 1650), Sf-9 cells (ATCC No. CRL 1711), CHO cells (ATCC No. CCL-61) or 3T3 cells (ATCC No. CRL-6361).

### MUTATED ABC1 POLYPEPTIDES

According to another aspect, the invention relates to a polypeptide encoded by a mutated ABC1 gene, and more particularly a mutated ABC1 gene in patients suffering from a deficiency in the reverse transport of cholesterol, more particularly in patients suffering from Tangier disease.

As indicated above, seventeen mutations have been identified in the ABC1 gene according to the invention (see Table IV).

The polypeptides as depicted in SEQ ID NOs: 89-102 correspond to the non-silent ABC1 gene mutations, and are useful in particular for the preparation of antibodies specifically recognizing them. Such antibodies constitute a means for detecting the production of mutated ABC1 polypeptides in a sample obtained from a subject to be tested, preferably a patient having symptoms characteristic of a deficiency in the reverse transport of cholesterol, and more preferably in a patient having the symptoms characteristic of Tangier and/or FHD disease.

According to another aspect, the invention also relates to a polypeptide comprising an amino acid sequence of any one of SEQ ID NOs: 89-102.

According to another aspect, the invention also relates to a polypeptide comprising an amino acid sequence as depicted in any one of SEQ ID NOs: 89-102.

Generally, the polypeptides according to the invention are provided in an isolated or purified form.

### METHODS FOR PRODUCING ABC1 POLYPEPTIDES

The invention also relates to a method for the production of a polypeptide comprising an amino acid sequence of any one of SEQ ID NOs: 71 and 89-102, said method comprising the steps of:
a) inserting a nucleic acid encoding said polypeptide into an appropriate vector;
b) culturing, in an appropriate culture medium, a previously transformed host cell or transfecting a host cell with the recombinant vector of step a);
c) recovering the conditioned culture medium or lysing the host cell, for example by sonication or by osmotic shock;
d) separating and purifying said polypeptide from said culture medium or alternatively from the cell lysates obtained in step c); and
e) where appropriate, characterizing the recombinant polypeptide produced.

A specific embodiment of the invention relates to a method for producing a polypeptide comprising an amino acid sequence of amino acids 1-60 of SEQ ID NO: 71.

The polypeptides according to the invention may be characterized by binding to an immunoaffinity chromatography column on which the antibodies directed against this polypeptide or against a fragment or a variant thereof have been previously immobilized.

According to another aspect, a recombinant polypeptide according to the invention may be purified by passing it over an appropriate series of chromatography columns, according to methods known to persons skilled in the art and described for example in F. Ausubel et al (1989).

A polypeptide according to the invention may also be prepared by conventional chemical synthesis techniques either in homogeneous solution or in solid phase. By way of illustration, a polypeptide according to the invention may be prepared by the technique either in homogeneous solution described by Houben Weyl (1974) or the solid phase synthesis technique described by Merrifield (1965a; 1965b).

A polypeptide termed "homologous" to a polypeptide having an amino acid sequence comprising amino acids 1-60 of SEQ ID NO: 71 also forms part of the invention. Such a homologous polypeptide comprises an amino acid sequence possessing one or more substitutions of an amino acid by an equivalent amino acid, relative to amino acids 1-60 of SEQ ID NO: 71.

An "equivalent amino acid" according to the present invention will be understood to mean for example replacement of a residue in the L form by a residue in the D form or the replacement of a glutamic acid (E) by a pyro-glutamic acid according to techniques well known to persons skilled in the art. By way of illustration, the synthesis of peptide containing at least one residue in the D form is described by Koch (1977). According to another aspect, two amino acids belonging to the same class, that is to say two uncharged polar, nonpolar, basic or acidic amino acids, are also considered as equivalent amino acids.

Polypeptides comprising at least one nonpeptide bond such as a retro-inverse bond (NHCO), a carba bond (CH₂CH₂) or a ketomethylene bond (CO-CH₂) also form part of the invention.

Preferably, the polypeptides according to the invention comprising one or more additions, deletions, substitutions of at least one amino acid will retain their capacity to be recognized by antibodies directed against the nonmodified polypeptides.

### ANTIBODIES

The ABC1 polypeptides according to the invention, in particular 1) a polypeptide comprising an amino acid sequence of any one of SEQ ID NOs: 71 and 89-102, 2) a polypeptide comprising amino acids 1-60 of SEQ ID NO: 71, 3) a polypeptide fragment or variant of a polypeptide comprising an amino acid sequence of any one of SEQ ID NOs: 71 and 89-102, wherein the polypeptide fragment or variant comprises amino acids 1-60 of SEQ ID NO: 71, or 4) a polypeptide termed "homologous" to a polypeptide comprising amino acids 1-60 of SEQ ID NO: 71, may be used for the preparation of an antibody, in particular for detecting the production of a normal or altered form of an ABC1 polypeptide in a patient.

An antibody directed against a polypeptide comprising amino acids 1-60 of SEQ ID NO: 71, also forms part of the present invention.

An antibody directed against a polypeptide termed "homologous" to a polypeptide having an amino acid sequence comprising amino acids 1-60 of SEQ ID NO: 71 also forms part of the invention. Such an antibody is directed against a homologous polypeptide comprising an amino acid sequence possessing one or more substitutions of an amino acid by an equivalent amino acid, relative to amino acids 1-60 of SEQ ID NO: 71.

"Antibody" for the purposes of the present invention will be understood to mean in particular polyclonal or monoclonal antibodies or fragments (for example the F(ab)'₂ and Fab fragments) or any polypeptide comprising a domain of the initial antibody recognizing the target polypeptide or polypeptide fragment according to the invention.

Monoclonal antibodies may be prepared from hybridomas according to the technique described by Kohler and Milstein (1975).

According to the invention, a polypeptide produced recombinantly or by chemical synthesis, and fragments or other derivatives or analogs thereof, including fusion proteins, may be used as an immunogen to generate antibodies that recognize a polypeptide according to the invention. Such antibodies include but are not limited to polyclonal, monoclonal, chimeric, single chain, Fab fragments, and an Fab expression library. The anti-ABC1 antibodies of the invention may be cross reactive, *e.g.,* they may recognize an ABC1 polypeptide from different species. Polyclonal antibodies have greater likelihood of cross reactivity. Alternatively, an antibody of the invention may be specific for a single form of ABC1. Preferably, such an antibody is specific for human ABC1.

Various procedures known in the art may be used for the production of polyclonal antibodies to an ABC1 polypeptide or derivative or analog thereof. For the production of antibody, various host animals can be immunized by injection with an ABC1 polypeptide, or a derivative (*e.g.,* fragment or fusion protein) thereof, including but not limited to rabbits, mice, rats, sheep, goats, etc. In one embodiment, the ABC1 polypeptide or fragment thereof can be conjugated to an immunogenic carrier, *e.g.,* bovine serum albumin (BSA) or keyhole limpet hemocyanin (KLH). Various adjuvants may be used to increase the immunological response, depending on the host species, including but not limited to Freund's (complete and incomplete), mineral gels such as aluminum hydroxide, surface active substances such as lysolecithin, pluronic polyols, polyanions, peptides, oil emulsions, keyhole limpet hemocyanins, dinitrophenol, and potentially useful human adjuvants such as BCG (*bacille Calmette-Guerin)* and *Corynebacterium parvum.*

For preparation of monoclonal antibodies directed toward the ABC1 polypeptide, or fragment, analog, or derivative thereof, any technique that provides for the production of antibody molecules by continuous cell lines in culture may be used. These include but are not limited to the hybridoma technique originally developed by Kohler and Milstein (1975), as well as the trioma technique, the human B-cell hybridoma technique (Kozbor et al., 1983a); Cote et al. (1983), and the EBV-hybridoma technique to produce human monoclonal antibodies [Cole et al., 1985). In an additional embodiment of the invention, monoclonal antibodies can be produced in germ-free animals [International Patent Publication No. WO 89/12690, published 28 December 1989]. In fact, according to the invention, techniques developed for the production of "chimeric antibodies" [Morrison et al., (1984); Neuberger et al., (1984); Takeda et al., (1985)] by splicing the genes from a mouse antibody molecule specific for an ABC1 polypeptide together with genes from a human antibody molecule of appropriate biological activity can be used; such antibodies are within the scope of this invention. Such human or humanized chimeric antibodies are preferred for use in therapy of human diseases or disorders (described *infra*), since the human or humanized antibodies are much less likely than xenogenic antibodies to induce an immune response, in particular an allergic response, themselves.

According to the invention, techniques described for the production of single chain antibodies [U.S. Patent Nos. 5,476,786 and 5,132,405 to Huston; U.S. Patent 4,946,778] can be adapted to produce ABC1 polypeptide-specific single chain antibodies. An additional embodiment of the invention utilizes the techniques described for the construction of Fab expression libraries (Huse et al., 1989) to allow rapid and easy identification of monoclonal Fab fragments with the desired specificity for an ABC1 polypeptide, or its derivatives, or analogs.

Antibody fragments which contain the idiotype of the antibody molecule can be generated by known techniques. For example, such fragments include but are not limited to: the F(ab')₂ fragment which can be produced by pepsin digestion of the antibody molecule; the Fab' fragments which can be generated by reducing the disulfide bridges of the F(ab')2 fragment, and the Fab fragments which can be generated by treating the antibody molecule with papain and a reducing agent.

In the production of antibodies, screening for the desired antibody can be accomplished by techniques known in the art, *e.g.,* radioimmunoassay, ELISA (enzyme-linked immunosorbant assay), "sandwich" immunoassays, immunoradiometric assays, gel diffusion precipitin reactions, immunodiffusion assays, *in situ* immunoassays (using colloidal gold, enzyme or radioisotope labels, for example), western blots, precipitation reactions, agglutination assays (*e.g.,* gel agglutination assays, hemagglutination assays), complement fixation assays, immunofluorescence assays, protein A assays, and immunoelectrophoresis assays, etc. In one embodiment, antibody binding is detected by detecting a label on the primary antibody. In another embodiment, the primary antibody is detected by detecting binding of a secondary antibody or reagent to the primary antibody. In a further embodiment, the secondary antibody is labeled. Many means are known in the art for detecting binding in an immunoassay and are within the scope of the present invention. For example, to select antibodies which recognize a specific epitope of an ABC1 polypeptide, one may assay generated hybridomas for a product which binds to an ABC1 polypeptide fragment containing such epitope. For selection of an antibody specific to an ABC1 polypeptide from a particular species of animal, one can select on the basis of positive binding with an ABC1 polypeptide expressed by or isolated from cells of that species of animal.

The foregoing antibodies can be used in methods known in the art relating to the localization and activity of an ABC1 polypeptide, *e.g.,* for Western blotting, ABC1 polypeptide *in situ,* measuring levels thereof in appropriate physiological samples, etc. using any of the detection techniques mentioned above or known in the art.

In a specific embodiment, antibodies that agonize or antagonize the activity of an ABC1 polypeptide can be generated. Such antibodies can be tested using the assays described *infra* for identifying ligands.

The present invention relates to an antibody directed against 1) a polypeptide comprising an amino acid sequence of any one of SEQ ID NOs: 71 and 89-102, 2) a polypeptide comprising amino acids 1-60 of SEQ ID NO: 71, 3) a polypeptide fragment or variant of a polypeptide comprising an amino acid sequence of any one of SEQ ID NOs: 71 and 89-102, wherein the polypeptide fragment or variant comprises amino acids 1-60 of SEQ ID NO: 71, or 4) a polypeptide termed "homologous" to a polypeptide comprising amino acids 1-60 of SEQ ID NO: 71, also forms part of the invention, as produced in the trioma technique or the hybridoma technique described by Kozbor et al. (1983b).

The invention also relates to single-chain Fv antibody fragments (ScFv) as described in US patent No. 4,946,778 or by Martineau et al. (1998).

The antibodies according to the invention also comprise antibody fragments obtained with the aid of phage libraries as described by Ridder et al., (1995) or humanized antibodies as described by Reinmann et al. (1997) and Leger et al., (1997).

The antibody preparations according to the invention are useful in immunological detection tests intended for the identification of the presence and/or of the quantity of antigens present in a sample.

An antibody according to the invention may comprise, in addition, a detectable marker which is isotopic or nonisotopic, for example fluorescent, or may be coupled to a molecule such as biotin, according to techniques well known to persons skilled in the art.

Thus, the subject of the invention is, in addition, a method of detecting the presence of a polypeptide according to the invention in a sample, said method comprising the steps of:
a) bringing the sample to be tested into contact with an antibody directed against 1) a polypeptide comprising an amino acid sequence of any one of SEQ ID NOs: 71 and 89-102, 2) a polypeptide comprising amino acids 1-60 of SEQ ID NO: 71, 3) a polypeptide fragment or variant of a polypeptide comprising an amino acid sequence of any one of SEQ ID NOs: 71 and 89-102, wherein the polypeptide fragment or variant comprises amino acids 1-60 of SEQ ID NO: 71, or 4) a polypeptide termed "homologous" to a polypeptide comprising amino acids 1-60 of SEQ ID NO: 71, and
b) detecting the antigen/antibody complex formed.

The invention also relates to a box or kit for diagnosis or for detecting the presence of a polypeptide in accordance with the invention in a sample, said box comprising:
a) an antibody directed against 1) a polypeptide comprising an amino acid sequence of any one of SEQ ID NOs: 71 and 89-102, 2) a polypeptide comprising amino acids 1-60 of SEQ ID NO: 71, 3) a polypeptide fragment or variant of a polypeptide comprising an amino acid sequence of any one of SEQ ID NOs: 71 and 89-102, wherein the polypeptide fragment or variant comprises amino acids 1-60 of SEQ ID NO: 71, or 4) a polypeptide termed "homologous" to a polypeptide comprising amino acids 1-60 of SEQ ID NO: 71, and
b) a reagent allowing the detection of the antigen/antibody complexes formed.

### PHARMACEUTICAL COMPOSITIONS AND THERAPEUTIC METHODS OF TREATMENT

The invention also relates to pharmaceutical compositions intended for the prevention or treatment of a deficiency in the metabolism of cholesterol such as atherosclerosis, particularly in the transport of cholesterol, and still more particularly in the reverse transport of cholesterol, characterized in that they comprise a therapeutically effective quantity of a polynucleotide capable of giving rise to the production of an effective quantity of the normal ABC1 polypeptide, in particular a polypeptide comprising an amino acid sequence of SEQ ID NO: 71. In a preferred embodiment, the ABC1 polypeptide comprises amino acids 1-60 of SEQ ID NO: 71.

The subject of the invention is, in addition, pharmaceutical compositions intended for the prevention or treatment of a deficiency in the metabolism of cholesterol such as atherosclerosis, particularly in the transport of cholesterol, and still more particularly in the reverse transport of cholesterol, characterized in that they comprise a therapeutically effective quantity of the normal ABC1 polypeptide, in particular a polypeptide comprising an amino acid sequence of SEQ ID NO: 71. In a preferred embodiment, the ABC1 polypeptide comprises amino acids 1-60 of SEQ ID NO: 71.

The invention also relates to the use of the ABC1 polypeptide having an amino acid sequence of SEQ ID NO: 71 or amino acids 1-60 of SEQ ID NO: 71 for the manufacture of a medicament intended for the prevention of atherosclerosis in various forms or more particularly for the treatment of subjects affected by a dysfunction in the reverse transport of cholesterol.

The invention relates to a pharmaceutical composition for the prevention or treatment of subjects affected by a dysfunction in the reverse transport of cholesterol, comprising a therapeutically effective quantity of the polypeptide having an amino acid sequence of SEQ ID NO: 71 or amino acids 1-60 of SEQ ID NO: 71.

According to yet another aspect, the subject of the invention is also a preventive or curative therapeutic method of treating diseases caused by a deficiency in the metabolism of cholesterol, more particularly in the transport of cholesterol and still more particularly in the reverse transport of cholesterol, such a method comprising a step in which there is administered to a patient a therapeutically effective quantity of the ABC1 polypeptide in said patient, said polypeptide being, where appropriate, combined with one or more physiologically compatible vehicles and/or excipients.

Preferably, a pharmaceutical composition comprising a polypeptide according to the invention will be administered to the patient. Thus, the invention also relates to pharmaceutical compositions intended for the prevention or treatment of a deficiency in the metabolism of cholesterol such as atherosclerosis, particularly in the transport of cholesterol, and still more particularly in the reverse transport of cholesterol, characterized in that they comprise a therapeutically effective quantity of a polynucleotide capable of giving rise to the production of an effective quantity of a normal ABC1 polypeptide, in particular of a polypeptide having an amino acid sequence of SEQ ID NO: 71 or amino acids 1-60 of SEQ ID NO: 71.

The subject of the invention is, in addition, pharmaceutical compositions intended for the prevention or treatment of a deficiency in the metabolism of cholesterol such as atherosclerosis, particularly in the transport of cholesterol, and still more particularly in the reverse transport of cholesterol, characterized in that they comprise a therapeutically effective quantity of a normal ABC1 polypeptide, in particular of a polypeptide having an amino acid sequence of SEQ ID NO: 71 or amino acids 1-60 of SEQ ID NO: 71.

Such pharmaceutical compositions will be preferably suitable for the administration, for example by the parenteral route, of a quantity of the ABC1 polypeptide ranging from 1 µg/kg/day to 10 mg/kg/day, preferably at least 0.01 mg/kg/day and more preferably between 0.01 and 1 mg/kg/day.

The invention also provides pharmaceutical compositions comprising a nucleic acid encoding an ABC1 polypeptide according to the invention and pharmaceutical compositions comprising an ABC1 polypeptide according to the invention intended for the treatment of diseases linked to a deficiency in the reverse transport of cholesterol, such as Tangier and/or FHD disease.

The present invention also relates to a new therapeutic approach for the treatment of pathologies linked to the transport of cholesterol, comprising transferring and expressing *in vivo* nucleic acids encoding an ABC1 protein according to the invention. Specifically, the present invention provides a new therapeutic approach for the treatment and/or prevention of HDL deficiency, such as the HDL deficiency associated with Tangier and/or FHD disease, HDL deficiency, LCAT deficiency, malaria, and diabetes.

Thus, the present invention offers a new approach for the treatment and prevention of cardiovascular and neurological pathologies linked to the abnormalities of the transport and metabolism of cholesterol. Specifically, the present invention provides methods to restore or promote improved reverse transport of cholesterol within a patient or subject.

Consequently, the invention also relates to a pharmaceutical composition intended for the prevention of or treatment of subjects affected by, a dysfunction in the reverse transport of cholesterol, comprising a nucleic acid encoding the ABC1 protein, in combination with one or more physiologically compatible vehicle and/or excipient.

According to a specific embodiment of the invention, a composition is provided for the *in vivo* production of the ABC1 protein. This composition comprises a nucleic acid encoding the ABC1 polypeptide placed under the control of appropriate regulatory sequences, in solution in a physiologically acceptable vehicle and/or excipient.

Therefore, the present invention also relates to a composition comprising a nucleic acid encoding a polypeptide comprising an amino acid sequence of SEQ ID NO: 71, wherein the nucleic acid is placed under the control of appropriate regulatory elements.

The present invention also relates to a composition comprising a nucleic acid encoding a polypeptide comprising amino acids 1-60 of SEQ ID NO: 71, wherein the nucleic acid is placed under the control of appropriate regulatory elements.

Preferably, such a composition will comprise a nucleic acid comprising a polynucleotide sequence of either SEQ ID NO: 69 or SEQ ID NO: 70, placed under the control of appropriate regulatory elements.

According to another aspect, the subject of the invention is also a preventive or curative therapeutic method of treating diseases caused by a deficiency in the metabolism of cholesterol, more particularly in the transport of cholesterol and still more particularly in the reverse transport of cholesterol, such a method comprising a step in which there is administered to a patient a nucleic acid encoding an ABC1 polypeptide according to the invention in said patient, said nucleic acid being, where appropriate, combined with one or more physiologically compatible vehicles and/or excipients.

The invention also relates to a pharmaceutical composition intended for the prevention of or treatment of subjects affected by, a dysfunction in the reverse transport of cholesterol, comprising a recombinant vector according to the invention, in combination with one or more physiologically compatible excipients.

According to a specific embodiment, a method of introducing a nucleic acid according to the invention into a host cell, in particular a host cell obtained from a mammal, *in vivo,* comprises a step during which a preparation comprising a pharmaceutically compatible vector and a "naked" nucleic acid according to the invention, placed under the control of appropriate regulatory sequences, is introduced by local injection at the level of the chosen tissue, for example a smooth muscle tissue, the "naked" nucleic acid being absorbed by the cells of this tissue.

The invention also relates to the use of a nucleic acid according to the invention, encoding the ABC1 protein, for the manufacture of a medicament intended for the prevention or treatment of atherosclerosis in various forms or more particularly for the treatment of subjects affected by a dysfunction in the reverse transport of cholesterol.

The invention also relates to the use of a recombinant vector according to the invention, comprising a nucleic acid encoding the ABC1 protein, for the manufacture of a medicament intended for the prevention of atherosclerosis in various forms or more particularly for the treatment of subjects affected by a dysfunction in the reverse transport of cholesterol.

As indicated above, the present invention also relates to the use of a defective recombinant virus according to the invention for the preparation of a pharmaceutical composition for the treatment and/or prevention of pathologies linked to the transport of cholesterol.

The invention relates to the use of such a defective recombinant virus for the preparation of a pharmaceutical composition intended for the treatment and/or for the prevention of cardiovascular disease linked to a deficiency in the reverse transport of cholesterol. Thus, the present invention also relates to a pharmaceutical composition comprising one or more defective recombinant viruses according to the invention.

The present invention also relates to the use of cells genetically modified *ex vivo* with a virus according to the invention, or of producing cells such as viruses, implanted in the body, allowing a prolonged and effective expression in vivo of a biologically active ABC1 protein.

The present invention shows that it is possible to incorporate a nucleic acid encoding an ABC1 polypeptide into a viral vector, and that these vectors make it possible to effectively express a biologically active, mature form. More particularly, the invention shows that the in vivo expression of ABC1 may be obtained by direct administration of an adenovirus or by implantation of a producing cell or of a cell genetically modified by an adenovirus or by a retrovirus incorporating such a DNA.

Preferably, the pharmaceutical compositions of the invention comprise a pharmaceutically acceptable vehicle or physiologically compatible excipient for an injectable formulation, in particular for an intravenous injection, such as for example into the patient's portal vein. These may relate in particular to isotonic sterile solutions or dry, in particular, freeze-dried, compositions which, upon addition depending on the case of sterilized water or physiological saline, allow the preparation of injectable solutions. Direct injection into the patient's portal vein is preferred because it makes it possible to target the infection at the level of the liver and thus to concentrate the therapeutic effect at the level of this organ.

A "pharmaceutically acceptable vehicle or excipient " includes diluents and fillers which are pharmaceutically acceptable for method of administration, are sterile, and may be aqueous or oleaginous suspensions formulated using suitable dispersing or wetting agents and suspending agents. The particular pharmaceutically acceptable carrier and the ratio of active compound to carrier are determined by the solubility and chemical properties of the composition, the particular mode of administration, and standard pharmaceutical practice.

Any nucleic acid, polypeptide, vector, or host cell of the invention will preferably be introduced *in vivo* in a pharmaceutically acceptable vehicle or excipient. The phrase "pharmaceutically acceptable" refers to molecular entities and compositions that are physiologically tolerable and do not typically produce an allergic or similar untoward reaction, such as gastric upset, dizziness and the like, when administered to a human. Preferably, as used herein, the term "pharmaceutically acceptable" means approved by a regulatory agency of the Federal or a state government or listed in the U.S. Pharmacopeia or other generally recognized pharmacopeia for use in animals, and more particularly in humans. The term "excipient" refers to a diluent, adjuvant, excipient, or vehicle with which the compound is administered. Such pharmaceutical carriers can be sterile liquids, such as water and oils, including those of petroleum, animal, vegetable or synthetic origin, such as peanut oil, soybean oil, mineral oil, sesame oil and the like. Water or aqueous solution saline solutions and aqueous dextrose and glycerol solutions are preferably employed as excipients, particularly for injectable solutions. Suitable pharmaceutical excipients are described in "Remington's Pharmaceutical Sciences" by E.W. Martin.

The pharmaceutical compositions according to the invention may be equally well administered by the oral, rectal, parenteral, intravenous, subcutaneous or intradermal route.

The invention also relates to the use of the ABC1 polypeptide having an amino acid sequence of SEQ ID NO: 71 or amino acids 1-60 of SEQ ID NO: 71 for the manufacture of a medicament intended for the prevention of atherosclerosis in various forms or more particularly for the treatment of a patient or subject affected by a dysfunction in the reverse transport of cholesterol.

The invention finally relates to a pharmaceutical composition for the prevention or treatment of a patient or subject affected by a dysfunction in the reverse transport of cholesterol, comprising a therapeutically effective quantity of a polypeptide having an amino acid sequence of SEQ ID NO: 71 or a polypeptide comprising amino acids 1-60 of SEQ ID NO: 71, combined with one or more physiologically compatible vehicles and/or excipients.

According to another aspect, the subject of the invention is also a preventive or curative therapeutic method of treating diseases caused by a deficiency in the metabolism of cholesterol, more particularly in the transport of cholesterol and still more particularly in the reverse transport of cholesterol, such a method comprising a step in which there is administered to a patient or subject a nucleic acid encoding an ABC1 polypeptide in said patient, said nucleic acid being, where appropriate, combined with one or more physiologically compatible vehicles and/or excipients.

According to yet another aspect, the subject of the invention is also a preventive or curative therapeutic method of treating diseases caused by a deficiency in the metabolism of cholesterol, more particularly in the transport of cholesterol and still more particularly in the reverse transport of cholesterol, such a method comprising a step in which there is administered to a patient or subject a therapeutically effective quantity of an ABC1 polypeptide according to the invention in said patient or subject, said polypeptide being, where appropriate, combined with one or more physiologically compatible vehicles and/or excipients.

In another embodiment, the nucleic acids, polypeptides, recombinant vectors, and compositions according to the invention can be delivered in a vesicle, in particular a liposome (see Langer, 1990; Treat et al., 1989; and Lopez-Berestein, 1989).

In yet another embodiment, the nucleic acids, polypeptides, recombinant vectors, recombinant cells, and compositions according to the invention can be delivered in a controlled release system. For example, the polypeptide may be administered using intravenous infusion, an implantable osmotic pump, a transdermal patch, liposomes, or other modes of administration. In one embodiment, a pump may be used (see Langer, 1990; Sefton, 1987; Buchwald et al., 1980; Saudek et al., 1989). In another embodiment, polymeric materials can be used (Langer and Wise, 1974; Smolen and Ball, 1984; Ranger and Peppas, 1983; Levy et al., 1985; During et al., 1989; and Howard et al., 1989). In yet another embodiment, a controlled release system can be placed in proximity of the target tissue or organ, *i.e.,* the cardiovascular system, thus requiring only a fraction of the systemic dose (see Goodson, 1984). Other controlled release systems are discussed in the review by Langer (1990).

In a further aspect, recombinant cells that have been transformed with a nucleic acid according to the invention and that express high levels of an ABC1 polypeptide according to the invention can be transplanted in a subject in need of ABC1 polypeptide. Preferably autologous cells transformed with an ABC1 encoding nucleic acid according to the invention are transplanted to avoid rejection; alternatively, technology is available to shield non-autologous cells that produce soluble factors within a polymer matrix that prevents immune recognition and rejection.

Thus, the ABC1 polypeptide can be delivered by intravenous, intraarterial, intraperitoneal, intramuscular, or subcutaneous routes of administration. Alternatively, the ABC1 polypeptide, properly formulated, can be administered by nasal or oral administration. A constant supply of ABC1 can be ensured by providing a therapeutically effective dose (*i.e.,* a dose effective to induce metabolic changes in a subject) at the necessary intervals, *e.g.,* daily, every 12 hours, etc. These parameters will depend on the severity of the disease condition being treated, other actions, such as diet modification, that are implemented, the weight, age, and sex of the subject, and other criteria, which can be readily determined according to standard good medical practice by those of skill in the art.

A subject in whom administration of the nucleic acids, polypeptides, recombinant vectors, recombinant host cells, and compositions according to the invention is performed is preferably a human, but can be any animal. Thus, as can be readily appreciated by one of ordinary skill in the art, the methods and pharmaceutical compositions of the present invention are particularly suited to administration to any animal, particularly a mammal, and including, but by no means limited to, domestic animals, such as feline or canine subjects, farm animals, such as but not limited to bovine, equine, caprine, ovine, and porcine subjects, wild animals (whether in the wild or in a zoological garden), research animals, such as mice, rats, rabbits, goats, sheep, pigs, dogs, cats, etc., avian species, such as chickens, turkeys, songbirds, etc., *i.e.,* for veterinary medical use.

Preferably, a pharmaceutical composition comprising a nucleic acid, a recombinant vector, a polypeptide, or a recombinant host cell, as defined above, will be administered to the patient or subject.

### METHODS OF SCREENING AN AGONIST OR ANTAGONIST COMPOUND FOR THE ABC1 POLYPEPTIDE

According to another aspect, the invention also relates to various methods of screening compounds or small molecules for therapeutic use which are useful in the treatment of diseases due to a deficiency in the metabolism of cholesterol, particularly in the transport of cholesterol, still more particularly in the reverse transport of cholesterol, such as Tangier disease, or more generally FHD-type conditions.

The invention therefore also relates to the use of an ABC1 polypeptide, or of cells expressing an ABC1 polypeptide, for screening active ingredients for the prevention or treatment of diseases resulting from a dysfunction in the reverse transport of cholesterol. The catalytic sites and oligopeptide or immunogenic fragments of an ABC1 polypeptide can serve for screening product libraries by a whole range of existing techniques. The polypeptide fragment used in this type of screening may be free in solution, bound to a solid support, at the cell surface or in the cell. The formation of the binding complexes between the ABC1 polypeptide fragments and the tested agent can then be measured.

Another product screening technique which may be used in high-flux screenings giving access to products having affinity for the protein of interest is described in application WO84/03564. In this method, applied to an ABC1 protein, various products are synthesized on a solid surface. These products react with the ABC1 protein or fragments thereof and the complex is washed. The products binding the ABC1 protein are then detected by methods known to persons skilled in the art. Non-neutralizing antibodies can also be used to capture a peptide and immobilize it on a support.

Another possibility is to perform a product screening method using an ABC1 neutralizing antibody competition, an ABC1 protein and a product potentially binding the ABC1 protein. In this manner, the antibodies may be used to detect the presence of a peptide having a common antigenic unit with an ABC1 polypeptide or protein.

Of the products to be evaluated for their ability to increase ABC1 activity, there may be mentioned in particular kinase-specific ATP homologs involved in the activation of the molecules, as well as phosphatases, which may be able to avoid the dephosphorylation resulting from said kinases. There may be mentioned in particular inhibitors of the phosphodiesterase (PDE) theophylline and 3-isobutyl-1-methylxanthine type or the adenylcyclase forskolin activators.

Accordingly, this invention relates to the use of any method of screening products, *i.e.,* compounds, small molecules, and the like, based on the method of translocation of cholesterol (see Example 13) between the membranes or vesicles, this being in all synthetic or cellular types, that is to say of mammals, insects, bacteria, or yeasts expressing constitutively or having incorporated a human ABC1 encoding nucleic acid. To this effect, labeled lipid analogs may be used.

Likewise, it has been described that the ABC1 protein allowed anion transport (Becq et al., 1997 and Yamon et al., 1997) and this transport was activated by phosphatase inhibitors such as okadaic acid and orthovanadate as well as part of the elevation of cAMP by agents such as forskolin. The present invention also relates to the use of such a system for screening molecules modulating the activity of the ABC1 protein (see Example 14).

Yamon et al (1997) have demonstrated that the mouse ABC1 protein was involved in the secretion of a proinflammatory cytokine IL-lbeta in mouse peritoneal macrophages. It is therefore also possible to provide a method of screening products modulating the activity of the ABC1 protein by determining the release of IL-lbeta from any cell type expressing two proteins (see Example 15).

Furthermore, knowing that the disruption of numerous transporters have been described (van den Hazel et al., 1999), it is possible to think of using cellular mutants having a characteristic phenotype and to complement the function thereof with ABC1 and to use the whole for screening purposes.

The invention also relates to a method of screening a compound or small molecule active on the metabolism of cholesterol, an agonist or antagonist of an ABC1 polypeptide, said method comprising the following steps:
a) preparing a membrane vesicle comprising an ABC1 polypeptide and a lipid substrate comprising a detectable marker;
b) incubating the vesicle obtained in step a) with an agonist or antagonist candidate compound;
c) qualitatively and/or quantitatively measuring release of the lipid substrate comprising a detectable marker; and
d) comparing the release measurement obtained in step b) with a measurement of release of labeled lipid substrate by a vesicle that has not been previously incubated with the agonist or antagonist candidate compound.

In a first specific embodiment, the ABC1 polypeptide comprises an amino acid sequence of SEQ ID NO: 71.

In a second specific embodiment, the ABC1 polypeptide comprises amino acids 1-60 of SEQ ID NO: 71.

According to a first aspect of the above screening method, the membrane vesicle is a synthetic lipid vesicle, which may be prepared according to techniques well known to a person skilled in the art. According to this particular aspect, the ABC1 protein may be a recombinant ABC1 protein.

According to a second aspect, the membrane vesicle is a vesicle of a plasma membrane derived from cells expressing an ABC1 polypeptide. These may be cells naturally expressing an ABC1 polypeptide or cells transfected with a nucleic acid encoding an ABC1 polypeptide or recombinant vector comprising a nucleic acid encoding an ABC1 polypeptide.

According to a third aspect of the above screening method, the lipid substrate is chosen from cholesterol or phosphatidylcholine.

According to a fourth aspect, the lipid substrate is radioactively labeled, for example with an isotope chosen from ³H or ¹²⁵I.

According to a fifth aspect, the lipid substrate is labeled with a fluorescent compound, such as NBD or pyrene.

According to a sixth aspect, the membrane vesicle comprising the labeled lipid substrate and the ABC1 polypeptide is immobilized at the surface of a solid support prior to step b).

According to a seventh aspect, the measurement of the fluorescence or of the radioactivity released by the vesicle is the direct reflection of the activity of lipid substrate transport by the ABC1 polypeptide.

The invention also relates to a method of screening a compound or small molecule active on the metabolism of cholesterol, an agonist or antagonist of an ABC1 polypeptide, said method comprising the following steps:
a) obtaining cells, for example a cell line, that, either naturally or after transfecting the cell with an ABC1 encoding nucleic acid, expresses an ABC1 polypeptide;
b) incubating the cells of step a) in the presence of an anion labeled with a detectable marker;
c) washing the cells of step b) in order to remove the excess of the labeled anion which has not penetrated into these cells;
d) incubating the cells obtained in step c) with an agonist or antagonist candidate compound for the ABC1 polypeptide;
e) measuring efflux of the labeled anion; and
t) comparing the value of efflux of the labeled anion determined in step e) with a value of the efflux of a labeled anion measured with cells that have not been previously incubated in the presence of the agonist or antagonist candidate compound for the ABC1 polypeptide.

In a first specific embodiment, the ABC1 polypeptide comprises an amino acid sequence of SEQ ID NO: 71.

In a second specific embodiment, the ABC1 polypeptide comprises amino acids 1-60 of SEQ ID NO: 71.

According to a first aspect of the above screening method, the cells used are cells naturally expressing an ABC1 polypeptide. They may be human monocytes in primary culture, purified from a population of human blood mononuclear cells. They may also be human monocytic cell lines, such as the monocytic leukemia line THP1.

According to a second aspect, the cells used in the screening method described above may be cells not naturally expressing, or alternatively expressing at a low level, an ABC1 polypeptide, said cells being transfected with a recombinant vector according to the invention capable of directing the expression of a nucleic acid encoding an ABC1 polypeptide.

According to a third aspect, the cells may be cells having a natural deficiency in anion transport, or cells pretreated with one or more anion channel inhibitors such as Verapamil™ or tetraethylammonium.

According to a fourth aspect of said screening method, the anion is a radioactively labeled iodide, such as the salts K¹²⁵I or Na¹²⁵I.

According to a fifth aspect, the measurement of efflux of the labeled anion is determined periodically over time during the experiment, thus making it possible to also establish a kinetic measurement of this efflux.

According to a sixth aspect, the value of efflux of the labeled anion is determined by measuring the quantity of labeled anion present at a given time in the cell culture supernatant.

According to a seventh aspect, the value of efflux of the labeled anion is determined as the proportion of radioactivity found in the cell culture supernatant relative to the total radioactivity corresponding to the sum of the radioactivity found in the cell lysate and the radioactivity found in the cell culture supernatant.

The subject of the invention is also a method of screening a compound or small molecule active on the metabolism of cholesterol, an agonist or antagonist of an ABC1 polypeptide, said method comprising the following steps:
a) culturing cells of a human monocytic line in an appropriate culture medium, in the presence of purified human albumin;
b) incubating the cells of step a) simultaneously in the presence of a compound stimulating the production of IL-1 beta and of an agonist or antagonist candidate compound;
c) incubating the cells obtained in step b) in the presence of an appropriate concentration of ATP;
d) measuring IL-1 beta released into the cell culture supernatant; and
e) comparing the value of the release of the IL-1 beta obtained in step d) with the value of the IL-1 beta released into the culture supernatant of cells which have not been previously incubated in the presence of the agonist or antagonist candidate compound.

According to a first aspect of the screening method described above, the cells used belong to the human leukemic monocytic line THP1.

According to a second aspect of the screening method, the compound stimulating the production of IL-1 beta is a lipopolysaccharide.

According to a third aspect of said method, the production of IL-1 alpha, IL-6 and TNF alpha by these cells is also qualitatively and/or quantitatively determined.

According to a fourth aspect, the level of expression of the messenger RNA encoding IL-1 beta is also determined.

The following examples are intended to further illustrate the present invention but do not limit the invention.

### EXAMPLES

### EXAMPLE 1: Tissue distribution of the transcripts of the ABC1 gene according to the invention.

The profile of expression of the polynucleotides according to the present invention is determined according to the protocols for PCR-coupled reverse transcription and Northern blot analysis described in particular by Sambrook et al. (1989).

For example, in the case of an analysis by reverse transcription, a pair of primers as described above may be synthesized from a cDNA of the human ABC1 gene comprising a polynucleotide sequence as depicted in SEQ ID NOs: 69 and 70. This primer pair may be used to detect the corresponding ABC1 cDNA. Specifically, two oligonucleotide primers specific for ABC1 and comprising either 1) a sequence contained within nucleotide region 1-184 of SEQ ID NO: 69 and a complementary sequence contained within nucleotide region 6968-9741 of SEQ ID NO: 69, or 2) a sequence contained within nucleotide region 1-297 of SEQ ID NO: 70 and a complementary sequence contained within nucleotide region 7081-9854 of SEQ ID NO: 70, may be used to isolate the ABC1 cDNA.

The polymerase chain reaction (PCR) is carried out on cDNA templates corresponding to retrotranscribed polyA⁺ mRNAs (Clontech). The reverse transcription to cDNA is carried out with the enzyme SUPERSCRIPT II (GibcoBRL, Life Technologies) according to the conditions described by the manufacturer. The polymerase chain reaction is carried out according to standard conditions, in 20 µl of reaction mixture with 25 ng of cDNA preparation. The reaction mixture is composed of 400 µM of each of the dNTPs, 2 units of Thermus aquaticus (Taq) DNA polymerase (Ampli Taq Gold; Perkin Elmer), 0.5 µM of each primer, 2.5 mM MgCl2, and PCR buffer. Thirty four PCR cycles [denaturing 30 seconds at 94°C, annealing of 30 seconds divided up as follows during the 34 cycles: 64°C (2 cycles), 61°C (2 cycles), 58°C (2 cycles), and 55°C (28 cycles), and an extension of one minute per kilobase at 72°C] are carried out after a first step of denaturing at 94°C for 10 minutes using a Perkin Elmer 9700 thermocycler. The PCR reactions are visualized on agarose gel by electrophoresis. The cDNA fragments obtained may be used as probes for a Northern blot analysis and may also be used for the exact determination of the polynucleotide sequence.

In the case of a Northern Blot analysis, a cDNA probe produced as described above is labeled with ³²P by means of the DNA labeling system High Prime (Boehringer) according to the instructions indicated by the manufacturer. After labeling, the probe is purified on a Sephadex G50 microcolumn (Pharmacia) according to the instructions indicated by the manufacturer. The labeled and purified probe is then used for the detection of the expression of the mRNAs in various tissues.

The Northern blot containing samples of RNA of different human tissues (Multiple Tissue Northern, MTN, Clontech) Blot 2, reference 77759-1) is hybridized with the labeled probe. The protocol followed for the hybridizations and washes may be either directly as that described by the manufacturer (Instruction manual PT1200-1) or an adaptation of this protocol using methods known to persons skilled in the art and described for example in F. Ausubel et al (1999). It is thus possible to vary, for example, the prehybridization and hybridization temperatures in the presence of formamide.

For example, it may be possible to use the following protocol:
1- Membrane competition and PREHYBRIDIZATION:
   - Mix: 40 µl salmon sperm DNA (10 mg/ml) + 40 µl human placental DNA (10 mg/ml)
   - Denature for 5 minutes at 96°C, then immerse the mixture in ice.
   - Remove the 2X SSC and pour 4 ml of formamide mix in the hybridization tube containing the membranes.
   - Add the mixture of the two denatured DNAs.
   - Incubation at 42°C for 5 to 6 hours, with rotation.
2- Labeled probe competition:
   - Add to the labeled and purified probe 10 to 50 µl Cot I DNA, depending on the quantity of repeat sequences.
   - Denature for 7 to 10 minutes at 95°C.
   - Incubate at 65°C for 2 to 5 hours.
3- HYBRIDIZATION:
   - Remove the prehybridization mix.
   - Mix 40 µl salmon sperm DNA + 40 µl human placental DNA; denature for 5 min at 96°C, then immerse in ice.
   - Add to the hybridization tube 4 ml of formamide mix, the mixture of the two DNAs and the denatured labeled probe/Cot I DNA.
   - Incubate 15 to 20 hours at 42°C, with rotation.
4- Washes:
   - One wash at room temperature in 2X SSC, to rinse.
   - Twice 5 minutes at room temperature 2X SSC and 0.1% SDS at 65°C.
   - Twice 15 minutes at 65°C IX SSC and 0.1% SDS at 65°C.

After hybridization and washing, the blot is analyzed after overnight exposure in contact with a phosphorus screen revealed with the aid of Storm (Molecular Dynamics, Sunnyvale, CA).

### EXAMPLE 2 : 5' Extension of the human ABC1 cDNA.

This Example describes the isolation and identification of cDNA molecules encoding the full length human ABC1 protein. 5' extension of the partial ABC1 cDNA sequence (GenBank Accession # AJ012376, Langmann et al., 1999) was performed using a modified 5' RACE approach.

### Experimental design:

A human monocytic THP1 cDNA library (in the Lambda phage vector lZip-lox, Life Tech®) which had previously been made from THP1 cells that were differentiated with phorbol ester and then loaded with acetylated LDL to convert them to the foam cell phenotype was used in this Example. It has been shown that THP1 cells, when loaded with cholesterol, strongly up-regulate the expression of the human ABC1 gene (Langmann et al., 1999). This THP1 cDNA library was used to 5' extend the human ABC1 cDNA, since it should be enriched for ABC1 cDNA.

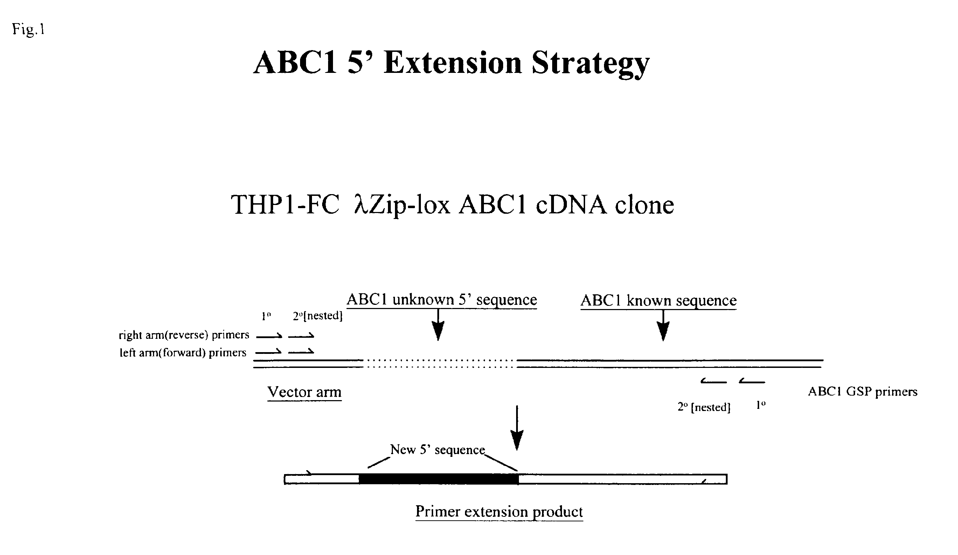

A set of nested PCR primers (SEQ ID NOs: 155 and 156) which hybridize to nucleotides of the 5' published sequence region of human ABC1 cDNA (Langmann et al., 1999) was used to elongate the ABC cDNA in the 5' direction (see Table VII). Two sets of nested vector arm specific primers (SEQ ID NOs: 2, 140, 153, and 154) were designed for the reverse synthesis in the direction towards the human ABC1 cDNA specific primer (see Table VII and Figure 1). Within Table VII, the term "Comp" refers to the complementary nucleotide sequence of the nucleotide positions indicated. In this manner, a PCR product corresponding to the true 5' end of the human ABC1 cDNA can be obtained, regardless of its orientation within the vector.

**TABLE VII**

| **5' ABC1 cDNA EXTENSION PRIMERS** | | | | |
|---|---|---|---|---|
| PCR Primer | PCR Primer SEQ ID NO: | Primary, Secondary, or Sequencing Reaction Use | Position in SEQ ID NO: 69 | Orientation Relative to Human ABC1 cDNA |
| ABC1-3' | 155 | Primary PCR | Comp nt 803-824 | antisense |
| ABC 1-9 | 156 | Secondary PCR | Comp nt 703-727 | antisense |
| ABC 1-7 | 139 | Sequencing | Comp nt 448-468 | antisense |
| λZip-forward | 140 | Primary PCR | ---- | sense |
| λZip-reverse | 153 | Primary PCR | ---- | sense |
| λZip-seq-1 | 154 | Secondary PCR | ---- | sense |
| λZip-seq-2 | 2 | Secondary PCR | ---- | sense |

### Materials and Methods:

Primary PCR. An aliquot of the THP1 cDNA library (cleared bacterial lysate, containing approximately 5 x10⁹ pfu/ml of λZip-lox-THP1-FC library phage, 2.5x10⁶ recombinants, with an average insert size of 1.5kb) was heated to 95°C for 10 minutes to lyse the phage and release its DNA. Four ul of the lysed phage was then used in a 50ul PCR reaction containing 20mM Tris pH8.4, 50mM KCI, 1.5mM MgCl₂, 0.2mM dNTPs, 2.5units of Platinum® Taq ploymerase-HF (BRL), and 0.2uM of each primer [ABCl-3' (SEQ ID NO: 155), and either λZip-forward (SEQ ID NO: 140) or λZip-reverse (SEQ ID NO: 153)]. Amplification was performed for 35 cycles of 94°C for 10 seconds, 58°C for 20 seconds, and 72°C for 2.5 minutes. Five ul of the PCR reaction were electrophoresed on a 0.8% agarose /TBE gel containing .02 ug/ml of ethidium bromide. The gel was photographed and examined for evidence of product.

Secondary PCR. From selected primary PCR reactions, lul of the reaction was removed and diluted into 50ul of 10mM Tris pH 8.5. One ul of the diluted primary PCR reaction was used in a secondary PCR reaction using a nested ABC1 primer (ABC1-9, SEQ ID NO: 156) and the appropriate nested arm primer ®Zip-seq-1 (SEQ ID NO: 154) or λZip-seq-2 (SEQ ID NO: 2). The PCR reaction was amplified as above except the cycling parameters were adjusted as follows: 25 cycles at 94°C for 20 seconds, 56°C for 20 seconds, and 72°C for 1.5 minutes. Five ul of the secondary PCR reaction were analyzed on an agarose gel as described above.

Gel Purification of PCR product. The secondary PCR reactions which appeared to have a product of the appropriate size were then analyzed on an agarose gel and the bands corresponding to the ABC1 cDNA 5' extension product were excised and purified using the Qiagen Qiaquick™ gel extraction purification system. The purified DNA fragments were eluted in a volume as 30ul of 10mM Tris pH 8.5. Five ul of the eluted PCR products were analyzed on an agarose gel to determine the approximate yields.

Sequencing. Five ul of each of the purified PCR products along with 20ng of ABC1-7 sequencing primer (SEQ ID NO: 139) using BigDye™ terminator cycle sequencing on an ABI 377 sequencing apparatus. The purified 5' extended human ABC1 cDNA PCR products' sequence results were analyzed using the Lasergene DNA Star software package and the BLAST program at the NCBI web site (http://www.ncbi.nlm.nih.gov).

### Results and Discussion:

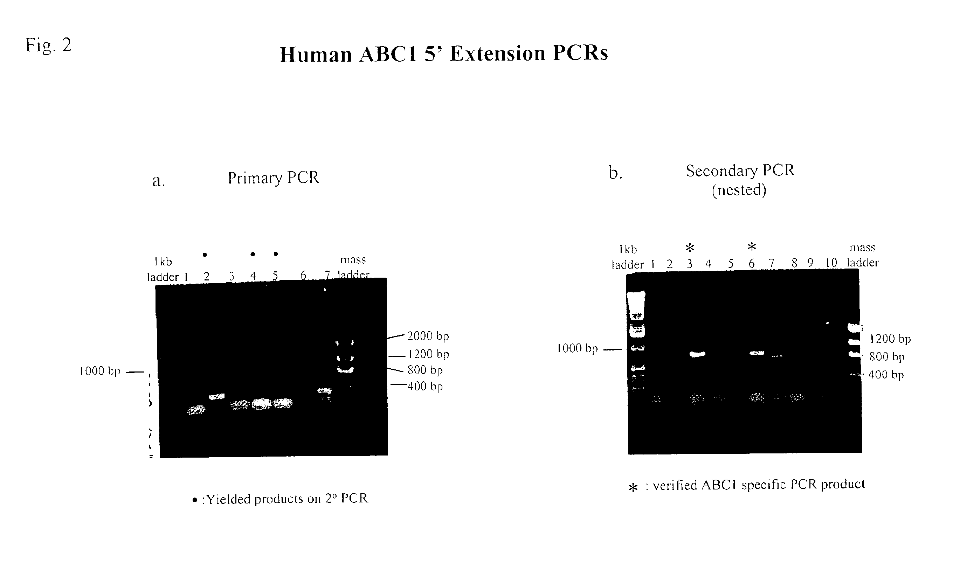

The primary PCR reactions yielded only faint bands, other than the primers staining (see Figure 2-a). Therefore, a secondary nested PCR reaction was performed. The secondary PCR reactions (Figure 2-b) produced an intensely stained, single band of approximately 800bp using primers ABC1-9 (SEQ ID NO: 156) and λZip-seq-2 (SEQ ID NO: 2) in two of the reactions (see lanes 3 and 6, Figure 2-b), while there was a fainter band of approximately 750bp in lane 7 using primers ABC1-9 (SEQ ID NO: 156) and λZip-seq-1 (SEQ ID NO: 154).

To verify that the PCR products were authentic and specific for human ABC1 cDNA, an additional nested PCR reaction was performed using the diluted primary PCR reactions that had yielded the bands obtained in the secondary PCR reactions (lanes 3, 6, and 7 of Figure 2-b) using primers ABC1-7 (SEQ ID NO: 139) and λZip-seq-2 (SEQ ID NO: 154) or λZip-seq-1 (SEQ ID NO: 2), respectively. This additional nested PCR reaction should yield a product that is 254bp smaller than the one obtained in the secondary PCR reactions. This was indeed the case for the samples corresponding to lanes 3 and 6. However, the sample of lane 7 yielded no such product and therefore, was not likely to be ABC1 cDNA specific and was not analyzed further.

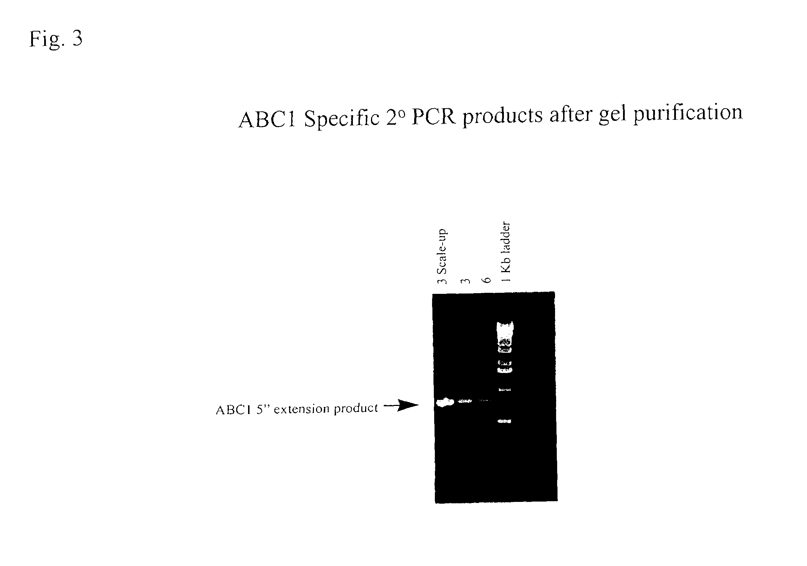

An additional 100ul PCR reaction was performed to generate more of the product obtained in the secondary PCR reaction corresponding to the sample in lane 3. The secondary PCR reactions consisting of the first sample 3, the 100ul scale-up of sample 3, and sample 6 were gel purified in preparation for cloning and sequencing (see Figure 3). Following purification, 5ul of the three gel purified samples were sequenced using the ABC 1-7 oligonucleotide (SEQ ID NO: 139) as a primer. In addition, the purified products were each cloned into the plasmid, PCR2.1 (Invitrogen).

The sequencing results revealed that a nucleic acid comprising an additional 244 nucleotides (SEQ ID NO: 3) 5' of the published partial human ABC1 cDNA had been obtained. An open reading frame search was performed on this nucleic acid and a new ORF was identified that was contiguous with the previously published open reading frame. This new open reading frame extended the human ABC1 protein by an additional 60 amino acid residues (amino acids 1-60 of SEQ ID NO: 71) as compared to the sequence of Langmann et al. (1999). The newly identified initiator ATG codon (nucleotides 185-187 of SEQ ID NO: 3) conformed well with the Kozak consensus sequence for eukaryotic translation initiation and significantly, there was an in-frame TGA terminator nine bases upstream (nucleotides 176-178 of SEQ ID NO: 3) of the new ATG initiation codon. Thus, the invention provides nucleic acids encoding the full length human ABC1 protein (SEQ ID NO: 71). In particular, the present invention provides nucleic acids encoding a polypeptide comprising amino acids 1-60 of SEQ ID NO: 71.

In addition, two, third position nucleotide differences were also identified compared to the published sequence at new positions 260 and 262 of SEQ ID NO: 3 [corresponding to nucleotide positions 16 and 18 of Langmann et al. (1999)]. These nucleotide differences are conservative changes: 260:T→C and 262:A→G; and do not lead to any codon changes. SEQ ID NOs: 69 and 70 comprise the new 5' extended ABC1 cDNA region (SEQ ID NO: 3) obtained within this Example. Specifically, nucleotides 1-244 of SEQ ID NO: 3 correspond to nucleotides 1-244 of SEQ ID NO: 69 and nucleotides 114-357 of SEQ ID NO: 70, respectively.

Various other approaches may be used to isolate the cDNA corresponding to the complete cDNA of ABC 1. For example, a complete clone may be directly isolated by hybridization by screening a cDNA library by means of a polynucleotide probe specific for the sequence of the gene of interest. In particular, a specific probe of 30-40 nucleotides is synthesized using a synthesizer of the Applied Biosystem/Perkin Elmer trademark depending on the chosen sequence. The oligonucleotide obtained is radiolabeled, for example with [γ-³²P]ATP using T4 polynucleotide kinase and is purified according to the customary methods (e.g. Maniatis et al., 1982 or F. Ausubel et al., 1989).

The clone library containing the cDNA that is desired to screen is established on a culture medium in a Petri dish (1.5% agar) containing the appropriate antibiotics according to the customary methods cited above (F. Ausubel et al.). The colonies thus produced after incubation are transferred on nitrocellulose filters and screened by means of the radiolabeled nucleotide probe, according to the customary methods and the colonies hybridizing with the probe are isolated and subcloned.

The DNA of the clones thus identified is prepared and analyzed by sequencing. The clones containing the fragments corresponding to the complete cDNA are purified and recloned into the vector pcDNA3 according to the protocols known to persons skilled in the art and presented for example in F. Ausubel et al (1989).

Various methods are known for identifying the 5' and 3' ends of the cDNA corresponding to the genes described in the present application. These methods include but are not limited to hybridization cloning, to cloning using protocols similar or identical to 3' or 5' RACE-PCR (Rapid Amplification of cDNA End-PCR) which are well known to persons skilled in the art.

For example, it will be possible to use the kit marketed by the company Clontech (Marathon Ready™ cDNA kit, protocol identified by the reference PT1156-1) or alternatively a method similar to 5'RACE is available for characterizing any absent 5' end of a cDNA (Fromont-Racine et al., 1993). Briefly, an RNA oligonucleotide is ligated to the 5' end of an mRNA population. After retrotranscription to cDNA, a set of primers specific respectively for the adaptor ligated in 5' and for a sequence situated in 3' of the gene of interest is used in PCR to amplify the 5' portion of the desired cDNA. The amplified fragment is then used to reconstruct the complete cDNA.

### EXAMPLE 3 : Analysis of the gene expression profile for Tangier disease

The verification of the impairment of the level of expression of the ABC1 gene causing the Tangier cellular phenotype may be determined by hybridizing these sequences with probes corresponding to the mRNAs obtained from fibroblasts of subjects suffering or otherwise from the disease, according to the methods described below:

### 1. Preparation of the total RNAs, of the poly(A)⁺ mRNAs and of cDNA probes

The total RNAs are obtained from cell cultures of the fibroblasts of normal subjects or subjects suffering from Tangier disease by the guanidine isothiocyanate method (Chomczynski & Sacchi, 1987). The poly(A)⁺ mRNAs are obtained by affinity chromatography on oligo(dT)-cellulose columns (Sambrook et al., 1989) and the cDNAs used as probes are obtained by RT-PCR (DeRisi et al., 1997) with oligonucleotides labeled with a fluorescent product (Amersham Pharmacia Biotech ; CyDye™).

### 2. Hydridization and detection of the expression levels

The glass membranes containing the sequences presented in this patent application, corresponding to the Tangier gene are hybridized with the cDNA probes obtained from fibroblasts (Iyer et al., 1999). The use of the Amersham/molecular Dynamics system (Avalanche Microscanner™) allows the quantification of the expressions of the products of sequences on healthy or affected cell types.

### EXAMPLE 4 : Construction of the expression vector containing the complete cDNA of ABC1 in mammalian cells

The ABC1 gene may be expressed in mammalian cells. A typical eukaryotic expression vector contains a promoter which allows the initiation of the transcription of the mRNA, a sequence encoding the protein, and the signals required for the termination of the transcription and for the polyadenylation of the transcript. It also contains additional signals such as enhancers, the Kozak sequence and sequences necessary for the splicing of the mRNA. An effective transcription is obtained with the early and late elements of the SV40 virus promoters, the retroviral LTRs or the CMV virus early promoter. However, cellular elements such as the actin promoter may also be used. Many expression vectors may be used to carry out the present invention, an example of such a vector is pcDNA3 (Invitrogen).

### EXAMPLE 5 : Production of normal and mutated ABC1 polypeptides.

The normal ABC1 polypeptide encoded by the complete cDNA of ABC1 whose isolation is described in Example 2 (cloning of the complete cDNA), or the mutated ABC1 polypeptides whose complete cDNA may also be obtained according to the techniques described in Example 2, may be easily produced in a bacterial or insect cell expression system using the baculovirus vectors or in mammalian cells with or without the vaccinia virus vectors. All the methods are now widely described and are known to persons skilled in the art. A detailed description thereof will be found for example in F. Ausubel et al. (1989).

### EXAMPLE 6: Production of an antibody directed against one of the mutated ABC1 polypeptides.

The antibodies in the present invention may be prepared by various methods (Current Protocols In Molecular Biology Volume 1 edited by Frederick M. Ausubel, Roger Brent, Robert E. Kingston, David D. Moore, J.G. Seidman, John A. Smith, Kevin Struhl - Massachusetts General Hospital Harvard Medical School, chapter 11, 1989). For example, the cells expressing a polypeptide of the present invention are injected into an animal in order to induce the production of serum containing the antibodies. In one of the methods described, the proteins are prepared and purified so as to avoid contaminations. Such a preparation is then introduced into the animal with the aim of producing polyclonal antisera having a higher activity.

In the preferred method, the antibodies of the present invention are monoclonal antibodies. Such monoclonal antibodies may be prepared using the hybridoma technique (Kohler et al, 1975 ; Köhler et al, 1976 ; Kohler et al, 1976 ; Hammeling et al., 1981). In general, such methods involve immunizing the animal (preferably a mouse) with a polypeptide or better still with a cell expressing the polypeptide. These cells may be cultured in a suitable tissue culture medium. However, it is preferable to culture the cells in an Eagle medium (modified Earle) supplemented with 10% fetal bovine serum (inactivated at 56°C) and supplemented with about 10 g /l of nonessential amino acids, 1000 U/ml of penicillin and about 100 µg/ml of streptomycin.

The splenocytes of these mice are extracted and fused with a suitable myeloma cell line. However, it is preferable to use the parental myeloma cell line (SP2O) available from the ATCC. After fusion, the resulting hybridoma cells are selectively maintained in HAT medium and then cloned by limiting dilution as described by Wands et al. (1981). The hybridoma cells obtained after such a selection are tested in order to identify the clones secreting antibodies capable of binding to the polypeptide.

Moreover, other antibodies capable of binding to the polypeptide may be produced according to a 2-stage procedure using anti-idiotype antibodies such a method is based on the fact that the antibodies are themselves antigens and consequently it is possible to obtain an antibody recognizing another antibody. According to this method, the antibodies specific for the protein are used to immunize an animal, preferably a mouse. The splenocytes of this animal are then used to produce hybridoma cells, and the latter are screened in order to identify the clones which produce an antibody whose capacity to bind to the specific antibody-protein complex may be blocked by the polypeptide. These antibodies may be used to immunize an animal in order to induce the formation of antibodies specific for the protein in a large quantity.

It is preferable to use Fab and F(ab')2 and the other fragments of the antibodies of the present invention according to the methods described here. Such fragments are typically produced by proteolytic cleavage with the aid of enzymes such as Papain (in order to produce the Fab fragments) or Pepsin (in order to produce the F(ab')2 fragments). Otherwise, the secreted fragments recognizing the protein may be produced by applying the recombinant DNA or synthetic chemistry technology.

For the in vivo use of antibodies in humans, it would be preferable to use "humanized" chimeric monoclonal antibodies. Such antibodies may be produced using genetic constructs derived from hybridoma cells producing the monoclonal antibodies described above. The methods for producing the chimeric antibodies are known to persons skilled in the art (for a review, see : Morrison, (1985); Oi et al., (1986); Cabilly et al., US patent No. 4,816,567 ; Taniguchi et al., EP 171496 ; Morrison et al., EP 173494 ; Neuberger et al., WO 8601533 ; Robinson et al., WO 8702671 ; Boulianne et al; (1984); and Neuberger et al., (1985).

### EXAMPLE 7 : Correction of the cellular phenotype of the Tangier disease

The Tangier disease is characterized by an accelerated catabolism of the high-density lipoprotein (HDL) particles and an accumulation of cholesterol in the tissues. In particular, the fibroblasts of the skin of patients suffering from Tangier disease have a reduced capacity to eliminate their cholesterol content by the process of efflux of cholesterol carried out by apolipoprotein A-I (apoA-I), the major protein of the HDLs (Francis et al., 1995). This characteristic corresponding to a loss of function is also found in other fibroblast cells of patients suffering from familial HDL deficiency (Marcil et al., 1999a).

The correction of the phenotype of the Tangier fibroblasts can be carried out by the transfection of the complete cDNA of ABC1 according to the invention, into said cells. The cDNA is inserted into an expression vector which is then transfected according to the methods described below :

### 1. Preparation of the fibroblast cultures of normal subjects and of subjects suffering from Tangier disease

The primary fibroblasts of human skin are obtained by culturing a skin biopsy obtained from the forearm. These biopsies are performed on patients suffering from Tangier disease having the clinical and biochemical features of the "homozygotes", that is to say orange-colored tonsils, plasma concentrations of apoA-I and of cholesterol-HDL less than the 5^{th} percentile. The normal fibroblast lines are obtained from the American Type Culture Collection (Rockville, MD). The fibroblasts are cultured in an EMMEM (Eagle-modified minimium essential medium ; GIBCO) medium supplemented with 10% fetal calf serum, 2 mM glutamine, 100 IU/ml of penicillin and 100 µg/ml of steptomycin (medium designated EMMEM 10). In order to carry out the study of the efflux of cholesterol, these cells are preloaded with cholesterol by incubating for 24 hours with 50 µg/ml of cholesterol in the medium described above without calf serum but containing 2 mg/ml of bovine albumin (BSA, fraction V).

### 2. Study of the efflux of cholesterol

The fibroblasts preloaded with cholesterol at confluence on 24-well plates are incubated in the EMMEM10 medium and 1 µCi/ml of 1,2-³H-cholesterol (50 Ci/mmol; Dupont; Wilmington, DE) for 48 hours. About 100,000 counts per minute are obtained per well or 1000 counts per minute and per µg of cellular protein. The cells are washed three times with EMMEM/BSA medium, and incubated with this medium for 24 hours before transfecting the gene of interest and starting the efflux by adding 10 µg/ml of proteoliposome containing apoA-I in EMMEM/BSA medium. These proteoliposomes are prepared by sonication of phosphatidylcholine and purified human apoA-I (Jonas, 1986). The cell transfection is carried out by the calcium phosphate precipitation technique (Sambrook et al., 1989). After the period of efflux, in general 20 hours, the medium is collected, centrifuged (1000 g, 5 min), and the radioactivity determined by liquid scintillation counting. The residual radioactivity in the cells is also determined overnight after extraction of the lipids in isopropanol. The percentage efflux is calculated by dividing the radioactivity measured in the supernatant by the sum of the radioactivities measured, in the supernatant and the cellular extract. An internal standard is prepared by transfection of a marker gene and incubation for 24 hours with an EMMEM/BSA medium without proteoliposome containing apoA-I. The efflux of cellular cholesterol from normal fibroblasts transfected with a control gene correspond to 6±2% whereas that obtained from fibroblasts suffering from Tangier disease and transfected with this control gene is less than 1%. On the other hand, the transfection of the fibroblasts suffering from Tangier disease with a plasmid containing the complete cDNA or the genomic DNA for ABC1 according to the invention could make it possible to restore the capacity of these cells to eliminate their excess of cholesterol at a level corresponding to that of normal fibroblasts.

### EXAMPLE 8: Isolation and characterization of genomic fragments of the human ABC1 gene.

A fragment of about 3 kb of the human ABC1 cDNA was obtained from the cDNA clone designated "pf10" containing the first ATP-binding domain of ABC1, this cDNA clone being described in the article by Luciani et al. (1994).

This cDNA fragment obtained by digestion of the clone pflO with the aid of the restriction endonuclease EcoRI, was isolated on an agarose gel after electrophoresis, then labeled with digoxigenin according to the manufacturer's instructions (kit marketed by Boehringer Mannheim, reference 1 585 614)

The labeled cDNA fragment was used to screen the LLNL (Lawrence Livermore National Labs) cosmid library of chromosome 9, immobilized on a Nylon™ filter.

Six positive clones were identified. For these six cosmids, the probe hybridized with single colonies.

A representative clone was isolated from each of these colonies.

The clones LLNLC 131J087 Q2 (designated here cos3a) and LLNLc 13101165 Q2 (designated here cos6f) were analyzed in greater detail.

The clone cos3a was subcloned in the form of an EcoRI fragment into the vector Gen3zf(-) and sequenced at both ends using the Big Dye Terminator technology on an ABI377 type sequencer (Applied Biosystems, Perkin Elmer).

The clones containing distinct inserts (determined after sequencing of the ends of the various inserts or by determining the size thereof) which were too long to be completely sequenced with the aid of the primers hybridizing with the sequences of the vector, were analyzed more before by the technique of transposon insertion and then of sequencing with the aid of primers specific to the transposon ("GPS" system marketed by the company New England Biolabs).

In this manner, genomic sequences corresponding to the human ABC1 gene were isolated and characterized. These sequences were compared with human and mouse sequences identified by references in the databases making it possible to determine the intron-exon junctions.

Primers for the amplification of the DNA of the patients were designed from nonrepetitive sequences of the intron DNA of the ABC1 gene, in such a way that an amplification of the intron-exon junctions as well as the bases essential for the formation of the secondary structure during the splicing step are included in the amplified fragments.

The genomic DNA of the patients was amplified with the aid of the primers described above using Qiagen's Star Taq kit or the Supertaq kit, using the hybridization conditions and the amplification cycle conditions recommended by the manufacturer.

The amplified PCR products were then purified using a kit marketed by the company Qiagen, and then sequenced by the Big Dye Terminator method on an ABI377 sequencer.

### EXAMPLE 9 : Determination of polymorphisms/mutations in the ABC1 gene.

The detection of polymorphisms or of mutations in the sequences of the transcripts or in the genomic sequence of the ABC1 gene may be carried out according to various protocols. The preferred method is direct sequencing.

For patients from whom it is possible to obtain an mRNA preparation, the preferred method consists in preparing the cDNAs and sequencing them directly. For patients for whom only DNA is available, and in the case of a transcript where the structure of the corresponding gene is unknown or partially known, it is necessary to precisely determine its intron-exon structure as well as the genomic sequence of the corresponding gene. This therefore involves, in a first instance, isolating the genomic DNA BAC or cosmid clone(s) corresponding to the transcript studied according to the method described in Example 8, sequencing the insert of the corresponding clone(s) and determining the intron-exon structure by comparing the cDNA sequence to that of the genomic DNA obtained.

The technique of detection of mutations by direct sequencing consists in comparing the genomic sequences of the ABC1 gene obtained from homozygotes for the disease or from at least 8 individuals (4 individuals affected by the pathology studied and 4 individuals not affected). The sequence divergences constitute polymorphisms. All those modifying the amino acid sequence of the wild-type protein may be mutations capable of affecting the function of said protein which it is preferred to consider more particularly for the study of cosegregation of the mutation and of the disease (denoted genotype-phenotype correlation) in the pedigree or in the studies of case/control association for the analysis of the sporadic cases.

### EXAMPLE 10 : Identification of a causal gene for a disease linked to a deficiency in the reverse transport of cholesterol by causal mutation or a transcriptional difference

Among the mutations identified according to the method described in Example 9, all those associated with the disease phenotype are capable of being causal. Validation of these results is made by sequencing the gene in all the affected individuals and their relations (whose DNA is available).

Moreover, Northern blot or RT-PCR analysis, according to the methods described in Example 1, using RNA specific to affected or nonaffected individuals makes it possible to detect notable variations in the level of expression of the gene studied, in particular in the absence of transcription of the gene.

### EXAMPLE 11 : Identification of biallelic polymorphisms in the ABC1 gene

Primers for the amplification of the DNA of the patients were designed from nonrepetitive sequences of the intron DNA of the ABC1 gene, in such a way that an amplification of the intron-exon junctions as well as the bases essential for the formation of the secondary structure during the RNA splicing step are included in the amplified fragments.

The various pairs of primers specifically developed are presented in Table VI. The results found on the DNA from 7 families containing cases of Tangier or FHD disease are shown in Table V.

The genomic DNA of the patients was amplified with the aid of the primers described above using Qiagen's Star Taq kit or the Supertaq kit, using the hybridization conditions and the amplification cycle conditions recommended by the manufacturer.

The amplified PCR products were then purified using a kit marketed by the company Qiagen, and then sequenced by the Big Dye Terminator method on an ABI377 sequencer (Applied Biosystems, Perkin Elmer).

### EXAMPLE 12 : Construction of recombinant vectors comprising a nucleic acid encoding a ABC1 protein

### I. Synthesis of a nucleic acid encoding a human ABC1 protein:

Total RNA (500 ng) isolated from a human cell (i.e., placental tissue, Clontech, Palo Alto, CA, USA, or THP1 cells) may be used as source for the synthesis of the cDNA of the human ABC1 gene. Methods to reverse transcribe mRNA to cDNA are well known in the art. For example, one may use the system "Superscript one step RT-PCR (Life Technologies, Gaithersburg, MD, USA).

Oligonucleotide primers specific for ABC1 cDNA may be used for this purpose. Specifically, two oligonucleotide primers specific for ABC1 (0.25 µM) comprising either 1) a sequence contained within nucleotide region 1-184 of SEQ ID NO: 69 and a complementary sequence contained within nucleotide region 6968-9741 of SEQ ID NO: 69, or 2) a sequence contained within nucleotide region 1-297 of SEQ ID NO: 70 and a complementary sequence contained within nucleotide region 7081-9854 of SEQ ID NO: 70, may be used to isolate a nucleic acid encoding an ABC1 protein.

These oligonucleotide primers may be synthesized by the phosphoramidite method on a DNA synthesizer of the ABI 394 type (Applied Biosystems, Foster City, CA, USA).

Sites recognized by the restriction enzyme NotI may be incorporated into the amplified ABC1 cDNA to flank the ABC1 cDNA region desired for insertion into the recombinant vector by a second amplification step using 50 ng of human ABC1 cDNA as template, and 0.25 µM of the ABC1 specific oligonucleotide primers used above containing, at their 5' end, the site recognized by the restriction enzyme NotI (5'-GCGGCCGC-3'), in the presence of 200 µM of each of said dideoxynucleotides dATP, dCTP, dTTP and dGTP as well as the *Pyrococcus furiosus* DNA polymerase (Stratagene, Inc. La Jolla, CA, USA).

The PCR reaction may be carried out over 30 cycles each comprising a step of denaturation at 95°C for one minute, a step of renaturation at 50°C for one minute and a step of extension at 72°C for two minutes, in a thermocycler apparatus for PCR (Cetus Perkin Elmer Norwalk, CT, USA).

### II. Cloning of the cDNA of the human ABC1 gene into an expression vector:

The human ABC1 cDNA insert may then be cloned into the NotI restriction site of an expression vector, for example, the pCMV vector containing a cytomegalovirus (CMV) early promoter and an enhancer sequence as well as the SV40 polyadenylation signal (Beg et al., 1990; Applebaum-Boden, 1996), in order to produce an expression vector designated pABCl.

The sequence of the cloned cDNA can be confirmed by sequencing on the two strands using the reaction set "ABI Prism Big Dye Terminator Cycle Sequencing ready" (marketed by Applied Biosystems, Foster City, CA, USA) in a capillary sequencer of the ABI 310 type (Applied Biosystems, Foster City, CA, USA).

### III. Construction of a recombinant adenoviral vector containing the cDNA of the human ABC1 gene:

### A- Modification of the expression vector pCMV-β:

The β-galactosidase cDNA of the expression vector pCMV-β (Clontech, Palo Alto, CA, USA, Gene Bank Accession No. U02451) may be deleted by digestion with the restriction endonuclease NotI and replaced with a multiple cloning site containing, from the 5' end to the 3' end, the following sites: NotI, AscI, RsrII, AvrII, SwaI, and NotI, cloned at the region of the NotI restriction site. The sequence of this multiple cloning site is:

5'-CGGCCGCGGCGCGCCCGGACCGCCTAGGATTTAAATCGCGGCCCGCG-3' (SEQ ID
NO: 66).

The DNA fragment between the EcoRI and SanI sites of the modified expression vector pCMV may be isolated and cloned into the modified XbaI site of the shuttle vector pXCXII (McKinnon et al., 1982; McGrory et al., 1988).

### B-modification of the shuttle vector pXCXII:

A multiple cloning site comprising, from the 5' end to the 3 end the XbaI, EcoRI, SfiI, PmeI, NheI, SrfI, PacI, Sail and XbaI restriction sites having the sequence: 
5’-GCTCTAGAATTCGGCCTCCGTGGCCGTTTAAACGCTAGCGCCCGGGCTTAATTAA
GTCGACTCTAGAGC-3’ (SEQ ID NO: 67).
 may be inserted at the level of the Xbal site (nucleotide at position 3329) of the vector pXCXII (McKinnon et al., 1982; McGrory et al., 1988).

The EcoRI-SalI DNA fragment isolated from the modified vector pCMV-β containing the CMV promoter/enhancer, the donor and acceptor splicing sites of FV40 and the polyadenylation signal of FV40 may then be cloned into the EcoRI-SalI site of the modified shuttle vector pXCX, designated pCMV-11.

### C- Preparation of the shuttle vector pAD12-ABC1:

The human ABC1 cDNA is obtained by an RT-PCR reaction, as described above, and cloned at the level of the NotI site into the vector pCMV-12, resulting in the obtaining of the vector pCMV-ABC1.

### D. Construction of the ABC1 recombinant adenovirus:

The ABC1 -rldV recombinant adenovirus containing the human ABC1 cDNA may be constructed according to the technique described by McGrory et al. (1988).

Briefly, the vector pAD 12-ABC1 is cotransfected with the vector tGM17 according to the technique of Chen and Okayama (1987).

Likewise, the vector pAD12-Luciferase was constructed and cotransfected with the vector pJM17.

The recombinant adenoviruses are identified by PCR amplification and subjected to two purification cycles before a large-scale amplification in the human embryonic kidney cell line HEK 293 (American Type Culture Collection, Rockville, MD, USA).

The infected cells are collected 48 to 72 hours after their infection with the adenoviral vectors and subjected to five freeze-thaw lysing cycles.

The crude lysates are extracted with the aid of Freon (Halocarbone 113, Matheson Product, Scaucus, N.J. USA), sedimented twice in cesium chloride supplemented with 0.2% murine albumine (Sigma Chemical Co., St Louis, MO, USA) and dialysed extensively against buffer composed of 150 nM NaCI, 10 mM Hepes (pH 7,4), 5 mM KCI, 1 mM MgCl₂, and 1 mM CaCl₂.

The recombinant adenoviruses are stored at -70°C and titrated before their administration to animals or their incubation with cells in culture.

The absence of wild-type contaminating adenovirus is confirmed by screening with the aid of PCR amplification using oligonucleotide primers located in the structural portion of the deleted region.

### IV Validation of the expression of the human ABC1 cDNA:

Polyclonal antibodies specific for a human ABC1 polypeptide may be prepared as described above in rabbits and chicks by injecting a synthetic polypeptide fragment derived from an ABC1 protein, comprising all or part of an amino acid sequence as described in SEQ ID NO: 71. These polyclonal antibodies are used to detect and/or quantify the expression of the human ABC1 gene in cells and animal models by immunoblotting and/or immunodetection.

The biological activity of ABC1 may be monitored by quantifying the cholesterol fluxes induced by apoA-I using cells transfected with the vector pCMV-ABC1 which have been loaded with cholesterol (Remaley et al., 1997).

### V. Expression in vitro of the human ABC1 cDNA in cells:

Cells of the HEK293 line and of the COS-7 line (American Tissue Culture Collection, Bethesda, MD, USA), as well as fibroblasts in primary culture derived from Tangier patients or from patients suffering from hypo-alphalipoproteinemia are transfected with the expression vector pCMV-ABC1 (5-25 µg) using Lipofectamine (BRL, Gaithersburg, MD, USA) or by coprecipitation with the aid of calcium chloride (Chen et al., 1987).

These cells may also be infected with the vector pABC1-AdV (Index of infection, MOI=10).

The expression of human ABC1 may be monitored by immunoblotting as well as by quantification of the efflux of cholesterol induced by apoA-1 using transfected and/or infected cells.

The complementation of the genetic defect from which the Tangier patients and the hypo-alphalipoproteinemic patients are suffering using fibroblasts of these patients, may be confirmed by the detection of the expression of the normal ABC1 gene, which makes it possible to establish the functional importance of this receptor.

### VI. Expression in vivo of the ABC1 gene in various animal models:

An appropriate volume (100 to 300 µl) of a medium containing the purified recombinant adenovirus (pABC1-AdV or pLucif-AdV) containing from 10⁸ to 10⁹ lysis plaque-forming units (pfu) are infused into the Saphenous vein of mice (C57BL/6, both control mice and models of transgenic or knock-out mice) on day 0 of the experiment.

The evaluation of the physiological role of the ABC1 protein in the metabolism of lipoproteins is carried out by determining the total quantity of cholesterol, of triglycerides, of phospholipids and of free cholesterol (Sigma and Wako Chemicals, Richmond, VA, USA), of cholesterol-HDL (CIBA-Corning, Oberlin, OH, USA) and apolipoproteins A-I, A-II, E and B from mice (Foger et al., 1997), before (day zero) and after (days 2, 4, 7, 10, 14) the administration of the adenovirus.

Kinetic studies with the aid of radioactively labelled produces such as apoA-I-HDL, CE-HDL as well as apoB-LDL and CE-LDL are carried out on day 5 after the administration of the vectors rLucif-AdV and rABC1-AdV in order to evaluate the effect of the expression of ABC1 on the metabolism of the HDLs and of the LDLs as well as on the release of cholesterol toward the liver.

The effect of the expression of ABC1 on the development of atherosclerosis may be evaluated by quantifying the mean surface area of aortic lesion in apoE mice after administration of the vector rABC1-Adv.

Furthermore, transgenic mice and rabbits overexpressing the ABC1 gene may be produced, in accordance with the teaching of Vaisman (1995) and Hoeg (1996) using constructs containing the human ABC1 cDNA under the control of endogenous promoters such as ABC 1, CMV or apoE.

The evaluation of the long-term effect of the expression of ABC1 on the kinetics of the plasma lipids, lipoproteins and apolipoproteins and on atherosclerosis may be carried out as described above.

### EXAMPLE 13 : Use of vesicles for screening for agonist and antagonist molecules of an ABC1 protein.

The basis of this screening test is the reconstitution of membranes which have incorporated the ABC1 protein and containing substrates such as cholesterol or phospholipids. The ABC1 protein may then be activated or its function repressed by the addition of molecules of interest. The outflow of the substrates through the channel formed by the ABC1 protein is then detected.
**a) Reconstitution of a membrane comprising an ABC1 protein and a labeled lipid substrate.**
   Various strategies may be used to manufacture these membranes, methods using organic solvents, mechanical means such as sonication, the "French press", or by freeze-thaw cycles or using detergents (cholates, Chaps, Chapso) (see Rigaud et al., 1995).
   More particularly, a lipid substrate such as phospholipids, cholesterol or cholesterol ester, a radioactive substrate of the ³H-cholesterol, ¹²⁵-cholesterol or ³H-phosphatidylcholine type or a fluorescent substrate with NBD or pyrene (Molecular Probes ; http ://www.probes.com) and phospatidylcholine from eggs (1 mM) are dried on the pellet of a glass flask. Sodium cholate and the ABC1 protein are mixed in this flask in a mol to mol ratio of 0.3. The whole is vortex-mixed for 5 minutes and then incubated at 25°C for 30 minutes and then dialysed against a saline buffer. The proteoliposome produced according to this protocol is monitored by turbidimetry in order to verify that its manufacture is good.
**b) Capture of the proteoliposome on a solid surface.**
   This step may be carried out by incorporating binding proteins of the integrin type. In this protocol, a capture by the antibodies directed against the ABC1 protein and previous adsorbed on a 96- or 384-well plate is used.
   A solution containing these antibodies at the concentration of 100 µg/l are absorbed on these multi-well plates by incubating overnight at 4°C. After washing, the plate is then saturated with bovine albumin at 1 mg/ml incubated for 2 hours at 37°C. The whole is then washed and incubated with the proteoliposomes containing ABC1 for 2 hours at 37°C.
**c) Binding to the molecules of interest.**
   This step is carried out by incubation of products for 1 hour at 37°C.
**d) Determination of the activation or inhibition of the ABC1 protein.**
   If the substrate if fluorescent, the fluorescence of the supernatant shows us the activity of a product in inducing a transport of lipid to the outside of the proteoliposome. Alternatively, the use of a Confocal system gives us information on the quantities of substrate inside and outside the proteoliposome. If the substrate is radioactive, the use of CytoStar-type plates having a bottom with scintillation liquid makes it possible to reveal the substrate still sequestered in the proteoliposome.

### EXAMPLE 14 : Use of anion transport for screening of agonist and antagonist molecules of an ABC1 protein.

The principle of this test lies in the property that the ABC1 protein has for transporting the anions during its activation.
a) The macrophage cells of the THP-1 lines, monocytic leukemia human cells, are a model of differentiated macrophages. The cells are cultured in an RPMI 1640 medium supplemented with 10% fetal calf serum in 48-multiwell plates at the density of 2 x 10⁵ cells per well. The fibroblast cells of patients suffering from Tangier disease may be used as a negative control because their ABC1 protein is not functional. Another negative control may be obtained by the addition of anti-ABC1 antibodies.
b) The use of anion transport defective cells or cells treated with anion channel inhibitors (Verapamil type, an inhibitor of P-glycoprotein or tetraethylammonium, a potassium channel inhibitor) may also be used.
c) For the actual test itself, the cells are then washed with an Earles's modified salt solution (ESS) medium preloaded with I ml of KI at 1 µmol/L (0.1 µCi/ml of NaI125) in this ESS medium for 30 minutes at 37°C. The products are then added to the extracellular medium. The cells are then washed with the ESS medium.
d) The quantity of iodide in the medium is detected every minute for 11 minutes. The first two points correspond to the basal efflux. At the end of the incubation, the medium is taken up and the quantity of iodine remaining in the cells is counted following lysis of the cells in 1 molar NaOH.
e) The total quantity of radioactivity at time zero is equal to the sum of the radioactivity found in the supernatant and the residual radioactivity in the cells. The efflux curves are constructed by plotting the percentage of radioactivity released into the medium as a function of time.

### EXAMPLE 15 : Use of THP-1 macrophages expressing IL-lbeta to screen for agonist and antagonist molecules of an ABC1 protein.

The principle of this test is that any substance that modulates the activity of an ABC1 protein will have an effect on the synthesis of IL-lbeta.
a) The macrophage cells of the THP-1 lines, monocytic leukemia human cells, are a model of differentiated macrophages. The cells are cultured in an RPMI 1640 medium supplemented with 10% fetal calf serum in multiwell plates at the density of 2 105 cells per well.
b) For the actual test itself, the cells are then washed and placed in an RPMI 1640 medium containing 1 mg/ml of purified human albumin fraction IV.
c) The products are added to the extracellular medium. Simultaneously, the cells are then activated by addition of lipopolysaccharide (LPS) over 3 hours at 1 µg/ml followed by an incubation of 30 minutes in the presence of ATP at 5 mmol/L.
d) The concentrations of IL-1beta and of control IL-1alpha, tumor necrosis factor alpha (TNFalpha) and IL-6 are determined by ELISA kits according to the manufacturers' instructions (R&D Sytem ; human IL-lbeta Chemiluminescent ELISA reference QLBO0). The variations of mRNA for IL-lbeta which is not supposed to be affected are evaluated by the Northern blotting technique with the corresponding probe.

### REFERENCES CITED

Adames et al., 1985, Nature 318:533-538.
Agbedana et al., 1990. Ann Trop Med Parasitol., 84:529-30.
Alexander et al., 1987, Mol. Cell. Biol. 7:1436-1444.
Altschul et al, 1990. J. Mol. Biol., 215:403-410.
Altschul et al, 1997. Nucleic Acids Res., 25:3389-3402.
Applebaum-Boden, 1996. JCI 97.
Ausubel et al., 1989. Current Protocols in Molecular Biology, Green Publishing Associates and Wiley Interscience, N.Y.
Baptista et al., 1996. Parasite, 3:335-40.
Beard et al., 1990. Virology, 75:81.
Beaucage et al., 1981. Tetrahedron Lett, 22: 1859-1862.
Becq et al., 1997. Journal of Biological Chemistry, 272:2695-2699.
Beg et al., 1990. PNAS, 87:3473.
Bender et al., 1987. J. Virol., 61:1639.
Benoist and Chambon, 1981, Nature 290:304-310.
Benton and Davis, 1977, Science 196:180.
Bernstein et al., 1985. Genet. Eng., 7:235.
Bodzioch et al., 1999. Nature Genetics, 22:347-351.
Boulianne et al., 1984. Nature, 312:643.
Breakfield et al., 1991. New Biologist, 3:203.
Brinster et al., 1982, Nature 296:39-42.
Brooks et al., 1999. Nature Genetics, 22:336-345.
Brown EL, Belagaje R, Ryan MJ, Khorana HG, 1979. Methods Enzymol, 68:109-151.
Buchwald et al., 1980. Surgery 88:507.
Cabilly et al., US patent No. 4,816,567.
Chander et al., 1998. Indian J Exp Biol., 36:371-4.
Chen and Kwok, 1997. Nucleic Acids Research 25:347-353.
Chen and Okayama, 1987. Mol Cell Biol., 7:2745-2752.
Chen et al., 1997. Proc. Natl. Acad. Sci. USA, 94/20:10756-10761.
Chen et al., 1987. Mol. Cell. Biol., 7 : 2745-2752.
Chomczynski, P. and Sacchi, 1987. Anal. Biochem., 162:156-159.
Cole et al., 1985. In: *Monoclonal Antibodies and Cancer Therapy,* Alan R. Liss, Inc., pp. 77-96.
Cote et al., 1983. Proc. Natl. Acad. Sci. U.S.A. 80:2026-2030.
Cuisinier et al., 1990. Med Trop (Mars). 50:91-5.
Curiel et al., 1992. Hum. Gene Ther. 3:147-154.
Davis et al., 1993. J. Infect. 26:279-85.
Davis et al., 1995. J. Infect. 31:181-8.
DeBoer, et al., 1983, Proc. Natl. Acad. Sci. U.S.A. 80:21-25.
DeRisi J. et al., 1997. Science, 278:680-686.
Dib C. et al., 1996. Nature, 380:152-154.
Djoumessi, 1989. Pathol Biol., 37:909-11.
During et al., 1989. Ann. Neurol. 25:351.
Efthimiou et al., 1992. Wein Klin Wochenschr., 104:705-6.
Erel et al., 1998. Haematologia (Budap)., 29:207-12.
Felgner et al., 1987. PNAS 84:7413.
Felgner and Ringold, 1989. Science 337:387-388.
Flotte et al., 1992. Am. J. Respir. Cell Mol. Biol., 7:349-356.
Foger et al., 1997. J. Biol. Chem., 272:27393-400.
Forsell Y. et al., 1997. Biol. Psychiatry, 42:898-903.
Fraley et al., 1979. Proc. Natl. Acad. Sci. USA, 76:3348-3352.
Fraley et al., 1980. J.Biol.Chem., 255:10431.
Francis et al., 1995. J. Clin. Invest., 96:78-87.
French et al., 1993. Am J Cardiol. 71:505-510.
Freshney (ed.), 1986. *Immobilized Cells And Enzymes,* IRL Press.
Fromont-Racine et al, 1993. Nucleic Acid Res.21(7):1683-1684.
Fuller S.A. et al., 1996. *Immunology, In: Current Protocols in Molecular Biology,* Ausubel et al.(eds.).
Gait (ed.), 1984. *Nucleic Acid Hybridization.*
Glover (ed.), 1985. *DNA Cloning: A Practical Approach, Volumes I and II Oligonucleotide Synthesis,* MRL Press, Ltd., Oxford, U.K.
Goodson, 1984. In: *Controlled Drug Bioavailability, Drug Product Design and Performance,* Smolen and Ball (eds.), Wiley: New York , vol. 2, pp. 115-138.
Gopal, 1985. Mol. Cell. Biol., 5:1188-1190.
Graham et al., 1973. Virology, 52:456-457.
Graham et al., 1977. J. Gen. Virol., 36:59.
Graham, 1984. EMBO J., 3:2917.
Grellier et al., 1997. Vox Sang., 72:211-20.
Grosschedl et al., 1984, Cell 38:647-658.
Grunstein and Hogness, 1975, Proc.Natl. Acad. Sci. U.S.A. 72:3961.
Haff L.A. and Smirnov I.P., 1997. Genome Research, 7:378-388.
Hager and Hajduk, 1997. Nature 385:823-6.
Ham, 1980. Methods Cell. Biol., 21a:255.
Hames and Higgins (eds.), 1984. *Animal Cell Culture.*
Hames and Higgins (eds.), 1985. *Transcription And Translation.*
Hames BD and Higgins SJ, 1985. *Nucleic acid hybridization : a practical approach,* Hames and Higgins Ed., IRL Press, Oxford.
Hammeling et al., 1981. In : Monoclonal Antibodies and T-Cell Hybridomas, Elsevier, N.Y., pp. 563-681.
Hammer et al., 1987, Science 235:53-58.
Hanahan, 1985, Nature 315:115-122.
Harland et al., 1985. J. Cell. Biol., 101:1094-1095.
Hartmut et al., Canadian Patent Application No.2,012,311, filed March 15, 1990.
Higuchi, 1989, "Using PCR to Engineer DNA", in *PCR Technology: Principles and Applications for DNA Amplification,* H. Erlich, ed., Stockton Press, Chapter 6, pp. 61-70.
Hoeg, 1996. PNAS, 93:11448.
Horvath S. et al., 1998. Am. J. Hum. Genet., 63:1886-1897.
Houben Weyl, 1974. In: Meuthode der Organischen Chemie, E. Wunsch Ed., 15-I:15-II. Howard et al., 1989. J. Neurosurg. 71:105.
Huse et al., 1989. Science 246:1275-1281.
Hutchinson, C., et al., 1978, J. Biol. Chem. 253:6551.
Hutchinson et al., 1986, Proc. Natl. Acad. Sci. U.S.A. 83:710.
Huygen et al., 1996. Nature Medicine, 2(8):893-898.
Jonas et al., 1986. Methods Enzymol., 128:553-82.
Kaneda et al., 1989. Science 243:375.
Kaufman, 1991. *Current Protocols in Molecular Biology,* 16.12.
Kelsey et al., 1987, Genes and Devel. 1:161-171.
Kim et al., 1996. Genomics, 34:213-218.
Kittl, et al., 1992. Wein Klin Wochenschr 104:21-4.
Koch Y., 1977. Biochem. Biophys. Res. Commun., 74:488-491.
Kohler et al, 1976. Eur. J. Immunol., 6:511.
Kohler et al., 1976. Eur. J. Immunol. 6:292.
Kohler et al., 1975. Nature, 256:495.
Kohler G. and Milstein C., 1975. Nature, 256:495-497.
Kollias et al., 1986, Cell 46:89-94.
Kozbor et al., 1983a. Immunology Today, 4:72.
Kozbor et al., 1983b. Hybridoma, 2(1):7-16.
Krumlauf et al., 1985, Mol. Cell. Biol. 5:1639-1648.
Kwiterovich, 1995. Ann NY Acad Sci., 748:313-30; discussion 331-2.
Lander and Schork, 1994. Science, 265:2037-2048.
Langer, 1990. Science, 249:1527-1533.
Langer and Wise (eds.), 1974. In: *Medical Applications of Controlled Release,* CRC Press: Boca Raton, Florida.
Langmann T. et al., 1999. Biochem. Biophys. Res. Comm., 257:29-33.
Lawn et al., 1999. J. Clin. Invest., 104:R25-R31.
Leder et al., 1986, Cell 45:485-495.
Leger OJ, et al., 1997. Hum Antibodies, 8(1):3-16.
Levrero et al., 1991. Gene 101.
Levy et al., 1985. *Science* 228:190.
Lopez-Berestein, 1989. In: *Liposomes in the Therapy of Infectious Disease and Cancer,* Lopez-Berestein and Fidler (eds.), Liss: New York, pp. 317-327.
Luciani M.F.et al., 1994. Genomics, 21:150-159.
Lyer V. et al., 1999. Science, 283:83-87.
MacDonald, 1987, Hepatology 7:425-515.
Mackey, et al., 1988. Proc. Natl. Acad. Sci. U.S.A. 85:8027-8031.
MacKinnon et al., 1982. Gene 19:33.
Maniatis et al., 1982. Molecular cloning : A Laboratory Manual, Cold Spring Harbor Press, Cold Spring, NY.
Marcil M. et al., 1999a. Artreriosclerosis Throbosis and Vascular Biology, 17:1813-1821.
Marcil et al., 1999b. Lancet, 354:1341-46.
Martineau P, Jones P, Winter G, 1998. J Mol Biol, 280(1):117-127.
Mason et al., 1986, Science 234:1372-1378.
Maurois et al., 1985. Biochimie., 67:227-39.
McCormick, 1985. BioTechnology, 3:689.
McGrory et al, 1988. Virology, 163:614.
McLaughlin BA et al., 1996. Am. J. Hum. Genet., 59:561-569.
Merrifield RB, 1965a. Nature, 207(996):522-523.
Merrifield RB., 1965b. Science, 150(693):178-185.
Mogram et al., 1985, Nature 315:338-340.
Mohanty, et al., 1992. Ann Trop Med Parasitol., 86:601-6.
Morrison et al., EP 173494.
Morrison et al., 1984. J. Bacteriol. 159:870.
Morrison, 1985. Science 229:1202.
Narang SA, Hsiung HM, Brousseau R, 1979. Methods Enzymol, 68:90-98.
Neuberger et al., 1984. Nature, 312:604-608.
Neuberger et al., 1985. Nature, 314:268.
Neuberger et al., WO 8601533.
Nickerson D.A. et al., 1992. Genomics, 12:377-387.
Nicolau C. et al., 1987. Methods Enzymol., 149:157-76.
Nilsson et al., 1990. J Intern Med., 227:151-5.
Oi et al., 1986. Biotechnique, 4:214.
Okayama, 1987. Mol Cell. Biol., 7:2745.
Oliphant et al., 1986, Gene 44:177.
Ornitz et al., 1986, Cold Spring Harbor Symp. Quant. Biol. 50:399-409.
Pagano et al., 1967. J.Virol., 1:891.
Perbal, 1984. *A Practical Guide To Molecular Cloning.*
Pinkert et al., 1987, Genes and Devel. 1:268-276.
Pirich et al., 1993. Semin Thromb Hemost., 19:138-43.
Potter et al., 1984. Proc Natl Acad Sci U S A., 81(22):7161-5.
Ranger and Peppas, 1983. J. Macromol. Sci. Rev. Macromol. Chem. 23:61.
Readhead et al., 1987, Cell 48:703-712.
Reeck et al., 1987, Cell 50:667.
Reimann KA, et al., 1997. AIDS Res Hum Retroviruses, 13(11):933-943.
Remaley et al., 1997. ATVB, 17:1813.
Ridder R, Schmitz R, Legay F, Gram H, 1995. Biotechnology (NY), 13(3):255-260.
Rigaud et al., 1995. Biochimica et Biophysica Acta, 1231:223-246.
Robinson et al., WO 8702671.
Rust S. et al., 1998. Nature Genetics, 20:96-98.
Rust et al., 1999. Nature Genetics, 22:352-355.
Sambrook, J. Fritsch, E. F., and T. Maniatis, 1989. *Molecular cloning: a laboratory manual.* 2ed. Cold Spring Harbor Laboratory, Cold spring Harbor, New York.
Samulski et al., 1989. J. Virol., 63:3822-3828.
Sanchez-Pescador R., 1988. J. Clin. Microbiol., 26(10):1934-1938.
Sani, 1985, Nature 314:283-286.
Saudek et al., 1989 N. Engl. J. Med. 321:574.
Sefton, 1987. CRC Crit. Ref. Biomed. Eng. 14:201.
Sham P.C. et al., 1995. Ann. Hum. Genet., 59:323-336.
Smith *et al.,* 1988, Gene 67:31-40.
Smolen and Ball (eds.), 1984. *Controlled Drug Bioavailability, Drug Product Design and Performance,* Wiley: New York .
Sternberg N.L., 1992. Trends Genet., 8:1-16.
Sternberg N.L., 1994. Mamm. Genome, 5:397-404.
Strautnieks S.S. et al., 1998. Nature Genetics, 20:233-235.
Swift et al., 1984, Cell 38:639-646.
Syvanne et al., 1995a. Circulation, 92:364-70.
Syvanne et al., 1995b. J. Lipid Res., 36:573-82.
Tacson et al., 1996. Nature Medicine, 2(8):888-892.
Takeda et al., 1985. Nature 314:452-454.
Taniguchi et al., EP 171496.
Tomlinson and Raper, 1996. Nat. Biotechnol., 14:717-21.
Treat et al., 1989. In: *Liposomes in the Therapy of Infectious Disease and Cancer,* Lopez-Berestein and Fidler (eds.), Liss: New York, pp. 353-365.
Tur-Kaspa et al, 1986. Mol. Cell. Biol., 6:716-718.
Ulmer et al., 1993. Science 259:1745-1748.
Urdea M.S., 1988. Nucleic Acids Research, 11:4937-4957.
Urdea MS et al., 1991. Nucleic Acids Symp Ser., 24:197-200.
Vaisman, 1995. J.Biol. Chem., 270:12269.
Van, den Hazel. H., H. Pichler, V. M. M. do, E. Leitner, A. Goffeau, and G. Daum, 1999. J Biol Chem., 274:1934-41.
Villa-Kamaroff, et al., 1978, Proc. Natl. Acad. Sci. U.S.A. 75:3727-3731.
Wagner et al., 1981, Proc. Natl. Acad. Sci. U.S.A. 78:1441-1445.
Wands et al., 1981. Gastroenterology, 80:225-232.
Williams et al., 1991. Proc. Natl. Acad. Sci. USA 88:2726-2730.
Wu et al., 1992, J. Biol. Chem. 267:963-967.
Wu and Wu, 1987. J. Biol. Chem. 262:4429-4432.
Wu and Wu, 1988, J. Biol. Chem. 263:14621-14624.
Xiong M. et al., 1999. Am. J. Hum. Genet., 64:629-640.
Yamamoto, et al., 1980, Cell 22:787-797.
Yamon et al., 1997. Blood, 90:2911-2915.
Zoller and Smith, 1984, DNA 3:479-488.

The present invention is not to be limited in scope by the specific embodiments described herein. Indeed, various modifications of the invention in addition to those described herein will become apparent to those skilled in the art from the foregoing description and the accompanying figures. Such modifications are intended to fall within the scope of the appended claims.

Various publications are cited herein, the disclosures of which are incorporated by reference in their entireties.

## Claims

1. An isolated nucleic acid comprising any one of SEQ ID NOs: 1, 4-65, 68-70, 72-88, 103, 109-118, and 137, or of a complementary polynucleotide sequence.

2. An isolated nucleic acid comprising at least eight consecutive nucleotides of a polynucleotide sequence of a) any one of SEQ ID NOs: 1, 4-8, 11-16, 18-26, 28-35, 37-52, 54-65, 68, and 137, or of a complementary polynucleotide sequence; b) nucleotides 3154-3200 of SEQ ID NO: 9 or of a complementary polynucleotide sequence; c) nucleotides 1-242 of SEQ ID NO: 10 or of a complementary polynucleotide sequence; d) nucleotides 1-1851 of SEQ ID NO: 17; or of a complementary polynucleotide sequence; e) nucleotides 152-198 of SEQ ID NO: 27, or of a complementary polynucleotide sequence; f) nucleotides 1-1657 of SEQ ID NO: 36, or of a complementary polynucleotide sequence; or g) nucleotides 1-242 of SEQ ID NO: 53, or of a complementary polynucleotide sequence.

3. An isolated nucleic acid comprising at least 80% nucleotide identity with a nucleic acid comprising a) any one of SEQ ID NOs: 1, 4-8, 11-16, 18-26, 28-35, 37-52, 54-65, 68, and 137, or of a complementary polynucleotide sequence; b) nucleotides 3154-3200 of SEQ ID NO: 9 or of a complementary polynucleotide sequence; c) nucleotides 1-242 of SEQ ID NO: 10 or of a complementary polynucleotide sequence; d) nucleotides 1-1851 of SEQ ID NO: 17; or of a complementary polynucleotide sequence; e) nucleotides 152-198 of SEQ ID NO: 27, or of a complementary polynucleotide sequence; f) nucleotides 1-1657 of SEQ ID NO: 36, or of a complementary polynucleotide sequence; or g) nucleotides 1-242 of SEQ ID NO: 53, or of a complementary polynucleotide sequence.

4. The isolated nucleic acid according to claim 3, wherein the nucleic acid comprises an 85%, 90%, 95%, or 98% nucleotide identity with the nucleic acid comprising a) any one of SEQ ID NOs: 1, 4-8, 11-16, 18-26, 28-35, 37-52, 54-65, 68, and 137, or of a complementary polynucleotide sequence; b) nucleotides 3154-3200 of SEQ ID NO: 9 or of a complementary polynucleotide sequence; c) nucleotides 1-242 of SEQ ID NO: 10 or of a complementary polynucleotide sequence; d) nucleotides 1-1851 of SEQ ID NO: 17; or of a complementary polynucleotide sequence; e) nucleotides 152-198 of SEQ ID NO: 27, or of a complementary polynucleotide sequence; f) nucleotides 1-1657 of SEQ ID NO: 36, or of a complementary polynucleotide sequence; or g) nucleotides 1-242 of SEQ ID NO: 53, or of a complementary polynucleotide sequence.

5. An isolated nucleic acid that hybridizes under high stringency conditions with a nucleic acid comprising a) any one of SEQ ID NOs: 1, 4-8, 11-16, 18-26, 28-35, 37-52, 54-65, 68, and 137, or of a complementary polynucleotide sequence; b) nucleotides 3154-3200 of SEQ ID NO: 9 or of a complementary polynucleotide sequence; c) nucleotides 1-242 of SEQ ID NO: 10 or of a complementary polynucleotide sequence; d) nucleotides 1-1851 of SEQ ID NO: 17; or of a complementary polynucleotide sequence; e) nucleotides 152-198 of SEQ ID NO: 27, or of a complementary polynucleotide sequence; f) nucleotides 1-1657 of SEQ ID NO: 36, or of a complementary polynucleotide sequence; or g) nucleotides 1-242 of SEQ ID NO: 53, or of a complementary polynucleotide sequence.

6. An isolated nucleic acid comprising a polynucleotide sequence as depicted in any one of SEQ ID NOs: 1, 4-65, 68-70, 72-88, 103, 109-118, and 137, or of a complementary polynucleotide sequence.

7. An isolated nucleic acid comprising nucleotides 1-244 of SEQ ID NO: 69 or nucleotides 1-357 of SEQ ID NO: 70, or a complementary polynucleotide sequence.

8. An isolated nucleic acid comprising at least eight consecutive nucleotides of nucleotides 1-244 of SEQ ID NO: 69 or nucleotides 1-357 of SEQ ID NO: 70, or a complementary polynucleotide sequence.

9. An isolated nucleic acid comprising at least 80% nucleotide identity with nucleotides 1-244 of SEQ ID NO: 69 or nucleotides 1-357 of SEQ ID NO: 70, or a complementary polynucleotide sequence.

10. The isolated nucleic acid according to claim 9 comprising an 85%, 90%, 95%, or 98% nucleotide identity with nucleotides 1-244 of SEQ ID NO: 69 or nucleotides 1-357 of SEQ ID NO: 70, or a complementary polynucleotide sequence.

11. An isolated nucleic acid that hybridizes under high stringency conditions with nucleotides 1-244 of SEQ ID NO: 69 or nucleotides 1-357 of SEQ ID NO: 70, or a complementary polynucleotide sequence.

12. A nucleotide probe or primer specific for an ABC1 gene, wherein the nucleotide probe or primer comprises at least 15 consecutive nucleotides of a polynucleotide sequence of a) any one of SEQ ID NOs: 1, 4-8, 11-16, 18-26, 28-35, 37-52, 54-65, 68, and 137, or of a complementary polynucleotide sequence; b) nucleotides 3154-3200 of SEQ ID NO: 9 or of a complementary polynucleotide sequence; c) nucleotides 1-242 of SEQ ID NO: 10 or of a complementary polynucleotide sequence; d) nucleotides 1-1851 of SEQ ID NO: 17; or of a complementary polynucleotide sequence; e) nucleotides 152-198 of SEQ ID NO: 27, or of a complementary polynucleotide sequence; f) nucleotides 1-1657 of SEQ ID NO: 36, or of a complementary polynucleotide sequence; or g) nucleotides 1-242 of SEQ ID NO: 53, or of a complementary polynucleotide sequence.

13. A nucleotide probe or primer specific for an ABC1 gene, wherein the nucleotide probe or primer comprises at least 15 consecutive nucleotides of nucleotides 1-244 of SEQ ID NO: 69 or nucleotides 1-357 of SEQ ID NO: 70, or a complementary polynucleotide sequence.

14. A nucleotide probe or primer specific for an ABC1 gene, wherein the nucleotide probe or primer comprises any one of SEQ ID NOs: 119-136, 138, and 141-152, or of a complementary polynucleotide sequence.

15. A method of amplifying a region of the nucleic acid according to claim 1, wherein the method comprises:
a) contacting the nucleic acid with two nucleotide primers, wherein the first nucleotide primer hybridizes at a position 5' of the region of the nucleic acid, and the second nucleotide primer hybridizes at a position 3' of the region of the nucleic acid, in the presence of reagents necessary for an amplification reaction; and
b) detecting the amplified nucleic acid region.

16. A method of amplifying a region of the nucleic acid according to claim 15, wherein the two nucleotide primers are selected from the group consisting of
A) a nucleotide primer comprising at least 15 consecutive nucleotides of a polynucleotide sequence of a) any one of SEQ ID NOs: 1, 4-8, 11-16, 18-26, 28-35, 37-52, 54-65, 68, and 137, or of a complementary polynucleotide sequence; b) nucleotides 3154-3200 of SEQ ID NO: 9 or of a complementary polynucleotide sequence; c) nucleotides 1-242 of SEQ ID NO: 10 or of a complementary polynucleotide sequence; d) nucleotides 1-1851 of SEQ ID NO: 17; or of a complementary polynucleotide sequence; e) nucleotides 152-198 of SEQ ID NO: 27, or of a complementary polynucleotide sequence; f) nucleotides 1-1657 of SEQ ID NO: 36, or of a complementary polynucleotide sequence; or g) nucleotides 1-242 of SEQ ID NO: 53, or of a complementary polynucleotide sequence,
B) a nucleotide primer comprising at least 15 consecutive nucleotides of nucleotides 1-244 of SEQ ID NO: 69 or nucleotides 1-357 of SEQ ID NO: 70, or a nucleic acid having a complementary sequence, and
C) a nucleotide primer comprising a polynucleotide sequence of any one of SEQ ID NOs: 119-136, 138, and 141-152, or a nucleic acid having a complementary sequence.

17. A kit for amplifying the nucleic acid according to claim 1, wherein the kit comprises:
a) two nucleotide primers whose hybridization position is located respectively 5' and 3' of the region of the nucleic acid; and optionally,
b) reagents necessary for an amplification reaction.

18. The kit according to claim 17, wherein the two nucleotide primers are selected from the group consisting of
A) a nucleotide primer comprising at least 15 consecutive nucleotides of a polynucleotide sequence of a) any one of SEQ ID NOs: 1, 4-8, 11-16, 18-26, 28-35, 37-52, 54-65, 68, and 137, or of a complementary polynucleotide sequence; b) nucleotides 3154-3200 of SEQ ID NO: 9 or of a complementary polynucleotide sequence; c) nucleotides 1-242 of SEQ ID NO: 10 or of a complementary polynucleotide sequence; d) nucleotides 1-1851 of SEQ ID NO: 17; or of a complementary polynucleotide sequence; e) nucleotides 152-198 of SEQ ID NO: 27, or of a complementary polynucleotide sequence; f) nucleotides 1-1657 of SEQ ID NO: 36, or of a complementary polynucleotide sequence; or g) nucleotides 1-242 of SEQ ID NO: 53, or of a complementary polynucleotide sequence,
B) a nucleotide primer comprising at least 15 consecutive nucleotides of nucleotides 1-244 of SEQ ID NO: 69 or nucleotides 1-357 of SEQ ID NO: 70, or a complementary polynucleotide sequence, and
C) a nucleotide primer comprising a polynucleotide sequence of any one of SEQ ID NOs: 119-136, 138, and 141-152, or of a complementary polynucleotide sequence.

19. The nucleotide probe or primer according to claim 12, wherein the nucleotide probe or primer comprises a marker compound.

20. The nucleotide probe or primer according to claim 13, wherein the nucleotide probe or primer comprises a marker compound.

21. The nucleotide probe or primer according to claim 14, wherein the nucleotide probe or primer comprises a marker compound.

22. A method of detecting a nucleic acid according to claim 1, wherein the method comprises:
A) contacting the nucleic acid with a nucleotide probe selected from the group consisting of
1) a nucleotide probe comprising at least 15 consecutive nucleotides of a polynucleotide sequence of a) any one of SEQ ID NOs: 1,4-8, 11-16, 18-26, 28-35, 37-52, 54-65, 68, and 137, or of a complementary polynucleotide sequence; b) nucleotides 3154-3200 of SEQ ID NO: 9 or of a complementary polynucleotide sequence; c) nucleotides 1-242 of SEQ ID NO: 10 or of a complementary polynucleotide sequence; d) nucleotides 1-1851 of SEQ ID NO: 17; or of a complementary polynucleotide sequence; e) nucleotides 152-198 of SEQ ID NO: 27, or of a complementary polynucleotide sequence; f) nucleotides 1-1657 of SEQ ID NO: 36, or of a complementary polynucleotide sequence; or g) nucleotides 1-242 of SEQ ID NO: 53, or of a complementary polynucleotide sequence,
2) a nucleotide primer comprising at least 15 consecutive nucleotides of nucleotides 1-244 of SEQ ID NO: 69 or nucleotides 1-357 of SEQ ID NO: 70, or a complementary polynucleotide sequence, and
3) a nucleotide probe comprising a polynucleotide sequence of any one of SEQ ID NOs: 119-136, 138, and 141-152, or of a complementary polynucleotide sequence, and
B) detecting a complex formed between the nucleic acid and the probe.

23. The method of detection according to claim 22, wherein the probe is immobilized on a support.

24. A kit for detecting the nucleic acid according to claim 1, wherein the kit comprises
A) a nucleotide probe selected from the group consisting of
1) a nucleotide probe comprising at least 15 consecutive nucleotides of a polynucleotide sequence of a) any one of SEQ ID NOs: 1, 4-8, 11-16, 18-26, 28-35, 37-52, 54-65, 68, and 137, or of a complementary polynucleotide sequence; b) nucleotides 3154-3200 of SEQ ID NO: 9 or of a complementary polynucleotide sequence; c) nucleotides 1-242 of SEQ ID NO: 10 or of a complementary polynucleotide sequence; d) nucleotides 1-1851 of SEQ ID NO: 17; or of a complementary polynucleotide sequence; e) nucleotides 152-198 of SEQ ID NO: 27, or of a complementary polynucleotide sequence; f) nucleotides 1-1657 of SEQ ID NO: 36, or of a complementary polynucleotide sequence; or g) nucleotides 1-242 of SEQ ID NO: 53, or of a complementary polynucleotide sequence,
2) a nucleotide primer comprising at least 15 consecutive nucleotides of nucleotides 1-244 of SEQ ID NO: 69 or nucleotides 1-357 of SEQ ID NO: 70, or a complementary polynucleotide sequence, and
3) a nucleotide probe comprising any one of SEQ ID NOs: 119-136, 138, and 141-152, or of a complementary polynucleotide sequence,
and optionally,
B) reagents necessary for a hybridization reaction.

25. The kit according to claim 24, wherein the probe is immobilized on a support.

26. A recombinant vector comprising the nucleic acid according claim 1.

27. The vector according to claim 26, wherein the vector is an adenovirus.

28. A recombinant vector comprising the nucleic acid according claim 6.

29. The vector according to claim 28, wherein the vector is an adenovirus.

30. A recombinant vector comprising the nucleic acid according claim 7.

31. The vector according to claim 30, wherein the vector is an adenovirus.

32. A recombinant host cell comprising the nucleic acid according claim 1.

33. A recombinant host cell comprising the recombinant vector according to claim 26.

34. A recombinant host cell comprising the nucleic acid according claim 6.

35. A recombinant host cell comprising the recombinant vector according to claim 28.

36. A recombinant host cell comprising the nucleic acid according claim 7.

37. A recombinant host cell comprising the recombinant vector according to claim 30.

38. An isolated nucleic acid encoding a polypeptide comprising an amino acid sequence of SEQ ID NO: 71.

39. An isolated nucleic acid encoding a polypeptide comprising amino acids 1-60 of SEQ ID NO: 71.

40. A recombinant vector comprising the nucleic acid according to claim 38.

41. A recombinant vector comprising the nucleic acid according to claim 39.

42. A recombinant host cell comprising the nucleic acid according to claim 38.

43. A recombinant host cell comprising the nucleic acid according to claim 39.

44. A recombinant host cell comprising the recombinant vector according to claim 40.

45. A recombinant host cell comprising the recombinant vector according to claim 41.

46. An isolated polypeptide selected from the group consisting of
a) a polypeptide comprising an amino acid sequence of any one of SEQ ID NOs: 71 and 89-102,
b) a polypeptide comprising amino acids 1-60 of SEQ ID NO: 71,
c) a polypeptide fragment or variant of a polypeptide comprising an amino acid sequence of any one of SEQ ID NOs: 71 and 89-102, wherein the polypeptide fragment or variant comprises amino acids 1-60 of SEQ ID NO: 71, and
d) a polypeptide homologous to a polypeptide comprising amino acids 1-60 of SEQ ID NO: 71.

47. An antibody directed against the isolated polypeptide according to claim 46.

48. The antibody according to claim 47, wherein the antibody comprises a detectable compound.

49. A method of detecting a polypeptide, wherein the method comprises
a) contacting the polypeptide with an antibody according to claim 47; and
b) detecting an antigen/antibody complex formed between the polypeptide and the antibody.

50. A diagnostic kit for detecting a polypeptide, wherein the kit comprises
a) the antibody according to claim 47; and
b) a reagent allowing detection of an antigen/antibody complex formed between the polypeptide and the antibody.

51. A pharmaceutical composition comprising the nucleic acid according to claim 1 and a physiologically compatible excipient.

52. A pharmaceutical composition comprising the nucleic acid according to claim 6 and a physiologically compatible excipient.

53. A pharmaceutical composition comprising the nucleic acid according to claim 7 and a physiologically compatible excipient.

54. A pharmaceutical composition comprising the nucleic acid according to claim 38 and a physiologically compatible excipient.

55. A pharmaceutical composition comprising the nucleic acid according to claim 39 and a physiologically compatible excipient.

56. A pharmaceutical composition comprising the recombinant vector according to claim 26 and a physiologically compatible excipient.

57. A pharmaceutical composition comprising the recombinant vector according to claim 28 and a physiologically compatible excipient.

58. A pharmaceutical composition comprising the recombinant vector according to claim 30 and a physiologically compatible excipient.

59. A pharmaceutical composition comprising the recombinant vector according to claim 40 and a physiologically compatible excipient.

60. A pharmaceutical composition comprising the recombinant vector according to claim 41 and a physiologically compatible excipient.

61. Use of the nucleic acid according to claim 1 for the manufacture of a medicament intended for the prevention of atherosclerosis in various forms or more particularly for the treatment of a subject affected by a dysfunction in the reverse transport of cholesterol.

62. Use of the nucleic acid according to claim 6 for the manufacture of a medicament for the prevention of atherosclerosis in various forms or more particularly for the treatment of a subject affected by a dysfunction in the reverse transport of cholesterol.

63. Use of the nucleic acid according to claim 7 for the manufacture of a medicament for the prevention of atherosclerosis in various forms or more particularly for the treatment of a subject affected by a dysfunction in the reverse transport of cholesterol.

64. Use of the nucleic acid according to claim 38 for the manufacture of a medicament for the prevention of atherosclerosis in various forms or more particularly for the treatment of a subject affected by a dysfunction in the reverse transport of cholesterol.

65. Use of the nucleic acid according to claim 39 for the manufacture of a medicament for the prevention of atherosclerosis in various forms or more particularly for the treatment of a subject affected by a dysfunction in the reverse transport of cholesterol.

66. Use of a recombinant vector according to claim 26 for the manufacture of a medicament for the prevention of atherosclerosis in various forms or more particularly for the treatment of subjects affected by a dysfunction in the reverse transport of cholesterol.

67. Use of a recombinant vector according to claim 28 for the manufacture of a medicament intended for the prevention of atherosclerosis in various forms or more particularly for the treatment of subjects affected by a dysfunction in the reverse transport of cholesterol.

68. Use of a recombinant vector according to claim 30 for the manufacture of a medicament for the prevention of atherosclerosis in various forms or more particularly for the treatment of subjects affected by a dysfunction in the reverse transport of cholesterol.

69. Use of a recombinant vector according to claim 40 for the manufacture of a medicament for the prevention of atherosclerosis in various forms or more particularly for the treatment of subjects affected by a dysfunction in the reverse transport of cholesterol.

70. Use of a recombinant vector according to claim 41 for the manufacture of a medicament for the prevention of atherosclerosis in various forms or more particularly for the treatment of subjects affected by a dysfunction in the reverse transport of cholesterol.

71. Use of an isolated ABC1 polypeptide comprising an amino acid sequence of SEQ ID NO: 71 or amino acids 1-60 of SEQ ID NO: 71 for the manufacture of a medicament intended for the prevention of atherosclerosis in various forms or more particularly for the treatment of subjects affected by a dysfunction in the reverse transport of cholesterol.

72. A pharmaceutical composition comprising a polypeptide comprising an amino acid sequence of SEQ ID NO: 71 and a physiologically compatible excipient.

73. A pharmaceutical composition comprising a polypeptide comprising amino acids 1-60 of SEQ ID NO: 71 and a physiologically compatible excipient.

74. A pharmaceutical composition comprising a polypeptide comprising an amino acid sequence of any one of SEQ ID NOs: 71 and 89-102, and a physiologically compatible excipient.

75. Use of an isolated ABC1 polypeptide comprising an amino acid sequence of any one of SEQ ID NOs: 71 and 89-102 for screening an active ingredient for the prevention or treatment of a disease resulting from a dysfunction in the reverse transport of cholesterol.

76. Use of a recombinant host cell expressing an ABC1 polypeptide comprising an amino acid sequence of SEQ ID NOs: 71 and 89-102 for screening an active ingredient for the prevention or treatment of a disease resulting from a dysfunction in the reverse transport of cholesterol.

77. A method of screening a compound active on cholesterol metabolism, an agonist, or an antagonist of an ABC1 polypeptide, wherein the method comprises
a) preparing a membrane vesicle comprising an ABC1 polypeptide and a lipid substrate comprising a detectable marker;
b) incubating the vesicle obtained in step a) with an agonist or antagonist candidate compound;
c) qualitatively and/or quantitatively measuring a release of the lipid substrate comprising the detectable marker; and
d) comparing the release of the lipid substrate measured in step b) with a measurement of a release of a labeled lipid substrate by a membrane vesicle that has not been previously incubated with the agonist or antagonist candidate compound.

78. A method of screening a compound active on cholesterol metabolism, an agonist, or an antagonist of a ABC1 polypeptide, wherein the method comprises
a) incubating a cell that expresses an ABC1 polypeptide with an anion labeled with a detectable marker;
b) washing the cell of step a) whereby excess labeled anion that has not penetrated into the cell is removed;
c) incubating the cell obtained in step b) with an agonist or antagonist candidate compound for the ABC1 polypeptide;
d) measuring efflux of the labeled anion from the cell; and
e) comparing the efflux of the labeled anion determined in step d) with efflux of a labeled anion measured with a cell that has not been previously incubated with the agonist or antagonist candidate compound.

79. A method of screening a compound active on cholesterol metabolism, an agonist, or an antagonist of an ABC1 polypeptide, wherein the method comprises
a) culturing a cell of a human monocytic line in an appropriate culture medium with a purified human albumin;
b) incubating the cell of step a) simultaneously with a compound that stimulates the production of IL-1 beta and an agonist or antagonist candidate compound;
c) incubating the cell obtained in step b) with an appropriate concentration of ATP;
d) measuring IL-1 beta released by the cell obtained in step c) into the cell culture supernatant, and
e) comparing the released IL-1 beta obtained in step d) with IL-1 beta released into a culture supernatant of a cell that has not been previously incubated with the agonist or antagonist candidate compound.

80. An implant comprising the recombinant host cell according to claim 32.

81. An implant comprising the recombinant host cell according to claim 34.

82. An implant comprising the recombinant host cell according to claim 36.


SEQUENCE LISTING

<110> DENEFLE, Patrice
      ROSIER-MONTUS, Marie-Francoise
      ARNOULD-REGUIGNE, Isabelle
      PRADES, Catherine
      NAUDIN, Laurent
      LEMOINE, Cendrine
      DUVERGER, Nicolas
      JAYE, Michael
      SEARFOSS, III, George
      REMALEY, Alan
      BREWER, H. Bryan
      DEAN, Michael

<120> NUCLEIC ACIDS OF THE HUMAN ABC1 GENE AND THEIR
      THERAPEUTIC AND DIAGNOSTIC APPLICATION

<130> A3738-EP

<140>
<141>

<160> 156

<170> PatentIn Ver. 2.1

<210> 1
<211> 11754
<212> DNA
<213> Homo sapiens

<400> 1
ccattccacc tagtggggcc atttcctacc tgagtcaccc gtggtgtcaa atagaatgtc 60
atgtggcctc ctgagttggg tagaattggc tgctcatctc aaccccgcta ctgactatct 120
ctgtgattta cccttcctcc agccttagcc ttgctacata taaaatcaag acaataatgt 180
ttcctatctc acagggttgt cctgaggatt aaattaagta attaatataa aatgtgcctt 240
gtacatattg ggccctaaat aaacagtagc tactatttat ccttaaagta caaatggtag 300
tttcagagct tcaaggctga tggctattta tcttactcat actctttgtt tagcttcatt 360
tttttcccct aatttcatta gtattttctt ttcttttttt tttttttttt tttttttttt 420
tttggaggtg aagtctcact ctgttgccca ggctggagtg caatggagcg atcttggctc 480
accccaacct ctgtctcctg ggttcaaaca gttctcctgc ctcagcctcc cgagtagctg 540
ggattacagg ctcccgccac catgcccagc tatttttttg tattttcagt agagatgggg 600
tttcaccctt ttgaccaggc tggtcttgaa ctcctgacct catgatcaac ccacctcagc 660
ctcccaaagt gctgggatta caggtgtgag ccaccacgcc cggcctcata agtattttct 720
aaatttattt acagtcatgc catttaaaag gaaagttgta ttcctgtctt tgttaatatt 780
tataagtgat tttattcagc tacaagcttg gaatggcata taattttgta ttctgctttt 840
ttcacttaat attacatggc taatgatttc tgtgtttcat aaacattatt ctgatgatgg 900
catgatatat tgttgagtac atgtaccata attgaatcat ttccctattg ctatgcaatt 960
aagttgtttc caatattttg caattataat gtttcaatga atgaataact ttatgcatat 1020
agctttttga tatcttaagt tcagtttcct aggatgaatt tccaggaata gtaattgggc 1080
aaatgggata aacatgactc ttgaatacgt attgttaaca ttgctttccc aaagggctca 1140
actgatttat atttccgtgt tcattatctt ttaaaccagc tcatttactc accaaacatt 1200
tttaaagcca ttatcatgtg gtaggcttag taagaagaaa gtgaccctaa gggagaagct 1260
tatatataaa tagggtccct ggtgtaccaa gtgctgatac agacacaaag tacctgggga 1320
aattgagatg agggagtcct ggctcagctg ggagaaaagt tcattttcat agagtcatgg 1380
ttttgttctt tggcagaaag aaaattgctt tcttccccac ccccaccccc agctttattg 1440
aggtataatt gacaaataaa aattgtatat ctttaagata tgcaatgtga tatatatgta 1500

tatctcaact taaaaaataa gctacagaat aaaaaggtgt ttgctattaa aaaaaaagaa 1560
aaggctgaat gtcattccca agcttggaaa tttgagtatg ttgcctcttt gggattattt 1620
acagaaatat tagcaagacc agccccatct ttggtcttga gtactccact gtcagcatgc 1680
tttcttccag agagggatcc atttgccttt atttttcatt ctgttgtgcc gtctatgcaa 1740
actattcttg atagttttat ggtaacagtg tttttttgtt ccatgagata atttatacat 1800
gctcattgtg gaaaatttag aaaagacagg aaagtattaa aaacatcact tttttttttt 1860
tttttttttt ttttttttta agagacagag tcttgctctg tcgcccaggc cggagtgcag 1920
tggcgtgatc tcagctcaca gcaacctccg cttcccaggt ttaagtgatt ctcctgcctc 1980
agcctcccaa gtagctggga gtacaggcat gcaccaccac gcccggctaa ttttgtattt 2040
ttagtagaga tggggtttca ccatgttggc caggctggtc tcaaactcct gacctcaggt 2100
gatccgcctg ccttggcctc gcaaagttct gggattatag gcaggagcca ctgcgccagc 2160
cacacctacg ttcttatcat cctagtacat ccactgtcat tatcttgctg tatttccttc 2220
tgcccagtct cactctgatc atgcagtggc gtgatcatgc agtgatctcg gctcactgca 2280
acctaggcct tctgggttcg agtgattctc ctgccttagc ctcctgggtt caagtgattc 2340
tcttgccttg gcctcccaag tagctgggat tacaggcata cacccccatg cccatctaat 2400
ttttgtattt ttagtagaca cagcgtttca ctaaaatttt gtatttttag tagagatggg 2460
gtttcaccat gttggccagg ctggtctcca actcctgacc tcaggtgatc cgcctgcctt 2520
ggcctcacaa agtgattaca ggcatgagcc actgcatcca tcgccaaaaa gattttttaa 2580
aagagtttaa tgtagaacca tatcaaaggt ctttggaaat aaaaaacagt tttttaaaaa 2640
tatcagaaat aaaacaacaa ataaataaat aaataaaaaa cccaaaacaa tctgaagcac 2700
gagcacctag cagaaaggtt caattatgat ctattcatag agtggaatat caagtagaca 2760
ttacaggaca tgttttaaga ttatatttta tgtcatggga aatgctctcc cagtatgatg 2820
ttaaatgaaa aaacagaata caaaagtata tatgctgcat agtctcaata ttgtagagaa 2880
aaaatattat ttatgtatgc atgaaaaaag acaaaagatg ttaacagaga tccattgtta 2940
cttcagttta ctagggattg tctctgggag gtaggattaa ggtgatttat atttaccttt 3000
ttaaactttt ctgtattttt ttattttcaa attttccata aaaatataag gacttgaaga 3060
tcaagaaaaa atttctgctt tggctcagtg cagtggctca cgcctgtaat cccagcagtt 3120
tgggagccct aggggagagg atcacttgaa cccaagagtt tgacgttcca gtgagctatg 3180
atctccggat cgtaccgcct ggacgatgga gcaagaccct gtctcaaaaa aaaaaatctt 3240
tgcttttttt ttttgtttgt ttttgagacg gagtctctct ctgttgcccc agctggagta 3300
cagtggcaca atctcagctc accgcaacct ctgcctcctg ggttcaagcg attctcttgc 3360
ctcagcctcc caagtacctg ggattccatg cacccaccac tatgcccagc tacttttttg 3420
tattttcagt agagacaggg tttcaccatg ttggccaggc tggtctcgaa ttcctgacct 3480
cagctgatcc accggccttg gcctcccaaa gtgctgggat tacaggcatg agccactgtg 3540
cccagcccaa tcttttgctt tttttaaaaa aagaagacaa aaagggattt tataccagta 3600
ttatcttggc tgtgtgactc tgaagccaca gttgtaagtt ataattactc tgaaacacaa 3660
ggccctgtga ctcttttggg ctctttggtg tttatcttga ttacaacgtt ggaatataga 3720
aatgaaagga atgggagagg tgatagactt caggcagtgt aactagttgt ctgaacacta 3780
ctggctcaat tatattgtgt ctagtgattt ccatcttgtc cgtctgctaa tttatcgcct 3840
ggtaactcac tgaggcaggg ttttcctttg gagaaacctc attgttttaa ccagtgtatc 3900
atgcttgttt agaagttcaa tgatcttttt aactcatcgg agaagatgat gaccagacct 3960
ggacagatgg ggaaggactt tgcactctct ctttacagtc ctgagtgcac acaggtcaat 4020
atggaactat gtgtgaattt tcattgtctt tgagagccct cttctctgcc ccatagggag 4080
cagctttgtg tgcaattaga ggagcaaggg ttgtgtgtat ttagcacagc aggttggcct 4140
ggtcctctcc tctcaacata gtcaccacat acctggcact atgctaaggc tgggaatgca 4200
gacagatggg tgcctgcttt cagagtgctc aatgtgctga ggaagccagc aacagaaaca 4260
gatgatttca ggagctccag gaaaatgcta caggaggagt gtgcctgggt tactggagta 4320
gcacaggagg agggcttcta gctcaggctg agattttagt aaaggaaatt atgccacgat 4380
gaatcctgaa gaatgaatag aagtgaacca gataaagcac gataggaagc atcttccctt 4440
acctaaggga agacacagag gtatatggaa tggtatgtta aaaggttggg actccaaaca 4500
gttctgttaa agcttagaga gtggtgggag agactggaga agttgattaa ttagtaaatg 4560
aagttgtctg tggatttccc agatcccagt ggcattggat atccgtatca ttttaaatta 4620
cagtgttcta tcttatttcc actcagtgtc agctgctgct ggaagtggcc tggcctctat 4680
ttatcttcct gatcctgatc tctgttcggc tgagctaccc accctatgaa caacatgaat 4740
gtaagtaact gtggatgttg cctgagactc accaatggca gggaaaatcc aggcaattaa 4800
cgtgggctaa attggacttt tccaaagatg ctgtctttgg gaaacatcac acatgctttg 4860
gatcagaaaa cctaggcttc taatttgttg ataaggcatg aactcaggag actgttttca 4920
gtcctagtga atggtgataa ttgtaattat aacagtagac aacatctctt ttacacattt 4980

taaatcatga aaatagaata accttactga taattttaga aagtggtgat taaaagcaca 5040
tttaagataa tgccttaaca cctagtcttt tccatatgca tgatgtctta atcacacatt 5100
gcaaatcatg gaacacagaa ttttaagcag catttgtgta gaacttctca gttttactaa 5160
tattatttta ttttattctc ataacaacct tgaatagaac tcagatcatc tgtcaatcat 5220
gtattttgat aacagccttt acagtgagca tagaaaatac agtagtggct aacaacacag 5280
gctccagatg tcaggttatc tgggtatgaa ttctggtgtc agcattcact aagcatatga 5340
ccttggacaa gtgatttaag tttcttttaa acagagaata gtaataccta cctcatatta 5400
ttattgtcag tgtatcatct tacaatcaca gtctttctct tagggctggg ctcagtgggt 5460
ggattgacac tgcagaaatg gccagatcta aaggatcaac atttacgtag ctgggaaatg 5520
tagctgggac ttcagtttca ctgcgctagt gatttttcct accactaagc agctcagtcc 5580
atacccctac gagacccaca agcttatgag atactgttct tccaggaaag cagtggggcc 5640
agggccacct tttaattgtg tttcttggcc tggtcccatc tttctcacaa tatatagcaa 5700
cagttattta cttgctgatt ttctaatgca catcacacat agtcatatta aacacacaca 5760
cacacacaca cacacacaca cacccctcaa gaaacatttt ctgagacgtg atttcctgat 5820
ttcatcaaaa aagaaaagag cgggccaggc acagtgggaa gtcaaggtgg gtggatcact 5880
tgaggtcagg agtttgaaac cagcctggcc aacacggtgg aacctcgtct ctactaaaaa 5940
tacaaaaatt agccaggcgt ggtggcgcac acctgtaatc ccagctactg gggaggctga 6000
ggcaggagaa ttgcttcaac ctgcgaggct gaggttgcag tgagccgaga ttgcgccatt 6060
gcactccagc ctgggcaaca gagtgagact ctgtctcaaa aaaaaaaaaa aaaaaaaaag 6120
cataaactga aatttatatg caatttatat gcctgtgaga taattctgtt ttctcttttg 6180
gaaccccaaa gagatttttt tgattgatga gcaaatacat tttagatttt atttaagcat 6240
tatgccaagc accactgaag tataagtttc aagggcaaac tcagtttttt catctactag 6300
acgaatgatt ttctggaatg attacaagca ggcaagatgg tgtagtggaa atagcaaatg 6360
tcttcggcat cagacaagtt ggggtttgtt tgtatcctgc ctctgccctt caccgaggtt 6420
gtgatcttgg gcagattgtt gagttttaac ctagattcct ctgactccag atcataaatt 6480
ttcagaaaag ttctgaaatt cttgtatata ctgatggtaa atgagacttt tccttacatc 6540
tatgcacttc tttgtttgtt tgttttgaga tggtcttgct ctgttgccca gactggagtg 6600
cagtagtgca atctccgctc actacaatgt ctgcctccca ggttccagtg agcctcctgc 6660
ctcagcctcc caaatagctg agactacagg catgtgccac cacgtccggc taatttttgt 6720
atttttagta gagacagggt tttgccatgt tgaccacact ggtctcgaac tcctggcctc 6780
aggtgattcg cccgcctcag cctcccaaag tgctgggatt acaggcatga gccaccatgc 6840
ccggccatat ccatgcactt cttgcaacct taccttcttt tctcatcacc ctccagggac 6900
ctagttggaa gagcagagtt aaaagttaag gtgaaacttg gagaggtgtc ttgtccctag 6960
gaacaaagga ctggtttgaa attctctgta aatcttcccc agttcaaacc agagttatca 7020
aggtcttaaa aacttccctg ggtcctgaga gcccattata ttatttactt gtcttcctgt 7080
acacccactg cctagtcctg atcctacttt tgtttgcaaa taggatgggg cacaacgtac 7140
aaggaagggc ctttgccacc cctgctaagg gataacctga aataccttca ccatcactgc 7200
cctgtgctgc ttttcaccta tgccagtctg tctacagtgc cagtgtctcc tggcattgaa 7260
aggggagaat cttttggtcc tttgagtatt tggttgggtt acataaatct ccctgaatga 7320
agagcagctg acttaggcaa ggggccttgt ttggttttcc ttgaactatt aacaggaaga 7380
tagggagatt aactgtgtaa atgttcaata ggccagagtc cctgcagagg gtggccacag 7440
tgatcagatc ttatcacatc cttgctttgg gtgttgcctc tctggttgga gtatggatag 7500
aaaagaaaga aagaccctat attgaaatgc aaagtgcagc aagtcctgac tttggattaa 7560
cttctcagcc catttgcatg aaaataaaaa gatgaataaa acaaggttcc cactttggag 7620
ggaggtggta gctgtgagat ggaaggagtg ttcctgctgg gcaacagcag agtaagtgct 7680
ggggtagatt cactcccaca gtgcctggaa aatcctcata ggctcatttg ttgagtcttt 7740
gtcctacacc aggcactctg caaaaacgct ttgcctgcaa ggtctcatgc gatgctcacc 7800
acagctctgt gaagttaatt gtacttttat caccatttta cagatgagaa aactgagggt 7860
atggggtcaa tgacttggct aaagtcactg cttagcaagc tgcagggact ggatgtgaat 7920
tccaattggt ttgactccaa agcctgtgaa gctacttgtt cttcaccacc tagagctgtg 7980
gttcttgata actgtgaact cttttggggt cacaaatagc cctgagaata tgatagaagc 8040
aggagctctg gcctttctgt ccatacctga acaggtcctt gggttaagag cccctcgtcc 8100
agggcctatt aatcttgatc ctcataagca gcatccatgt ataaaggccg caaaccaaac 8160
tgtgccagac cgaatcctag gaccaagccc aaatatgtcc catcatcctt ttggtaagaa 8220
gctcattgta agaaagaaag aggagagcaa gaggatgacc tagtgcatgg ggcctcattg 8280
ttttaattag tgacaaaaca acaataataa caacaaaacc cccgaagctt cacagatgac 8340
atcagacccc aagcctgtgt gtttttcagg tgcccttgag gagctttgta gctggcagag 8400
gaggtgaaac tgacaaatgt ttggcagatg gaggagagta ccagaggggt ttgagatgag 8460

ctaaattcca atctaaccgc aggtgtgagg aagaggcttg gattgggacc atggagatgg 8520
gggttctact cccagtcacg ccagctgact ttgcgagtgt tctttgtcag tcactttatc 8580
ttattttatt tatttttatt tttttgaaat ggagtttcgc tcttgtcgcc caggctggag 8640
tgaaatggcg cgatcttggc tcactgcaac ctccccctcc tgagttcaag cgattctcct 8700
gcctcagcct ccagagtacc tgggattaca ggcgcctgcc accaagccca tcgaattttt 8760
gtatgcttag tagagacagg gtttcgccat gttggccagg gtggtcttga actcctgacc 8820
tcaggtgatc cgcccacctt ggcctcccaa agtgctggga ttacaggcgc gagccactgt 8880
gcccagccca cttcatctta ccgtagttac ctccttagag tatgaaaaaa taggcttagg 8940
gcatccccaa gtcccctcta tgtctgagag ctgaggctgg ctgtcaaaga ggaactaagg 9000
atgccaggga ctttctgctt aggacccctc tcatcacttc tccaacgctg gtatcatgaa 9060
ccccattcta cagatgatgt ccactagatt aagaatggca tgtgaggcca agtttccacc 9120
tgagagtcag ttttattcag aagagacagg tctctgggat gtggggaatg ggacggacag 9180
acttggcatg aagcattgta taaatggagc ctcaaaatcg cttcagggaa ttaatgtttc 9240
tccctgtgtt tttctactcc tcgatttgaa caggccattt tccaaataaa gccatgccct 9300
ctgcaggaac acttccttgg gttcagggga ttatctgtaa tgccaacaac ccctgtttcc 9360
gttacccgac tcctggggag gctcccggag ttgttggaaa ctttaacaaa tccatgtaag 9420
tatcagatca ggttttcttt ccaaacttgt cagttaatcc ttttccttcc tttcttgtcc 9480
tctggagaat tttgaatggc tggatttaag tgaagttgtt tttgtaaatg cttgtgtgat 9540
agagtctgca gaatgaggga agggagaatt ttggagaatt tggggtattt ggggtatcca 9600
tcacctcgag tatttatcat ttctgtatgt tgtgaacatt tcaagtcctg tctgctagct 9660
attttggaat atactatatg ttgttaatga tatcatgcag cagacgtgca tctgaatggg 9720
ctggctctag gagctagagg gtaggggctg gcacaaagat gcatgctgga agggtccttg 9780
cccataagaa gctgacagcc aaggctaggg gagttctgtc ttctctgcat caggtcacct 9840
ctctcacctc tgtcactgcc ccatcagact acaatgtctg caggtctttc tcccctgagt 9900
gtgagctccc tgagcaaagc aggatgctgc cccttccctt tgtattcctg gctcctggct 9960
tcagtgcctg gacataagta tgggcataat aagtgtcccc caaatgagac attgaggatt 10020
cttcaaatgc acaggaccgt gatgtgagtt aggacggagt aaggacgatg ggatgtggct 10080
caggacaatc ctgaggaagc tgcagctgcg gcacgcaggg ccacactgtc atgttcatgg 10140
accctagact ggctttgtag cctccatggg ccccttccat acacaaatat taaaaattat 10200
atttcatgac tgcattggta taaagatgaa tataatccag accagattca tgattattca 10260
tacattttta gtgtattaac ttttaattct gcttttaaaa taaattaaaa cattctaata 10320
tgcccttaag agtatcccag gcccaggcca ctgagcctac tgtggttcat ggataagttg 10380
gccctggggg catgtgtgtg catgcatgtg tgtgcacatg catgatgagc cgggccttga 10440
agggtggtaa gatttgggtg tgtagaccaa tggagaaagg catttggggc agtgatgatg 10500
ggtgggggag ggaacatggt gatgaatgga gctgggtgtg gggagccatg ggagtgggtt 10560
agggccagcc tgtggaggac ctgggagcca ggctgagttc tatgcacttg gcagtcactt 10620
ctgtaaagca gcagaggcag ttggcctagc taaagccttt cgccttttct tgcacccttt 10680
acagtgtggc tcgcctgttc tcagatgctc ggaggcttct tttatacagc cagaaagaca 10740
ccagcatgaa ggacatgcgc aaagttctga gaacattaca gcagatcaag aaatccagct 10800
caagtaagta aaaaccttct ctgcatccgt ttataattgg aaattgacct gcaccaggga 10860
aagagagtag cccaggtgtc tggggcttgt tcccattaga tcttccccaa ggggtttttc 10920
tccttggtgg ctggcctgtg gggcccctct ccaggaggca ttggtgaaga aactagggga 10980
gctggttgcc acagacagtg atgtactaat cttctctggg aagacagaag aaaagtcccc 11040
agggaagaat actacagact tggccttagg gacagctagg ggtgcagatt gctgccaact 11100
gcattttttc tgaagttggc catatggttg cagtgaatgg atttatagac agagtatttc 11160
tgtgcatata agagcaatta cagttgtaag ttgatatgga taagtgaaag ttaagcactt 11220
ctttctaaaa agagaatgca attcattttc ccctaatcat ttcaattagt ctgatgggca 11280
tttgaacttg ttgtctttaa aaagtgaaat ctttacctct gatctggtaa gtatccaggc 11340
aatttcttgt gtgccaccca ggaggtatct ggggagtggg cattttctga ctgaggcatt 11400
ggctgccata gcatcagagc agccttccag gcagtggcct ggcaagggga cagaggctgg 11460
tgggagcagc tggctgagtg cagccagtaa tggcatgtgc atggtctgta gagaatgtag 11520
aagcaataat gaagccgata aaagctggtc tgcattttat tattatcatg cgccggtggt 11580
tctaaacaat gtcagtgata aattactcct ccccatcatg gaccaatggc tgccactgct 11640
ccagggaagt gctttttatt ccgtttggtg tttagggagg gatggagttg gctggccttt 11700
gctgaaaggc ctaccagttt gttttctatt tggcaaaaga agaaatgata aagt 11754

<210> 2

<211> 21
<212> DNA
<213> Artificial Sequence

<220>
<223> Description of Artificial Sequence:
      Oligonucleotide Primer

<400> 2
atttaggtga cactatagaa g                                                21


<210> 3
<211> 446
<212> DNA
<213> Artificial Sequence

<220>
<223> Description of Artificial Sequence:
      Oligonucleotide Primer

<400> 3
cttgtttttt ccccggttct gttttctccc cttctccgga aggcttgtca aggggtagga 60
gaaagagacg caaacacaaa agtggaaaac agttaatgac cagccacggc gtccctgctg 120
tgagctctgg ccgctgcctt ccagggctcc cgagccacac gctgggggtg ctggctgagg 180
gaacatggct tgttggcctc agctgaggtt gctgctgtgg aagaacctca ctttcagaag 240
aagacaaaca tgtcagctgc tgctggaagt ggcctggcct ctatttatct tcctgatcct 300
gatctctgtt cggctgagct acccacccta tgaacaacat gaatgccatt ttccaaataa 360
agccatgccc tctgcaggaa cacttccttg ggttcagggg attatctgta atgccaacaa 420
cccctgtttc cgttacccga ttctgg                                          446


<210> 4
<211> 571
<212> DNA
<213> Homo sapiens

<400> 4
ttcntggttc cgtttttttc ttcctttcag gtgtcgtgaa aagcttgaat tcggcgcgcc 60
agatatcaca cgtgccaagg ggctggctca ctgatatggc gatgctcccc tatctcattc 120
ctgtgtgtgt ttagccaggc aactgttgat catcaatatt atgataacgt ttctccactg 180
tcccattgtg cccacttttt tttttttttt gaggttactt actaaataaa aataaaacac 240
tatttctcaa tagacttgaa gcttcaagat ttcctggtgg acaatgaaac cttctctggg 300
ttcctgtatc acaacctctc tctcccaaag tctactgtgg acaagatgct gagggctgat 360
gtcattctcc acaaggtaag ctgatgcctc cagcttcctc agtagggctg atggcaatta 420
cgttgtgcag ctactggaaa gaaatgaata aacccttgtc cttgtaatgg tggtgaaggg 480
gagggaggta gtttgaatac aacttcactt aattttactt ccctattcag gcaggaattg 540
ccaaaccatc caggactcca atatgcaacc t                                    571


<210> 5
<211> 428
<212> DNA
<213> Homo sapiens

<400> 5
gtcagtaaag agacctgatg cattggactg ttgcaatgga acctgtttta agatcttcaa 60
agctgtattg atatgaagtt ctccaaaaga cttcaaggac ccagcttcca atcttcataa 120

tcctcttgtg cttgtctctc tttgcatgaa atgcttccag gtatttttgc aaggctacca 180
gttacatttg acaagtctgt gcaatggatc aaaatcagaa gagatgattc aacttggtga 240
ccaagaagtt tctgagcttt gtggcctacc aagggagaaa ctggctgcag cagagcgagt 300
acttcgttcc aacatggaca tcctgaagcc aatcctggtg agtagacttg ctcactggag 360
aaacttcaag cactaatgct ttcggaatgt gaggcttttc cttggacagc atgactttgt 420
tttgtagc 428

<210> 6
<211> 213
<212> DNA
<213> Homo sapiens

<400> 6
tgttaaactc aagattgtga acgagtttta attcacttgt ttatttccaa tagagatttc 60
aggtttacat ttgaattcag aaacaaagtt ttctttctca ttacagagaa cactaaactc 120
tacatctccc ttcccgagca aggagctggc cgaagccaca aaaacattgc tgcatagtct 180
tgggactctg gcccaggagg taagttgtgt ctt 213

<210> 7
<211> 1402
<212> DNA
<213> Homo sapiens

<400> 7
ttcattgcnt gacactttac tgagaccaag atagggaact tcacatacca gaccttttct 60
cccaaggcgg aagagggctg ttcaatttct acactagagt tcggggagtt ttagaaatga 120
gccagttgtc gaggatgaga gcagttcctg ataggctcaa ccacaatgag atgtagctgt 180
tcagagaaag cattcttatc tataaactgg aagataatcc cggtgaaaca aagcccagcc 240
ccaggggctt cactaactcc aggctgtgct tctcaaactt tagtgagcat aggaatcacc 300
tgggcatctt gtgaagctgt agatttgaat tctgcaggtc ggcagagggg tctcagaatc 360
cgcatttcca acaatgtctc cagtaatgct gatgctgctc gtccctggac cacagattgg 420
gtagccaggt tctggcaagc tcatcccaag gctttgagat gacatcagac aaaatatgtt 480
ctgggacatg gcttttgaga ggtcaagaaa ataagatgtt tctttctctt ctcatcccca 540
acccttgcac tgcccttttc tcccttcccc taccctcctt tctgtcccca tccctgacgc 600
cagctgttca gcatgagaag ctggagtgac atgcgacagg aggtgatgtt tctgaccaat 660
gtgaacagct ccagctcctc cacccaaatc taccaggctg tgtctcgtat tgtctgcggg 720
catcccgagg gaggggggct gaagatcaag tctctcaact ggtatgagga caacaactac 780
aaagccctct ttggaggcaa tggcactgag gaagatgctg aaaccttcta tgacaactct 840
acaagtgagt gtccatgcag accccagccc tgtccccaac cccatccctc ccttagttct 900
ggccttggcc tgtgtcatct cctccctctg tagcagcgtt agatgtctac atgcccattt 960
gcccaccaga ctgagctctt cctagaggag agaggcttct cttgaatagc tacctgtccc 1020
cagttctctg aatgcagcct ggcacatctc aggtgcacag tagtgtttat caatggaatg 1080
aatgattgac agccaacctt ctggttttct gggggatgtg gaagggtggc ttccagggtg 1140
atcaagaatg agataatggc agaaggacaa atcctgcaag atctcactta tatatggaat 1200
atatgtaagg tagaaagtgt cagtttcaca tgatgaataa gttcctggga tcttgatgta 1260
catcgtgatg actatagtta gtaacactgt atagtatact tgaaatttgc taagagagta 1320
gatccgaagt gttcacacta cacaaaaaag gcaactatga ggtgatggat ttattaacaa 1380
gcttggattg nggtgatcct tt 1402

<210> 8
<211> 1808
<212> DNA
<213> Homo sapiens

<400> 8

cctantttgg tatttttaga aaagacaggg tttttccatg ttggtcaggc tggtctcgaa 60
ctcccaccct caggtggttc gcccgccttg gcctcccaaa gtgctgggat tgcaggcatg 120
agccactgcg cccagcccca aattttggtt tttgcttgaa aactgaggtc tgaattcagc 180
cttctggttg cccctcaaga gtcagtttaa atgttggtca tgttagttgt cagtgaaaac 240
aatggtgagg ctggcatgag agtgtgaacc tggatgggag ggcttgtgct tcacgaaaac 300
atttttccag atcagctcag tcgtgagtta tccgtcattg acgttataat aagctctgat 360
tatttatcaa gcatcattct ttatagatat ctcagtttaa tctgagataa tcttctccac 420
atctctccac atagatgtta tgaattttac ttttacagag gagccaactg aggctcagat 480
aagttactta ttatatgact agtagtggna gagctggggt ttcaaactaa gaactntctg 540
gctccaaagc ccttgtaagt ttctatcagt atatgaccat gcatatgagc atttgtctct 600
cctcttcttc atagctcctt actgcaatga tttgatgaag aatttggagt ctagtcctct 660
ttcccgcatt atctggaaag ctctgaagcc gctgctcgtt gggaagatcc tgtatacacc 720
tgacactcca gccacaaggc aggtcatggc tgaggtaagc tgcccccagc ccaagactcc 780
ctccccagaa tctccccaga actgggggca aaaaactcaa ggtagcttca gaggtgtgcg 840
ctaagtatac tcacggctct tctggaattc ccagagtgaa aacctcaagt ctgatgcaga 900
ccagagctgg gccagctccc cagtcgtggg tatagaatca tagttacaag caggcatttc 960
ttggggatgg ggaggactgg cacagggctg ctgtgatggg gtatcttttc agggaggagc 1020
caaagcgctc attgtctgtg cttctccttt ttctgcggtc cctggctccc cacctgactc 1080
caggtgaaca agaccttcca ggaactggct gtgttccatg atctggaagg catgtgggag 1140
gaactcagcc ccaagatctg gaccttcatg gagaacagcc aagaaatgga ccttgtccgg 1200
gtgagtgtcc ctcccattat taccatgtgc ctgcttgata ctggagaggt gagtttctgg 1260
tcactttccc aggtgtgagt gaggtgagaa ttctttcagt ttatctagct gggggaaatt 1320
gtagtgagca tagctaaaag tcacatgggg caccaacctc tccatgaagt acaggccatg 1380
gtgcaagaga taacgcntgt gcatatnagc catcccatgc ccacntcanc ggtccaaata 1440
agcagttttc tgcaaaactt agtgagggct ggtgtttgga agtggagttg agtaattgca 1500
gtaccctatt ttcctttttg gctgcagcct ctcagccagc cacagcatct ccctgtgtct 1560
tggtaggttt tggaaagaag tgtgggagca aaagcatgat gttacatgta gactggcctg 1620
agatactcat tctcagggca ctgtgtgaat gatgagctgc tgttactgtg tggaggggaa 1680
atgcacttag tgcttcagag ccacttgaaa gggataagtg ctctagagac aattggattc 1740
aaatgtggag caggctgagc aagaacagaa tgtctccttt gcctgagcct gagtgctggt 1800
aatcacat 1808

<210> 9
<211> 3215
<212> DNA
<213> Homo sapiens

<400> 9
acatggcaat ggcattcatt aggaatctag ctgggaaaat ccagtgtgta tgcttggaaa 60
tgagggatct ggggctggag agaaaggcat gggcatgcct tggagggact tgtgtgtcaa 120
gctgaggacc tttactttaa gctctagggg accaggcaag gggagatgta gatacgttac 180
tctgatgggg tggatgaatt gaagaaggat gaggcaagaa tgaaggcaga gaccagggag 240
gaggctctcc aagtggccaa ggcataaagc aagaaatgag gcctggtgac tgcttagtgg 300
cagagcagtg aaagagaggg aggcatcaaa gtgagtctcg atttctagct gggtgggtgg 360
tagcgatgtc cagtaggcca gtggctactg aggtctgcag tggaggaggg tggttgggct 420
ggagacagat gatgagggag tcatcagcct gtgggtggaa gaaaagggaa cctcttccaa 480
ctgttttctt tgcttcttcc ctctctttct cttttttttt ttttttggac agagtcttgc 540
tctgtcaccc aggctgaaat gcagtggcat gatcttggct caccacagcc tccgcctcct 600
gggttcaagc aattctcctg tctcagcctc cagagtagct gggattacag gcacatatca 660
ctgtgcccgg ctaatttttg tattttcagt ggagatggga tttcaccatg ttggtcgggc 720
tggaatgaac tcctgacctc aagtgatcca cctgcctcag cctcccaaag tgttgggatt 780
acaggcattg agccaccgcg cccggccttt cttccctctc ttaaagagtg tttatttaat 840
tccacaaaca tgagcttgtc accccctgta gcctggcatc tcctacacga ggtgatggct 900
gaggcttctg cttctgctgg ggtagctctg atctttctgc tttctctggc actgtctacc 960
catgttgcct caccccacag gtcccagggc acctctctcg ggcaagtctt ggaaccctct 1020
gacactgatt tgctctcttt tctgagctgc ttttagccac ccatcctcgg gacctgtttt 1080
ctctctgcct ccacccctgc gggcagtctt aggtctcctg cccctcacga gcaccccaga 1140

gaggccacgt gctcagtgat ctcagtgggc gcatctttct agtcttgcta ttctttttgg 1200
ccatgttgtt cagaaaccat actgggcagg gccgacttca ccctaaaggc tgcgtctctt 1260
cactctgctt ttgtttgttc caaataaagt ggcttcagaa ttgctaaccc tagcctctgt 1320
gaacttgtga ggtacaattt tgtgtctgtt atgttaacaa aaatacatac ataccttcct 1380
ggtgatggta taaattgcta ttctctattg gaaagcaatt tggaatgaaa atttaaagaa 1440
ccattttaaa atatgctatc ctgcgtacct ccattccacc cacccccagg gatgtagcct 1500
actgaaataa ttttaaagaa gtcaccatat gagagaaaat gttattgcta tattgttatt 1560
gtgagaaatt ggaaatagac taaatgttca gcactatagg aataattaat gaaattacat 1620
atactctata caatcattat gctgccattg aaataataaa tacaaaggcg caagggggga 1680
aaagcttata atgttagtga aactaagact gattttttta taaagcagca gttttcagac 1740
ccttggagac tccaattcgg tagaaccaga gcttcatctt ctctgtcgaa gctgtgacag 1800
gagttgcaaa tgcctctcct ttttgctgag tttgcagctg ctgtttttcc ggcagcacat 1860
ctgtgcaggc ctctgcctcg gcccctctgg atctgctgat tgagcagcgg attgatctgt 1920
ccttctcttt cgtgttgacc catgtgagga accaactggc aagggaacaa gaaatggaaa 1980
taggcctcct ttgcatcatg acctgtacat cctgcaattg gaaaagattg tactttagtt 2040
ggtttaacca gcagcattat ttttctaaac taagcagtaa gaaggaatta ggttttatgt 2100
gggatcaaca gactgggtct caaaagagga aggtgataga acacagtggg gagggggagg 2160
tgcactagaa acagagggcc tatgctttca ttctggcttt gctacttaat agctgtgtga 2220
cccaatctta gagacttaac ctctctgaac ttccattttc tcatgtataa aatgggaaat 2280
attaaaggat actcactggg ctggtggctt gtgcctgtaa tcccagcact tggggaggtt 2340
gaggtgggag gatcacttga gcccaggtgt tcaagaccag cccaggcaac atggcaagac 2400
tctgtctcta tgaaaaaatt aaaaattagc caggtgtggt ggtgtgcacc tgtagtctta 2460
gctacttggt aggctgagat gggaggatca cttgggcttg ggaggtcaag gctgcggtga 2520
gctgtgattc catcactgca ctccagcccg ggcggcagag cgagacactg aatccaaacg 2580
acaacaacaa caaaaggcaa aaaaataaaa gtgccctctt tatggagttg tgtaaggtga 2640
agcatataca ctattcaaca tagtaactat ataaaggaag tattgttgtt gttactgtag 2700
ttaataccat taagtgagat gtttcgtata gtggaaagca catggactct gaattcagac 2760
tggtctgact ttgagtctca gctccacatc tagtaatact atgaccaagc cctggttaaa 2820
atcatgtttt tttttcttca gccttagtct tctcacatat aaaataggga cactgtcatt 2880
tacctcagtt ttctgtgagg ataaaacaac gacagtgtat atgcaagtat tttgtaaatt 2940
ttgtagtgct cctcaagatt tagttggtgt ttactacttg tactttctca ctggaatggc 3000
agatgctgtt ggacagcagg gacaatgacc acttttggga acagcagttg gatggcttag 3060
attggacagc ccaagacatc gtggcgtttt tggccaagca cccagaggat gtccagtcca 3120
gtaatggttc tgtgtacacc tggagagaag ctttcaacga gactaaccag gcaatccgga 3180
ccatatctcg cttcatggag gtaatctgtt gctgg 3215

<210> 10
<211> 9181
<212> DNA
<213> Homo sapiens

<400> 10
   tttggtcctt ggcaaagttc acaaatactg gatttgataa caaaaggnac ttaataaatg 60
   ttagttgagt ggttgaatga gtaaataaac ctctagcttt agtaaattaa ctctagcctt 120
   attctatata ggctcaagag aatatttcta cccattttct tctaggtttt cctatctcag 180
   tgactaatgg tagcaaagca ttcccttaaa aaggcattat ttgtgaaact tatctaaaat 240
   cgaattcggg tccaattaaa tttttgaaat tttatattaa aaattatatt agtagggatg 300
   ggtaagaggt gttttggtct ggttggttgg ttagttgcta tgactcagaa ttgctaagaa 360
   aacagaaaag taagataaga tcattgtttt aacctctttt cctccacaaa atcaataaat 420
   aacatatccc taaattactc ttagaatttc tcttaaattg cagtgaaaaa ccaaaatcct 480
   tcattcttgg ttgaaggttg gaaaactacg ttagagagga ttagagagag aggatgagca 540
   atcgtgtagt cagcccttgc ctcctagtgt aggatttgtc tcagccactg cttgttgtcc 600
   tggctgccaa cgttctcatg aaggctgttc ttctatcagt gtgtcaacct gaacaagcta 660
   gaacccatag caacagaagt ctggctcatc aacaagtcca tggagctgct ggatgagagg 720
   aagttctggg ctggtattgt gttcactgga attactccag gcagcattga gctgccccat 780
   catgtcaagt acaagatccg aatggacatt gacaatgtgg agaggacaaa taaaatcaag 840
   gatgggtaag tggaatccca tcacaccagc ctggtcttgg ggaggtccag agcacctatt 900
   atattaggac aagaggtact ttattttaac taaaaatttg gtagaaattt caacaacaac 960
   aaaaaaactc aacttggtgt catgattttg gtgaaattgg tacatgactt gctggaaggt 1020
   ttttcatagg tcataaaata acagtatctt ttgatttagc atttctactc aagggaatta 1080
   attccaggaa ttttggtggc aggcacctgt aatcccagct actcgggagg ctgaggcagg 1140
   agaattgctt gaacccagga ggcagaggtt gcagtgagct aagatcgcat cattgcactc 1200
   ccgcctgggc aataagagtg aaactccatc tcaaaaaaaa aaaaagatac aaaaatagaa 1260
   aaaggggctt ggtaagggta gtagggtttt gggcaatttt tttttttttt tttttttatt 1320
   gtatggttct aaaggaatgg ttgattacct gtggtttggt tttaggtact gggaccctgg 1380
   tcctcgagct gacccctttg aggacatgcg gtacgtctgg gggggcttcg cctacttgca 1440
   ggatgtggtg gagcaggcaa tcatcagggt gctgacgggc accgagaaga aaactggtgt 1500
   ctatatgcaa cagatgccct atccctgtta cgttgatgac atgtaagtta cctgcaagcc 1560
   actgttttta accagtttat actgtgccag atgggggtgt atatatgtgt gtgcatgtgc 1620
   atgcatgtgt gaatgatctg gaaataagat gccagatgta agttgtcaac agttgcagcc 1680
   acatgacaga catagatata tgtgcacaca ctagtaaacc tctttccttc tcatccatgg 1740
   ttgccacttt tatcttttta tttttatttt tttttttgag atggagtctc gctctgacgc 1800
   ccaggctgga gtgcagtggc tcgatctcgg ctcactgcaa cctttgcctc ccgggttcaa 1860
   gctattctcc tgcctcagcc tccacagtag ctgggactac aggctcatgc tgccacgccc 1920
   ggctgacttt ttgtatttta gtagagacga ggtttcacca tgttacccag gctagacttc 1980
   aactcctgag ctcaggcaat ccaccctcct tggcctccca aagtgctggg attacaggtg 2040
   tgagccactg cacccagccc accactttaa ttttttacac tctacccttt tggtcaaaat 2100
   ttgctcaatc tgcaagctta aaatgtgtca tgacaaacac atgcaagcac atactcacac 2160
   atagatgcag aaacagcgtc taaacttata aaagcacagt ttatgtaaat gtgtgcactt 2220
   cttctcccta ggtggtaaac cacatttcaa aacaacccaa ataaaactga acaaagcttc 2280
   ttcctcttag actttttaga aaatctttca gtgctgagtc actaagctgc caagttctca 2340
   ttgtgggaac tatgcctttg gatgtaatga tttcttctaa gacaatgggc ggaggtgtag 2400
   ttattgcaga catctgaaat atgtaatgtt tcttccagat tctggaaatt ctcttattct 2460
   ctgtggttgg tggtggtggt gggatgtgtg tgtgtgtgtg tgtgtgtgtg tgtgtgtgtg 2520
   tgtagggatc aggatgcggg aggagctggg ttctgcttgt attggttctc tgttttgcat 2580
   tgaatagtgt gtttccttgt atggctatct atagcttttc aaggtcacca gaaattatcc 2640
   tgtttttcac cttctaaaca attagctgga atttttcaaa ggaagacttt tacaaagacc 2700
   cctaagctaa ggtttactct agaaaggatg tcttaagaca gggcacagga gttcagaggc 2760
   attaagagct ggtgcctgtt gtcatgtagt gagtatgtgc ctacatggta aagctttgac 2820
   gtgaacctca agttcagggt ccaaaatctg tgtgcctttt tactttgcac atctgcattt 2880
   tctattctag cttggaatct gaaacattga caagagctgc ctgaaatgta tgtctgtggt 2940
   gtgattagag ttacgataag caagtcaata gtgagatgac cttggagatg ttgaactttt 3000
   gtgagagaat gagttgtttt tttgttttgg tttttagtac tttaacataa tctaccttta 3060
   gtttaagtat cgctcacagt tacctagtta ctgaagcaag cccccaaaga aatttggttt 3120
   ggcaacactt tgttagcctc gtttttctct ctacattgca ttgctcgtga agcattggat 3180
   catacgtaca tttcagagtc tagagggcct gtccttctgt ggcccagatg tggtgctccc 3240
   tctagcatgc aggctcagag gccttggccc atcaccctgg ctcacgtgtg tctttctttc 3300
   tccccttgtc cttccttggg gcctccagct ttctgcgggt gatgagccgg tcaatgcccc 3360
   tcttcatgac gctggcctgg atttactcag tggctgtgat catcaagggc atcgtgtatg 3420
   agaaggaggc acggctgaaa gagaccatgc ggatcatggg cctggacaac agcatcctct 3480
   ggtttagctg gttcattagt agcctcattc ctcttcttgt gagcgctggc ctgctagtgg 3540
   tcatcctgaa ggtaaggcag cctcactcgc tcttccctgc caggaaactc cgaaatagct 3600
   caacacgggc taagggagga gaagaagaaa aaaaatccaa gcctctggta gagaaggggt 3660
   catacctgtc atttcctgca atttcatcca tttatagttg gggaaagtga ggcccagaga 3720
   ggggcagtga cttgcccaag gtcaacccag ccgggtagca gctaagtagg atgagagtgc 3780
   agggttcatg ctttccagat aaccacatgc tcaactgtgc catgctgtct cattggtagt 3840
   ggttcatggc agcatctgaa agctatttat tttcttagat atattgggtg gcgattcttc 3900
   ctaagtttct aagaacaata atcagaagga tatatattgt tgcaggttag actgtctgga 3960
   agcagaggct gaaatagagt ttgatgtatg ggtatttatg agggctcaat acctatgaag 4020
   agatatggaa gatgcaggat tgggcagagg gaggagttga actgtgatat agggccaacc 4080
   ccgtggggca ctctanagaa tatgcagctt gttggagttg ttnttcatcg agctgaaaca 4140
   tccagccctt tgtgctcccc caaggcctcc ctcctgacac cacctacctc agccctctca 4200
   atcaatcact ggatgtgggc tgccctggga aggtcgtgcc ccagggccta catggctctc 4260
   tgctgctgtg acaaacccag agttgctgat gcctgaggcc gtctactgac agctgggcaa 4320
   caaggcttcc ctgaatgggg actctgggca gtgcagtttt gtgtctgaac catacattaa 4380
   tatatttata tccgaatttt ctttctctgc aagcatttca tataaagaca catcaggtaa 4440
   aaataaatgt ttttgaagca aaaggagtac aaagagataa gaactaacta atttaatact 4500
   agttaccatc tgttacaaat agttcctact gattgccaag gactgtttaa acacatcaca 4560
   tgggcttctt cttctatcct cactaaccct tttaacagac aaggaaatga ggctcaggaa 4620
   ggtcaaggac tttattgagg ttccacagta ggatacagtt cttgctaaaa gcaacccctc 4680
   cctcatgctc tgttatctaa ctgcaagggg aaggtcagtg gcagaggtag tggtcccatg 4740
   gttggtgcat aagagctgct ctgagacaac tgcatgctgg tgggtcctgc agacatgtac 4800
   ccatcagccg gagataggct caaaatatcc acaagagttt ggatgattgt gggaatgcag 4860
   aatccatggt gatcaagagg gaaagtcaag ttgcctggcc attttccttg gcttttagac 4920
   agaaaagtta cgtgggatat tatctcccac agctcttctg tggtgccacc agtcatagtc 4980
   cttatataag gagaaaccag ttgaaattac ctattgaaga aacaaagagc aaactcgccc 5040
   actgaaatgc gtagaaagcc ctggactctg ttgtattcat aactctgcca ttatttttct 5100
   gcgtagtttt gggtaagtca cttatcttct ttaggatggt aatgatcagt tgcctcatca 5160
   gaaagatgaa cagcattacg cctctgcatt gtctctaaca tgagtaggaa taaaccctgt 5220
   cttttttctg tagatcatac aagtgagtgc ttgggattgt tgaggcagca catttgatgt 5280
   gtctcttcct tcccagttag gaaacctgct gccctacagt gatcccagcg tggtgtttgt 5340
   cttcctgtcc gtgtttgctg tggtgacaat cctgcagtgc ttcctgatta gcacactctt 5400
   ctccagagcc aacctggcag cagcctgtgg gggcatcatc tacttcacgc tgtacctgcc 5460
   ctacgtcctg tgtgtggcat ggcaggacta cgtgggcttc acactcaaga tcttcgctgt 5520
   gagtacctct ggcctttctt cagtggctgt aggcatttga ccttcctttg gagtccctga 5580
   ataaaagcag caagttgaga acagaagatg attgtctttt ccaatgggac atgaacctta 5640
   gctctagatt ctaagctctt taagggtaag ggcaagcatt gtgttttatt aaattgttta 5700
   cctttagtct tctcagtgaa tcctggttga attgaattga atggaatttt tccgagagcc 5760
   agactgcatc ttgaactggg ctggggataa atggcattga ggaatggctt caggcaacag 5820
   atgccatctc tgccctttat ctcccagctc tgttggctat gttaagctca tgacaaagcc 5880
   aaggccacaa atagaactga aaactcttga tgtcagagat gacctctctt gtcttccttg 5940
   tgtccagtat ggtgttttgc ttgagtaatg ttttctgaac taagcacaac tgaggagcag 6000
   gtgcctcatc ccacaaattc ctgacttgga cacttccttc cctcgtacag agcaggggga 6060
   tatcttggag agtgtgtgag cccctacaag tgcaagttgt cagatgtccc caggtcactt 6120
   atcaggaaag ctaagagtga ctcataggat gctcctgttg cctcagtctg ggcttcatag 6180
   gcatcagcag ccccaaacag gcacctctga tcctgagcca tccttggctg agcagggagc 6240
   ctcagaagac tgtgggtatg cgcatgtgtg tgggggaaca ggattgctga gccttggggc 6300
   atctttggaa acataaagtt ttaaaagttt tatgcttcac tgtatatgca tttctgaaat 6360
   gtttgtatat aatgagtggt tacaaatgga atcattttat atgttacttg gtagcccacc 6420
   actcccctaa agggactcta taggtaaata ctacttctgc accttatgat tgatccattt 6480
   tgcaaattca aatttctcca ggtataattt acactagaag agatagaaaa atgagactga 6540
   ccaggaaatg gataggtgac tttgcctgtt tctcacagag cctgctgtct cctgtggctt 6600
   ttgggtttgg ctgtgagtac tttgcccttt ttgaggagca gggcattgga gtgcagtggg 6660
   acaacctgtt tgagagtcct gtggaggaag atggcttcaa tctcaccact tcggtctcca 6720
   tgatgctgtt tgacaccttc ctctatgggg tgatgacctg gtacattgag gctgtctttc 6780
   caggtacact gctttgggca tctgtttgga aaatatgact tctagctgat gtcctttctt 6840
   tgtgctagaa tctctgcagt gcatgggctt ccctgggaag tggtttgggc tatagatcta 6900
   tagtaaacag atagtccaag gacaggcagc tgatgctgaa agtacaattg tcactacttg 6960
   tacagcactt gtttcttgaa aactgtgtgc caggcagcat gcaaaatgtt ttatacacat 7020
   tgcttcattt aattctcaca aggctactct gaagtagtta ctataataac cagcaatttt 7080
   caaatgagag aactgtgact caaagacgtt aagtaaccag ctttggtcac acaactgtta 7140
   aatgttggta cgtggaggtg aatccacttc ggttacactg ggtcaataag cccaggcgaa 7200
   tcctcccaat gctcacccaa ttctgtattt ctgtgtcctc agagggggta caactaggag 7260
   aggttctgtt tcctgagtac aggttgttaa taattaaata tactagctct aaggcctgcc 7320
   tgtgatttaa ttagcattca ataaaaattc atgttgaatt tttctttagt acttctttct 7380
   taatataata catcttcttg accaagtcca agaggaacct gcgttggaca gttttcatat 7440
   gagatcaaat tctgagagag caagatttaa ccctttttgg ttcaccttct gatcctcccc 7500
   taaggaggta tacatgaaat atttattact cctgcctgaa cttctttcat tgaatatgca 7560
   attttgcagc atgcagattc tggatttaaa ttctgagtct taacttactg gctgagggac 7620
   cttggatagg ctccttatcc ctcagtttcc tcatctctaa aatggggatg gcacctgccc 7680
   cgtgggttgt tggaaggact tacagaggtg cagaatgtac gttgtacata gcaggtttca 7740
   gcaaatgtta gctccctctt tccccacatc cattcaaatc tgttccttct ccaaaggatg 7800
   tgtcaaggag gaaatggacc tggctgggaa accctcagaa tactgggatg atgctgagct 7860
   tggctcatac ctgtgctttg ctttcaggcc agtacggaat tcccaggccc tggtattttc 7920
   cttgcaccaa gtcctactgg tttggcgagg aaagtgatga gaagagccac cctggttcca 7980
   accagaagag aatatcagaa agtaagtgct gttgacctcc tgctctttct ttaacctagt 8040
   gctgctgcct ctgctaactg ttgggggcaa gcgatgtctc ctgcctttct aaaagactgt 8100
   gaaaccactc caggggcaga gaaatcacat gcagtgtccc tttccaaatc ctcccatgcc 8160
   atttatgtcc aatgctgttg acctattggg agttcacggt ctcgatccct gagggacatt 8220
   ttctttgttg tcttggcttc tagaagagta tcttttactt gccccctccc aaacacacat 8280
   ttcatggtct cctaacaagc tagaagaaag aggtaaagac aagcgtgatt gtggaaccat 8340
   agcctcgctg cctgcctgtg acatggtgac ctgtgtatca gcctgtgtgg gctgagacca 8400
   agtggctacc acagagctca gcctatgctt cataatgtaa tcattaccca gatccctaat 8460
   cctctcttgg ctcttaactg cagacagaga tgtccacagc tcatcaaagg ctctgccttc 8520
   tgggttcttt gtgcttagag tggcttccta aatatttaat aggtcccttt tctgccagtc 8580
   tcttctgtgc ccatcccctg attgcccttg gtaaaagtat gatgcccctt agtgtagcac 8640
   gcttgcctgc tgttcctaat catcttctcc tacctcctct ttacacctag ctcctgtttc 8700
   agtcacctag aaatgctcac agtcgctgga atatgtcatg ttcttccaca cctccatgcc 8760
   tttgtaggta ctgtttgctc tcacaggaga actttctctc taacttgcct atcttctcaa 8820
   ctcctccttt ctctccaaga tctagttccg gatcccctcc cctgagcatc cctccttggt 8880
   tctcaggtag tcagtcactc tctgccctga acttccatgg cacgtgaaag aaaatctttt 8940
   tattttaaaa caattacaga ctcacaagaa gtaatacaaa ttacatgagg gggttccctt 9000
   aaacctttca tccagtttcc ccaatggtag cagcatgtgt aactgtagaa tagtatcaaa 9060
   accatgaaat tgacataggt acaattcaca aaccttcttc agatttcact agctttatgt 9120
   gcgctcattt gtgtgtgtgt gtgcgtattt agttctatgc aattttatca tgtgtgaatt 9180
   c 9181

<210> 11
<211> 578
<212> DNA
<213> Homo sapiens

<400> 11
ttagatctat ccaagttgtg tgtgtcaaca gttcattcct ctntcactgc tgagtagtgt 60
tccctgggag gggtgtatca cagttccatg gcanttttta gatgtatttt ttaaacagct 120
ttcagcatcc tctattttaa ttgtntcatc aagtcctttt tcccaataga ctctgaatgc 180
tcctttatca tcgtanttcc catcaccaac atcagtaccc aaataggccc taaataaaca 240
tttatangcc tcctgcctgc ctgagaaacc agggtggaca tggagagaag gcacttcntg 300
aaagttcaag cgcagtgccc tgtgtcctta cactccactc ctcagtgcnt ttctgtgggt 360
tcatttctgt cttctctcct gtcacagtct gcatggagga ggaacccacc cacttgaagc 420
tgggcgtgtc cattcagaac ctggtaaaag tctaccgaga tgggatgaag gtggctgtcg 480
atggcctggc actgaatttt tatgaggcca agatcacctc cttcctgggc cacaatggag 540
cggggaagac gaccaccatg tcaatgaggg cgggtagc 578

<210> 12
<211> 384
<212> DNA
<213> Homo sapiens

<400> 12
tagaagctga ataatatcaa tgatatcctg aagtccgtgt tcttgatctt cccacatttt 60
tgcctgggac gagggctcat cgacatggtg aaaaaccagg caatggctga tgccctggaa 120
aggtttggtg agtgaagcag tggctgtagg atgctttaat ggagatggca ctctgcatag 180
gccttggtac cctgaacttt gttttggaaa gaagcaggtg actaagcaca ggatgttccc 240
ccacccccat gcccagtaac agggctcatg ccaacacagc tggttgtggc atgggttttg 300
tgacacaacc atttgtctgt gtctctgata gcattagaaa agtaaagggc agttttaagg 360
taaggaaaaa ggttttgctg gaca 384

<210> 13
<211> 386
<212> DNA
<213> Homo sapiens

<400> 13
tggctagctg gctttcatta ctgtttttca cacattaact ttggcctcaa cttgacaact 60
caaataatat ttataaatac agccacactt aaaatggtcc cattatgaaa tacatattta 120
aatatctata cgatgtgtta aaaccaagaa aatatttgat tcttctctga tatttaagaa 180
ttgaaggttt gaggtagtta cgtgttaggg gcatttatat tcatgttttt agagtntgct 240
tancaactta atctttcctt ttcagtgctt tgggctcctg ggagttaatg gggctggaaa 300
atcatcaact ttcaagatgt taacaggaga taccactgtt accagaggag atgctttcct 360
taacagaaat aggtgagaaa agaagt 386

<210> 14
<211> 345
<212> DNA
<213> Homo sapiens

<400> 14
tcttgtcctt tgcagtatct tatcaaacat ccatgaagta catcagaaca tgggctactg 60
ccctcagttt gatgccatca cagagctgtt gactgggaga gaacacgtgg agttctttgc 120
ccttttgaga ggagtcccag agaaagaagt tggcaaggta ctagtgnggc acctgaaagc 180
cagcctagtc tcctttggca tcctagacaa tatatacctt atgagctttc ccacacgcca 240
ttagacttca gagctagttt ttccttcatg atanncagca gcacaaaatg cctgagttct 300
ttgtatctgc tttcagangt nggaaaccnt ggtaacgagt gagtg 345

<210> 15
<211> 618
<212> DNA
<213> Homo sapiens

<400> 15
gtgcctgctc ccaccacacg agtcccttct ccctgctttg gctcctcacc agttgtcagg 60
ttatgattat agaatctagt cctactcagt gaaagaactt tcatacatgt atgtgtagga 120
cagcatgata aaattcccaa gccagaccaa agtcaaggtg ctttttatca ctgtaggttg 180
gtgagtgggc gattcggaaa ctgggcctcg tgaagtatgg agaaaaatat gctggtaact 240
atagtggagg caacaaacgc aagctctcta cagccatggc tttgatcggc gggcctcctg 300
tggtgtttct ggtataactg tggatggaaa actgttgttc tggcctgagt ggaaaacatg 360
actgttcaaa agtcctatat gtccagggct gttgtatgat tggcttgtct tcccccaggg 420
acagcagagc aaccttggaa aagcagaggg aagcttctcc cttggcacac actggggtgg 480
ctgtaccatg cctgcagatg ctcccaaata gaggcactcc aagcacnttt gtttcttagc 540
gtgattgagg ctggatatgt gatttgatct ttctctggaa cattctttct aatcatcttt 600
gtgttcattc cctgaaaa 618

<210> 16
<211> 1329
<212> DNA
<213> Homo sapiens

<400> 16
aaaggaattt caccccaatc ttatactgac ttcaatagag ctttcagaca aaaagttgtt 60
ttgtatatac tnatcagtca tgaaaagata attacaacta aatggccttt ttccttccct 120
atttatttgg agaaatttaa ttacataaga aagtactcag aatatgtgag tttcctgcat 180
caataagaca tttataataa tgaccntgtt tacaaatgaa tttgaaagtn actctaattc 240

tttgattcat caagaaataa ctagaatggc aagttaaaat ttaagctgtt tcaaagatgc 300
ttcngcantt agaaactcnn ttatctttga tnttctgtcc cncnagcaaa taanacttan 360
tntattntna tancaggatg aacccaccac aggcatggat cccaaagccc ggcggttctt 420
gtggaattgt gccctaagtg ttgtcaagga ggggagatca gtagtgctta catctcatag 480
gtccgtagta aagtcttggg ttcctcactg tgggatgttt taactttcca agtagaatat 540
gcgatcattt tgtaaaaatt agaaaataca gaaaagcaaa gagtaaaaca attattacct 600
gaaattatat atgcatattc ttacaaaaat gcaagcccag tataaatact gctctttttc 660
acttaatata ttgtaaacat tattccaagt cagtgcattt aggtgtcatt tcttatagct 720
ggatagtatt ccattaggat atactcttat ttaactattc ccccttttgt agacatttgg 780
attatttcca acttgttcac aattgtanac nccaccacac tgaacagcat catccctata 840
tccacatgna cttgtaacag aatacaattc cctaggaagc tggaatgctg gaagtcatgg 900
tgatgttctc atggtnacag agaatctctc taganctaan acctctctct gttttaccgc 960
agtatggaag aatgtgaagc tctttgcact aggatggcaa tcatggtcaa tggaaggttc 1020
aggtgccttg gcagtgtcca gcatctaaaa aataggtaat aaagataatt tctttgggat 1080
angtgcctag tgagaaggct tgatatttat tcttttgtng agtatataaa tggtgcctct 1140
aaaataaagg gaaataaaac tgagcaaaac agtgtagtgg aaagaatgag ggctttgaag 1200
tccgaactgc attcaaattc tgtctttacc atttactggt tctgtgactc ttgggcaagt 1260
tacttaacta ctgtaagagt tagtttccct ggaagatcta cctcctagct ttgtgctata 1320
gatgaaatg 1329

<210> 17
<211> 5352
<212> DNA
<213> Homo sapiens

<400> 17
aagtctattt ttaagttgtt attgcttttt tcaagtattc ttccctcctt cacacacagt 60
tttctagtta atccatttat gtaattctgt atgctcctac ttgacctaat ttcaacatct 120
ggaaaaatag aactagaata aagaatgagc aagttgagtg gtatttataa aggtccatct 180
taatctttta acaggtattt gtgaactttg ccaaggacca aagtgatgat gaccacttaa 240
aagacctctc attacacaaa aaccagacag tagtggacgt tgcagttctc acatcttttc 300
tacaggatga gaaagtgaaa gaaagctatg tatgaagaat cctgttcata cggggtggct 360
gaaagtaaag aggnactaga ctttcctttg caccatgtga agtgttgtgg agaaaagagc 420
cagaagttga tgtgggaaga agtaaactgg atactgtact gatactattc aatgcaatgc 480
aattcaatgc aatgaaaaca aaattccatt acaggggcag tgcctttgta gcctatgtct 540
tgtatggctc tcaagtgaaa gacttgaatt tagtttttta cctataccta tgtgaaactc 600
tattatggaa cccaatggac atatgggttt gaactcacac tttttttttt tttttgttcc 660
tgtgtattct cattggggtt gcaacaataa ttcatcaagt aatcatggcc agcgattatt 720
gatcaaaatc aaaaggtaat gcacatcctc attcactaag ccatgccatg cccaggagac 780
tggtttcccg gtgacacatc cattgctggc aatgagtgtg ccagagttat tagtgccaag 840
tttttcagaa agtttgaagc accatggtgt gtcatgctca cttttgtgaa agctgctctg 900
ctcagagtct atcaacattg aatatcagtt gacagaatgg tgccatgcgt ggctaacatc 960
ctgctttgat tccctctgat aagctgttct ggtggcagta acatgcaaca aaaatgtggg 1020
tgtctctagg cacgggaaac ttggttccat tgttatattg tcctatgctt cgagccatgg 1080
gtctacaggg tcatccttat gagactctta aatatactta gatcctggta agaggcaaag 1140
aatcaacagc caaactgctg gggctgcaag ctgctgaagc cagggcatgg gattaaagag 1200
attgtgcgtt caaacctagg gaagcctgtg cccatttgtc ctgactgtct gctaacatgg 1260
tacactgcat ctcaagatgt ttatctgaca caagtgtatt atttctggct ttttgaatta 1320
atctagaaaa tgaaaagatg gagttgtatt ttgacaaaaa tgtttgtact ttttaatgtt 1380
atttggaatt ttaagttcta tcagtgactt ctgaatcctt agaatggcct ctttgtagaa 1440
ccctgtggta tagaggagta tggccactgc cccactattt ttattttctt atgtaagttt 1500
gcatatcagt catgactagt gcctagaaag caatgtgatg gtcaggatct catgacatta 1560
tatttgagtt tctttcagat catttaggat actcttaatc tcacttcatc aatcaaatat 1620
tttttgagtg tatgctgtag ctgaaagagt atgtacgtac gtataagact agagagatat 1680
taagtctcag tacacttcct gtgccatgtt attcagctca ctggtttaca aatataggtt 1740
gtcttgtggt tgtaggagcc cactgtaaca atactgggca gccttttttt ttttttttaa 1800
ttgcaacaat gcaaaagcca agaaagtata agggtcacaa gtctaaacaa tgaattcttc 1860

aacagggaaa acagctagct tgaaaacttg ctgaaaaaca caacttgtgt ttatggcatt 1920
tagtaccttc aaataattgg ctttgcagat attggatacc ccattaaatc tgacagtctc 1980
aaatttttca tctcttcaat cactagtcaa gaaaaatata aaaacaacaa atacttccat 2040
atggagcatt tttcagagtt ttctaaccca gtcttatttt tctagtcagt aaacatttgt 2100
aaaaatactg tttcactaat acttactgtt aactgtcttg agagaaaaga aaaatatgag 2160
agaactattg tttggggaag ttcaagtgat ctttcaatat cattactaac ttcttccact 2220
ttttccaaaa tttgaatatt aacgctaaag gtgtaagact tcagatttca aattaatctt 2280
tctatatttt ttaaatttac agaatattat ataacccact gctgaaaaag aaaaaaatga 2340
ttgttttaga agttaaagtc aatattgatt ttaaatataa gtaatgaagg catatttcca 2400
ataactagtg atatggcatc gttgcatttt acagtatctt caaaaataca gaatttatag 2460
aataatttct cctcatttaa tatttttcaa aatcaaagtt atggtttcct cattttacta 2520
aaatcgtatt ctaattcttc attatagtaa atctatgagc aactccttac ttcggttcct 2580
ctgatttcaa ggccatattt taaaaaatca aaaggcactg tgaactattt tgaagaaaac 2640
acaacatttt aatacagatt gaaaggacct cttctgaagc tagaaacaat ctatagttat 2700
acatcttcat taatactgtg ttacctttta aaatagtaat tttttacatt ttcctgtgta 2760
aacctaattg tggtagaaat ttttaccaac tctatactca atcaagcaaa atttctgtat 2820
attccctgtg gaatgtacct atgtgagttt cagaaattct caaaatacgt gttcaaaaat 2880
ttctgctttt gcatctttgg gacacctcag aaaacttatt aacaactgtg aatatgagaa 2940
atacagaaga aaataataag ccctctatac ataaatgccc agcacaattc attgttaaaa 3000
aacaaccaaa cctcacacta ctgtatttca ttatctgtac tgaaagcaaa tgctttgtga 3060
ctattaaatg ttgcacatca ttcattcact gtatagtaat cattgactaa agccatttgt 3120
ctgtgttttc ttcttgtggt tgtatatatc aggtaaaata ttttccaaag agccatgtgt 3180
catgtaatac tgaaccactt tgatattgag acattaattt gtaccctgtg ttattatcta 3240
ctagtaataa tgtaatactg tagaaatatt gctctaattc ttttcaaaat tgttgcatcc 3300
cccttagaat gtttctattt ccataaggat ttaggtatgc tattatccct tcttataccc 3360
taagatgaag ctgtttttgt gctctttgtt catcattggc cctcattcca agcactttac 3420
gctgtctgta atgggatcta tttttgcact ggaatatctg agaattgcaa aactagacaa 3480
aagtttcaca acagattttc taagttaaat cattttcatt aaaaggaaaa aagaaaaaaa 3540
atttttgtat gtcaataacc tttatatgaa gtattaaaat gcatatttct atgttgtaat 3600
ataatgagtc acaaaataaa gctgtgacag ttctgttggt ctacagaaat ttacttttgt 3660
gcatttgtgg caccacctac tgttgaaggg ttataaagcc attagaaaag tagaggggaa 3720
gtgatttgga tcaaaaggaa aaactttaga aaagattcaa atgttccctt aatcataaaa 3780
gagaactgag gggactactt gaaaataaaa ggttgttttg tattttcatg ttggttaaga 3840
tactgagtaa ctggtattaa gtgttagagg tttttagata aatattctgc ttaatgatta 3900
tgaagctgca ctgagatttc tgaaaatgct ctgtagctga gcttatttaa taaatgttca 3960
cttggtatag gggaagctac aaaggcagcc ttcagtgtcc ttttgtttat tcaaccaaaa 4020
atataaggac acaatgtagc agttatactg ggaaggtgct gggggtggtg gcaatggtga 4080
gcaggaaggc gaagtagata tggaaacaga aatgatacta atatcggtga ttccttcctt 4140
ttttcctgta ataagtgctg tgcagacaac atatgagcag tgctgataaa tgtaaatgta 4200
tttttcatag ctcattaaga atcagtttca gaaagagatg tctgcttatt ttgctacttg 4260
aagaatccct gtcaaacagt ccttttgagg aagtacaaga ggctgtctct atttgtgacc 4320
tcaggaatgg ctgtgacagt gtcgtgagca gtccttttcc tgtggcacag atctgaactt 4380
tgtgtgcaga aaaatcttgg cttcaagtga gccaagatgc cccctgagca tcagcatcac 4440
aacttcatcc tcctatcttg aagttcatgt tatagtgact ttaatgaaat catagaacac 4500
tgtttcttcg tgaacaatga cgagggagag gaaaaaactt tattgaaaaa taaaaaggca 4560
ggtaatttag atgaaaatat gttacccatg aggttttgtt tttgcttttt gtttttgttt 4620
ttgagaaaca gaatctcgct ctgtcgtcca ggctggagtg cagcggcatg atcttggctc 4680
actgcaacct ccgcctcccg ggttcaagcg attctcctca gcttcccaag tagctggtac 4740
tacaggcatg cgccaccaca accagctaat ttttgtattt ttagtagaga tggggtttca 4800
ctatacgttg gccaggctgg tctcaaactc ctgacctaag gtgatccttc tgccttgggc 4860
tcccaaagtg ctgggattac aggcatgagc caccttgcct ggccctaccc atgagccttg 4920
actaaaacat tcttctatct gtagaaaagc ccaaaagaac ttttccagat tcaaaaaact 4980
tggcactttg taatggtaat gtttacatta agtaaaaaaa aaaaaaaaaa acccacttag 5040
cttcagtttt caagtgttta ctgtgttgtc atgcacttca tttaattctc aacacctgcc 5100
ctatgaggta aaaagtacca ttttacatat gagtaaatta cagctcagtg gataagaaac 5160
tcgtccaaag gtacaggttc agtcaagtgg cagagggttc tttttgttga agttaggtat 5220
cagttaaaat tgaccttgta aaatcacatc agcatcaata tacattaatt taacaaatat 5280
ttattgaact ttactgtatg ccagatactt ctctaggtac tagggggtac aatgtagaag 5340

aaaatagaat tc 5352

<210> 18
<211> 94
<212> DNA
<213> Homo sapiens

<400> 18
tgtcagctgc tgctggaagt ggcctggcct ctatttatct tcctgatcct gatctctgtt 60
cggctgagct acccacccta tgaacaacat gaat 94

<210> 19
<211> 142
<212> DNA
<213> Homo sapiens

<400> 19
gccattttcc aaataaagcc atgccctctg caggaacact tccttgggtt caggggatta 60
tctgtaatgc caacaacccc tgtttccgtt acccgactcc tggggaggct cccggagttg 120
ttggaaactt taacaaatcc at 142

<210> 20
<211> 119
<212> DNA
<213> Homo sapiens

<400> 20
tgtggctcgc ctgttctcag atgctcggag gcttctttta tacagccaga aagacaccag 60
catgaaggac atgcgcaaag ttctgagaac attacagcag atcaagaaat ccagctcaa 119

<210> 21
<211> 122
<212> DNA
<213> Homo sapiens

<400> 21
acttgaagct tcaagatttc ctggtggaca atgaaacctt ctctgggttc ctgtatcaca 60
acctctctct cccaaagtct actgtggaca agatgctgag ggctgatgtc attctccaca 120
ag 122

<210> 22
<211> 177
<212> DNA
<213> Homo sapiens

<400> 22
gtatttttgc aaggctacca gttacatttg acaagtctgt gcaatggatc aaaatcagaa 60
gagatgattc aacttggtga ccaagaagtt tctgagcttt gtggcctacc aagggagaaa 120
ctggctgcag cagagcgagt acttcgttcc aacatggaca tcctgaagcc aatcctg 177

<210> 23
<211> 93

<212> DNA
<213> Homo sapiens

<400> 23
agaacactaa actctacatc tcccttcccg agcaaggagc tggccgaagc cacaaaaaca 60
ttgctgcata gtcttgggac tctggcccag gag 93

<210> 24
<211> 241
<212> DNA
<213> Homo sapiens

<400> 24
ctgttcagca tgagaagctg gagtgacatg cgacaggagg tgatgtttct gaccaatgtg 60
aacagctcca gctcctccac ccaaatctac caggctgtgt ctcgtattgt ctgcgggcat 120
cccgagggag gggggctgaa gatcaagtct ctcaactggt atgaggacaa caactacaaa 180
gccctctttg gaggcaatgg cactgaggaa gatgctgaaa ccttctatga caactctaca 240
a 241

<210> 25
<211> 140
<212> DNA
<213> Homo sapiens

<400> 25
ctccttactg caatgatttg atgaagaatt tggagtctag tcctctttcc cgcattatct 60
ggaaagctct gaagccgctg ctcgttggga agatcctgta tacacctgac actccagcca 120
caaggcaggt catggctgag 140

<210> 26
<211> 117
<212> DNA
<213> Homo sapiens

<400> 26
gtgaacaaga ccttccagga actggctgtg ttccatgatc tggaaggcat gtgggaggaa 60
ctcagcccca agatctggac cttcatggag aacagccaag aaatggacct tgtccgg 117

<210> 27
<211> 198
<212> DNA
<213> Homo sapiens

<400> 27
atgctgttgg acagcaggga caatgaccac ttttgggaac agcagttgga tggcttagat 60
tggacagccc aagacatcgt ggcgtttttg gccaagcacc cagaggatgt ccagtccagt 120
aatggttctg tgtacacctg gagagaagct ttcaacgaga ctaaccaggc aatccggacc 180
atatctcgct tcatggag 198

<210> 28
<211> 114
<212> DNA
<213> Homo sapiens

<400> 28
gccagtacgg aattcccagg ccctggtatt ttccttgcac caagtcctac tggtttggcg 60
aggaaagtga tgagaagagc caccctggtt ccaaccagaa gagaatatca gaaa 114

<210> 29
<211> 172
<212> DNA
<213> Homo sapiens

<400> 29
tctgcatgga ggaggaaccc acccacttga agctgggcgt gtccattcag aacctggtaa 60
aagtctaccg agatgggatg aaggtggctg tcgatggcct ggcactgaat ttttatgagg 120
gccagatcac ctccttcctg ggccacaatg gagcggggaa gacgaccacc at 172

<210> 30
<211> 124
<212> DNA
<213> Homo sapiens

<400> 30
aagctgaata atatcaatga tatcctgaag tccgtgttct tgatcttccc acatttttgc 60
ctgggacgag ggctcatcga catggtgaaa aaccaggcaa tggctgatgc cctggaaagg 120
tttg 124

<210> 31
<211> 107
<212> DNA
<213> Homo sapiens

<400> 31
tgctttgggc tcctgggagt taatggggct ggaaaatcat caactttcaa gatgttaaca 60
ggagatacca ctgttaccag aggagatgct ttccttaaca gaaatag 107

<210> 32
<211> 142
<212> DNA
<213> Homo sapiens

<400> 32
tatcttatca aacatccatg aagtacatca gaacatgggc tactgccctc agtttgatgc 60
catcacagag ctgttgactg ggagagaaca cgtggagttc tttgcccttt tgagaggagt 120
cccagagaaa gaagttggca ag 142

<210> 33
<211> 135
<212> DNA
<213> Homo sapiens

<400> 33
gttggtgagt gggcgattcg gaaactgggc ctcgtgaagt atggagaaaa atatgctggt 60
aactatagtg gaggcaacaa acgcaagctc tctacagcca tggctttgat cggcgggcct 120
cctgtggtgt ttctg 135

<210> 34
<211> 104
<212> DNA
<213> Homo sapiens

<400> 34
gatgaaccca ccacaggcat ggatcccaaa gcccggcggt tcttgtggaa ttgtgcccta 60
agtgttgtca aggaggggag atcagtagtg cttacatctc atag 104


<210> 35
<211> 93
<212> DNA
<213> Homo sapiens

<400> 35
tatggaagaa tgtgaagctc tttgcactag gatggcaatc atggtcaatg gaaggttcag 60
gtgccttggc agtgtccagc atctaaaaaa tag 93


<210> 36
<211> 2894
<212> DNA
<213> Homo sapiens

<400> 36
gtatttgtga actttgccaa ggaccaaagt gatgatgacc acttaaaaga cctctcatta 60
cacaaaaacc agacagtagt ggacgttgca gttctcacat cttttctaca ggatgagaaa 120
gtgaaagaaa gctatgtatg aagaatcctg ttcatacggg gtggctgaaa gtaaagaggn 180
actagacttt cctttgcacc atgtgaagtg ttgtggagaa aagagccaga agttgatgtg 240
ggaagaagta aactggatac tgtactgata ctattcaatg caatgcaatt caatgcaatg 300
aaaacaaaat tccattacag gggcagtgcc tttgtagcct atgtcttgta tggctctcaa 360
gtgaaagact tgaatttagt tttttaccta tacctatgtg aaactctatt atggaaccca 420
atggacatat gggtttgaac tcacactttt tttttttttt tgttcctgtg tattctcatt 480
ggggttgcaa caataattca tcaagtaatc atggccagcg attattgatc aaaatcaaaa 540
ggtaatgcac atcctcattc actaagccat gccatgccca ggagactggt ttcccggtga 600
cacatccatt gctggcaatg agtgtgccag agttattagt gccaagtttt tcagaaagtt 660
tgaagcacca tggtgtgtca tgctcacttt tgtgaaagct gctctgctca gagtctatca 720
acattgaata tcagttgaca gaatggtgcc atgcgtggct aacatcctgc tttgattccc 780
tctgataagc tgttctggtg gcagtaacat gcaacaaaaa tgtgggtgtc tctaggcacg 840
ggaaacttgg ttccattgtt atattgtcct atgcttcgag ccatgggtct acagggtcat 900
ccttatgaga ctcttaaata tacttagatc ctggtaagag gcaaagaatc aacagccaaa 960
ctgctggggc tgcaagctgc tgaagccagg gcatgggatt aaagagattg tgcgttcaaa 1020
cctagggaag cctgtgccca tttgtcctga ctgtctgcta acatggtaca ctgcatctca 1080
agatgtttat ctgacacaag tgtattattt ctggcttttt gaattaatct agaaaatgaa 1140
aagatggagt tgtattttga caaaaatgtt tgtacttttt aatgttattt ggaattttaa 1200
gttctatcag tgacttctga atccttagaa tggcctcttt gtagaaccct gtggtataga 1260
ggagtatggc cactgcccca ctatttttat tttcttatgt aagtttgcat atcagtcatg 1320
actagtgcct agaaagcaat gtgatggtca ggatctcatg acattatatt tgagtttctt 1380
tcagatcatt taggatactc ttaatctcac ttcatcaatc aaatattttt tgagtgtatg 1440
ctgtagctga aagagtatgt acgtacgtat aagactagag agatattaag tctcagtaca 1500
cttcctgtgc catgttattc agctcactgg tttacaaata taggttgtct tgtggttgta 1560
ggagcccact gtaacaatac tgggcagcct tttttttttt ttttaattgc aacaatgcaa 1620
aagccaagaa agtataaggg tcacaagtct aaacaatgaa ttcttcaaca gggaaaacag 1680
ctagcttgaa aacttgctga aaaacacaac ttgtgtttat ggcatttagt accttcaaat 1740
aattggcttt gcagatattg gataccccat taaatctgac agtctcaaat ttttcatctc 1800

ttcaatcact agtcaagaaa aatataaaaa caacaaatac ttccatatgg agcatttttc 1860
agagttttct aacccagtct tatttttcta gtcagtaaac atttgtaaaa atactgtttc 1920
actaatactt actgttaact gtcttgagag aaaagaaaaa tatgagagaa ctattgtttg 1980
gggaagttca agtgatcttt caatatcatt actaacttct tccacttttt ccaaaatttg 2040
aatattaacg ctaaaggtgt aagacttcag atttcaaatt aatctttcta tattttttaa 2100
atttacagaa tattatataa cccactgctg aaaaagaaaa aaatgattgt tttagaagtt 2160
aaagtcaata ttgattttaa atataagtaa tgaaggcata tttccaataa ctagtgatat 2220
ggcatcgttg cattttacag tatcttcaaa aatacagaat ttatagaata atttctcctc 2280
atttaatatt tttcaaaatc aaagttatgg tttcctcatt ttactaaaat cgtattctaa 2340
ttcttcatta tagtaaatct atgagcaact ccttacttcg gttcctctga tttcaaggcc 2400
atattttaaa aaatcaaaag gcactgtgaa ctattttgaa gaaaacacaa cattttaata 2460
cagattgaaa ggacctcttc tgaagctaga aacaatctat agttatacat cttcattaat 2520
actgtgttac cttttaaaat agtaattttt tacattttcc tgtgtaaacc taattgtggt 2580
agaaattttt accaactcta tactcaatca agcaaaattt ctgtatattc cctgtggaat 2640
gtacctatgt gagtttcaga aattctcaaa atacgtgttc aaaaatttct gcttttgcat 2700
ctttgggaca cctcagaaaa cttattaaca actgtgaata tgagaaatac agaagaaaat 2760
aataagccct ctatacataa atgcccagca caattcattg ttaaaaaaca accaaacctc 2820
acactactgt atttcattat ctgtactgaa agcaaatgct ttgtgactat taaatgttgc 2880
acatcattca ttca 2894

<210> 37
<211> 4646
<212> DNA
<213> Homo sapiens

<400> 37
ccattccacc tagtggggcc atttcctacc tgagtcaccc gtggtgtcaa atagaatgtc 60
atgtggcctc ctgagttggg tagaattggc tgctcatctc aaccccgcta ctgactatct 120
ctgtgattta cccttcctcc agccttagcc ttgctacata taaaatcaag acaataatgt 180
ttcctatctc acagggttgt cctgaggatt aaattaagta attaatataa aatgtgcctt 240
gtacatattg ggccctaaat aaacagtagc tactatttat ccttaaagta caaatggtag 300
tttcagagct tcaaggctga tggctattta tcttactcat actctttgtt tagcttcatt 360
tttttcccct aatttcatta gtattttctt ttcttttttt tttttttttt tttttttttt 420
tttggaggtg aagtctcact ctgttgccca ggctggagtg caatggagcg atcttggctc 480
accccaacct ctgtctcctg ggttcaaaca gttctcctgc ctcagcctcc cgagtagctg 540
ggattacagg ctcccgccac catgcccagc tatttttttg tattttcagt agagatgggg 600
tttcaccctt ttgaccaggc tggtcttgaa ctcctgacct catgatcaac ccacctcagc 660
ctcccaaagt gctgggatta caggtgtgag ccaccacgcc cggcctcata agtattttct 720
aaatttattt acagtcatgc catttaaaag gaaagttgta ttcctgtctt tgttaatatt 780
tataagtgat tttattcagc tacaagcttg gaatggcata taattttgta ttctgctttt 840
ttcacttaat attacatggc taatgatttc tgtgtttcat aaacattatt ctgatgatgg 900
catgatatat tgttgagtac atgtaccata attgaatcat ttccctattg ctatgcaatt 960
aagttgtttc caatattttg caattataat gtttcaatga atgaataact ttatgcatat 1020
agctttttga tatcttaagt tcagtttcct aggatgaatt tccaggaata gtaattgggc 1080
aaatgggata aacatgactc ttgaatacgt attgttaaca ttgctttccc aaagggctca 1140
actgatttat atttccgtgt tcattatctt ttaaaccagc tcatttactc accaaacatt 1200
tttaaagcca ttatcatgtg gtaggcttag taagaagaaa gtgaccctaa gggagaagct 1260
tatatataaa tagggtccct ggtgtaccaa gtgctgatac agacacaaag tacctgggga 1320
aattgagatg agggagtcct ggctcagctg ggagaaaagt tcattttcat agagtcatgg 1380
ttttgttctt tggcagaaag aaaattgctt tcttccccac ccccaccccc agctttattg 1440
aggtataatt gacaaataaa aattgtatat ctttaagata tgcaatgtga tatatatgta 1500
tatctcaact taaaaaataa gctacagaat aaaaaggtgt ttgctattaa aaaaaaagaa 1560
aaggctgaat gtcattccca agcttggaaa tttgagtatg ttgcctcttt gggattattt 1620
acagaaatat tagcaagacc agccccatct ttggtcttga gtactccact gtcagcatgc 1680
tttcttccag agagggatcc atttgccttt atttttcatt ctgttgtgcc gtctatgcaa 1740
actattcttg atagttttat ggtaacagtg tttttttgtt ccatgagata atttatacat 1800
gctcattgtg gaaaatttag aaaagacagg aaagtattaa aaacatcact tttttttttt 1860

tttttttttt ttttttttta agagacagag tcttgctctg tcgcccaggc cggagtgcag 1920
tggcgtgatc tcagctcaca gcaacctccg cttcccaggt ttaagtgatt ctcctgcctc 1980
agcctcccaa gtagctggga gtacaggcat gcaccaccac gcccggctaa ttttgtattt 2040
ttagtagaga tggggtttca ccatgttggc caggctggtc tcaaactcct gacctcaggt 2100
gatccgcctg ccttggcctc gcaaagttct gggattatag gcaggagcca ctgcgccagc 2160
cacacctacg ttcttatcat cctagtacat ccactgtcat tatcttgctg tatttccttc 2220
tgcccagtct cactctgatc atgcagtggc gtgatcatgc agtgatctcg gctcactgca 2280
acctaggcct tctgggttcg agtgattctc ctgccttagc ctcctgggtt caagtgattc 2340
tcttgccttg gcctcccaag tagctgggat tacaggcata cacccccatg cccatctaat 2400
ttttgtattt ttagtagaca cagcgtttca ctaaaatttt gtatttttag tagagatggg 2460
gtttcaccat gttggccagg ctggtctcca actcctgacc tcaggtgatc cgcctgcctt 2520
ggcctcacaa agtgattaca ggcatgagcc actgcatcca tcgccaaaaa gattttttaa 2580
aagagtttaa tgtagaacca tatcaaaggt ctttggaaat aaaaaacagt tttttaaaaa 2640
tatcagaaat aaaacaacaa ataaataaat aaataaaaaa cccaaaacaa tctgaagcac 2700
gagcacctag cagaaaggtt caattatgat ctattcatag agtggaatat caagtagaca 2760
ttacaggaca tgttttaaga ttatatttta tgtcatggga aatgctctcc cagtatgatg 2820
ttaaatgaaa aaacagaata caaaagtata tatgctgcat agtctcaata ttgtagagaa 2880
aaaatattat ttatgtatgc atgaaaaaag acaaaagatg ttaacagaga tccattgtta 2940
cttcagttta ctagggattg tctctgggag gtaggattaa ggtgatttat atttaccttt 3000
ttaaactttt ctgtattttt ttattttcaa attttccata aaaatataag gacttgaaga 3060
tcaagaaaaa atttctgctt tggctcagtg cagtggctca cgcctgtaat cccagcagtt 3120
tgggagccct aggggagagg atcacttgaa cccaagagtt tgacgttcca gtgagctatg 3180
atctccggat cgtaccgcct ggacgatgga gcaagaccct gtctcaaaaa aaaaaatctt 3240
tgcttttttt ttttgtttgt ttttgagacg gagtctctct ctgttgcccc agctggagta 3300
cagtggcaca atctcagctc accgcaacct ctgcctcctg ggttcaagcg attctcttgc 3360
ctcagcctcc caagtacctg ggattccatg cacccaccac tatgcccagc tacttttttg 3420
tattttcagt agagacaggg tttcaccatg ttggccaggc tggtctcgaa ttcctgacct 3480
cagctgatcc accggccttg gcctcccaaa gtgctgggat tacaggcatg agccactgtg 3540
cccagcccaa tcttttgctt tttttaaaaa aagaagacaa aaaggatttt tataccagta 3600
ttatcttggc tgtgtgactc tgaagccaca gttgtaagtt ataattactc tgaaacacaa 3660
ggccctgtga ctcttttggg ctctttggtg tttatcttga ttacaacgtt ggaatataga 3720
aatgaaagga atgggagagg tgatagactt caggcagtgt aactagttgt ctgaacacta 3780
ctggctcaat tatattgtgt ctagtgattt ccatcttgtc cgtctgctaa tttatcgcct 3840
ggtaactcac tgaggcaggg ttttcctttg gagaaacctc attgttttaa ccagtgtatc 3900
atgcttgttt agaagttcaa tgatcttttt aactcatcgg agaagatgat gaccagacct 3960
ggacagatgg ggaaggactt tgcactctct ctttacagtc ctgagtgcac acaggtcaat 4020
atggaactat gtgtgaattt tcattgtctt tgagagccct cttctctgcc ccatagggag 4080
cagctttgtg tgcaattaga ggagcaaggg ttgtgtgtat ttagcacagc aggttggcct 4140
ggtcctctcc tctcaacata gtcaccacat acctggcact atgctaaggc tgggaatgca 4200
gacagatggg tgcctgcttt cagagtgctc aatgtgctga ggaagccagc aacagaaaca 4260
gatgatttca ggagctccag gaaaatgcta caggaggagt gtgcctgggt tactggagta 4320
gcacaggagg agggcttcta gctcaggctg agattttagt aaaggaaatt atgccacgat 4380
gaatcctgaa gaatgaatag aagtgaacca gataaagcac gataggaagc atcttccctt 4440
acctaaggga agacacagag gtatatggaa tggtatgtta aaaggttggg actccaaaca 4500
gttctgttaa agcttagaga gtggtgggag agactggaga agttgattaa ttagtaaatg 4560
aagttgtctg tggatttccc agatcccagt ggcattggat atccgtatca ttttaaatta 4620
cagtgttcta tcttatttcc actcag 4646

<210> 38
<211> 4533
<212> DNA
<213> Homo sapiens

<400> 38
gtaagtaact gtggatgttg cctgagactc accaatggca gggaaaatcc aggcaattaa 60
cgtgggctaa attggacttt tccaaagatg ctgtctttgg gaaacatcac acatgctttg 120
gatcagaaaa cctaggcttc taatttgttg ataaggcatg aactcaggag actgttttca 180

gtcctagtga atggtgataa ttgtaattat aacagtagac aacatctctt ttacacattt 240
taaatcatga aaatagaata accttactga taattttaga aagtggtgat taaaagcaca 300
tttaagataa tgccttaaca cctagtcttt tccatatgca tgatgtctta atcacacatt 360
gcaaatcatg gaacacagaa ttttaagcag catttgtgta gaacttctca gttttactaa 420
tattatttta ttttattctc ataacaacct tgaatagaac tcagatcatc tgtcaatcat 480
gtattttgat aacagccttt acagtgagca tagaaaatac agtagtggct aacaacacag 540
gctccagatg tcaggttatc tgggtatgaa ttctggtgtc agcattcact aagcatatga 600
ccttggacaa gtgatttaag tttcttttaa acagagaata gtaataccta cctcatatta 660
ttattgtcag tgtatcatct tacaatcaca gtctttctct tagggctggg ctcagtgggt 720
ggattgacac tgcagaaatg gccagatcta aaggatcaac atttacgtag ctgggaaatg 780
tagctgggac ttcagtttca ctgcgctagt gatttttcct accactaagc agctcagtcc 840
atacccctac gagacccaca agcttatgag atactgttct tccaggaaag cagtggggcc 900
agggccacct tttaattgtg tttcttggcc tggtcccatc tttctcacaa tatatagcaa 960
cagttattta cttgctgatt ttctaatgca catcacacat agtcatatta aacacacaca 1020
cacacacaca cacacacaca cacccctcaa gaaacatttt ctgagacgtg atttcctgat 1080
ttcatcaaaa aagaaaagag cgggccaggc acagtgggaa gtcaaggtgg gtggatcact 1140
tgaggtcagg agtttgaaac cagcctggcc aacacggtgg aacctcgtct ctactaaaaa 1200
tacaaaaatt agccaggcgt ggtggcgcac acctgtaatc ccagctactg gggaggctga 1260
ggcaggagaa ttgcttcaac ctgcgaggct gaggttgcag tgagccgaga ttgcgccatt 1320
gcactccagc ctgggcaaca gagtgagact ctgtctcaaa aaaaaaaaaa aaaaaaaaag 1380
cataaactga aatttatatg caatttatat gcctgtgaga taattctgtt ttctcttttg 1440
gaaccccaaa gagatttttt tgattgatga gcaaatacat tttagatttt atttaagcat 1500
tatgccaagc accactgaag tataagtttc aagggcaaac tcagtttttt catctactag 1560
acgaatgatt ttctggaatg attacaagca ggcaagatgg tgtagtggaa atagcaaatg 1620
tcttcggcat cagacaagtt ggggtttgtt tgtatcctgc ctctgccctt caccgaggtt 1680
gtgatcttgg gcagattgtt gagttttaac ctagattcct ctgactccag atcataaatt 1740
ttcagaaaag ttctgaaatt cttgtatata ctgatggtaa atgagacttt tccttacatc 1800
tatgcacttc tttgtttgtt tgttttgaga tggtcttgct ctgttgccca gactggagtg 1860
cagtagtgca atctccgctc actacaatgt ctgcctccca ggttccagtg agcctcctgc 1920
ctcagcctcc caaatagctg agactacagg catgtgccac cacgtccggc taatttttgt 1980
atttttagta gagacagggt tttgccatgt tgaccacact ggtctcgaac tcctggcctc 2040
aggtgattcg cccgcctcag cctcccaaag tgctgggatt acaggcatga gccaccatgc 2100
ccggccatat ccatgcactt cttgcaacct taccttcttt tctcatcacc ctccagggac 2160
ctagttggaa gagcagagtt aaaagttaag gtgaaacttg gagaggtgtc ttgtccctag 2220
gaacaaagga ctggtttgaa attctctgta aatcttcccc agttcaaacc agagttatca 2280
aggtcttaaa aacttccctg ggtcctgaga gcccattata ttatttactt gtcttcctgt 2340
acacccactg cctagtcctg atcctacttt tgtttgcaaa taggatgggg cacaacgtac 2400
aaggaagggc ctttgccacc cctgctaagg gataacctga aataccttca ccatcactgc 2460
cctgtgctgc ttttcaccta tgccagtctg tctacagtgc cagtgtctcc tggcattgaa 2520
aggggagaat cttttggtcc tttgagtatt tggttgggtt acataaatct ccctgaatga 2580
agagcagctg acttaggcaa ggggccttgt ttggttttcc ttgaactatt aacaggaaga 2640
tagggagatt aactgtgtaa atgttcaata ggccagagtc cctgcagagg gtggccacag 2700
tgatcagatc ttatcacatc cttgctttgg gtgttgcctc tctggttgga gtatggatag 2760
aaaagaaaga aagaccctat attgaaatgc aaagtgcagc aagtcctgac tttggattaa 2820
cttctcagcc catttgcatg aaaataaaaa gatgaataaa acaaggttcc cactttggag 2880
ggaggtggta gctgtgagat ggaaggagtg ttcctgctgg gcaacagcag agtaagtgct 2940
ggggtagatt cactcccaca gtgcctggaa aatcctcata ggctcatttg ttgagtcttt 3000
gtcctacacc aggcactctg caaaaacgct ttgcctgcaa ggtctcatgc gatgctcacc 3060
acagctctgt gaagttaatt gtacttttat caccatttta cagatgagaa aactgagggt 3120
atggggtcaa tgacttggct aaagtcactg cttagcaagc tgcaggactt ggatgtgaat 3180
tccaattggt ttgactccaa agcctgtgaa gctacttgtt cttcaccacc tagagctgtg 3240
gttcttgata actgtgaact cttttggggt cacaaatagc cctgagaata tgatagaagc 3300
aggagctctg gcctttctgt ccatacctga acaggtcctt gggttaagag cccctcgtcc 3360
agggcctatt aatcttgatc ctcataagca gcatccatgt ataaaggccg caaaccaaac 3420
tgtgccagac cgaatcctag gaccaagccc aaatatgtcc catcatcctt ttggtaagaa 3480
gctcattgta agaaagaaag aggagagcaa gaggatgacc tagtgcatgg ggcctcattg 3540
ttttaattag tgacaaaaca acaataataa caacaaaacc cccgaagctt cacagatgac 3600
atcagacccc aagcctgtgt gtttttcagg tgcccttgag gagctttgta gctggcagag 3660

gaggtgaaac tgacaaatgt ttggcagatg gaggagagta ccagaggggt ttgagatgag 3720
ctaaattcca atctaaccgc aggtgtgagg aagaggcttg gattgggacc atggagatgg 3780
gggttctact cccagtcacg ccagctgact ttgcgagtgt tctttgtcag tcactttatc 3840
ttattttatt tatttttatt tttttgaaat ggagtttcgc tcttgtcgcc caggctggag 3900
tgaaatggcg cgatcttggc tcactgcaac ctccccctcc tgagttcaag cgattctcct 3960
gcctcagcct ccagagtacc tgggattaca ggcgcctgcc accaagccca tcgaattttt 4020
gtatgcttag tagagacagg gtttcgccat gttggccagg gtggtcttga actcctgacc 4080
tcaggtgatc cgcccacctt ggcctcccaa agtgctggga ttacaggcgc gagccactgt 4140
gcccagccca cttcatctta ccgtagttac ctccttagag tatgaaaaaa taggcttagg 4200
gcatccccaa gtcccctcta tgtctgagag ctgaggctgg ctgtcaaaga ggaactaagg 4260
atgccaggga ctttctgctt aggacccctc tcatcacttc tccaacgctg gtatcatgaa 4320
ccccattcta cagatgatgt ccactagatt aagaatggca tgtgaggcca agtttccacc 4380
tgagagtcag ttttattcag aagagacagg tctctgggat gtggggaatg ggacggacag 4440
acttggcatg aagcattgta taaatggagc ctcaaaatcg cttcagggaa ttaatgtttc 4500
tccctgtgtt tttctactcc tcgatttgaa cag 4533

<210> 39
<211> 1269
<212> DNA
<213> Homo sapiens

<400> 39
gtaagtatca gatcaggttt tctttccaaa cttgtcagtt aatccttttc cttcctttct 60
tgtcctctgg agaattttga atggctggat ttaagtgaag ttgtttttgt aaatgcttgt 120
gtgatagagt ctgcagaatg agggaaggga gaattttgga gaatttgggg tatttggggt 180
atccatcacc tcgagtattt atcatttctg tatgttgtga acatttcaag tcctgtctgc 240
tagctatttt ggaatatact atatgttgtt aatgatatca tgcagcagac gtgcatctga 300
atgggctggc tctaggagct agagggtagg ggctggcaca aagatgcatg ctggaagggt 360
ccttgcccat aagaagctga cagccaaggc taggggagtt ctgtcttctc tgcatcaggt 420
cacctctctc acctctgtca ctgccccatc agactacaat gtctgcaggt ctttctcccc 480
tgagtgtgag ctccctgagc aaagcaggat gctgcccctt ccctttgtat tcctggctcc 540
tggcttcagt gcctggacat aagtatgggc ataataagtg tcccccaaat gagacattga 600
ggattcttca aatgcacagg accgtgatgt gagttaggac ggagtaagga cgatgggatg 660
tggctcagga caatcctgag gaagctgcag ctgcggcacg cagggccaca ctgtcatgtt 720
catggaccct agactggctt tgtagcctcc atgggcccct tccatacaca aatattaaaa 780
attatatttc atgactgcat tggtataaag atgaatataa tccagaccag attcatgatt 840
attcatacat ttttagtgta ttaactttta attctgcttt taaaataaat taaaacattc 900
taatatgccc ttaagagtat cccaggccca ggccactgag cctactgtgg ttcatggata 960
agttggccct gggggcatgt gtgtgcatgc atgtgtgtgc acatgcatga tgagccgggc 1020
cttgaagggt ggtaagattt gggtgtgtag accaatggag aaaggcattt ggggcagtga 1080
tgatgggtgg gggagggaac atggtgatga atggagctgg gtgtggggag ccatgggagt 1140
gggttagggc cagcctgtgg aggacctggg agccaggctg agttctatgc acttggcagt 1200
cacttctgta aagcagcaga ggcagttggc ctagctaaag cctttcgcct tttcttgcac 1260
cctttacag 1269

<210> 40
<211> 951
<212> DNA
<213> Homo sapiens

<400> 40
   gtaagtaaaa accttctctg catccgttta taattggaaa ttgacctgca ccagggaaag 60
   agagtagccc aggtgtctgg ggcttgttcc cattagatct tccccaaggg gtttttctcc 120
   ttggtggctg gcctgtgggg cccctctcca ggaggcattg gtgaagaaac taggggagct 180
   ggttgccaca gacagtgatg tactaatctt ctctgggaag acagaagaaa agtccccagg 240
   gaagaatact acagacttgg ccttagggac agctaggggt gcagattgct gccaactgca 300
   ttttttctga agttggccat atggttgcag tgaatggatt tatagacaga gtatttctgt 360
   gcatataaga gcaattacag ttgtaagttg atatggataa gtgaaagtta agcacttctt 420
   tctaaaaaga gaatgcaatt cattttcccc taatcatttc aattagtctg atgggcattt 480
   gaacttgttg tctttaaaaa gtgaaatctt tacctctgat ctggtaagta tccaggcaat 540
   ttcttgtgtg ccacccagga ggtatctggg gagtgggcat tttctgactg aggcattggc 600
   tgccatagca tcagagcagc cttccaggca gtggcctggc aaggggacag aggctggtgg 660
   gagcagctgg ctgagtgcag ccagtaatgg catgtgcatg gtctgtagag aatgtagaag 720
   caataatgaa gccgataaaa gctggtctgc attttattat tatcatgcgc cggtggttct 780
   aaacaatgtc agtgataaat tactcctccc catcatggac caatggctgc cactgctcca 840
   gggaagtgct ttttattccg tttggtgttt agggagggat ggagttggct ggcctttgct 900
   gaaaggccta ccagtttgtt ttctatttgg caaaagaaga aatgataaag t 951

<210> 41
<211> 253
<212> DNA
<213> Homo sapiens

<400> 41
ttcntggttc cgtttttttc ttcctttcag gtgtcgtgaa aagcttgaat tcggcgcgcc 60
agatatcaca cgtgccaagg ggctggctca ctgatatggc gatgctcccc tatctcattc 120
ctgtgtgtgt ttagccaggc aactgttgat catcaatatt atgataacgt ttctccactg 180
tcccattgtg cccacttttt tttttttttt gaggttactt actaaataaa aataaaacac 240
tatttctcaa tag 253

<210> 42
<211> 196
<212> DNA
<213> Homo sapiens

<400> 42
gtaagctgat gcctccagct tcctcagtag ggctgatggc aattacgttg tgcagctact 60
ggaaagaaat gaataaaccc ttgtccttgt aatggtggtg aaggggaggg aggtagtttg 120
aatacaactt cacttaattt tacttcccta ttcaggcagg aattgccaaa ccatccagga 180
ctccaatatg caacct 196

<210> 43
<211> 160
<212> DNA
<213> Homo sapiens

<400> 43
gtcagtaaag agacctgatg cattggactg ttgcaatgga acctgtttta agatcttcaa 60
agctgtattg atatgaagtt ctccaaaaga cttcaaggac ccagcttcca atcttcataa 120
tcctcttgtg cttgtctctc tttgcatgaa atgcttccag 160

<210> 44
<211> 91
<212> DNA
<213> Homo sapiens

<400> 44
gtgagtagac ttgctcactg gagaaacttc aagcactaat gctttcggaa tgtgaggctt 60
ttccttggac agcatgactt tgttttgtag c 91

<210> 45
<211> 106
<212> DNA
<213> Homo sapiens

<400> 45
tgttaaactc aagattgtga acgagtttta attcacttgt ttatttccaa tagagatttc 60
aggtttacat ttgaattcag aaacaaagtt ttctttctca ttacag 106

<210> 46
<211> 14
<212> DNA
<213> Homo sapiens

<400> 46
gtaagttgtg tctt 14

<210> 47
<211> 603
<212> DNA
<213> Homo sapiens

<400> 47
ttcattgcnt gacactttac tgagaccaag atagggaact tcacatacca gaccttttct 60
cccaaggcgg aagagggctg ttcaatttct acactagagt tcggggagtt ttagaaatga 120
gccagttgtc gaggatgaga gcagttcctg ataggctcaa ccacaatgag atgtagctgt 180
tcagagaaag cattcttatc tataaactgg aagataatcc cggtgaaaca aagcccagcc 240
ccaggggctt cactaactcc aggctgtgct tctcaaactt tagtgagcat aggaatcacc 300
tgggcatctt gtgaagctgt agatttgaat tctgcaggtc ggcagagggg tctcagaatc 360
cgcatttcca acaatgtctc cagtaatgct gatgctgctc gtccctggac cacagattgg 420
gtagccaggt tctggcaagc tcatcccaag gctttgagat gacatcagac aaaatatgtt 480
ctgggacatg gcttttgaga ggtcaagaaa ataagatgtt tctttctctt ctcatcccca 540
acccttgcac tgcccttttc tcccttcccc taccctcctt tctgtcccca tccctgacgc 600
cag 603

<210> 48
<211> 558
<212> DNA
<213> Homo sapiens

<400> 48
gtgagtgtcc atgcagaccc cagccctgtc cccaacccca tccctccctt agttctggcc 60
ttggcctgtg tcatctcctc cctctgtagc agcgttagat gtctacatgc ccatttgccc 120
accagactga gctcttccta gaggagagag gcttctcttg aatagctacc tgtccccagt 180
tctctgaatg cagcctggca catctcaggt gcacagtagt gtttatcaat ggaatgaatg 240
attgacagcc aaccttctgg ttttctgggg gatgtggaag ggtggcttcc agggtgatca 300
agaatgagat aatggcagaa ggacaaatcc tgcaagatct cacttatata tggaatatat 360
gtaaggtaga aagtgtcagt ttcacatgat gaataagttc ctgggatctt gatgtacatc 420
gtgatgacta tagttagtaa cactgtatag tatacttgaa atttgctaag agagtagatc 480
cgaagtgttc acactacaca aaaaaggcaa ctatgaggtg atggatttat taacaagctt 540
ggattgnggt gatccttt 558

<210> 49

<211> 614
<212> DNA
<213> Homo sapiens

<400> 49
cctantttgg tatttttaga aaagacaggg tttttccatg ttggtcaggc tggtctcgaa 60
ctcccaccct caggtggttc gcccgccttg gcctcccaaa gtgctgggat tgcaggcatg 120
agccactgcg cccagcccca aattttggtt tttgcttgaa aactgaggtc tgaattcagc 180
cttctggttg cccctcaaga gtcagtttaa atgttggtca tgttagttgt cagtgaaaac 240
aatggtgagg ctggcatgag agtgtgaacc tggatgggag ggcttgtgct tcacgaaaac 300
atttttccag atcagctcag tcgtgagtta tccgtcattg acgttataat aagctctgat 360
tatttatcaa gcatcattct ttatagatat ctcagtttaa tctgagataa tcttctccac 420
atctctccac atagatgtta tgaattttac ttttacagag gagccaactg aggctcagat 480
aagttactta ttatatgact agtagtggna gagctggggt ttcaaactaa gaactntctg 540
gctccaaagc ccttgtaagt ttctatcagt atatgaccat gcatatgagc atttgtctct 600
cctcttcttc atag 614

<210> 50
<211> 329
<212> DNA
<213> Homo sapiens

<400> 50
gtaagctgcc cccagcccaa gactccctcc ccagaatctc cccagaactg ggggcaaaaa 60
actcaaggta gcttcagagg tgtgcgctaa gtatactcac ggctcttctg gaattcccag 120
agtgaaaacc tcaagtctga tgcagaccag agctgggcca gctccccagt cgtgggtata 180
gaatcatagt tacaagcagg catttcttgg ggatggggag gactggcaca gggctgctgt 240
gatggggtat cttttcaggg aggagccaaa gcgctcattg tctgtgcttc tcctttttct 300
gcggtccctg gctccccacc tgactccag 329

<210> 51
<211> 608
<212> DNA
<213> Homo sapiens

<400> 51
gtgagtgtcc ctcccattat taccatgtgc ctgcttgata ctggagaggt gagtttctgg 60
tcactttccc aggtgtgagt gaggtgagaa ttctttcagt ttatctagct gggggaaatt 120
gtagtgagca tagctaaaag tcacatgggg caccaacctc tccatgaagt acaggccatg 180
gtgcaagaga taacgcntgt gcatatnagc catcccatgc ccacntcanc ggtccaaata 240
agcagttttc tgcaaaactt agtgagggct ggtgtttgga agtggagttg agtaattgca 300
gtaccctatt ttcctttttg gctgcagcct ctcagccagc cacagcatct ccctgtgtct 360
tggtaggttt tggaaagaag tgtgggagca aaagcatgat gttacatgta gactggcctg 420
agatactcat tctcagggca ctgtgtgaat gatgagctgc tgttactgtg tggaggggaa 480
atgcacttag tgcttcagag ccacttgaaa gggataagtg ctctagagac aattggattc 540
aaatgtggag caggctgagc aagaacagaa tgtctccttt gcctgagcct gagtgctggt 600
aatcacat 608

<210> 52
<211> 15
<212> DNA
<213> Homo sapiens

<400> 52
gtaatctgtt gctgg 15

<210> 53
<211> 639
<212> DNA
<213> Homo sapiens

<400> 53
tttggtcctt ggcaaagttc acaaatactg gatttgataa caaaaggnac ttaataaatg 60
ttagttgagt ggttgaatga gtaaataaac ctctagcttt agtaaattaa ctctagcctt 120
attctatata ggctcaagag aatatttcta cccattttct tctaggtttt cctatctcag 180
tgactaatgg tagcaaagca ttcccttaaa aaggcattat ttgtgaaact tatctaaaat 240
cgaattcggg tccaattaaa tttttgaaat tttatattaa aaattatatt agtagggatg 300
ggtaagaggt gttttggtct ggttggttgg ttagttgcta tgactcagaa ttgctaagaa 360
aacagaaaag taagataaga tcattgtttt aacctctttt cctccacaaa atcaataaat 420
aacatatccc taaattactc ttagaatttc tcttaaattg cagtgaaaaa ccaaaatcct 480
tcattcttgg ttgaaggttg gaaaactacg ttagagagga ttagagagag aggatgagca 540
atcgtgtagt cagcccttgc ctcctagtgt aggatttgtc tcagccactg cttgttgtcc 600
tggctgccaa cgttctcatg aaggctgttc ttctatcag 639

<210> 54
<211> 387
<212> DNA
<213> Homo sapiens

<400> 54
ttagatctat ccaagttgtg tgtgtcaaca gttcattcct ctntcactgc tgagtagtgt 60
tccctgggag gggtgtatca cagttccatg gcanttttta gatgtatttt ttaaacagct 120
ttcagcatcc tctattttaa ttgtntcatc aagtcctttt tcccaataga ctctgaatgc 180
tcctttatca tcgtanttcc catcaccaac atcagtaccc aaataggccc taaataaaca 240
tttatangcc tcctgcctgc ctgagaaacc agggtggaca tggagagaag gcacttcntg 300
aaagttcaag cgcagtgccc tgtgtcctta cactccactc ctcagtgcnt ttctgtgggt 360
tcatttctgt cttctctcct gtcacag 387

<210> 55
<211> 19
<212> DNA
<213> Homo sapiens

<400> 55
gtcaatgagg gcgggtagc 19

<210> 56
<211> 3
<212> DNA
<213> Homo sapiens

<400> 56
tag 3

<210> 57
<211> 257
<212> DNA
<213> Homo sapiens

<400> 57
gtgagtgaag cagtggctgt aggatgcttt aatggagatg gcactctgca taggccttgg 60
taccctgaac tttgttttgg aaagaagcag gtgactaagc acaggatgtt cccccacccc 120
catgcccagt aacagggctc atgccaacac agctggttgt ggcatgggtt ttgtgacaca 180
accatttgtc tgtgtctctg atagcattag aaaagtaaag ggcagtttta aggtaaggaa 240
aaaggttttg ctggaca 257

<210> 58
<211> 265
<212> DNA
<213> Homo sapiens

<400> 58
tggctagctg gctttcatta ctgtttttca cacattaact ttggcctcaa cttgacaact 60
caaataatat ttataaatac agccacactt aaaatggtcc cattatgaaa tacatattta 120
aatatctata cgatgtgtta aaaccaagaa aatatttgat tcttctctga tatttaagaa 180
ttgaaggttt gaggtagtta cgtgttaggg gcatttatat tcatgttttt agagtntgct 240
tancaactta atctttcctt ttcag 265

<210> 59
<211> 14
<212> DNA
<213> Homo sapiens

<400> 59
gtgagaaaag aagt 14

<210> 60
<211> 15
<212> DNA
<213> Homo sapiens

<400> 60
tcttgtcctt tgcag 15

<210> 61
<211> 188
<212> DNA
<213> Homo sapiens

<400> 61
gtactagtgn ggcacctgaa agccagccta gtctcctttg gcatcctaga caatatatac 60
cttatgagct ttcccacacg ccattagact tcagagctag tttttccttc atgatannca 120
gcagcacaaa atgcctgagt tctttgtatc tgctttcaga ngtnggaaac cntggtaacg 180
agtgagtg 188

<210> 62
<211> 176
<212> DNA
<213> Homo sapiens

<400> 62

gtgcctgctc ccaccacacg agtcccttct ccctgctttg gctcctcacc agttgtcagg 60
ttatgattat agaatctagt cctactcagt gaaagaactt tcatacatgt atgtgtagga 120
cagcatgata aaattcccaa gccagaccaa agtcaaggtg ctttttatca ctgtag 176

<210> 63
<211> 307
<212> DNA
<213> Homo sapiens

<400> 63
gtataactgt ggatggaaaa ctgttgttct ggcctgagtg gaaaacatga ctgttcaaaa 60
gtcctatatg tccagggctg ttgtatgatt ggcttgtctt cccccaggga cagcagagca 120
accttggaaa agcagaggga agcttctccc ttggcacaca ctggggtggc tgtaccatgc 180
ctgcagatgc tcccaaatag aggcactcca agcacntttg tttcttagcg tgattgaggc 240
tggatatgtg atttgatctt tctctggaac attctttcta atcatctttg tgttcattcc 300
ctgaaaa 307

<210> 64
<211> 376
<212> DNA
<213> Homo sapiens

<400> 64
aaaggaattt caccccaatc ttatactgac ttcaatagag ctttcagaca aaaagttgtt 60
ttgtatatac tnatcagtca tgaaaagata attacaacta aatggccttt ttccttccct 120
atttatttgg agaaatttaa ttacataaga aagtactcag aatatgtgag tttcctgcat 180
caataagaca tttataataa tgaccntgtt tacaaatgaa tttgaaagtn actctaattc 240
tttgattcat caagaaataa ctagaatggc aagttaaaat ttaagctgtt tcaaagatgc 300
ttcngcantt agaaactcnn ttatctttga tnttctgtcc cncnagcaaa taanacttan 360
tntattntna tancag 376

<210> 65
<211> 482
<212> DNA
<213> Homo sapiens

<400> 65
gtccgtagta aagtcttggg ttcctcactg tgggatgttt taactttcca agtagaatat 60
gcgatcattt tgtaaaaatt agaaaataca gaaaagcaaa gagtaaaaca attattacct 120
gaaattatat atgcatattc ttacaaaaat gcaagcccag tataaatact gctctttttc 180
acttaatata ttgtaaacat tattccaagt cagtgcattt aggtgtcatt tcttatagct 240
ggatagtatt ccattaggat atactcttat ttaactattc ccccttttgt agacatttgg 300
attatttcca acttgttcac aattgtanac nccaccacac tgaacagcat catccctata 360
tccacatgna cttgtaacag aatacaattc cctaggaagc tggaatgctg gaagtcatgg 420
tgatgttctc atggtnacag agaatctctc taganctaan acctctctct gttttaccgc 480
ag 482

<210> 66
<211> 47
<212> DNA
<213> Artificial Sequence

<220>
<223> Description of Artificial Sequence: Multiple

Cloining Site

<400> 66
cggccgcggc gcgcccggac cgcctaggat ttaaatcgcg gcccgcg 47

<210> 67
<211> 69
<212> DNA
<213> Artificial Sequence

<220>
<223> Description of Artificial Sequence: Multiple
      Cloning Site

<400> 67
gctctagaat tcggcctccg tggccgttta aacgctagcg cccgggctta attaagtcga 60
ctctagagc 69

<210> 68
<211> 194
<212> DNA
<213> Homo sapiens

<400> 68
aagtctattt ttaagttgtt attgcttttt tcaagtattc ttccctcctt cacacacagt 60
tttctagtta atccatttat gtaattctgt atgctcctac ttgacctaat ttcaacatct 120
ggaaaaatag aactagaata aagaatgagc aagttgagtg gtatttataa aggtccatct 180
taatctttta acag 194

<210> 69
<211> 9741
<212> DNA
<213> Homo sapiens

<400> 69
cttgtttttt ccccggttct gttttctccc cttctccgga aggcttgtca aggggtagga 60
gaaagagacg caaacacaaa agtggaaaac agttaatgac cagccacggc gtccctgctg 120
tgagctctgg ccgctgcctt ccagggctcc cgagccacac gctgggggtg ctggctgagg 180
gaacatggct tgttggcctc agctgaggtt gctgctgtgg aagaacctca ctttcagaag 240
aagacaaaca tgtcagctgc tgctggaagt ggcctggcct ctatttatct tcctgatcct 300
gatctctgtt cggctgagct acccacccta tgaacaacat gaatgccatt ttccaaataa 360
agccatgccc tctgcaggaa cacttccttg ggttcagggg attatctgta atgccaacaa 420
cccctgtttc cgttacccga ctcctgggga ggctcccgga gttgttggaa actttaacaa 480
atccattgtg gctcgcctgt tctcagatgc tcggaggctt cttttataca gccagaaaga 540
caccagcatg aaggacatgc gcaaagttct gagaacatta cagcagatca agaaatccag 600
ctcaaacttg aagcttcaag atttcctggt ggacaatgaa accttctctg ggttcctgta 660
tcacaacctc tctctcccaa agtctactgt ggacaagatg ctgagggctg atgtcattct 720
ccacaaggta tttttgcaag gctaccagtt acatttgaca agtctgtgca atggatcaaa 780
atcagaagag atgattcaac ttggtgacca agaagtttct gagctttgtg gcctaccaag 840
ggagaaactg gctgcagcag agcgagtact tcgttccaac atggacatcc tgaagccaat 900
cctgagaaca ctaaactcta catctccctt cccgagcaag gagctggccg aagccacaaa 960
aacattgctg catagtcttg ggactctggc ccaggagctg ttcagcatga gaagctggag 1020
tgacatgcga caggaggtga tgtttctgac caatgtgaac agctccagct cctccaccca 1080
aatctaccag gctgtgtctc gtattgtctg cgggcatccc gagggagggg ggctgaagat 1140
caagtctctc aactggtatg aggacaacaa ctacaaagcc ctctttggag gcaatggcac 1200

tgaggaagat gctgaaacct tctatgacaa ctctacaact ccttactgca atgatttgat 1260
gaagaatttg gagtctagtc ctctttcccg cattatctgg aaagctctga agccgctgct 1320
cgttgggaag atcctgtata cacctgacac tccagccaca aggcaggtca tggctgaggt 1380
gaacaagacc ttccaggaac tggctgtgtt ccatgatctg gaaggcatgt gggaggaact 1440
cagccccaag atctggacct tcatggagaa cagccaagaa atggaccttg tccggatgct 1500
gttggacagc agggacaatg accacttttg ggaacagcag ttggatggct tagattggac 1560
agcccaagac atcgtggcgt ttttggccaa gcacccagag gatgtccagt ccagtaatgg 1620
ttctgtgtac acctggagag aagctttcaa cgagactaac caggcaatcc ggaccatatc 1680
tcgcttcatg gagtgtgtca acctgaacaa gctagaaccc atagcaacag aagtctggct 1740
catcaacaag tccatggagc tgctggatga gaggaagttc tgggctggta ttgtgttcac 1800
tggaattact ccaggcagca ttgagctgcc ccatcatgtc aagtacaaga tccgaatgga 1860
cattgacaat gtggagagga caaataaaat caaggatggg tactgggacc ctggtcctcg 1920
agctgacccc tttgaggaca tgcggtacgt ctgggggggc ttcgcctact tgcaggatgt 1980
ggtggagcag gcaatcatca gggtgctgac gggcaccgag aagaaaactg gtgtctatat 2040
gcaacagatg ccctatccct gttacgttga tgacatcttt ctgcgggtga tgagccggtc 2100
aatgcccctc ttcatgacgc tggcctggat ttactcagtg gctgtgatca tcaagggcat 2160
cgtgtatgag aaggaggcac ggctgaaaga gaccatgcgg atcatgggcc tggacaacag 2220
catcctctgg tttagctggt tcattagtag cctcattcct cttcttgtga gcgctggcct 2280
gctagtggtc atcctgaagt taggaaacct gctgccctac agtgatccca gcgtggtgtt 2340
tgtcttcctg tccgtgtttg ctgtggtgac aatcctgcag tgcttcctga ttagcacact 2400
cttctccaga gccaacctgg cagcagcctg tgggggcatc atctacttca cgctgtacct 2460
gccctacgtc ctgtgtgtgg catggcagga ctacgtgggc ttcacactca agatcttcgc 2520
tagcctgctg tctcctgtgg cttttgggtt tggctgtgag tactttgccc tttttgagga 2580
gcagggcatt ggagtgcagt gggacaacct gtttgagagt cctgtggagg aagatggctt 2640
caatctcacc acttcggtct ccatgatgct gtttgacacc ttcctctatg gggtgatgac 2700
ctggtacatt gaggctgtct ttccaggcca gtacggaatt cccaggccct ggtattttcc 2760
ttgcaccaag tcctactggt ttggcgagga aagtgatgag aagagccacc ctggttccaa 2820
ccagaagaga atatcagaaa tctgcatgga ggaggaaccc acccacttga agctgggcgt 2880
gtccattcag aacctggtaa aagtctaccg agatgggatg aaggtggctg tcgatggcct 2940
ggcactgaat ttttatgagg gccagatcac ctccttcctg ggccacaatg gagcggggaa 3000
gacgaccacc atgtcaatcc tgaccgggtt gttccccccg acctcgggca ccgcctacat 3060
cctgggaaaa gacattcgct ctgagatgag caccatccgg cagaacctgg gggtctgtcc 3120
ccagcataac gtgctgtttg acatgctgac tgtcgaagaa cacatctggt tctatgcccg 3180
cttgaaaggg ctctctgaga agcacgtgaa ggcggagatg gagcagatgg ccctggatgt 3240
tggtttgcca tcaagcaagc tgaaaagcaa aacaagccag ctgtcaggtg gaatgcagag 3300
aaagctatct gtggccttgg cctttgtcgg gggatctaag gttgtcattc tggatgaacc 3360
cacagctggt gtggaccctt actcccgcag gggaatatgg gagctgctgc tgaaataccg 3420
acaaggccgc accattattc tctctacaca ccacatggat gaagcggacg tcctggggga 3480
caggattgcc atcatctccc atgggaagct gtgctgtgtg ggctcctccc tgtttctgaa 3540
gaaccagctg ggaacaggct actacctgac cttggtcaag aaagatgtgg aatcctccct 3600
cagttcctgc agaaacagta gtagcactgt gtcatacctg aaaaaggagg acagtgtttc 3660
tcagagcagt tctgatgctg gcctgggcag cgaccatgag agtgacacgc tgaccatcga 3720
tgtctctgct atctccaacc tcatcaggaa gcatgtgtct gaagcccggc tggtggaaga 3780
catagggcat gagctgacct atgtgctgcc atatgaagct gctaaggagg gagcctttgt 3840
ggaactcttt catgagattg atgaccggct ctcagacctg ggcatttcta gttatggcat 3900
ctcagagacg accctggaag aaatattcct caaggtggcc gaagagagtg gggtggatgc 3960
tgagacctca gatggtacct tgccagcaag acgaaacagg cgggccttcg gggacaagca 4020
gagctgtctt cgcccgttca ctgaagatga tgctgctgat ccaaatgatt ctgacataga 4080
cccagaatcc agagagacag acttgctcag tgggatggat ggcaaagggt cctaccaggt 4140
gaaaggctgg aaacttacac agcaacagtt tgtggccctt ttgtggaaga gactgctaat 4200
tgccagacgg agtcggaaag gattttttgc tcagattgtc ttgccagctg tgtttgtctg 4260
cattgccctt gtgttcagcc tgatcgtgcc accctttggc aagtacccca gcctggaact 4320
tcagccctgg atgtacaacg aacagtacac atttgtcagc aatgatgctc ctgaggacac 4380
gggaaccctg gaactcttaa acgccctcac caaagaccct ggcttcggga cccgctgtat 4440
ggaaggaaac ccaatcccag acacgccctg ccaggcaggg gaggaagagt ggaccactgc 4500
cccagttccc cagaccatca tggacctctt ccagaatggg aactggacaa tgcagaaccc 4560
ttcacctgca tgccagtgta gcagcgacaa aatcaagaag atgctgcctg tgtgtccccc 4620
aggggcaggg gggctgcctc ctccacaaag aaaacaaaac actgcagata tccttcagga 4680

cctgacagga agaaacattt cggattatct ggtgaagacg tatgtgcaga tcatagccaa 4740
aagcttaaag aacaagatct gggtgaatga gtttaggtat ggcggctttt ccctgggtgt 4800
cagtaatact caagcacttc ctccgagtca agaagttaat gatgccacca aacaaatgaa 4860
gaaacaccta aagctggcca aggacagttc tgcagatcga tttctcaaca gcttgggaag 4920
atttatgaca ggactggaca ccagaaataa tgtcaaggtg tggttcaata acaagggctg 4980
gcatgcaatc agctctttcc tgaatgtcat caacaatgcc attctccggg ccaacctgca 5040
aaagggagag aaccctagcc attatggaat tactgctttc aatcatcccc tgaatctcac 5100
caagcagcag ctctcagagg tggctccgat gaccacatca gtggatgtcc ttgtgtccat 5160
ctgtgtcatc tttgcaatgt ccttcgtccc agccagcttt gtcgtattcc tgatccagga 5220
gcgggtcagc aaagcaaaac acctgcagtt catcagtgga gtgaagcctg tcatctactg 5280
gctctctaat tttgtctggg atatgtgcaa ttacgttgtc cctgccacac tggtcattat 5340
catcttcatc tgcttccagc agaagtccta tgtgtcctcc accaatctgc ctgtgctagc 5400
ccttctactt ttgctgtatg ggtggtcaat cacacctctc atgtacccag cctcctttgt 5460
gttcaagatc cccagcacag cctatgtggt gctcaccagc gtgaacctct tcattggcat 5520
taatggcagc gtggccacct ttgtgctgga gctgttcacc gacaataagc tgaataatat 5580
caatgatatc ctgaagtccg tgttcttgat cttcccacat ttttgcctgg gacgagggct 5640
catcgacatg gtgaaaaacc aggcaatggc tgatgccctg gaaaggtttg gggagaatcg 5700
ctttgtgtca ccattatctt gggacttggt gggacgaaac ctcttcgcca tggccgtgga 5760
aggggtggtg ttcttcctca ttactgttct gatccagtac agattcttca tcaggcccag 5820
acctgtaaat gcaaagctat ctcctctgaa tgatgaagat gaagatgtga ggcgggaaag 5880
acagagaatt cttgatggtg gaggccagaa tgacatctta gaaatcaagg agttgacgaa 5940
gatatataga aggaagcgga agcctgctgt tgacaggatt tgcgtgggca ttcctcctgg 6000
tgagtgcttt gggctcctgg gagttaatgg ggctggaaaa tcatcaactt tcaagatgtt 6060
aacaggagat accactgtta ccagaggaga tgctttcctt aacagaaata gtatcttatc 6120
aaacatccat gaagtacatc agaacatggg ctactgccct cagtttgatg ccatcacaga 6180
gctgttgact gggagagaac acgtggagtt ctttgccctt ttgagaggag tcccagagaa 6240
agaagttggc aaggttggtg agtgggcgat tcggaaactg ggcctcgtga agtatggaga 6300
aaaatatgct ggtaactata gtggaggcaa caaacgcaag ctctctacag ccatggcttt 6360
gatcggcggg cctcctgtgg tgtttctgga tgaacccacc acaggcatgg atcccaaagc 6420
ccggcggttc ttgtggaatt gtgccctaag tgttgtcaag gaggggagat cagtagtgct 6480
tacatctcat agtatggaag aatgtgaagc tctttgcact aggatggcaa tcatggtcaa 6540
tggaaggttc aggtgccttg gcagtgtcca gcatctaaaa aataggtttg gagatggtta 6600
tacaatagtt gtacgaatag cagggtccaa cccggacctg aagcctgtcc aggatttctt 6660
tggacttgca tttcctggaa gtgttccaaa agagaaacac cggaacatgc tacaatacca 6720
gcttccatct tcattatctt ctctggccag gatattcagc atcctctccc agagcaaaaa 6780
gcgactccac atagaagact actctgtttc tcagacaaca cttgaccaag tatttgtgaa 6840
ctttgccaag gaccaaagtg atgatgacca cttaaaagac ctctcattac acaaaaacca 6900
gacagtagtg gacgttgcag ttctcacatc ttttctacag gatgagaaag tgaaagaaag 6960
ctatgtatga agaatcctgt tcatacgggg tggctgaaag taaagaggna ctagactttc 7020
ctttgcacca tgtgaagtgt tgtggagaaa agagccagaa gttgatgtgg gaagaagtaa 7080
actggatact gtactgatac tattcaatgc aatgcaattc aatgcaatga aaacaaaatt 7140
ccattacagg ggcagtgcct ttgtagccta tgtcttgtat ggctctcaag tgaaagactt 7200
gaatttagtt ttttacctat acctatgtga aactctatta tggaacccaa tggacatatg 7260
ggtttgaact cacacttttt tttttttttt gttcctgtgt attctcattg gggttgcaac 7320
aataattcat caagtaatca tggccagcga ttattgatca aaatcaaaag gtaatgcaca 7380
tcctcattca ctaagccatg ccatgcccag gagactggtt tcccggtgac acatccattg 7440
ctggcaatga gtgtgccaga gttattagtg ccaagttttt cagaaagttt gaagcaccat 7500
ggtgtgtcat gctcactttt gtgaaagctg ctctgctcag agtctatcaa cattgaatat 7560
cagttgacag aatggtgcca tgcgtggcta acatcctgct ttgattccct ctgataagct 7620
gttctggtgg cagtaacatg caacaaaaat gtgggtgtct ctaggcacgg gaaacttggt 7680
tccattgtta tattgtccta tgcttcgagc catgggtcta cagggtcatc cttatgagac 7740
tcttaaatat acttagatcc tggtaagagg caaagaatca acagccaaac tgctggggct 7800
gcaagctgct gaagccaggg catgggatta aagagattgt gcgttcaaac ctagggaagc 7860
ctgtgcccat ttgtcctgac tgtctgctaa catggtacac tgcatctcaa gatgtttatc 7920
tgacacaagt gtattatttc tggctttttg aattaatcta gaaaatgaaa agatggagtt 7980
gtattttgac aaaaatgttt gtacttttta atgttatttg gaattttaag ttctatcagt 8040
gacttctgaa tccttagaat ggcctctttg tagaaccctg tggtatagag gagtatggcc 8100
actgccccac tatttttatt ttcttatgta agtttgcata tcagtcatga ctagtgccta 8160

gaaagcaatg tgatggtcag gatctcatga cattatattt gagtttcttt cagatcattt 8220
aggatactct taatctcact tcatcaatca aatatttttt gagtgtatgc tgtagctgaa 8280
agagtatgta cgtacgtata agactagaga gatattaagt ctcagtacac ttcctgtgcc 8340
atgttattca gctcactggt ttacaaatat aggttgtctt gtggttgtag gagcccactg 8400
taacaatact gggcagcctt tttttttttt tttaattgca acaatgcaaa agccaagaaa 8460
gtataagggt cacaagtcta aacaatgaat tcttcaacag ggaaaacagc tagcttgaaa 8520
acttgctgaa aaacacaact tgtgtttatg gcatttagta ccttcaaata attggctttg 8580
cagatattgg ataccccatt aaatctgaca gtctcaaatt tttcatctct tcaatcacta 8640
gtcaagaaaa atataaaaac aacaaatact tccatatgga gcatttttca gagttttcta 8700
acccagtctt atttttctag tcagtaaaca tttgtaaaaa tactgtttca ctaatactta 8760
ctgttaactg tcttgagaga aaagaaaaat atgagagaac tattgtttgg ggaagttcaa 8820
gtgatctttc aatatcatta ctaacttctt ccactttttc caaaatttga atattaacgc 8880
taaaggtgta agacttcaga tttcaaatta atctttctat attttttaaa tttacagaat 8940
attatataac ccactgctga aaaagaaaaa aatgattgtt ttagaagtta aagtcaatat 9000
tgattttaaa tataagtaat gaaggcatat ttccaataac tagtgatatg gcatcgttgc 9060
attttacagt atcttcaaaa atacagaatt tatagaataa tttctcctca tttaatattt 9120
ttcaaaatca aagttatggt ttcctcattt tactaaaatc gtattctaat tcttcattat 9180
agtaaatcta tgagcaactc cttacttcgg ttcctctgat ttcaaggcca tattttaaaa 9240
aatcaaaagg cactgtgaac tattttgaag aaaacacaac attttaatac agattgaaag 9300
gacctcttct gaagctagaa acaatctata gttatacatc ttcattaata ctgtgttacc 9360
ttttaaaata gtaatttttt acattttcct gtgtaaacct aattgtggta gaaattttta 9420
ccaactctat actcaatcaa gcaaaatttc tgtatattcc ctgtggaatg tacctatgtg 9480
agtttcagaa attctcaaaa tacgtgttca aaaatttctg cttttgcatc tttgggacac 9540
ctcagaaaac ttattaacaa ctgtgaatat gagaaataca gaagaaaata ataagccctc 9600
tatacataaa tgcccagcac aattcattgt taaaaaacaa ccaaacctca cactactgta 9660
tttcattatc tgtactgaaa gcaaatgctt tgtgactatt aaatgttgca catcattcat 9720
tcaaaaaaaa aaaaaaaaaa a 9741

<210> 70
<211> 9854
<212> DNA
<213> Homo sapiens

<400> 70
gtgagtgggg ccgggacccg cagagccgag ccgacccttc tctcccgggc tgcggcaggg 60
cagggcgggg agctccgcgc accaacagag ccggttctca gggcgctttg ctccttgttt 120
tttccccggt tctgttttct ccccttctcc ggaaggcttg tcaaggggta ggagaaagag 180
acgcaaacac aaaagtggaa aacagttaat gaccagccac ggcgtccctg ctgtgagctc 240
tggccgctgc cttccagggc tcccgagcca cacgctgggg gtgctggctg agggaacatg 300
gcttgttggc ctcagctgag gttgctgctg tggaagaacc tcactttcag aagaagacaa 360
acatgtcagc tgctgctgga agtggcctgg cctctattta tcttcctgat cctgatctct 420
gttcggctga gctacccacc ctatgaacaa catgaatgcc attttccaaa taaagccatg 480
ccctctgcag gaacacttcc ttgggttcag gggattatct gtaatgccaa caacccctgt 540
ttccgttacc cgactcctgg ggaggctccc ggagttgttg gaaactttaa caaatccatt 600
gtggctcgcc tgttctcaga tgctcggagg cttcttttat acagccagaa agacaccagc 660
atgaaggaca tgcgcaaagt tctgagaaca ttacagcaga tcaagaaatc cagctcaaac 720
ttgaagcttc aagatttcct ggtggacaat gaaaccttct ctgggttcct gtatcacaac 780
ctctctctcc caaagtctac tgtggacaag atgctgaggg ctgatgtcat tctccacaag 840
gtatttttgc aaggctacca gttacatttg acaagtctgt gcaatggatc aaaatcagaa 900
gagatgattc aacttggtga ccaagaagtt tctgagcttt gtggcctacc aagggagaaa 960
ctggctgcag cagagcgagt acttcgttcc aacatggaca tcctgaagcc aatcctgaga 1020
acactaaact ctacatctcc cttcccgagc aaggagctgg ccgaagccac aaaaacattg 1080
ctgcatagtc ttgggactct ggcccaggag ctgttcagca tgagaagctg gagtgacatg 1140
cgacaggagg tgatgtttct gaccaatgtg aacagctcca gctcctccac ccaaatctac 1200
caggctgtgt ctcgtattgt ctgcgggcat cccgagggag gggggctgaa gatcaagtct 1260
ctcaactggt atgaggacaa caactacaaa gccctctttg gaggcaatgg cactgaggaa 1320
gatgctgaaa ccttctatga caactctaca actccttact gcaatgattt gatgaagaat 1380

ttggagtcta gtcctctttc ccgcattatc tggaaagctc tgaagccgct gctcgttggg 1440
aagatcctgt atacacctga cactccagcc acaaggcagg tcatggctga ggtgaacaag 1500
accttccagg aactggctgt gttccatgat ctggaaggca tgtgggagga actcagcccc 1560
aagatctgga ccttcatgga gaacagccaa gaaatggacc ttgtccggat gctgttggac 1620
agcagggaca atgaccactt ttgggaacag cagttggatg gcttagattg gacagcccaa 1680
gacatcgtgg cgtttttggc caagcaccca gaggatgtcc agtccagtaa tggttctgtg 1740
tacacctgga gagaagcttt caacgagact aaccaggcaa tccggaccat atctcgcttc 1800
atggagtgtg tcaacctgaa caagctagaa cccatagcaa cagaagtctg gctcatcaac 1860
aagtccatgg agctgctgga tgagaggaag ttctgggctg gtattgtgtt cactggaatt 1920
actccaggca gcattgagct gccccatcat gtcaagtaca agatccgaat ggacattgac 1980
aatgtggaga ggacaaataa aatcaaggat gggtactggg accctggtcc tcgagctgac 2040
ccctttgagg acatgcggta cgtctggggg ggcttcgcct acttgcagga tgtggtggag 2100
caggcaatca tcagggtgct gacgggcacc gagaagaaaa ctggtgtcta tatgcaacag 2160
atgccctatc cctgttacgt tgatgacatc tttctgcggg tgatgagccg gtcaatgccc 2220
ctcttcatga cgctggcctg gatttactca gtggctgtga tcatcaaggg catcgtgtat 2280
gagaaggagg cacggctgaa agagaccatg cggatcatgg gcctggacaa cagcatcctc 2340
tggtttagct ggttcattag tagcctcatt cctcttcttg tgagcgctgg cctgctagtg 2400
gtcatcctga agttaggaaa cctgctgccc tacagtgatc ccagcgtggt gtttgtcttc 2460
ctgtccgtgt ttgctgtggt gacaatcctg cagtgcttcc tgattagcac actcttctcc 2520
agagccaacc tggcagcagc ctgtgggggc atcatctact tcacgctgta cctgccctac 2580
gtcctgtgtg tggcatggca ggactacgtg ggcttcacac tcaagatctt cgctagcctg 2640
ctgtctcctg tggcttttgg gtttggctgt gagtactttg ccctttttga ggagcagggc 2700
attggagtgc agtgggacaa cctgtttgag agtcctgtgg aggaagatgg cttcaatctc 2760
accacttcgg tctccatgat gctgtttgac accttcctct atggggtgat gacctggtac 2820
attgaggctg tctttccagg ccagtacgga attcccaggc cctggtattt tccttgcacc 2880
aagtcctact ggtttggcga ggaaagtgat gagaagagcc accctggttc caaccagaag 2940
agaatatcag aaatctgcat ggaggaggaa cccacccact tgaagctggg cgtgtccatt 3000
cagaacctgg taaaagtcta ccgagatggg atgaaggtgg ctgtcgatgg cctggcactg 3060
aatttttatg agggccagat cacctccttc ctgggccaca atggagcggg gaagacgacc 3120
accatgtcaa tcctgaccgg gttgttcccc ccgacctcgg gcaccgccta catcctggga 3180
aaagacattc gctctgagat gagcaccatc cggcagaacc tgggggtctg tccccagcat 3240
aacgtgctgt ttgacatgct gactgtcgaa gaacacatct ggttctatgc ccgcttgaaa 3300
gggctctctg agaagcacgt gaaggcggag atggagcaga tggccctgga tgttggtttg 3360
ccatcaagca agctgaaaag caaaacaagc cagctgtcag gtggaatgca gagaaagcta 3420
tctgtggcct tggcctttgt cgggggatct aaggttgtca ttctggatga acccacagct 3480
ggtgtggacc cttactcccg caggggaata tgggagctgc tgctgaaata ccgacaaggc 3540
cgcaccatta ttctctctac acaccacatg gatgaagcgg acgtcctggg ggacaggatt 3600
gccatcatct cccatgggaa gctgtgctgt gtgggctcct ccctgtttct gaagaaccag 3660
ctgggaacag gctactacct gaccttggtc aagaaagatg tggaatcctc cctcagttcc 3720
tgcagaaaca gtagtagcac tgtgtcatac ctgaaaaagg aggacagtgt ttctcagagc 3780
agttctgatg ctggcctggg cagcgaccat gagagtgaca cgctgaccat cgatgtctct 3840
gctatctcca acctcatcag gaagcatgtg tctgaagccc ggctggtgga agacataggg 3900
catgagctga cctatgtgct gccatatgaa gctgctaagg agggagcctt tgtggaactc 3960
tttcatgaga ttgatgaccg gctctcagac ctgggcattt ctagttatgg catctcagag 4020
acgaccctgg aagaaatatt cctcaaggtg gccgaagaga gtggggtgga tgctgagacc 4080
tcagatggta ccttgccagc aagacgaaac aggcgggcct tcggggacaa gcagagctgt 4140
cttcgcccgt tcactgaaga tgatgctgct gatccaaatg attctgacat agacccagaa 4200
tccagagaga cagacttgct cagtgggatg gatggcaaag ggtcctacca ggtgaaaggc 4260
tggaaactta cacagcaaca gtttgtggcc cttttgtgga agagactgct aattgccaga 4320
cggagtcgga aaggattttt tgctcagatt gtcttgccag ctgtgtttgt ctgcattgcc 4380
cttgtgttca gcctgatcgt gccacccttt ggcaagtacc ccagcctgga acttcagccc 4440
tggatgtaca acgaacagta cacatttgtc agcaatgatg ctcctgagga cacgggaacc 4500
ctggaactct taaacgccct caccaaagac cctggcttcg ggacccgctg tatggaagga 4560
aacccaatcc cagacacgcc ctgccaggca ggggaggaag agtggaccac tgccccagtt 4620
ccccagacca tcatggacct cttccagaat gggaactgga caatgcagaa cccttcacct 4680
gcatgccagt gtagcagcga caaaatcaag aagatgctgc ctgtgtgtcc cccaggggca 4740
ggggggctgc ctcctccaca aagaaaacaa aacactgcag atatccttca ggacctgaca 4800
ggaagaaaca tttcggatta tctggtgaag acgtatgtgc agatcatagc caaaagctta 4860

aagaacaaga tctgggtgaa tgagtttagg tatggcggct tttccctggg tgtcagtaat 4920
actcaagcac ttcctccgag tcaagaagtt aatgatgcca ccaaacaaat gaagaaacac 4980
ctaaagctgg ccaaggacag ttctgcagat cgatttctca acagcttggg aagatttatg 5040
acaggactgg acaccagaaa taatgtcaag gtgtggttca ataacaaggg ctggcatgca 5100
atcagctctt tcctgaatgt catcaacaat gccattctcc gggccaacct gcaaaaggga 5160
gagaacccta gccattatgg aattactgct ttcaatcatc ccctgaatct caccaagcag 5220
cagctctcag aggtggctcc gatgaccaca tcagtggatg tccttgtgtc catctgtgtc 5280
atctttgcaa tgtccttcgt cccagccagc tttgtcgtat tcctgatcca ggagcgggtc 5340
agcaaagcaa aacacctgca gttcatcagt ggagtgaagc ctgtcatcta ctggctctct 5400
aatttgtctt gggatatgtg caattacgtt gtccctgcca cactggtcat tatcatcttc 5460
atctgcttcc agcagaagtc ctatgtgtcc tccaccaatc tgcctgtgct agcccttcta 5520
cttttgctgt atgggtggtc aatcacacct ctcatgtacc cagcctcctt tgtgttcaag 5580
atccccagca cagcctatgt ggtgctcacc agcgtgaacc tcttcattgg cattaatggc 5640
agcgtggcca cctttgtgct ggagctgttc accgacaata agctgaataa tatcaatgat 5700
atcctgaagt ccgtgttctt gatcttccca catttttgcc tgggacgagg gctcatcgac 5760
atggtgaaaa accaggcaat ggctgatgcc ctggaaaggt ttggggagaa tcgctttgtg 5820
tcaccattat cttgggactt ggtgggacga aacctcttcg ccatggccgt ggaaggggtg 5880
gtgttcttcc tcattactgt tctgatccag tacagattct tcatcaggcc cagacctgta 5940
aatgcaaagc tatctcctct gaatgatgaa gatgaagatg tgaggcggga aagacagaga 6000
attcttgatg gtggaggcca gaatgacatc ttagaaatca aggagttgac gaagatatat 6060
agaaggaagc ggaagcctgc tgttgacagg atttgcgtgg gcattcctcc tggtgagtgc 6120
tttgggctcc tgggagttaa tggggctgga aaatcatcaa ctttcaagat gttaacagga 6180
gataccactg ttaccagagg agatgctttc cttaacagaa atagtatctt atcaaacatc 6240
catgaagtac atcagaacat gggctactgc cctcagtttg atgccatcac agagctgttg 6300
actgggagag aacacgtgga gttctttgcc cttttgagag gagtcccaga gaaagaagtt 6360
ggcaaggttg gtgagtgggc gattcggaaa ctgggcctcg tgaagtatgg agaaaaatat 6420
gctggtaact atagtggagg caacaaacgc aagctctcta cagccatggc tttgatcggc 6480
gggcctcctg tggtgtttct ggatgaaccc accacaggca tggatcccaa agcccggcgg 6540
ttcttgtgga attgtgccct aagtgttgtc aaggagggga gatcagtagt gcttacatct 6600
catagtatgg aagaatgtga agctctttgc actaggatgg caatcatggt caatggaagg 6660
ttcaggtgcc ttggcagtgt ccagcatcta aaaaataggt ttggagatgg ttatacaata 6720
gttgtacgaa tagcagggtc caacccggac ctgaagcctg tccaggattt ctttggactt 6780
gcatttcctg gaagtgttcc aaaagagaaa caccggaaca tgctacaata ccagcttcca 6840
tcttcattat cttctctggc caggatattc agcatcctct cccagagcaa aaagcgactc 6900
cacatagaag actactctgt ttctcagaca acacttgacc aagtatttgt gaactttgcc 6960
aaggaccaaa gtgatgatga ccacttaaaa gacctctcat tacacaaaaa ccagacagta 7020
gtggacgttg cagttctcac atcttttcta caggatgaga aagtgaaaga aagctatgta 7080
tgaagaatcc tgttcatacg gggtggctga aagtaaagag gnactagact ttcctttgca 7140
ccatgtgaag tgttgtggag aaaagagcca gaagttgatg tgggaagaag taaactggat 7200
actgtactga tactattcaa tgcaatgcaa ttcaatgcaa tgaaaacaaa attccattac 7260
aggggcagtg cctttgtagc ctatgtcttg tatggctctc aagtgaaaga cttgaattta 7320
gttttttacc tatacctatg tgaaactcta ttatggaacc caatggacat atgggtttga 7380
actcacactt tttttttttt tttgttcctg tgtattctca ttggggttgc aacaataatt 7440
catcaagtaa tcatggccag cgattattga tcaaaatcaa aaggtaatgc acatcctcat 7500
tcactaagcc atgccatgcc caggagactg gtttcccggt gacacatcca ttgctggcaa 7560
tgagtgtgcc agagttatta gtgccaagtt tttcagaaag tttgaagcac catggtgtgt 7620
catgctcact tttgtgaaag ctgctctgct cagagtctat caacattgaa tatcagttga 7680
cagaatggtg ccatgcgtgg ctaacatcct gctttgattc cctctgataa gctgttctgg 7740
tggcagtaac atgcaacaaa aatgtgggtg tctctaggca cgggaaactt ggttccattg 7800
ttatattgtc ctatgcttcg agccatgggt ctacagggtc atccttatga gactcttaaa 7860
tatacttaga tcctggtaag aggcaaagaa tcaacagcca aactgctggg gctgcaagct 7920
gctgaagcca gggcatggga ttaaagagat tgtgcgttca aacctaggga agcctgtgcc 7980
catttgtcct gactgtctgc taacatggta cactgcatct caagatgttt atctgacaca 8040
agtgtattat ttctggcttt ttgaattaat ctagaaaatg aaaagatgga gttgtatttt 8100
gacaaaaatg tttgtacttt ttaatgttat ttggaatttt aagttctatc agtgacttct 8160
gaatccttag aatggcctct ttgtagaacc ctgtggtata gaggagtatg gccactgccc 8220
cactattttt attttcttat gtaagtttgc atatcagtca tgactagtgc ctagaaagca 8280
atgtgatggt caggatctca tgacattata tttgagtttc tttcagatca tttaggatac 8340

tcttaatctc acttcatcaa tcaaatattt tttgagtgta tgctgtagct gaaagagtat 8400
gtacgtacgt ataagactag agagatatta agtctcagta cacttcctgt gccatgttat 8460
tcagctcact ggtttacaaa tataggttgt cttgtggttg taggagccca ctgtaacaat 8520
actgggcagc cttttttttt ttttttaatt gcaacaatgc aaaagccaag aaagtataag 8580
ggtcacaagt ctaaacaatg aattcttcaa cagggaaaac agctagcttg aaaacttgct 8640
gaaaaacaca acttgtgttt atggcattta gtaccttcaa ataattggct ttgcagatat 8700
tggatacccc attaaatctg acagtctcaa atttttcatc tcttcaatca ctagtcaaga 8760
aaaatataaa aacaacaaat acttccatat ggagcatttt tcagagtttt ctaacccagt 8820
cttatttttc tagtcagtaa acatttgtaa aaatactgtt tcactaatac ttactgttaa 8880
ctgtcttgag agaaaagaaa aatatgagag aactattgtt tggggaagtt caagtgatct 8940
ttcaatatca ttactaactt cttccacttt ttccaaaatt tgaatattaa cgctaaaggt 9000
gtaagacttc agatttcaaa ttaatctttc tatatttttt aaatttacag aatattatat 9060
aacccactgc tgaaaaagaa aaaaatgatt gttttagaag ttaaagtcaa tattgatttt 9120
aaatataagt aatgaaggca tatttccaat aactagtgat atggcatcgt tgcattttac 9180
agtatcttca aaaatacaga atttatagaa taatttctcc tcatttaata tttttcaaaa 9240
tcaaagttat ggtttcctca ttttactaaa atcgtattct aattcttcat tatagtaaat 9300
ctatgagcaa ctccttactt cggttcctct gatttcaagg ccatatttta aaaaatcaaa 9360
aggcactgtg aactattttg aagaaaacac aacattttaa tacagattga aaggacctct 9420
tctgaagcta gaaacaatct atagttatac atcttcatta atactgtgtt accttttaaa 9480
atagtaattt tttacatttt cctgtgtaaa cctaattgtg gtagaaattt ttaccaactc 9540
tatactcaat caagcaaaat ttctgtatat tccctgtgga atgtacctat gtgagtttca 9600
gaaattctca aaatacgtgt tcaaaaattt ctgcttttgc atctttggga cacctcagaa 9660
aacttattaa caactgtgaa tatgagaaat acagaagaaa ataataagcc ctctatacat 9720
aaatgcccag cacaattcat tgttaaaaaa caaccaaacc tcacactact gtatttcatt 9780
atctgtactg aaagcaaatg ctttgtgact attaaatgtt gcacatcatt cattcaaaaa 9840
aaaaaaaaaa aaaa 9854

<210> 71
<211> 2261
<212> PRT
<213> Homo sapiens

<400> 71
Met Ala Cys Trp Pro Gln Leu Arg Leu Leu Leu Trp Lys Asn Leu Thr
1               5                   10                  15

Phe Arg Arg Arg Gln Thr Cys Gln Leu Leu Leu Glu Val Ala Trp Pro
            20                  25                  30

Leu Phe Ile Phe Leu Ile Leu Ile Ser Val Arg Leu Ser Tyr Pro Pro
        35                  40                  45

Tyr Glu Gln His Glu Cys His Phe Pro Asn Lys Ala Met Pro Ser Ala
    50                  55                  60

Gly Thr Leu Pro Trp Val Gln Gly Ile Ile Cys Asn Ala Asn Asn Pro
65                  70                  75                  80

Cys Phe Arg Tyr Pro Thr Pro Gly Glu Ala Pro Gly Val Val Gly Asn
                85                  90                  95

Phe Asn Lys Ser Ile Val Ala Arg Leu Phe Ser Asp Ala Arg Arg Leu
            100                 105                 110

Leu Leu Tyr Ser Gln Lys Asp Thr Ser Met Lys Asp Met Arg Lys Val
        115                 120                 125

Leu Arg Thr Leu Gln Gln Ile Lys Lys Ser Ser Ser Asn Leu Lys Leu
    130                 135                 140

Gln Asp Phe Leu Val Asp Asn Glu Thr Phe Ser Gly Phe Leu Tyr His
145                 150                 155                 160

Asn Leu Ser Leu Pro Lys Ser Thr Val Asp Lys Met Leu Arg Ala Asp
                165                 170                 175

Val Ile Leu His Lys Val Phe Leu Gln Gly Tyr Gln Leu His Leu Thr
            180                 185                 190

Ser Leu Cys Asn Gly Ser Lys Ser Glu Glu Met Ile Gln Leu Gly Asp
        195                 200                 205

Gln Glu Val Ser Glu Leu Cys Gly Leu Pro Arg Glu Lys Leu Ala Ala
    210                 215                 220

Ala Glu Arg Val Leu Arg Ser Asn Met Asp Ile Leu Lys Pro Ile Leu
225                 230                 235                 240

Arg Thr Leu Asn Ser Thr Ser Pro Phe Pro Ser Lys Glu Leu Ala Glu
                245                 250                 255

Ala Thr Lys Thr Leu Leu His Ser Leu Gly Thr Leu Ala Gln Glu Leu
            260                 265                 270

Phe Ser Met Arg Ser Trp Ser Asp Met Arg Gln Glu Val Met Phe Leu
        275                 280                 285

Thr Asn Val Asn Ser Ser Ser Ser Ser Thr Gln Ile Tyr Gln Ala Val
    290                 295                 300

Ser Arg Ile Val Cys Gly His Pro Glu Gly Gly Gly Leu Lys Ile Lys
305                 310                 315                 320

Ser Leu Asn Trp Tyr Glu Asp Asn Asn Tyr Lys Ala Leu Phe Gly Gly
                325                 330                 335

Asn Gly Thr Glu Glu Asp Ala Glu Thr Phe Tyr Asp Asn Ser Thr Thr
            340                 345                 350

Pro Tyr Cys Asn Asp Leu Met Lys Asn Leu Glu Ser Ser Pro Leu Ser
        355                 360                 365

Arg Ile Ile Trp Lys Ala Leu Lys Pro Leu Leu Val Gly Lys Ile Leu
    370                 375                 380

Tyr Thr Pro Asp Thr Pro Ala Thr Arg Gln Val Met Ala Glu Val Asn
385                 390                 395                 400

Lys Thr Phe Gln Glu Leu Ala Val Phe His Asp Leu Glu Gly Met Trp
                405                 410                 415

Glu Glu Leu Ser Pro Lys Ile Trp Thr Phe Met Glu Asn Ser Gln Glu
            420                 425                 430

Met Asp Leu Val Arg Met Leu Leu Asp Ser Arg Asp Asn Asp His Phe

435                 440                 445

Trp Glu Gln Gln Leu Asp Gly Leu Asp Trp Thr Ala Gln Asp Ile Val
    450                 455                 460

Ala Phe Leu Ala Lys His Pro Glu Asp Val Gln Ser Ser Asn Gly Ser
465                 470                 475                 480

Val Tyr Thr Trp Arg Glu Ala Phe Asn Glu Thr Asn Gln Ala Ile Arg
                485                 490                 495

Thr Ile Ser Arg Phe Met Glu Cys Val Asn Leu Asn Lys Leu Glu Pro
            500                 505                 510

Ile Ala Thr Glu Val Trp Leu Ile Asn Lys Ser Met Glu Leu Leu Asp
        515                 520                 525

Glu Arg Lys Phe Trp Ala Gly Ile Val Phe Thr Gly Ile Thr Pro Gly
    530                 535                 540

Ser Ile Glu Leu Pro His His Val Lys Tyr Lys Ile Arg Met Asp Ile
545                 550                 555                 560

Asp Asn Val Glu Arg Thr Asn Lys Ile Lys Asp Gly Tyr Trp Asp Pro
                565                 570                 575

Gly Pro Arg Ala Asp Pro Phe Glu Asp Met Arg Tyr Val Trp Gly Gly
            580                 585                 590

Phe Ala Tyr Leu Gln Asp Val Val Glu Gln Ala Ile Ile Arg Val Leu
        595                 600                 605

Thr Gly Thr Glu Lys Lys Thr Gly Val Tyr Met Gln Gln Met Pro Tyr
    610                 615                 620

Pro Cys Tyr Val Asp Asp Ile Phe Leu Arg Val Met Ser Arg Ser Met
625                 630                 635                 640

Pro Leu Phe Met Thr Leu Ala Trp Ile Tyr Ser Val Ala Val Ile Ile
                645                 650                 655

Lys Gly Ile Val Tyr Glu Lys Glu Ala Arg Leu Lys Glu Thr Met Arg
            660                 665                 670

Ile Met Gly Leu Asp Asn Ser Ile Leu Trp Phe Ser Trp Phe Ile Ser
        675                 680                 685

Ser Leu Ile Pro Leu Leu Val Ser Ala Gly Leu Leu Val Val Ile Leu
    690                 695                 700

Lys Leu Gly Asn Leu Leu Pro Tyr Ser Asp Pro Ser Val Val Phe Val
705                 710                 715                 720

Phe Leu Ser Val Phe Ala Val Val Thr Ile Leu Gln Cys Phe Leu Ile
                725                 730                 735

Ser Thr Leu Phe Ser Arg Ala Asn Leu Ala Ala Ala Cys Gly Gly Ile
            740                 745                 750

Ile Tyr Phe Thr Leu Tyr Leu Pro Tyr Val Leu Cys Val Ala Trp Gln
        755                 760                 765

Asp Tyr Val Gly Phe Thr Leu Lys Ile Phe Ala Ser Leu Leu Ser Pro
    770                 775                 780

Val Ala Phe Gly Phe Gly Cys Glu Tyr Phe Ala Leu Phe Glu Glu Gln
785                 790                 795                 800

Gly Ile Gly Val Gln Trp Asp Asn Leu Phe Glu Ser Pro Val Glu Glu
                805                 810                 815

Asp Gly Phe Asn Leu Thr Thr Ser Val Ser Met Met Leu Phe Asp Thr
            820                 825                 830

Phe Leu Tyr Gly Val Met Thr Trp Tyr Ile Glu Ala Val Phe Pro Gly
        835                 840                 845

Gln Tyr Gly Ile Pro Arg Pro Trp Tyr Phe Pro Cys Thr Lys Ser Tyr
    850                 855                 860

Trp Phe Gly Glu Glu Ser Asp Glu Lys Ser His Pro Gly Ser Asn Gln
865                 870                 875                 880

Lys Arg Ile Ser Glu Ile Cys Met Glu Glu Glu Pro Thr His Leu Lys
                885                 890                 895

Leu Gly Val Ser Ile Gln Asn Leu Val Lys Val Tyr Arg Asp Gly Met
            900                 905                 910

Lys Val Ala Val Asp Gly Leu Ala Leu Asn Phe Tyr Glu Gly Gln Ile
        915                 920                 925

Thr Ser Phe Leu Gly His Asn Gly Ala Gly Lys Thr Thr Thr Met Ser
    930                 935                 940

Ile Leu Thr Gly Leu Phe Pro Pro Thr Ser Gly Thr Ala Tyr Ile Leu
945                 950                 955                 960

Gly Lys Asp Ile Arg Ser Glu Met Ser Thr Ile Arg Gln Asn Leu Gly
                965                 970                 975

Val Cys Pro Gln His Asn Val Leu Phe Asp Met Leu Thr Val Glu Glu
            980                 985                 990

His Ile Trp Phe Tyr Ala Arg Leu Lys Gly Leu Ser Glu Lys His Val
        995                 1000                1005

Lys Ala Glu Met Glu Gln Met Ala Leu Asp Val Gly Leu Pro Ser Ser
    1010                1015                1020

Lys Leu Lys Ser Lys Thr Ser Gln Leu Ser Gly Gly Met Gln Arg Lys
1025                1030                1035                1040

Leu Ser Val Ala Leu Ala Phe Val Gly Gly Ser Lys Val Val Ile Leu
                1045                1050                1055

Asp Glu Pro Thr Ala Gly Val Asp Pro Tyr Ser Arg Arg Gly Ile Trp
            1060                1065                1070

Glu Leu Leu Leu Lys Tyr Arg Gln Gly Arg Thr Ile Ile Leu Ser Thr
        1075                1080                1085

His His Met Asp Glu Ala Asp Val Leu Gly Asp Arg Ile Ala Ile Ile
    1090                1095                1100

Ser His Gly Lys Leu Cys Cys Val Gly Ser Ser Leu Phe Leu Lys Asn
1105                1110                1115                1120

Gln Leu Gly Thr Gly Tyr Tyr Leu Thr Leu Val Lys Lys Asp Val Glu
                1125                1130                1135

Ser Ser Leu Ser Ser Cys Arg Asn Ser Ser Ser Thr Val Ser Tyr Leu
            1140                1145                1150

Lys Lys Glu Asp Ser Val Ser Gln Ser Ser Ser Asp Ala Gly Leu Gly
        1155                1160                1165

Ser Asp His Glu Ser Asp Thr Leu Thr Ile Asp Val Ser Ala Ile Ser
    1170                1175                1180

Asn Leu Ile Arg Lys His Val Ser Glu Ala Arg Leu Val Glu Asp Ile
1185                1190                1195                1200

Gly His Glu Leu Thr Tyr Val Leu Pro Tyr Glu Ala Ala Lys Glu Gly
                1205                1210                1215

Ala Phe Val Glu Leu Phe His Glu Ile Asp Asp Arg Leu Ser Asp Leu
            1220                1225                1230

Gly Ile Ser Ser Tyr Gly Ile Ser Glu Thr Thr Leu Glu Glu Ile Phe
        1235                1240                1245

Leu Lys Val Ala Glu Glu Ser Gly Val Asp Ala Glu Thr Ser Asp Gly
    1250                1255                1260

Thr Leu Pro Ala Arg Arg Asn Arg Arg Ala Phe Gly Asp Lys Gln Ser
1265                1270                1275                1280

Cys Leu Arg Pro Phe Thr Glu Asp Asp Ala Ala Asp Pro Asn Asp Ser
                1285                1290                1295

Asp Ile Asp Pro Glu Ser Arg Glu Thr Asp Leu Leu Ser Gly Met Asp
            1300                1305                1310

Gly Lys Gly Ser Tyr Gln Val Lys Gly Trp Lys Leu Thr Gln Gln Gln
        1315                1320                1325

Phe Val Ala Leu Leu Trp Lys Arg Leu Leu Ile Ala Arg Arg Ser Arg
    1330                1335                1340

Lys Gly Phe Phe Ala Gln Ile Val Leu Pro Ala Val Phe Val Cys Ile
1345                1350                1355                1360

Ala Leu Val Phe Ser Leu Ile Val Pro Pro Phe Gly Lys Tyr Pro Ser

1365                1370                1375

Leu Glu Leu Gln Pro Trp Met Tyr Asn Glu Gln Tyr Thr Phe Val Ser
        1380                1385                1390

Asn Asp Ala Pro Glu Asp Thr Gly Thr Leu Glu Leu Leu Asn Ala Leu
    1395                1400                1405

Thr Lys Asp Pro Gly Phe Gly Thr Arg Cys Met Glu Gly Asn Pro Ile
  1410                1415                1420

Pro Asp Thr Pro Cys Gln Ala Gly Glu Glu Glu Trp Thr Thr Ala Pro
1425                1430                1435                1440

Val Pro Gln Thr Ile Met Asp Leu Phe Gln Asn Gly Asn Trp Thr Met
                1445                1450                1455

Gln Asn Pro Ser Pro Ala Cys Gln Cys Ser Ser Asp Lys Ile Lys Lys
        1460                1465                1470

Met Leu Pro Val Cys Pro Pro Gly Ala Gly Gly Leu Pro Pro Pro Gln
    1475                1480                1485

Arg Lys Gln Asn Thr Ala Asp Ile Leu Gln Asp Leu Thr Gly Arg Asn
  1490                1495                1500

Ile Ser Asp Tyr Leu Val Lys Thr Tyr Val Gln Ile Ile Ala Lys Ser
1505                1510                1515                1520

Leu Lys Asn Lys Ile Trp Val Asn Glu Phe Arg Tyr Gly Gly Phe Ser
                1525                1530                1535

Leu Gly Val Ser Asn Thr Gln Ala Leu Pro Pro Ser Gln Glu Val Asn
        1540                1545                1550

Asp Ala Thr Lys Gln Met Lys Lys His Leu Lys Leu Ala Lys Asp Ser
    1555                1560                1565

Ser Ala Asp Arg Phe Leu Asn Ser Leu Gly Arg Phe Met Thr Gly Leu
  1570                1575                1580

Asp Thr Arg Asn Asn Val Lys Val Trp Phe Asn Asn Lys Gly Trp His
1585                1590                1595                1600

Ala Ile Ser Ser Phe Leu Asn Val Ile Asn Asn Ala Ile Leu Arg Ala
                1605                1610                1615

Asn Leu Gln Lys Gly Glu Asn Pro Ser His Tyr Gly Ile Thr Ala Phe
        1620                1625                1630

Asn His Pro Leu Asn Leu Thr Lys Gln Gln Leu Ser Glu Val Ala Pro
    1635                1640                1645

Met Thr Thr Ser Val Asp Val Leu Val Ser Ile Cys Val Ile Phe Ala
  1650                1655                1660

Met Ser Phe Val Pro Ala Ser Phe Val Val Phe Leu Ile Gln Glu Arg
1665                1670                1675                1680

Val Ser Lys Ala Lys His Leu Gln Phe Ile Ser Gly Val Lys Pro Val
                1685                1690                1695

Ile Tyr Trp Leu Ser Asn Phe Val Trp Asp Met Cys Asn Tyr Val Val
            1700                1705                1710

Pro Ala Thr Leu Val Ile Ile Ile Phe Ile Cys Phe Gln Gln Lys Ser
        1715                1720                1725

Tyr Val Ser Ser Thr Asn Leu Pro Val Leu Ala Leu Leu Leu Leu Leu
    1730                1735                1740

Tyr Gly Trp Ser Ile Thr Pro Leu Met Tyr Pro Ala Ser Phe Val Phe
1745                1750                1755                1760

Lys Ile Pro Ser Thr Ala Tyr Val Val Leu Thr Ser Val Asn Leu Phe
                1765                1770                1775

Ile Gly Ile Asn Gly Ser Val Ala Thr Phe Val Leu Glu Leu Phe Thr
            1780                1785                1790

Asp Asn Lys Leu Asn Asn Ile Asn Asp Ile Leu Lys Ser Val Phe Leu
        1795                1800                1805

Ile Phe Pro His Phe Cys Leu Gly Arg Gly Leu Ile Asp Met Val Lys
    1810                1815                1820

Asn Gln Ala Met Ala Asp Ala Leu Glu Arg Phe Gly Glu Asn Arg Phe
1825                1830                1835                1840

Val Ser Pro Leu Ser Trp Asp Leu Val Gly Arg Asn Leu Phe Ala Met
                1845                1850                1855

Ala Val Glu Gly Val Val Phe Phe Leu Ile Thr Val Leu Ile Gln Tyr
            1860                1865                1870

Arg Phe Phe Ile Arg Pro Arg Pro Val Asn Ala Lys Leu Ser Pro Leu
        1875                1880                1885

Asn Asp Glu Asp Glu Asp Val Arg Arg Glu Arg Gln Arg Ile Leu Asp
    1890                1895                1900

Gly Gly Gly Gln Asn Asp Ile Leu Glu Ile Lys Glu Leu Thr Lys Ile
1905                1910                1915                1920

Tyr Arg Arg Lys Arg Lys Pro Ala Val Asp Arg Ile Cys Val Gly Ile
                1925                1930                1935

Pro Pro Gly Glu Cys Phe Gly Leu Leu Gly Val Asn Gly Ala Gly Lys
            1940                1945                1950

Ser Ser Thr Phe Lys Met Leu Thr Gly Asp Thr Thr Val Thr Arg Gly
        1955                1960                1965

Asp Ala Phe Leu Asn Arg Asn Ser Ile Leu Ser Asn Ile His Glu Val
    1970                1975                1980

His Gln Asn Met Gly Tyr Cys Pro Gln Phe Asp Ala Ile Thr Glu Leu
1985                1990                1995                2000

Leu Thr Gly Arg Glu His Val Glu Phe Phe Ala Leu Leu Arg Gly Val
                2005                2010                2015

Pro Glu Lys Glu Val Gly Lys Val Gly Glu Trp Ala Ile Arg Lys Leu
            2020                2025                2030

Gly Leu Val Lys Tyr Gly Glu Lys Tyr Ala Gly Asn Tyr Ser Gly Gly
        2035                2040                2045

Asn Lys Arg Lys Leu Ser Thr Ala Met Ala Leu Ile Gly Gly Pro Pro
    2050                2055                2060

Val Val Phe Leu Asp Glu Pro Thr Thr Gly Met Asp Pro Lys Ala Arg
2065                2070                2075                2080

Arg Phe Leu Trp Asn Cys Ala Leu Ser Val Val Lys Glu Gly Arg Ser
                2085                2090                2095

Val Val Leu Thr Ser His Ser Met Glu Glu Cys Glu Ala Leu Cys Thr
            2100                2105                2110

Arg Met Ala Ile Met Val Asn Gly Arg Phe Arg Cys Leu Gly Ser Val
        2115                2120                2125

Gln His Leu Lys Asn Arg Phe Gly Asp Gly Tyr Thr Ile Val Val Arg
    2130                2135                2140

Ile Ala Gly Ser Asn Pro Asp Leu Lys Pro Val Gln Asp Phe Phe Gly
2145                2150                2155                2160

Leu Ala Phe Pro Gly Ser Val Pro Lys Glu Lys His Arg Asn Met Leu
                2165                2170                2175

Gln Tyr Gln Leu Pro Ser Ser Leu Ser Ser Leu Ala Arg Ile Phe Ser
            2180                2185                2190

Ile Leu Ser Gln Ser Lys Lys Arg Leu His Ile Glu Asp Tyr Ser Val
        2195                2200                2205

Ser Gln Thr Thr Leu Asp Gln Val Phe Val Asn Phe Ala Lys Asp Gln
    2210                2215                2220

Ser Asp Asp Asp His Leu Lys Asp Leu Ser Leu His Lys Asn Gln Thr
2225                2230                2235                2240

Val Val Asp Val Ala Val Leu Thr Ser Phe Leu Gln Asp Glu Lys Val
                2245                2250                2255

Lys Glu Ser Tyr Val
            2260

<210> 72
<211> 122
<212> DNA

<213> Homo sapiens

<400> 72
acttgaagct tcaagatttc ctggtggaca atgaaacctt ctctgggttc ctatatcaca 60
acctctctct cccaaagtct actgtggaca agatgctgag ggctgatgtc attctccaca 120
ag 122

<210> 73
<211> 177
<212> DNA
<213> Homo sapiens

<400> 73
gtatttttgc aaggctacca gttacatttg acaagtctgt gcaatggatc aaaatcagaa 60
gagatgattc aacttggtga ccaagaagtt tctgagcttt gtggcctacc aaaggagaaa 120
ctggctgcag cagagcgagt acttcgttcc aacatggaca tcctgaagcc aatcctg 177

<210> 74
<211> 177
<212> DNA
<213> Homo sapiens

<400> 74
gtatttttgc aaggctacca gttacatttg acaagtctgt gcaatggatc aaaatcagaa 60
gagatgattc aacttggtga ccaagaagtt tctgagcttt gtggcctacc aaaggagaaa 120
ctggctgcag cagagtgagt acttcgttcc aacatggaca tcctgaagcc aatcctg 177

<210> 75
<211> 241
<212> DNA
<213> Homo sapiens

<400> 75
ctgttcagca tgagaagctg gagtgacatg tgacaggagg tgatgtttct gaccaatgtg 60
aacagctcca gctcctccac ccaaatctac caggctgtgt ctcgtattgt ctgcgggcat 120
cccgagggag gggggctgaa gatcaagtct ctcaactggt atgaggacaa caactacaaa 180
gccctctttg gaggcaatgg cactgaggaa gatgctgaaa ccttctatga caactctaca 240
a 241

<210> 76
<211> 241
<212> DNA
<213> Homo sapiens

<400> 76
ctgttcagca tgagaagctg gagtgacatg cgacaggagg tgatgtttct gaccaatgtg 60
aacagctcca gctcctccac ccaaatctac caggctgtgt ctcgtattgt ctgcgggcat 120
cctgagggag gggggctgaa gatcaagtct ctcaactggt atgaggacaa caactacaaa 180
gccctctttg gaggcaatgg cactgaggaa gatgctgaaa ccttctatga caactctaca 240
a 241

<210> 77
<211> 241

<212> DNA
<213> Homo sapiens

<400> 77
ctgttcagca tgagaagctg gagtgacatg cgacaggagg tgatgtttct gaccaatgtg 60
aacagctcca gctcctccac ccaaatctac caggctgtgt ctcgtattgt ctgcgggcat 120
cccgagggag ggggactgaa gatcaagtct ctcaactggt atgaggacaa caactacaaa 180
gccctctttg gaggcaatgg cactgaggaa gatgctgaaa ccttctatga caactctaca 240
a 241

<210> 78
<211> 176
<212> DNA
<213> Homo sapiens

<400> 78
gtactgggac cctggtcctc gagctgaccc ctttgaggac atgcggtacg tctggggggg 60
cttcgcctac ttgcaggatg tggtggagca ggcaatcatc agggtgctac gggcaccgag 120
aagaaaactg gtgtctatat gcaacagatg ccctatccct gttacgttga tgacat 176

<210> 79
<211> 222
<212> DNA
<213> Homo sapiens

<400> 79
ttaggaaacc tgctgcccta cagtgatccc agcgtggtgt ttgtcttcct gtccgtgttt 60
gctgtggtga caatcctgca gtgcttcctg attagcacac tcttctccag agccaacctg 120
gcagcagcct gtgggggcat catctacttc acgctgtacc tgccctacgt cctgtgtgtg 180
gcatggcagg actacgtggg cttcccactc aagatcttcg ct 222

<210> 80
<211> 114
<212> DNA
<213> Homo sapiens

<400> 80
gccagtacag aattcccagg ccctggtatt ttccttgcac caagtcctac tggtttggcg 60
aggaaagtga tgagaagagc caccctggtt ccaaccagaa gagaatatca gaaa 114

<210> 81
<211> 114
<212> DNA
<213> Homo sapiens

<400> 81
gccagtacgg aattcccagg ccctggtatt ttccttgcac caagtcctac tggtttggcg 60
aggaaagtga tgagaagagc caccctggtt ccaaccagaa gagaatgtca gaaa 114

<210> 82
<211> 219
<212> DNA
<213> Homo sapiens

<400> 82
gccgcaccat tattctctct acacaccaca tggatgaagc ggacgtcctg ggggacagga 60
ttgccatcat ctcccatggg aagctgtgct gtgtgggctc ccctgtttct gaagaaccag 120
ctgggaacag gctactacct gaccttggtc aagaaagatg tggaatcctc cctcagttcc 180
tgcagaaaca gtagtagcac tgtgtcatac ctgaaaaag 219

<210> 83
<211> 219
<212> DNA
<213> Homo sapiens

<400> 83
gccgcaccat tattctctct acacaccaca tggatgaagc ggacgtcctg ggggacagga 60
ttgccatcat ctcccatggg aagctgtgct gtgtgggctc ccctgtttct gaagaaccag 120
ctgggaacag gctactacct gaccttggtc aagaaagatg tggaatcctc cctcagttcc 180
tgcagaaaca gtagtagcac tgtgtcatac ctgaaaaag 219

<210> 84
<211> 149
<212> DNA
<213> Homo sapiens

<400> 84
aatccagaga gacagacttg ctcagtggga tggatggcaa agggtcctac caggtgaaag 60
gctggaaact tacacagcaa cagtttgtgg cccttttgtg gaagagactg ctaattgcca 120
gatggagtcg gaaaggattt tttgctcag 149

<210> 85
<211> 33
<212> DNA
<213> Homo sapiens

<400> 85
cttaaagaac aagatctgag tgaatgagtt tag 33

<210> 86
<211> 75
<212> DNA
<213> Homo sapiens

<400> 86
gacagttctg cagatcgatt tctcaacagc ttgggaagat ttatgacagg actggacacc 60
aaaaataatg tcaag 75

<210> 87
<211> 93
<212> DNA
<213> Homo sapiens

<400> 87
tgtggaagaa tgtgaagctc tttgcactag gatggcaatc atggtcaatg gaaggttcag 60
gtgccttggc agtgtccagc atctaaaaaa tag 93

<210> 88
<211> 92
<212> DNA
<213> Homo sapiens

<400> 88
tatggaagaa tgtgaagctc tttgcatagg atggcaatca tggtcaatgg aaggttcagg 60
tgccttggca gtgtccagca tctaaaaaat ag 92


<210> 89
<211> 2261
<212> PRT
<213> Homo sapiens

<400> 89
Met Ala Cys Trp Pro Gln Leu Arg Leu Leu Leu Trp Lys Asn Leu Thr
1               5                   10                  15

Phe Arg Arg Arg Gln Thr Cys Gln Leu Leu Leu Glu Val Ala Trp Pro
            20                  25                  30

Leu Phe Ile Phe Leu Ile Leu Ile Ser Val Arg Leu Ser Tyr Pro Pro
        35                  40                  45

Tyr Glu Gln His Glu Cys His Phe Pro Asn Lys Ala Met Pro Ser Ala
    50                  55                  60

Gly Thr Leu Pro Trp Val Gln Gly Ile Ile Cys Asn Ala Asn Asn Pro
65                  70                  75                  80

Cys Phe Arg Tyr Pro Thr Pro Gly Glu Ala Pro Gly Val Val Gly Asn
                85                  90                  95

Phe Asn Lys Ser Ile Val Ala Arg Leu Phe Ser Asp Ala Arg Arg Leu
            100                 105                 110

Leu Leu Tyr Ser Gln Lys Asp Thr Ser Met Lys Asp Met Arg Lys Val
        115                 120                 125

Leu Arg Thr Leu Gln Gln Ile Lys Lys Ser Ser Ser Asn Leu Lys Leu
    130                 135                 140

Gln Asp Phe Leu Val Asp Asn Glu Thr Phe Ser Gly Phe Leu Tyr His
145                 150                 155                 160

Asn Leu Ser Leu Pro Lys Ser Thr Val Asp Lys Met Leu Arg Ala Asp
                165                 170                 175

Val Ile Leu His Lys Val Phe Leu Gln Gly Tyr Gln Leu His Leu Thr
            180                 185                 190

Ser Leu Cys Asn Gly Ser Lys Ser Glu Glu Met Ile Gln Leu Gly Asp
        195                 200                 205

Gln Glu Val Ser Glu Leu Cys Gly Leu Pro Arg Glu Lys Leu Ala Ala

210                 215                 220

Ala Glu Arg Val Leu Arg Ser Asn Met Asp Ile Leu Lys Pro Ile Leu
225                 230                 235                 240

Arg Thr Leu Asn Ser Thr Ser Pro Phe Pro Ser Lys Glu Leu Ala Glu
                245                 250                 255

Ala Thr Lys Thr Leu Leu His Ser Leu Gly Thr Leu Ala Gln Glu Leu
            260                 265                 270

Phe Ser Met Arg Ser Trp Ser Asp Met Arg Gln Glu Val Met Phe Leu
        275                 280                 285

Thr Asn Val Asn Ser Ser Ser Ser Ser Thr Gln Ile Tyr Gln Ala Val
    290                 295                 300

Ser Arg Ile Val Cys Gly His Pro Glu Gly Gly Gly Leu Lys Ile Lys
305                 310                 315                 320

Ser Leu Asn Trp Tyr Glu Asp Asn Asn Tyr Lys Ala Leu Phe Gly Gly
                325                 330                 335

Asn Gly Thr Glu Glu Asp Ala Glu Thr Phe Tyr Asp Asn Ser Thr Thr
            340                 345                 350

Pro Tyr Cys Asn Asp Leu Met Lys Asn Leu Glu Ser Ser Pro Leu Ser
        355                 360                 365

Arg Ile Ile Trp Lys Ala Leu Lys Pro Leu Leu Val Gly Lys Ile Leu
    370                 375                 380

Tyr Thr Pro Asp Thr Pro Ala Thr Arg Gln Val Met Ala Glu Val Asn
385                 390                 395                 400

Lys Thr Phe Gln Glu Leu Ala Val Phe His Asp Leu Glu Gly Met Trp
                405                 410                 415

Glu Glu Leu Ser Pro Lys Ile Trp Thr Phe Met Glu Asn Ser Gln Glu
            420                 425                 430

Met Asp Leu Val Arg Met Leu Leu Asp Ser Arg Asp Asn Asp His Phe
        435                 440                 445

Trp Glu Gln Gln Leu Asp Gly Leu Asp Trp Thr Ala Gln Asp Ile Val
    450                 455                 460

Ala Phe Leu Ala Lys His Pro Glu Asp Val Gln Ser Ser Asn Gly Ser
465                 470                 475                 480

Val Tyr Thr Trp Arg Glu Ala Phe Asn Glu Thr Asn Gln Ala Ile Arg
                485                 490                 495

Thr Ile Ser Arg Phe Met Glu Cys Val Asn Leu Asn Lys Leu Glu Pro
            500                 505                 510

Ile Ala Thr Glu Val Trp Leu Ile Asn Lys Ser Met Glu Leu Leu Asp
        515                 520                 525

Glu Arg Lys Phe Trp Ala Gly Ile Val Phe Thr Gly Ile Thr Pro Gly
    530                 535                 540

Ser Ile Glu Leu Pro His His Val Lys Tyr Lys Ile Arg Met Asp Ile
545                 550                 555                 560

Asp Asn Val Glu Arg Thr Asn Lys Ile Lys Asp Gly Tyr Trp Asp Pro
                565                 570                 575

Gly Pro Arg Ala Asp Pro Phe Glu Asp Met Arg Tyr Val Trp Gly Gly
            580                 585                 590

Phe Ala Tyr Leu Gln Asp Val Val Glu Gln Ala Ile Ile Arg Val Leu
        595                 600                 605

Thr Gly Thr Glu Lys Lys Thr Gly Val Tyr Met Gln Gln Met Pro Tyr
    610                 615                 620

Pro Cys Tyr Val Asp Asp Ile Phe Leu Arg Val Met Ser Arg Ser Met
625                 630                 635                 640

Pro Leu Phe Met Thr Leu Ala Trp Ile Tyr Ser Val Ala Val Ile Ile
                645                 650                 655

Lys Gly Ile Val Tyr Glu Lys Glu Ala Arg Leu Lys Glu Thr Met Arg
            660                 665                 670

Ile Met Gly Leu Asp Asn Ser Ile Leu Trp Phe Ser Trp Phe Ile Ser
        675                 680                 685

Ser Leu Ile Pro Leu Leu Val Ser Ala Gly Leu Leu Val Val Ile Leu
    690                 695                 700

Lys Leu Gly Asn Leu Leu Pro Tyr Ser Asp Pro Ser Val Val Phe Val
705                 710                 715                 720

Phe Leu Ser Val Phe Ala Val Val Thr Ile Leu Gln Cys Phe Leu Ile
                725                 730                 735

Ser Thr Leu Phe Ser Arg Ala Asn Leu Ala Ala Ala Cys Gly Gly Ile
            740                 745                 750

Ile Tyr Phe Thr Leu Tyr Leu Pro Tyr Val Leu Cys Val Ala Trp Gln
        755                 760                 765

Asp Tyr Val Gly Phe Thr Leu Lys Ile Phe Ala Ser Leu Leu Ser Pro
    770                 775                 780

Val Ala Phe Gly Phe Gly Cys Glu Tyr Phe Ala Leu Phe Glu Glu Gln
785                 790                 795                 800

Gly Ile Gly Val Gln Trp Asp Asn Leu Phe Glu Ser Pro Val Glu Glu
                805                 810                 815

Asp Gly Phe Asn Leu Thr Thr Ser Val Ser Met Met Leu Phe Asp Thr
            820                 825                 830

Phe Leu Tyr Gly Val Met Thr Trp Tyr Ile Glu Ala Val Phe Pro Gly
        835                 840                 845

Gln Tyr Gly Ile Pro Arg Pro Trp Tyr Phe Pro Cys Thr Lys Ser Tyr
    850                 855                 860

Trp Phe Gly Glu Glu Ser Asp Glu Lys Ser His Pro Gly Ser Asn Gln
865                 870                 875                 880

Lys Arg Ile Ser Glu Ile Cys Met Glu Glu Glu Pro Thr His Leu Lys
                885                 890                 895

Leu Gly Val Ser Ile Gln Asn Leu Val Lys Val Tyr Arg Asp Gly Met
            900                 905                 910

Lys Val Ala Val Asp Gly Leu Ala Leu Asn Phe Tyr Glu Gly Gln Ile
        915                 920                 925

Thr Ser Phe Leu Gly His Asn Gly Ala Gly Lys Thr Thr Thr Met Ser
    930                 935                 940

Ile Leu Thr Gly Leu Phe Pro Pro Thr Ser Gly Thr Ala Tyr Ile Leu
945                 950                 955                 960

Gly Lys Asp Ile Arg Ser Glu Met Ser Thr Ile Arg Gln Asn Leu Gly
                965                 970                 975

Val Cys Pro Gln His Asn Val Leu Phe Asp Met Leu Thr Val Glu Glu
            980                 985                 990

His Ile Trp Phe Tyr Ala Arg Leu Lys Gly Leu Ser Glu Lys His Val
        995                 1000                1005

Lys Ala Glu Met Glu Gln Met Ala Leu Asp Val Gly Leu Pro Ser Ser
    1010                1015                1020

Lys Leu Lys Ser Lys Thr Ser Gln Leu Ser Gly Gly Met Gln Arg Lys
1025                1030                1035                1040

Leu Ser Val Ala Leu Ala Phe Val Gly Gly Ser Lys Val Val Ile Leu
                1045                1050                1055

Asp Glu Pro Thr Ala Gly Val Asp Pro Tyr Ser Arg Arg Gly Ile Trp
            1060                1065                1070

Glu Leu Leu Leu Lys Tyr Arg Gln Gly Arg Thr Ile Ile Leu Ser Thr
        1075                1080                1085

His His Met Asp Glu Ala Asp Val Leu Gly Asp Arg Ile Ala Ile Ile
    1090                1095                1100

Ser His Gly Lys Leu Cys Cys Val Gly Ser Ser Leu Phe Leu Lys Asn
1105                1110                1115                1120

Gln Leu Gly Thr Gly Tyr Tyr Leu Thr Leu Val Lys Lys Asp Val Glu
                1125                1130                1135

Ser Ser Leu Ser Ser Cys Arg Asn Ser Ser Ser Thr Val Ser Tyr Leu

1140                1145                1150

Lys Lys Glu Asp Ser Val Ser Gln Ser Ser Ser Asp Ala Gly Leu Gly
    1155                1160                1165

Ser Asp His Glu Ser Asp Thr Leu Thr Ile Asp Val Ser Ala Ile Ser
1170                1175                1180

Asn Leu Ile Arg Lys His Val Ser Glu Ala Arg Leu Val Glu Asp Ile
1185                1190                1195                1200

Gly His Glu Leu Thr Tyr Val Leu Pro Tyr Glu Ala Ala Lys Glu Gly
                1205                1210                1215

Ala Phe Val Glu Leu Phe His Glu Ile Asp Asp Arg Leu Ser Asp Leu
            1220                1225                1230

Gly Ile Ser Ser Tyr Gly Ile Ser Glu Thr Thr Leu Glu Glu Ile Phe
        1235                1240                1245

Leu Lys Val Ala Glu Glu Ser Gly Val Asp Ala Glu Thr Ser Asp Gly
    1250                1255                1260

Thr Leu Pro Ala Arg Arg Asn Arg Arg Ala Phe Gly Asp Lys Gln Ser
1265                1270                1275                1280

Cys Leu Arg Pro Phe Thr Glu Asp Asp Ala Ala Asp Pro Asn Asp Ser
                1285                1290                1295

Asp Ile Asp Pro Glu Ser Arg Glu Thr Asp Leu Leu Ser Gly Met Asp
            1300                1305                1310

Gly Lys Gly Ser Tyr Gln Val Lys Gly Trp Lys Leu Thr Gln Gln Gln
        1315                1320                1325

Phe Val Ala Leu Leu Trp Lys Arg Leu Leu Ile Ala Arg Arg Ser Arg
    1330                1335                1340

Lys Gly Phe Phe Ala Gln Ile Val Leu Pro Ala Val Phe Val Cys Ile
1345                1350                1355                1360

Ala Leu Val Phe Ser Leu Ile Val Pro Pro Phe Gly Lys Tyr Pro Ser
                1365                1370                1375

Leu Glu Leu Gln Pro Trp Met Tyr Asn Glu Gln Tyr Thr Phe Val Ser
            1380                1385                1390

Asn Asp Ala Pro Glu Asp Thr Gly Thr Leu Glu Leu Leu Asn Ala Leu
        1395                1400                1405

Thr Lys Asp Pro Gly Phe Gly Thr Arg Cys Met Glu Gly Asn Pro Ile
    1410                1415                1420

Pro Asp Thr Pro Cys Gln Ala Gly Glu Glu Glu Trp Thr Thr Ala Pro
1425                1430                1435                1440

Val Pro Gln Thr Ile Met Asp Leu Phe Gln Asn Gly Asn Trp Thr Met
                1445                1450                1455

Gln Asn Pro Ser Pro Ala Cys Gln Cys Ser Ser Asp Lys Ile Lys Lys
            1460                1465                1470

Met Leu Pro Val Cys Pro Pro Gly Ala Gly Gly Leu Pro Pro Pro Gln
        1475                1480                1485

Arg Lys Gln Asn Thr Ala Asp Ile Leu Gln Asp Leu Thr Gly Arg Asn
    1490                1495                1500

Ile Ser Asp Tyr Leu Val Lys Thr Tyr Val Gln Ile Ile Ala Lys Ser
1505                1510                1515                1520

Leu Lys Asn Lys Ile Trp Val Asn Glu Phe Arg Tyr Gly Gly Phe Ser
                1525                1530                1535

Leu Gly Val Ser Asn Thr Gln Ala Leu Pro Pro Ser Gln Glu Val Asn
            1540                1545                1550

Asp Ala Thr Lys Gln Met Lys Lys His Leu Lys Leu Ala Lys Asp Ser
        1555                1560                1565

Ser Ala Asp Arg Phe Leu Asn Ser Leu Gly Arg Phe Met Thr Gly Leu
    1570                1575                1580

Asp Thr Arg Asn Asn Val Lys Val Trp Phe Asn Asn Lys Gly Trp His
1585                1590                1595                1600

Ala Ile Ser Ser Phe Leu Asn Val Ile Asn Asn Ala Ile Leu Arg Ala
                1605                1610                1615

Asn Leu Gln Lys Gly Glu Asn Pro Ser His Tyr Gly Ile Thr Ala Phe
            1620                1625                1630

Asn His Pro Leu Asn Leu Thr Lys Gln Gln Leu Ser Glu Val Ala Pro
        1635                1640                1645

Met Thr Thr Ser Val Asp Val Leu Val Ser Ile Cys Val Ile Phe Ala
    1650                1655                1660

Met Ser Phe Val Pro Ala Ser Phe Val Val Phe Leu Ile Gln Glu Arg
1665                1670                1675                1680

Val Ser Lys Ala Lys His Leu Gln Phe Ile Ser Gly Val Lys Pro Val
                1685                1690                1695

Ile Tyr Trp Leu Ser Asn Phe Val Trp Asp Met Cys Asn Tyr Val Val
            1700                1705                1710

Pro Ala Thr Leu Val Ile Ile Ile Phe Ile Cys Phe Gln Gln Lys Ser
        1715                1720                1725

Tyr Val Ser Ser Thr Asn Leu Pro Val Leu Ala Leu Leu Leu Leu Leu
    1730                1735                1740

Tyr Gly Trp Ser Ile Thr Pro Leu Met Tyr Pro Ala Ser Phe Val Phe
1745                1750                1755                1760

Lys Ile Pro Ser Thr Ala Tyr Val Val Leu Thr Ser Val Asn Leu Phe
                1765                1770                1775

Ile Gly Ile Asn Gly Ser Val Ala Thr Phe Val Leu Glu Leu Phe Thr
            1780                1785                1790

Asp Asn Lys Leu Asn Asn Ile Asn Asp Ile Leu Lys Ser Val Phe Leu
        1795                1800                1805

Ile Phe Pro His Phe Cys Leu Gly Arg Gly Leu Ile Asp Met Val Lys
    1810                1815                1820

Asn Gln Ala Met Ala Asp Ala Leu Glu Arg Phe Gly Glu Asn Arg Phe
1825                1830                1835                1840

Val Ser Pro Leu Ser Trp Asp Leu Val Gly Arg Asn Leu Phe Ala Met
                1845                1850                1855

Ala Val Glu Gly Val Val Phe Phe Leu Ile Thr Val Leu Ile Gln Tyr
            1860                1865                1870

Arg Phe Phe Ile Arg Pro Arg Pro Val Asn Ala Lys Leu Ser Pro Leu
        1875                1880                1885

Asn Asp Glu Asp Glu Asp Val Arg Arg Glu Arg Gln Arg Ile Leu Asp
    1890                1895                1900

Gly Gly Gly Gln Asn Asp Ile Leu Glu Ile Lys Glu Leu Thr Lys Ile
1905                1910                1915                1920

Tyr Arg Arg Lys Arg Lys Pro Ala Val Asp Arg Ile Cys Val Gly Ile
                1925                1930                1935

Pro Pro Gly Glu Cys Phe Gly Leu Leu Gly Val Asn Gly Ala Gly Lys
            1940                1945                1950

Ser Ser Thr Phe Lys Met Leu Thr Gly Asp Thr Thr Val Thr Arg Gly
        1955                1960                1965

Asp Ala Phe Leu Asn Arg Asn Ser Ile Leu Ser Asn Ile His Glu Val
    1970                1975                1980

His Gln Asn Met Gly Tyr Cys Pro Gln Phe Asp Ala Ile Thr Glu Leu
1985                1990                1995                2000

Leu Thr Gly Arg Glu His Val Glu Phe Phe Ala Leu Leu Arg Gly Val
                2005                2010                2015

Pro Glu Lys Glu Val Gly Lys Val Gly Glu Trp Ala Ile Arg Lys Leu
            2020                2025                2030

Gly Leu Val Lys Tyr Gly Glu Lys Tyr Ala Gly Asn Tyr Ser Gly Gly
        2035                2040                2045

Asn Lys Arg Lys Leu Ser Thr Ala Met Ala Leu Ile Gly Gly Pro Pro
    2050                2055                2060

Val Val Phe Leu Asp Glu Pro Thr Thr Gly Met Asp Pro Lys Ala Arg

2065                2070                2075                2080

Arg Phe Leu Trp Asn Cys Ala Leu Ser Val Val Lys Glu Gly Arg Ser
                2085                2090                2095

Val Val Leu Thr Ser His Ser Met Glu Glu Cys Glu Ala Leu Cys Thr
            2100                2105                2110

Arg Met Ala Ile Met Val Asn Gly Arg Phe Arg Cys Leu Gly Ser Val
        2115                2120                2125

Gln His Leu Lys Asn Arg Phe Gly Asp Gly Tyr Thr Ile Val Val Arg
    2130                2135                2140

Ile Ala Gly Ser Asn Pro Asp Leu Lys Pro Val Gln Asp Phe Phe Gly
2145                2150                2155                2160

Leu Ala Phe Pro Gly Ser Val Pro Lys Glu Lys His Arg Asn Met Leu
                2165                2170                2175

Gln Tyr Gln Leu Pro Ser Ser Leu Ser Ser Leu Ala Arg Ile Phe Ser
            2180                2185                2190

Ile Leu Ser Gln Ser Lys Lys Arg Leu His Ile Glu Asp Tyr Ser Val
        2195                2200                2205

Ser Gln Thr Thr Leu Asp Gln Val Phe Val Asn Phe Ala Lys Asp Gln
    2210                2215                2220

Ser Asp Asp Asp His Leu Lys Asp Leu Ser Leu His Lys Asn Gln Thr
2225                2230                2235                2240

Val Val Asp Val Ala Val Leu Thr Ser Phe Leu Gln Asp Glu Lys Val
                2245                2250                2255

Lys Glu Ser Tyr Val
            2260


<210> 90
<211> 226
<212> PRT
<213> Homo sapiens

<400> 90
Met Ala Cys Trp Pro Gln Leu Arg Leu Leu Leu Trp Lys Asn Leu Thr
1               5                   10                  15

Phe Arg Arg Arg Gln Thr Cys Gln Leu Leu Leu Glu Val Ala Trp Pro
            20                  25                  30

Leu Phe Ile Phe Leu Ile Leu Ile Ser Val Arg Leu Ser Tyr Pro Pro
        35                  40                  45

Tyr Glu Gln His Glu Cys His Phe Pro Asn Lys Ala Met Pro Ser Ala
    50                  55                  60

Gly Thr Leu Pro Trp Val Gln Gly Ile Ile Cys Asn Ala Asn Asn Pro

65                  70                  75                  80

Cys Phe Arg Tyr Pro Thr Pro Gly Glu Ala Pro Gly Val Val Gly Asn
                85                  90                  95

Phe Asn Lys Ser Ile Val Ala Arg Leu Phe Ser Asp Ala Arg Arg Leu
            100                 105                 110

Leu Leu Tyr Ser Gln Lys Asp Thr Ser Met Lys Asp Met Arg Lys Val
        115                 120                 125

Leu Arg Thr Leu Gln Gln Ile Lys Lys Ser Ser Ser Asn Leu Lys Leu
    130                 135                 140

Gln Asp Phe Leu Val Asp Asn Glu Thr Phe Ser Gly Phe Leu Tyr His
145                 150                 155                 160

Asn Leu Ser Leu Pro Lys Ser Thr Val Asp Lys Met Leu Arg Ala Asp
                165                 170                 175

Val Ile Leu His Lys Val Phe Leu Gln Gly Tyr Gln Leu His Leu Thr
            180                 185                 190

Ser Leu Cys Asn Gly Ser Lys Ser Glu Glu Met Ile Gln Leu Gly Asp
        195                 200                 205

Gln Glu Val Ser Glu Leu Cys Gly Leu Pro Arg Glu Lys Leu Ala Ala
    210                 215                 220

Ala Glu
225


<210> 91
<211> 281
<212> PRT
<213> Homo sapiens

<400> 91
Met Ala Cys Trp Pro Gln Leu Arg Leu Leu Leu Trp Lys Asn Leu Thr
1               5                   10                  15

Phe Arg Arg Arg Gln Thr Cys Gln Leu Leu Leu Glu Val Ala Trp Pro
            20                  25                  30

Leu Phe Ile Phe Leu Ile Leu Ile Ser Val Arg Leu Ser Tyr Pro Pro
        35                  40                  45

Tyr Glu Gln His Glu Cys His Phe Pro Asn Lys Ala Met Pro Ser Ala
    50                  55                  60

Gly Thr Leu Pro Trp Val Gln Gly Ile Ile Cys Asn Ala Asn Asn Pro
65                  70                  75                  80

Cys Phe Arg Tyr Pro Thr Pro Gly Glu Ala Pro Gly Val Val Gly Asn
                85                  90                  95

Phe Asn Lys Ser Ile Val Ala Arg Leu Phe Ser Asp Ala Arg Arg Leu

100                 105                 110

Leu Leu Tyr Ser Gln Lys Asp Thr Ser Met Lys Asp Met Arg Lys Val
        115                 120                 125

Leu Arg Thr Leu Gln Gln Ile Lys Lys Ser Ser Ser Asn Leu Lys Leu
    130                 135                 140

Gln Asp Phe Leu Val Asp Asn Glu Thr Phe Ser Gly Phe Leu Tyr His
145                 150                 155                 160

Asn Leu Ser Leu Pro Lys Ser Thr Val Asp Lys Met Leu Arg Ala Asp
                165                 170                 175

Val Ile Leu His Lys Val Phe Leu Gln Gly Tyr Gln Leu His Leu Thr
            180                 185                 190

Ser Leu Cys Asn Gly Ser Lys Ser Glu Glu Met Ile Gln Leu Gly Asp
        195                 200                 205

Gln Glu Val Ser Glu Leu Cys Gly Leu Pro Arg Glu Lys Leu Ala Ala
    210                 215                 220

Ala Glu Arg Val Leu Arg Ser Asn Met Asp Ile Leu Lys Pro Ile Leu
225                 230                 235                 240

Arg Thr Leu Asn Ser Thr Ser Pro Phe Pro Ser Lys Glu Leu Ala Glu
                245                 250                 255

Ala Thr Lys Thr Leu Leu His Ser Leu Gly Thr Leu Ala Gln Glu Leu
            260                 265                 270

Phe Ser Met Arg Ser Trp Ser Asp Met
        275                 280


<210> 92
<211> 634
<212> PRT
<213> Homo sapiens

<400> 92
Met Ala Cys Trp Pro Gln Leu Arg Leu Leu Leu Trp Lys Asn Leu Thr
1               5                   10                  15

Phe Arg Arg Arg Gln Thr Cys Gln Leu Leu Leu Glu Val Ala Trp Pro
            20                  25                  30

Leu Phe Ile Phe Leu Ile Leu Ile Ser Val Arg Leu Ser Tyr Pro Pro
        35                  40                  45

Tyr Glu Gln His Glu Cys His Phe Pro Asn Lys Ala Met Pro Ser Ala
    50                  55                  60

Gly Thr Leu Pro Trp Val Gln Gly Ile Ile Cys Asn Ala Asn Asn Pro
65                  70                  75                  80

Cys Phe Arg Tyr Pro Thr Pro Gly Glu Ala Pro Gly Val Val Gly Asn

                85                  90                  95

Phe Asn Lys Ser Ile Val Ala Arg Leu Phe Ser Asp Ala Arg Arg Leu
            100                 105                 110

Leu Leu Tyr Ser Gln Lys Asp Thr Ser Met Lys Asp Met Arg Lys Val
        115                 120                 125

Leu Arg Thr Leu Gln Gln Ile Lys Lys Ser Ser Ser Asn Leu Lys Leu
    130                 135                 140

Gln Asp Phe Leu Val Asp Asn Glu Thr Phe Ser Gly Phe Leu Tyr His
145                 150                 155                 160

Asn Leu Ser Leu Pro Lys Ser Thr Val Asp Lys Met Leu Arg Ala Asp
                165                 170                 175

Val Ile Leu His Lys Val Phe Leu Gln Gly Tyr Gln Leu His Leu Thr
            180                 185                 190

Ser Leu Cys Asn Gly Ser Lys Ser Glu Glu Met Ile Gln Leu Gly Asp
        195                 200                 205

Gln Glu Val Ser Glu Leu Cys Gly Leu Pro Arg Glu Lys Leu Ala Ala
    210                 215                 220

Ala Glu Arg Val Leu Arg Ser Asn Met Asp Ile Leu Lys Pro Ile Leu
225                 230                 235                 240

Arg Thr Leu Asn Ser Thr Ser Pro Phe Pro Ser Lys Glu Leu Ala Glu
                245                 250                 255

Ala Thr Lys Thr Leu Leu His Ser Leu Gly Thr Leu Ala Gln Glu Leu
            260                 265                 270

Phe Ser Met Arg Ser Trp Ser Asp Met Arg Gln Glu Val Met Phe Leu
        275                 280                 285

Thr Asn Val Asn Ser Ser Ser Ser Ser Thr Gln Ile Tyr Gln Ala Val
    290                 295                 300

Ser Arg Ile Val Cys Gly His Pro Glu Gly Gly Gly Leu Lys Ile Lys
305                 310                 315                 320

Ser Leu Asn Trp Tyr Glu Asp Asn Asn Tyr Lys Ala Leu Phe Gly Gly
                325                 330                 335

Asn Gly Thr Glu Glu Asp Ala Glu Thr Phe Tyr Asp Asn Ser Thr Thr
            340                 345                 350

Pro Tyr Cys Asn Asp Leu Met Lys Asn Leu Glu Ser Ser Pro Leu Ser
        355                 360                 365

Arg Ile Ile Trp Lys Ala Leu Lys Pro Leu Leu Val Gly Lys Ile Leu
    370                 375                 380

Tyr Thr Pro Asp Thr Pro Ala Thr Arg Gln Val Met Ala Glu Val Asn
385                 390                 395                 400

Lys Thr Phe Gln Glu Leu Ala Val Phe His Asp Leu Glu Gly Met Trp
                405                 410                 415

Glu Glu Leu Ser Pro Lys Ile Trp Thr Phe Met Glu Asn Ser Gln Glu
            420                 425                 430

Met Asp Leu Val Arg Met Leu Leu Asp Ser Arg Asp Asn Asp His Phe
        435                 440                 445

Trp Glu Gln Gln Leu Asp Gly Leu Asp Trp Thr Ala Gln Asp Ile Val
    450                 455                 460

Ala Phe Leu Ala Lys His Pro Glu Asp Val Gln Ser Ser Asn Gly Ser
465                 470                 475                 480

Val Tyr Thr Trp Arg Glu Ala Phe Asn Glu Thr Asn Gln Ala Ile Arg
                485                 490                 495

Thr Ile Ser Arg Phe Met Glu Cys Val Asn Leu Asn Lys Leu Glu Pro
            500                 505                 510

Ile Ala Thr Glu Val Trp Leu Ile Asn Lys Ser Met Glu Leu Leu Asp
        515                 520                 525

Glu Arg Lys Phe Trp Ala Gly Ile Val Phe Thr Gly Ile Thr Pro Gly
    530                 535                 540

Ser Ile Glu Leu Pro His His Val Lys Tyr Lys Ile Arg Met Asp Ile
545                 550                 555                 560

Asp Asn Val Glu Arg Thr Asn Lys Ile Lys Asp Gly Tyr Trp Asp Pro
                565                 570                 575

Gly Pro Arg Ala Asp Pro Phe Glu Asp Met Arg Tyr Val Trp Gly Gly
            580                 585                 590

Phe Ala Tyr Leu Gln Asp Val Val Glu Gln Ala Ile Ile Arg Val Leu
        595                 600                 605

Arg Ala Pro Arg Arg Lys Leu Val Ser Ile Cys Asn Arg Cys Pro Ile
    610                 615                 620

Pro Val Thr Leu Met Thr Ser Phe Cys Gly
625                 630

<210> 93
<211> 2261
<212> PRT
<213> Homo sapiens

<400> 93
Met Ala Cys Trp Pro Gln Leu Arg Leu Leu Leu Trp Lys Asn Leu Thr
  1               5                  10                  15

Phe Arg Arg Arg Gln Thr Cys Gln Leu Leu Leu Glu Val Ala Trp Pro
             20                  25                  30

Leu Phe Ile Phe Leu Ile Leu Ile Ser Val Arg Leu Ser Tyr Pro Pro
        35                  40                  45

Tyr Glu Gln His Glu Cys His Phe Pro Asn Lys Ala Met Pro Ser Ala
    50                  55                  60

Gly Thr Leu Pro Trp Val Gln Gly Ile Ile Cys Asn Ala Asn Asn Pro
65                  70                  75                  80

Cys Phe Arg Tyr Pro Thr Pro Gly Glu Ala Pro Gly Val Val Gly Asn
                85                  90                  95

Phe Asn Lys Ser Ile Val Ala Arg Leu Phe Ser Asp Ala Arg Arg Leu
            100                 105                 110

Leu Leu Tyr Ser Gln Lys Asp Thr Ser Met Lys Asp Met Arg Lys Val
        115                 120                 125

Leu Arg Thr Leu Gln Gln Ile Lys Lys Ser Ser Ser Asn Leu Lys Leu
    130                 135                 140

Gln Asp Phe Leu Val Asp Asn Glu Thr Phe Ser Gly Phe Leu Tyr His
145                 150                 155                 160

Asn Leu Ser Leu Pro Lys Ser Thr Val Asp Lys Met Leu Arg Ala Asp
                165                 170                 175

Val Ile Leu His Lys Val Phe Leu Gln Gly Tyr Gln Leu His Leu Thr
            180                 185                 190

Ser Leu Cys Asn Gly Ser Lys Ser Glu Glu Met Ile Gln Leu Gly Asp
        195                 200                 205

Gln Glu Val Ser Glu Leu Cys Gly Leu Pro Arg Glu Lys Leu Ala Ala
    210                 215                 220

Ala Glu Arg Val Leu Arg Ser Asn Met Asp Ile Leu Lys Pro Ile Leu
225                 230                 235                 240

Arg Thr Leu Asn Ser Thr Ser Pro Phe Pro Ser Lys Glu Leu Ala Glu
                245                 250                 255

Ala Thr Lys Thr Leu Leu His Ser Leu Gly Thr Leu Ala Gln Glu Leu
            260                 265                 270

Phe Ser Met Arg Ser Trp Ser Asp Met Arg Gln Glu Val Met Phe Leu
        275                 280                 285

Thr Asn Val Asn Ser Ser Ser Ser Ser Thr Gln Ile Tyr Gln Ala Val
    290                 295                 300

Ser Arg Ile Val Cys Gly His Pro Glu Gly Gly Gly Leu Lys Ile Lys
305                 310                 315                 320

Ser Leu Asn Trp Tyr Glu Asp Asn Asn Tyr Lys Ala Leu Phe Gly Gly
                325                 330                 335

Asn Gly Thr Glu Glu Asp Ala Glu Thr Phe Tyr Asp Asn Ser Thr Thr
            340                 345                 350

Pro Tyr Cys Asn Asp Leu Met Lys Asn Leu Glu Ser Ser Pro Leu Ser
        355                 360                 365

Arg Ile Ile Trp Lys Ala Leu Lys Pro Leu Leu Val Gly Lys Ile Leu
    370                 375                 380

Tyr Thr Pro Asp Thr Pro Ala Thr Arg Gln Val Met Ala Glu Val Asn
385                 390                 395                 400

Lys Thr Phe Gln Glu Leu Ala Val Phe His Asp Leu Glu Gly Met Trp
                405                 410                 415

Glu Glu Leu Ser Pro Lys Ile Trp Thr Phe Met Glu Asn Ser Gln Glu
            420                 425                 430

Met Asp Leu Val Arg Met Leu Leu Asp Ser Arg Asp Asn Asp His Phe
        435                 440                 445

Trp Glu Gln Gln Leu Asp Gly Leu Asp Trp Thr Ala Gln Asp Ile Val
    450                 455                 460

Ala Phe Leu Ala Lys His Pro Glu Asp Val Gln Ser Ser Asn Gly Ser
465                 470                 475                 480

Val Tyr Thr Trp Arg Glu Ala Phe Asn Glu Thr Asn Gln Ala Ile Arg
                485                 490                 495

Thr Ile Ser Arg Phe Met Glu Cys Val Asn Leu Asn Lys Leu Glu Pro
            500                 505                 510

Ile Ala Thr Glu Val Trp Leu Ile Asn Lys Ser Met Glu Leu Leu Asp
        515                 520                 525

Glu Arg Lys Phe Trp Ala Gly Ile Val Phe Thr Gly Ile Thr Pro Gly
    530                 535                 540

Ser Ile Glu Leu Pro His His Val Lys Tyr Lys Ile Arg Met Asp Ile
545                 550                 555                 560

Asp Asn Val Glu Arg Thr Asn Lys Ile Lys Asp Gly Tyr Trp Asp Pro
                565                 570                 575

Gly Pro Arg Ala Asp Pro Phe Glu Asp Met Arg Tyr Val Trp Gly Gly
            580                 585                 590

Phe Ala Tyr Leu Gln Asp Val Val Glu Gln Ala Ile Ile Arg Val Leu
        595                 600                 605

Thr Gly Thr Glu Lys Lys Thr Gly Val Tyr Met Gln Gln Met Pro Tyr
    610                 615                 620

Pro Cys Tyr Val Asp Asp Ile Phe Leu Arg Val Met Ser Arg Ser Met
625                 630                 635                 640

Pro Leu Phe Met Thr Leu Ala Trp Ile Tyr Ser Val Ala Val Ile Ile

                645                 650                 655

Lys Gly Ile Val Tyr Glu Lys Glu Ala Arg Leu Lys Glu Thr Met Arg
            660                 665                 670

Ile Met Gly Leu Asp Asn Ser Ile Leu Trp Phe Ser Trp Phe Ile Ser
        675                 680                 685

Ser Leu Ile Pro Leu Leu Val Ser Ala Gly Leu Leu Val Val Ile Leu
    690                 695                 700

Lys Leu Gly Asn Leu Leu Pro Tyr Ser Asp Pro Ser Val Val Phe Val
705                 710                 715                 720

Phe Leu Ser Val Phe Ala Val Val Thr Ile Leu Gln Cys Phe Leu Ile
                725                 730                 735

Ser Thr Leu Phe Ser Arg Ala Asn Leu Ala Ala Ala Cys Gly Gly Ile
            740                 745                 750

Ile Tyr Phe Thr Leu Tyr Leu Pro Tyr Val Leu Cys Val Ala Trp Gln
        755                 760                 765

Asp Tyr Val Gly Phe Pro Leu Lys Ile Phe Ala Ser Leu Leu Ser Pro
    770                 775                 780

Val Ala Phe Gly Phe Gly Cys Glu Tyr Phe Ala Leu Phe Glu Glu Gln
785                 790                 795                 800

Gly Ile Gly Val Gln Trp Asp Asn Leu Phe Glu Ser Pro Val Glu Glu
                805                 810                 815

Asp Gly Phe Asn Leu Thr Thr Ser Val Ser Met Met Leu Phe Asp Thr
            820                 825                 830

Phe Leu Tyr Gly Val Met Thr Trp Tyr Ile Glu Ala Val Phe Pro Gly
        835                 840                 845

Gln Tyr Gly Ile Pro Arg Pro Trp Tyr Phe Pro Cys Thr Lys Ser Tyr
    850                 855                 860

Trp Phe Gly Glu Glu Ser Asp Glu Lys Ser His Pro Gly Ser Asn Gln
865                 870                 875                 880

Lys Arg Ile Ser Glu Ile Cys Met Glu Glu Glu Pro Thr His Leu Lys
                885                 890                 895

Leu Gly Val Ser Ile Gln Asn Leu Val Lys Val Tyr Arg Asp Gly Met
            900                 905                 910

Lys Val Ala Val Asp Gly Leu Ala Leu Asn Phe Tyr Glu Gly Gln Ile
        915                 920                 925

Thr Ser Phe Leu Gly His Asn Gly Ala Gly Lys Thr Thr Thr Met Ser
    930                 935                 940

Ile Leu Thr Gly Leu Phe Pro Pro Thr Ser Gly Thr Ala Tyr Ile Leu
945                 950                 955                 960

Gly Lys Asp Ile Arg Ser Glu Met Ser Thr Ile Arg Gln Asn Leu Gly
                965                 970                 975

Val Cys Pro Gln His Asn Val Leu Phe Asp Met Leu Thr Val Glu Glu
            980                 985                 990

His Ile Trp Phe Tyr Ala Arg Leu Lys Gly Leu Ser Glu Lys His Val
        995                 1000                1005

Lys Ala Glu Met Glu Gln Met Ala Leu Asp Val Gly Leu Pro Ser Ser
    1010                1015                1020

Lys Leu Lys Ser Lys Thr Ser Gln Leu Ser Gly Gly Met Gln Arg Lys
1025                1030                1035                1040

Leu Ser Val Ala Leu Ala Phe Val Gly Gly Ser Lys Val Val Ile Leu
                1045                1050                1055

Asp Glu Pro Thr Ala Gly Val Asp Pro Tyr Ser Arg Arg Gly Ile Trp
            1060                1065                1070

Glu Leu Leu Leu Lys Tyr Arg Gln Gly Arg Thr Ile Ile Leu Ser Thr
        1075                1080                1085

His His Met Asp Glu Ala Asp Val Leu Gly Asp Arg Ile Ala Ile Ile
    1090                1095                1100

Ser His Gly Lys Leu Cys Cys Val Gly Ser Ser Leu Phe Leu Lys Asn
1105                1110                1115                1120

Gln Leu Gly Thr Gly Tyr Tyr Leu Thr Leu Val Lys Lys Asp Val Glu
                1125                1130                1135

Ser Ser Leu Ser Ser Cys Arg Asn Ser Ser Ser Thr Val Ser Tyr Leu
            1140                1145                1150

Lys Lys Glu Asp Ser Val Ser Gln Ser Ser Ser Asp Ala Gly Leu Gly
        1155                1160                1165

Ser Asp His Glu Ser Asp Thr Leu Thr Ile Asp Val Ser Ala Ile Ser
    1170                1175                1180

Asn Leu Ile Arg Lys His Val Ser Glu Ala Arg Leu Val Glu Asp Ile
1185                1190                1195                1200

Gly His Glu Leu Thr Tyr Val Leu Pro Tyr Glu Ala Ala Lys Glu Gly
                1205                1210                1215

Ala Phe Val Glu Leu Phe His Glu Ile Asp Asp Arg Leu Ser Asp Leu
            1220                1225                1230

Gly Ile Ser Ser Tyr Gly Ile Ser Glu Thr Thr Leu Glu Glu Ile Phe
        1235                1240                1245

Leu Lys Val Ala Glu Glu Ser Gly Val Asp Ala Glu Thr Ser Asp Gly
    1250                1255                1260

Thr Leu Pro Ala Arg Arg Asn Arg Arg Ala Phe Gly Asp Lys Gln Ser
1265                1270                1275                1280

Cys Leu Arg Pro Phe Thr Glu Asp Asp Ala Ala Asp Pro Asn Asp Ser
                1285                1290                1295

Asp Ile Asp Pro Glu Ser Arg Glu Thr Asp Leu Leu Ser Gly Met Asp
            1300                1305                1310

Gly Lys Gly Ser Tyr Gln Val Lys Gly Trp Lys Leu Thr Gln Gln Gln
        1315                1320                1325

Phe Val Ala Leu Leu Trp Lys Arg Leu Leu Ile Ala Arg Arg Ser Arg
    1330                1335                1340

Lys Gly Phe Phe Ala Gln Ile Val Leu Pro Ala Val Phe Val Cys Ile
1345                1350                1355                1360

Ala Leu Val Phe Ser Leu Ile Val Pro Pro Phe Gly Lys Tyr Pro Ser
                1365                1370                1375

Leu Glu Leu Gln Pro Trp Met Tyr Asn Glu Gln Tyr Thr Phe Val Ser
            1380                1385                1390

Asn Asp Ala Pro Glu Asp Thr Gly Thr Leu Glu Leu Leu Asn Ala Leu
        1395                1400                1405

Thr Lys Asp Pro Gly Phe Gly Thr Arg Cys Met Glu Gly Asn Pro Ile
    1410                1415                1420

Pro Asp Thr Pro Cys Gln Ala Gly Glu Glu Glu Trp Thr Thr Ala Pro
1425                1430                1435                1440

Val Pro Gln Thr Ile Met Asp Leu Phe Gln Asn Gly Asn Trp Thr Met
                1445                1450                1455

Gln Asn Pro Ser Pro Ala Cys Gln Cys Ser Ser Asp Lys Ile Lys Lys
            1460                1465                1470

Met Leu Pro Val Cys Pro Pro Gly Ala Gly Gly Leu Pro Pro Pro Gln
        1475                1480                1485

Arg Lys Gln Asn Thr Ala Asp Ile Leu Gln Asp Leu Thr Gly Arg Asn
    1490                1495                1500

Ile Ser Asp Tyr Leu Val Lys Thr Tyr Val Gln Ile Ile Ala Lys Ser
1505                1510                1515                1520

Leu Lys Asn Lys Ile Trp Val Asn Glu Phe Arg Tyr Gly Gly Phe Ser
                1525                1530                1535

Leu Gly Val Ser Asn Thr Gln Ala Leu Pro Pro Ser Gln Glu Val Asn
            1540                1545                1550

Asp Ala Thr Lys Gln Met Lys Lys His Leu Lys Leu Ala Lys Asp Ser
        1555                1560                1565

Ser Ala Asp Arg Phe Leu Asn Ser Leu Gly Arg Phe Met Thr Gly Leu

1570                1575                1580

Asp Thr Arg Asn Asn Val Lys Val Trp Phe Asn Asn Lys Gly Trp His
1585                1590                1595                1600

Ala Ile Ser Ser Phe Leu Asn Val Ile Asn Asn Ala Ile Leu Arg Ala
                1605                1610                1615

Asn Leu Gln Lys Gly Glu Asn Pro Ser His Tyr Gly Ile Thr Ala Phe
            1620                1625                1630

Asn His Pro Leu Asn Leu Thr Lys Gln Gln Leu Ser Glu Val Ala Pro
        1635                1640                1645

Met Thr Thr Ser Val Asp Val Leu Val Ser Ile Cys Val Ile Phe Ala
    1650                1655                1660

Met Ser Phe Val Pro Ala Ser Phe Val Val Phe Leu Ile Gln Glu Arg
1665                1670                1675                1680

Val Ser Lys Ala Lys His Leu Gln Phe Ile Ser Gly Val Lys Pro Val
                1685                1690                1695

Ile Tyr Trp Leu Ser Asn Phe Val Trp Asp Met Cys Asn Tyr Val Val
            1700                1705                1710

Pro Ala Thr Leu Val Ile Ile Ile Phe Ile Cys Phe Gln Gln Lys Ser
        1715                1720                1725

Tyr Val Ser Ser Thr Asn Leu Pro Val Leu Ala Leu Leu Leu Leu Leu
    1730                1735                1740

Tyr Gly Trp Ser Ile Thr Pro Leu Met Tyr Pro Ala Ser Phe Val Phe
1745                1750                1755                1760

Lys Ile Pro Ser Thr Ala Tyr Val Val Leu Thr Ser Val Asn Leu Phe
                1765                1770                1775

Ile Gly Ile Asn Gly Ser Val Ala Thr Phe Val Leu Glu Leu Phe Thr
            1780                1785                1790

Asp Asn Lys Leu Asn Asn Ile Asn Asp Ile Leu Lys Ser Val Phe Leu
        1795                1800                1805

Ile Phe Pro His Phe Cys Leu Gly Arg Gly Leu Ile Asp Met Val Lys
    1810                1815                1820

Asn Gln Ala Met Ala Asp Ala Leu Glu Arg Phe Gly Glu Asn Arg Phe
1825                1830                1835                1840

Val Ser Pro Leu Ser Trp Asp Leu Val Gly Arg Asn Leu Phe Ala Met
                1845                1850                1855

Ala Val Glu Gly Val Val Phe Phe Leu Ile Thr Val Leu Ile Gln Tyr
            1860                1865                1870

Arg Phe Phe Ile Arg Pro Arg Pro Val Asn Ala Lys Leu Ser Pro Leu
        1875                1880                1885

Asn Asp Glu Asp Glu Asp Val Arg Arg Glu Arg Gln Arg Ile Leu Asp
    1890                1895                1900

Gly Gly Gly Gln Asn Asp Ile Leu Glu Ile Lys Glu Leu Thr Lys Ile
1905                1910                1915                1920

Tyr Arg Arg Lys Arg Lys Pro Ala Val Asp Arg Ile Cys Val Gly Ile
                1925                1930                1935

Pro Pro Gly Glu Cys Phe Gly Leu Leu Gly Val Asn Gly Ala Gly Lys
            1940                1945                1950

Ser Ser Thr Phe Lys Met Leu Thr Gly Asp Thr Thr Val Thr Arg Gly
        1955                1960                1965

Asp Ala Phe Leu Asn Arg Asn Ser Ile Leu Ser Asn Ile His Glu Val
    1970                1975                1980

His Gln Asn Met Gly Tyr Cys Pro Gln Phe Asp Ala Ile Thr Glu Leu
1985                1990                1995                2000

Leu Thr Gly Arg Glu His Val Glu Phe Phe Ala Leu Leu Arg Gly Val
                2005                2010                2015

Pro Glu Lys Glu Val Gly Lys Val Gly Glu Trp Ala Ile Arg Lys Leu
            2020                2025                2030

Gly Leu Val Lys Tyr Gly Glu Lys Tyr Ala Gly Asn Tyr Ser Gly Gly
        2035                2040                2045

Asn Lys Arg Lys Leu Ser Thr Ala Met Ala Leu Ile Gly Gly Pro Pro
    2050                2055                2060

Val Val Phe Leu Asp Glu Pro Thr Thr Gly Met Asp Pro Lys Ala Arg
2065                2070                2075                2080

Arg Phe Leu Trp Asn Cys Ala Leu Ser Val Val Lys Glu Gly Arg Ser
                2085                2090                2095

Val Val Leu Thr Ser His Ser Met Glu Glu Cys Glu Ala Leu Cys Thr
            2100                2105                2110

Arg Met Ala Ile Met Val Asn Gly Arg Phe Arg Cys Leu Gly Ser Val
        2115                2120                2125

Gln His Leu Lys Asn Arg Phe Gly Asp Gly Tyr Thr Ile Val Val Arg
    2130                2135                2140

Ile Ala Gly Ser Asn Pro Asp Leu Lys Pro Val Gln Asp Phe Phe Gly
2145                2150                2155                2160

Leu Ala Phe Pro Gly Ser Val Pro Lys Glu Lys His Arg Asn Met Leu
                2165                2170                2175

Gln Tyr Gln Leu Pro Ser Ser Leu Ser Ser Leu Ala Arg Ile Phe Ser
            2180                2185                2190

Ile Leu Ser Gln Ser Lys Lys Arg Leu His Ile Glu Asp Tyr Ser Val
        2195                2200                2205

Ser Gln Thr Thr Leu Asp Gln Val Phe Val Asn Phe Ala Lys Asp Gln
    2210                2215                2220

Ser Asp Asp Asp His Leu Lys Asp Leu Ser Leu His Lys Asn Gln Thr
2225                2230                2235                2240

Val Val Asp Val Ala Val Leu Thr Ser Phe Leu Gln Asp Glu Lys Val
                2245                2250                2255

Lys Glu Ser Tyr Val
            2260

<210> 94
<211> 2261
<212> PRT
<213> Homo sapiens

<400> 94
Met Ala Cys Trp Pro Gln Leu Arg Leu Leu Leu Trp Lys Asn Leu Thr
1               5                   10                  15

Phe Arg Arg Arg Gln Thr Cys Gln Leu Leu Leu Glu Val Ala Trp Pro
            20                  25                  30

Leu Phe Ile Phe Leu Ile Leu Ile Ser Val Arg Leu Ser Tyr Pro Pro
        35                  40                  45

Tyr Glu Gln His Glu Cys His Phe Pro Asn Lys Ala Met Pro Ser Ala
    50                  55                  60

Gly Thr Leu Pro Trp Val Gln Gly Ile Ile Cys Asn Ala Asn Asn Pro
65                  70                  75                  80

Cys Phe Arg Tyr Pro Thr Pro Gly Glu Ala Pro Gly Val Val Gly Asn
                85                  90                  95

Phe Asn Lys Ser Ile Val Ala Arg Leu Phe Ser Asp Ala Arg Arg Leu
            100                 105                 110

Leu Leu Tyr Ser Gln Lys Asp Thr Ser Met Lys Asp Met Arg Lys Val
        115                 120                 125

Leu Arg Thr Leu Gln Gln Ile Lys Lys Ser Ser Ser Asn Leu Lys Leu
    130                 135                 140

Gln Asp Phe Leu Val Asp Asn Glu Thr Phe Ser Gly Phe Leu Tyr His
145                 150                 155                 160

Asn Leu Ser Leu Pro Lys Ser Thr Val Asp Lys Met Leu Arg Ala Asp
                165                 170                 175

Val Ile Leu His Lys Val Phe Leu Gln Gly Tyr Gln Leu His Leu Thr
            180                 185                 190

Ser Leu Cys Asn Gly Ser Lys Ser Glu Glu Met Ile Gln Leu Gly Asp
        195                 200                 205

Gln Glu Val Ser Glu Leu Cys Gly Leu Pro Arg Glu Lys Leu Ala Ala
    210                 215                 220

Ala Glu Arg Val Leu Arg Ser Asn Met Asp Ile Leu Lys Pro Ile Leu
225                 230                 235                 240

Arg Thr Leu Asn Ser Thr Ser Pro Phe Pro Ser Lys Glu Leu Ala Glu
                245                 250                 255

Ala Thr Lys Thr Leu Leu His Ser Leu Gly Thr Leu Ala Gln Glu Leu
            260                 265                 270

Phe Ser Met Arg Ser Trp Ser Asp Met Arg Gln Glu Val Met Phe Leu
        275                 280                 285

Thr Asn Val Asn Ser Ser Ser Ser Ser Thr Gln Ile Tyr Gln Ala Val
    290                 295                 300

Ser Arg Ile Val Cys Gly His Pro Glu Gly Gly Gly Leu Lys Ile Lys
305                 310                 315                 320

Ser Leu Asn Trp Tyr Glu Asp Asn Asn Tyr Lys Ala Leu Phe Gly Gly
                325                 330                 335

Asn Gly Thr Glu Glu Asp Ala Glu Thr Phe Tyr Asp Asn Ser Thr Thr
            340                 345                 350

Pro Tyr Cys Asn Asp Leu Met Lys Asn Leu Glu Ser Ser Pro Leu Ser
        355                 360                 365

Arg Ile Ile Trp Lys Ala Leu Lys Pro Leu Leu Val Gly Lys Ile Leu
    370                 375                 380

Tyr Thr Pro Asp Thr Pro Ala Thr Arg Gln Val Met Ala Glu Val Asn
385                 390                 395                 400

Lys Thr Phe Gln Glu Leu Ala Val Phe His Asp Leu Glu Gly Met Trp
                405                 410                 415

Glu Glu Leu Ser Pro Lys Ile Trp Thr Phe Met Glu Asn Ser Gln Glu
            420                 425                 430

Met Asp Leu Val Arg Met Leu Leu Asp Ser Arg Asp Asn Asp His Phe
        435                 440                 445

Trp Glu Gln Gln Leu Asp Gly Leu Asp Trp Thr Ala Gln Asp Ile Val
    450                 455                 460

Ala Phe Leu Ala Lys His Pro Glu Asp Val Gln Ser Ser Asn Gly Ser
465                 470                 475                 480

Val Tyr Thr Trp Arg Glu Ala Phe Asn Glu Thr Asn Gln Ala Ile Arg
                485                 490                 495

Thr Ile Ser Arg Phe Met Glu Cys Val Asn Leu Asn Lys Leu Glu Pro

            500                 505                 510

Ile Ala Thr Glu Val Trp Leu Ile Asn Lys Ser Met Glu Leu Leu Asp
        515                 520                 525

Glu Arg Lys Phe Trp Ala Gly Ile Val Phe Thr Gly Ile Thr Pro Gly
    530                 535                 540

Ser Ile Glu Leu Pro His His Val Lys Tyr Lys Ile Arg Met Asp Ile
545                 550                 555                 560

Asp Asn Val Glu Arg Thr Asn Lys Ile Lys Asp Gly Tyr Trp Asp Pro
                565                 570                 575

Gly Pro Arg Ala Asp Pro Phe Glu Asp Met Arg Tyr Val Trp Gly Gly
            580                 585                 590

Phe Ala Tyr Leu Gln Asp Val Val Glu Gln Ala Ile Ile Arg Val Leu
        595                 600                 605

Thr Gly Thr Glu Lys Lys Thr Gly Val Tyr Met Gln Gln Met Pro Tyr
    610                 615                 620

Pro Cys Tyr Val Asp Asp Ile Phe Leu Arg Val Met Ser Arg Ser Met
625                 630                 635                 640

Pro Leu Phe Met Thr Leu Ala Trp Ile Tyr Ser Val Ala Val Ile Ile
                645                 650                 655

Lys Gly Ile Val Tyr Glu Lys Glu Ala Arg Leu Lys Glu Thr Met Arg
            660                 665                 670

Ile Met Gly Leu Asp Asn Ser Ile Leu Trp Phe Ser Trp Phe Ile Ser
        675                 680                 685

Ser Leu Ile Pro Leu Leu Val Ser Ala Gly Leu Leu Val Val Ile Leu
    690                 695                 700

Lys Leu Gly Asn Leu Leu Pro Tyr Ser Asp Pro Ser Val Val Phe Val
705                 710                 715                 720

Phe Leu Ser Val Phe Ala Val Val Thr Ile Leu Gln Cys Phe Leu Ile
                725                 730                 735

Ser Thr Leu Phe Ser Arg Ala Asn Leu Ala Ala Ala Cys Gly Gly Ile
            740                 745                 750

Ile Tyr Phe Thr Leu Tyr Leu Pro Tyr Val Leu Cys Val Ala Trp Gln
        755                 760                 765

Asp Tyr Val Gly Phe Thr Leu Lys Ile Phe Ala Ser Leu Leu Ser Pro
    770                 775                 780

Val Ala Phe Gly Phe Gly Cys Glu Tyr Phe Ala Leu Phe Glu Glu Gln
785                 790                 795                 800

Gly Ile Gly Val Gln Trp Asp Asn Leu Phe Glu Ser Pro Val Glu Glu
                805                 810                 815

Asp Gly Phe Asn Leu Thr Thr Ser Val Ser Met Met Leu Phe Asp Thr
            820                 825                 830

Phe Leu Tyr Gly Val Met Thr Trp Tyr Ile Glu Ala Val Phe Pro Gly
        835                 840                 845

Gln Tyr Arg Ile Pro Arg Pro Trp Tyr Phe Pro Cys Thr Lys Ser Tyr
    850                 855                 860

Trp Phe Gly Glu Glu Ser Asp Glu Lys Ser His Pro Gly Ser Asn Gln
865                 870                 875                 880

Lys Arg Ile Ser Glu Ile Cys Met Glu Glu Glu Pro Thr His Leu Lys
                885                 890                 895

Leu Gly Val Ser Ile Gln Asn Leu Val Lys Val Tyr Arg Asp Gly Met
            900                 905                 910

Lys Val Ala Val Asp Gly Leu Ala Leu Asn Phe Tyr Glu Gly Gln Ile
        915                 920                 925

Thr Ser Phe Leu Gly His Asn Gly Ala Gly Lys Thr Thr Thr Met Ser
    930                 935                 940

Ile Leu Thr Gly Leu Phe Pro Pro Thr Ser Gly Thr Ala Tyr Ile Leu
945                 950                 955                 960

Gly Lys Asp Ile Arg Ser Glu Met Ser Thr Ile Arg Gln Asn Leu Gly
                965                 970                 975

Val Cys Pro Gln His Asn Val Leu Phe Asp Met Leu Thr Val Glu Glu
            980                 985                 990

His Ile Trp Phe Tyr Ala Arg Leu Lys Gly Leu Ser Glu Lys His Val
        995                 1000                1005

Lys Ala Glu Met Glu Gln Met Ala Leu Asp Val Gly Leu Pro Ser Ser
    1010                1015                1020

Lys Leu Lys Ser Lys Thr Ser Gln Leu Ser Gly Gly Met Gln Arg Lys
1025                1030                1035                1040

Leu Ser Val Ala Leu Ala Phe Val Gly Gly Ser Lys Val Val Ile Leu
                1045                1050                1055

Asp Glu Pro Thr Ala Gly Val Asp Pro Tyr Ser Arg Arg Gly Ile Trp
            1060                1065                1070

Glu Leu Leu Leu Lys Tyr Arg Gln Gly Arg Thr Ile Ile Leu Ser Thr
        1075                1080                1085

His His Met Asp Glu Ala Asp Val Leu Gly Asp Arg Ile Ala Ile Ile
    1090                1095                1100

Ser His Gly Lys Leu Cys Cys Val Gly Ser Ser Leu Phe Leu Lys Asn
1105                1110                1115                1120

Gln Leu Gly Thr Gly Tyr Tyr Leu Thr Leu Val Lys Lys Asp Val Glu
                1125                1130                1135

Ser Ser Leu Ser Ser Cys Arg Asn Ser Ser Ser Thr Val Ser Tyr Leu
            1140                1145                1150

Lys Lys Glu Asp Ser Val Ser Gln Ser Ser Ser Asp Ala Gly Leu Gly
        1155                1160                1165

Ser Asp His Glu Ser Asp Thr Leu Thr Ile Asp Val Ser Ala Ile Ser
    1170                1175                1180

Asn Leu Ile Arg Lys His Val Ser Glu Ala Arg Leu Val Glu Asp Ile
1185                1190                1195                1200

Gly His Glu Leu Thr Tyr Val Leu Pro Tyr Glu Ala Ala Lys Glu Gly
                1205                1210                1215

Ala Phe Val Glu Leu Phe His Glu Ile Asp Asp Arg Leu Ser Asp Leu
            1220                1225                1230

Gly Ile Ser Ser Tyr Gly Ile Ser Glu Thr Thr Leu Glu Glu Ile Phe
        1235                1240                1245

Leu Lys Val Ala Glu Glu Ser Gly Val Asp Ala Glu Thr Ser Asp Gly
    1250                1255                1260

Thr Leu Pro Ala Arg Arg Asn Arg Arg Ala Phe Gly Asp Lys Gln Ser
1265                1270                1275                1280

Cys Leu Arg Pro Phe Thr Glu Asp Asp Ala Ala Asp Pro Asn Asp Ser
                1285                1290                1295

Asp Ile Asp Pro Glu Ser Arg Glu Thr Asp Leu Leu Ser Gly Met Asp
            1300                1305                1310

Gly Lys Gly Ser Tyr Gln Val Lys Gly Trp Lys Leu Thr Gln Gln Gln
        1315                1320                1325

Phe Val Ala Leu Leu Trp Lys Arg Leu Leu Ile Ala Arg Arg Ser Arg
    1330                1335                1340

Lys Gly Phe Phe Ala Gln Ile Val Leu Pro Ala Val Phe Val Cys Ile
1345                1350                1355                1360

Ala Leu Val Phe Ser Leu Ile Val Pro Pro Phe Gly Lys Tyr Pro Ser
                1365                1370                1375

Leu Glu Leu Gln Pro Trp Met Tyr Asn Glu Gln Tyr Thr Phe Val Ser
            1380                1385                1390

Asn Asp Ala Pro Glu Asp Thr Gly Thr Leu Glu Leu Leu Asn Ala Leu
        1395                1400                1405

Thr Lys Asp Pro Gly Phe Gly Thr Arg Cys Met Glu Gly Asn Pro Ile
    1410                1415                1420

Pro Asp Thr Pro Cys Gln Ala Gly Glu Glu Glu Trp Thr Thr Ala Pro

1425                1430                1435                1440

Val Pro Gln Thr Ile Met Asp Leu Phe Gln Asn Gly Asn Trp Thr Met
                1445                1450                1455

Gln Asn Pro Ser Pro Ala Cys Gln Cys Ser Ser Asp Lys Ile Lys Lys
            1460                1465                1470

Met Leu Pro Val Cys Pro Pro Gly Ala Gly Gly Leu Pro Pro Gln
        1475                1480                1485

Arg Lys Gln Asn Thr Ala Asp Ile Leu Gln Asp Leu Thr Gly Arg Asn
    1490                1495                1500

Ile Ser Asp Tyr Leu Val Lys Thr Tyr Val Gln Ile Ile Ala Lys Ser
1505                1510                1515                1520

Leu Lys Asn Lys Ile Trp Val Asn Glu Phe Arg Tyr Gly Gly Phe Ser
                1525                1530                1535

Leu Gly Val Ser Asn Thr Gln Ala Leu Pro Pro Ser Gln Glu Val Asn
            1540                1545                1550

Asp Ala Thr Lys Gln Met Lys Lys His Leu Lys Leu Ala Lys Asp Ser
        1555                1560                1565

Ser Ala Asp Arg Phe Leu Asn Ser Leu Gly Arg Phe Met Thr Gly Leu
    1570                1575                1580

Asp Thr Arg Asn Asn Val Lys Val Trp Phe Asn Asn Lys Gly Trp His
1585                1590                1595                1600

Ala Ile Ser Ser Phe Leu Asn Val Ile Asn Asn Ala Ile Leu Arg Ala
                1605                1610                1615

Asn Leu Gln Lys Gly Glu Asn Pro Ser His Tyr Gly Ile Thr Ala Phe
            1620                1625                1630

Asn His Pro Leu Asn Leu Thr Lys Gln Gln Leu Ser Glu Val Ala Pro
        1635                1640                1645

Met Thr Thr Ser Val Asp Val Leu Val Ser Ile Cys Val Ile Phe Ala
    1650                1655                1660

Met Ser Phe Val Pro Ala Ser Phe Val Val Phe Leu Ile Gln Glu Arg
1665                1670                1675                1680

Val Ser Lys Ala Lys His Leu Gln Phe Ile Ser Gly Val Lys Pro Val
                1685                1690                1695

Ile Tyr Trp Leu Ser Asn Phe Val Trp Asp Met Cys Asn Tyr Val Val
            1700                1705                1710

Pro Ala Thr Leu Val Ile Ile Ile Phe Ile Cys Phe Gln Gln Lys Ser
        1715                1720                1725

Tyr Val Ser Ser Thr Asn Leu Pro Val Leu Ala Leu Leu Leu Leu Leu
    1730                1735                1740

Tyr Gly Trp Ser Ile Thr Pro Leu Met Tyr Pro Ala Ser Phe Val Phe
1745                1750                1755                1760

Lys Ile Pro Ser Thr Ala Tyr Val Val Leu Thr Ser Val Asn Leu Phe
                1765                1770                1775

Ile Gly Ile Asn Gly Ser Val Ala Thr Phe Val Leu Glu Leu Phe Thr
            1780                1785                1790

Asp Asn Lys Leu Asn Asn Ile Asn Asp Ile Leu Lys Ser Val Phe Leu
        1795                1800                1805

Ile Phe Pro His Phe Cys Leu Gly Arg Gly Leu Ile Asp Met Val Lys
    1810                1815                1820

Asn Gln Ala Met Ala Asp Ala Leu Glu Arg Phe Gly Glu Asn Arg Phe
1825                1830                1835                1840

Val Ser Pro Leu Ser Trp Asp Leu Val Gly Arg Asn Leu Phe Ala Met
                1845                1850                1855

Ala Val Glu Gly Val Val Phe Phe Leu Ile Thr Val Leu Ile Gln Tyr
            1860                1865                1870

Arg Phe Phe Ile Arg Pro Arg Pro Val Asn Ala Lys Leu Ser Pro Leu
        1875                1880                1885

Asn Asp Glu Asp Glu Asp Val Arg Arg Glu Arg Gln Arg Ile Leu Asp
    1890                1895                1900

Gly Gly Gly Gln Asn Asp Ile Leu Glu Ile Lys Glu Leu Thr Lys Ile
1905                1910                1915                1920

Tyr Arg Arg Lys Arg Lys Pro Ala Val Asp Arg Ile Cys Val Gly Ile
                1925                1930                1935

Pro Pro Gly Glu Cys Phe Gly Leu Leu Gly Val Asn Gly Ala Gly Lys
            1940                1945                1950

Ser Ser Thr Phe Lys Met Leu Thr Gly Asp Thr Thr Val Thr Arg Gly
        1955                1960                1965

Asp Ala Phe Leu Asn Arg Asn Ser Ile Leu Ser Asn Ile His Glu Val
    1970                1975                1980

His Gln Asn Met Gly Tyr Cys Pro Gln Phe Asp Ala Ile Thr Glu Leu
1985                1990                1995                2000

Leu Thr Gly Arg Glu His Val Glu Phe Phe Ala Leu Leu Arg Gly Val
                2005                2010                2015

Pro Glu Lys Glu Val Gly Lys Val Gly Glu Trp Ala Ile Arg Lys Leu
            2020                2025                2030

Gly Leu Val Lys Tyr Gly Glu Lys Tyr Ala Gly Asn Tyr Ser Gly Gly
        2035                2040                2045

Asn Lys Arg Lys Leu Ser Thr Ala Met Ala Leu Ile Gly Gly Pro Pro
    2050                2055                2060

Val Val Phe Leu Asp Glu Pro Thr Thr Gly Met Asp Pro Lys Ala Arg
2065                2070                2075                2080

Arg Phe Leu Trp Asn Cys Ala Leu Ser Val Val Lys Glu Gly Arg Ser
                2085                2090                2095

Val Val Leu Thr Ser His Ser Met Glu Glu Cys Glu Ala Leu Cys Thr
            2100                2105                2110

Arg Met Ala Ile Met Val Asn Gly Arg Phe Arg Cys Leu Gly Ser Val
        2115                2120                2125

Gln His Leu Lys Asn Arg Phe Gly Asp Gly Tyr Thr Ile Val Val Arg
    2130                2135                2140

Ile Ala Gly Ser Asn Pro Asp Leu Lys Pro Val Gln Asp Phe Phe Gly
2145                2150                2155                2160

Leu Ala Phe Pro Gly Ser Val Pro Lys Glu Lys His Arg Asn Met Leu
                2165                2170                2175

Gln Tyr Gln Leu Pro Ser Ser Leu Ser Ser Leu Ala Arg Ile Phe Ser
            2180                2185                2190

Ile Leu Ser Gln Ser Lys Lys Arg Leu His Ile Glu Asp Tyr Ser Val
        2195                2200                2205

Ser Gln Thr Thr Leu Asp Gln Val Phe Val Asn Phe Ala Lys Asp Gln
    2210                2215                2220

Ser Asp Asp Asp His Leu Lys Asp Leu Ser Leu His Lys Asn Gln Thr
2225                2230                2235                2240

Val Val Asp Val Ala Val Leu Thr Ser Phe Leu Gln Asp Glu Lys Val
                2245                2250                2255

Lys Glu Ser Tyr Val
            2260

<210> 95
<211> 2261
<212> PRT
<213> Homo sapiens

<400> 95
Met Ala Cys Trp Pro Gln Leu Arg Leu Leu Leu Trp Lys Asn Leu Thr
1               5                   10                  15

Phe Arg Arg Arg Gln Thr Cys Gln Leu Leu Leu Glu Val Ala Trp Pro
            20                  25                  30

Leu Phe Ile Phe Leu Ile Leu Ile Ser Val Arg Leu Ser Tyr Pro Pro
        35                  40                  45

Tyr Glu Gln His Glu Cys His Phe Pro Asn Lys Ala Met Pro Ser Ala
    50                  55                  60

Gly Thr Leu Pro Trp Val Gln Gly Ile Ile Cys Asn Ala Asn Asn Pro
65                  70                  75                  80

Cys Phe Arg Tyr Pro Thr Pro Gly Glu Ala Pro Gly Val Val Gly Asn
                85                  90                  95

Phe Asn Lys Ser Ile Val Ala Arg Leu Phe Ser Asp Ala Arg Arg Leu
            100                 105                 110

Leu Leu Tyr Ser Gln Lys Asp Thr Ser Met Lys Asp Met Arg Lys Val
        115                 120                 125

Leu Arg Thr Leu Gln Gln Ile Lys Lys Ser Ser Ser Asn Leu Lys Leu
    130                 135                 140

Gln Asp Phe Leu Val Asp Asn Glu Thr Phe Ser Gly Phe Leu Tyr His
145                 150                 155                 160

Asn Leu Ser Leu Pro Lys Ser Thr Val Asp Lys Met Leu Arg Ala Asp
                165                 170                 175

Val Ile Leu His Lys Val Phe Leu Gln Gly Tyr Gln Leu His Leu Thr
            180                 185                 190

Ser Leu Cys Asn Gly Ser Lys Ser Glu Glu Met Ile Gln Leu Gly Asp
        195                 200                 205

Gln Glu Val Ser Glu Leu Cys Gly Leu Pro Arg Glu Lys Leu Ala Ala
    210                 215                 220

Ala Glu Arg Val Leu Arg Ser Asn Met Asp Ile Leu Lys Pro Ile Leu
225                 230                 235                 240

Arg Thr Leu Asn Ser Thr Ser Pro Phe Pro Ser Lys Glu Leu Ala Glu
                245                 250                 255

Ala Thr Lys Thr Leu Leu His Ser Leu Gly Thr Leu Ala Gln Glu Leu
            260                 265                 270

Phe Ser Met Arg Ser Trp Ser Asp Met Arg Gln Glu Val Met Phe Leu
        275                 280                 285

Thr Asn Val Asn Ser Ser Ser Ser Ser Thr Gln Ile Tyr Gln Ala Val
    290                 295                 300

Ser Arg Ile Val Cys Gly His Pro Glu Gly Gly Gly Leu Lys Ile Lys
305                 310                 315                 320

Ser Leu Asn Trp Tyr Glu Asp Asn Asn Tyr Lys Ala Leu Phe Gly Gly
                325                 330                 335

Asn Gly Thr Glu Glu Asp Ala Glu Thr Phe Tyr Asp Asn Ser Thr Thr
            340                 345                 350

Pro Tyr Cys Asn Asp Leu Met Lys Asn Leu Glu Ser Ser Pro Leu Ser

355                 360                 365

Arg Ile Ile Trp Lys Ala Leu Lys Pro Leu Leu Val Gly Lys Ile Leu
    370                 375                 380

Tyr Thr Pro Asp Thr Pro Ala Thr Arg Gln Val Met Ala Glu Val Asn
385                 390                 395                 400

Lys Thr Phe Gln Glu Leu Ala Val Phe His Asp Leu Glu Gly Met Trp
                405                 410                 415

Glu Glu Leu Ser Pro Lys Ile Trp Thr Phe Met Glu Asn Ser Gln Glu
            420                 425                 430

Met Asp Leu Val Arg Met Leu Leu Asp Ser Arg Asp Asn Asp His Phe
        435                 440                 445

Trp Glu Gln Gln Leu Asp Gly Leu Asp Trp Thr Ala Gln Asp Ile Val
    450                 455                 460

Ala Phe Leu Ala Lys His Pro Glu Asp Val Gln Ser Ser Asn Gly Ser
465                 470                 475                 480

Val Tyr Thr Trp Arg Glu Ala Phe Asn Glu Thr Asn Gln Ala Ile Arg
                485                 490                 495

Thr Ile Ser Arg Phe Met Glu Cys Val Asn Leu Asn Lys Leu Glu Pro
            500                 505                 510

Ile Ala Thr Glu Val Trp Leu Ile Asn Lys Ser Met Glu Leu Leu Asp
        515                 520                 525

Glu Arg Lys Phe Trp Ala Gly Ile Val Phe Thr Gly Ile Thr Pro Gly
    530                 535                 540

Ser Ile Glu Leu Pro His His Val Lys Tyr Lys Ile Arg Met Asp Ile
545                 550                 555                 560

Asp Asn Val Glu Arg Thr Asn Lys Ile Lys Asp Gly Tyr Trp Asp Pro
                565                 570                 575

Gly Pro Arg Ala Asp Pro Phe Glu Asp Met Arg Tyr Val Trp Gly Gly
            580                 585                 590

Phe Ala Tyr Leu Gln Asp Val Val Glu Gln Ala Ile Ile Arg Val Leu
        595                 600                 605

Thr Gly Thr Glu Lys Lys Thr Gly Val Tyr Met Gln Gln Met Pro Tyr
    610                 615                 620

Pro Cys Tyr Val Asp Asp Ile Phe Leu Arg Val Met Ser Arg Ser Met
625                 630                 635                 640

Pro Leu Phe Met Thr Leu Ala Trp Ile Tyr Ser Val Ala Val Ile Ile
                645                 650                 655

Lys Gly Ile Val Tyr Glu Lys Glu Ala Arg Leu Lys Glu Thr Met Arg
            660                 665                 670

Ile Met Gly Leu Asp Asn Ser Ile Leu Trp Phe Ser Trp Phe Ile Ser
        675                 680                 685

Ser Leu Ile Pro Leu Leu Val Ser Ala Gly Leu Leu Val Val Ile Leu
    690                 695                 700

Lys Leu Gly Asn Leu Leu Pro Tyr Ser Asp Pro Ser Val Val Phe Val
705                 710                 715                 720

Phe Leu Ser Val Phe Ala Val Val Thr Ile Leu Gln Cys Phe Leu Ile
                725                 730                 735

Ser Thr Leu Phe Ser Arg Ala Asn Leu Ala Ala Ala Cys Gly Gly Ile
            740                 745                 750

Ile Tyr Phe Thr Leu Tyr Leu Pro Tyr Val Leu Cys Val Ala Trp Gln
        755                 760                 765

Asp Tyr Val Gly Phe Thr Leu Lys Ile Phe Ala Ser Leu Leu Ser Pro
    770                 775                 780

Val Ala Phe Gly Phe Gly Cys Glu Tyr Phe Ala Leu Phe Glu Glu Gln
785                 790                 795                 800

Gly Ile Gly Val Gln Trp Asp Asn Leu Phe Glu Ser Pro Val Glu Glu
                805                 810                 815

Asp Gly Phe Asn Leu Thr Thr Ser Val Ser Met Met Leu Phe Asp Thr
            820                 825                 830

Phe Leu Tyr Gly Val Met Thr Trp Tyr Ile Glu Ala Val Phe Pro Gly
        835                 840                 845

Gln Tyr Gly Ile Pro Arg Pro Trp Tyr Phe Pro Cys Thr Lys Ser Tyr
    850                 855                 860

Trp Phe Gly Glu Glu Ser Asp Glu Lys Ser His Pro Gly Ser Asn Gln
865                 870                 875                 880

Lys Arg Met Ser Glu Ile Cys Met Glu Glu Glu Pro Thr His Leu Lys
                885                 890                 895

Leu Gly Val Ser Ile Gln Asn Leu Val Lys Val Tyr Arg Asp Gly Met
            900                 905                 910

Lys Val Ala Val Asp Gly Leu Ala Leu Asn Phe Tyr Glu Gly Gln Ile
        915                 920                 925

Thr Ser Phe Leu Gly His Asn Gly Ala Gly Lys Thr Thr Thr Met Ser
    930                 935                 940

Ile Leu Thr Gly Leu Phe Pro Pro Thr Ser Gly Thr Ala Tyr Ile Leu
945                 950                 955                 960

Gly Lys Asp Ile Arg Ser Glu Met Ser Thr Ile Arg Gln Asn Leu Gly
                965                 970                 975

Val Cys Pro Gln His Asn Val Leu Phe Asp Met Leu Thr Val Glu Glu
            980                 985                 990

His Ile Trp Phe Tyr Ala Arg Leu Lys Gly Leu Ser Glu Lys His Val
        995                 1000                1005

Lys Ala Glu Met Glu Gln Met Ala Leu Asp Val Gly Leu Pro Ser Ser
    1010                1015                1020

Lys Leu Lys Ser Lys Thr Ser Gln Leu Ser Gly Gly Met Gln Arg Lys
1025                1030                1035                1040

Leu Ser Val Ala Leu Ala Phe Val Gly Gly Ser Lys Val Val Ile Leu
                1045                1050                1055

Asp Glu Pro Thr Ala Gly Val Asp Pro Tyr Ser Arg Arg Gly Ile Trp
            1060                1065                1070

Glu Leu Leu Leu Lys Tyr Arg Gln Gly Arg Thr Ile Ile Leu Ser Thr
        1075                1080                1085

His His Met Asp Glu Ala Asp Val Leu Gly Asp Arg Ile Ala Ile Ile
    1090                1095                1100

Ser His Gly Lys Leu Cys Cys Val Gly Ser Ser Leu Phe Leu Lys Asn
1105                1110                1115                1120

Gln Leu Gly Thr Gly Tyr Tyr Leu Thr Leu Val Lys Lys Asp Val Glu
                1125                1130                1135

Ser Ser Leu Ser Ser Cys Arg Asn Ser Ser Ser Thr Val Ser Tyr Leu
            1140                1145                1150

Lys Lys Glu Asp Ser Val Ser Gln Ser Ser Ser Asp Ala Gly Leu Gly
        1155                1160                1165

Ser Asp His Glu Ser Asp Thr Leu Thr Ile Asp Val Ser Ala Ile Ser
    1170                1175                1180

Asn Leu Ile Arg Lys His Val Ser Glu Ala Arg Leu Val Glu Asp Ile
1185                1190                1195                1200

Gly His Glu Leu Thr Tyr Val Leu Pro Tyr Glu Ala Ala Lys Glu Gly
                1205                1210                1215

Ala Phe Val Glu Leu Phe His Glu Ile Asp Asp Arg Leu Ser Asp Leu
            1220                1225                1230

Gly Ile Ser Ser Tyr Gly Ile Ser Glu Thr Thr Leu Glu Glu Ile Phe
        1235                1240                1245

Leu Lys Val Ala Glu Glu Ser Gly Val Asp Ala Glu Thr Ser Asp Gly
    1250                1255                1260

Thr Leu Pro Ala Arg Arg Asn Arg Arg Ala Phe Gly Asp Lys Gln Ser
1265                1270                1275                1280

Cys Leu Arg Pro Phe Thr Glu Asp Asp Ala Ala Asp Pro Asn Asp Ser

1285                1290                1295

Asp Ile Asp Pro Glu Ser Arg Glu Thr Asp Leu Leu Ser Gly Met Asp
        1300                1305                1310

Gly Lys Gly Ser Tyr Gln Val Lys Gly Trp Lys Leu Thr Gln Gln Gln
    1315                1320                1325

Phe Val Ala Leu Leu Trp Lys Arg Leu Leu Ile Ala Arg Arg Ser Arg
1330                1335                1340

Lys Gly Phe Phe Ala Gln Ile Val Leu Pro Ala Val Phe Val Cys Ile
1345                1350                1355                1360

Ala Leu Val Phe Ser Leu Ile Val Pro Pro Phe Gly Lys Tyr Pro Ser
                1365                1370                1375

Leu Glu Leu Gln Pro Trp Met Tyr Asn Glu Gln Tyr Thr Phe Val Ser
        1380                1385                1390

Asn Asp Ala Pro Glu Asp Thr Gly Thr Leu Glu Leu Leu Asn Ala Leu
    1395                1400                1405

Thr Lys Asp Pro Gly Phe Gly Thr Arg Cys Met Glu Gly Asn Pro Ile
1410                1415                1420

Pro Asp Thr Pro Cys Gln Ala Gly Glu Glu Glu Trp Thr Thr Ala Pro
1425                1430                1435                1440

Val Pro Gln Thr Ile Met Asp Leu Phe Gln Asn Gly Asn Trp Thr Met
                1445                1450                1455

Gln Asn Pro Ser Pro Ala Cys Gln Cys Ser Ser Asp Lys Ile Lys Lys
        1460                1465                1470

Met Leu Pro Val Cys Pro Pro Gly Ala Gly Gly Leu Pro Pro Pro Gln
    1475                1480                1485

Arg Lys Gln Asn Thr Ala Asp Ile Leu Gln Asp Leu Thr Gly Arg Asn
1490                1495                1500

Ile Ser Asp Tyr Leu Val Lys Thr Tyr Val Gln Ile Ile Ala Lys Ser
1505                1510                1515                1520

Leu Lys Asn Lys Ile Trp Val Asn Glu Phe Arg Tyr Gly Gly Phe Ser
                1525                1530                1535

Leu Gly Val Ser Asn Thr Gln Ala Leu Pro Pro Ser Gln Glu Val Asn
        1540                1545                1550

Asp Ala Thr Lys Gln Met Lys Lys His Leu Lys Leu Ala Lys Asp Ser
    1555                1560                1565

Ser Ala Asp Arg Phe Leu Asn Ser Leu Gly Arg Phe Met Thr Gly Leu
1570                1575                1580

Asp Thr Arg Asn Asn Val Lys Val Trp Phe Asn Asn Lys Gly Trp His
1585                1590                1595                1600

Ala Ile Ser Ser Phe Leu Asn Val Ile Asn Asn Ala Ile Leu Arg Ala
                1605                1610                1615

Asn Leu Gln Lys Gly Glu Asn Pro Ser His Tyr Gly Ile Thr Ala Phe
            1620                1625                1630

Asn His Pro Leu Asn Leu Thr Lys Gln Gln Leu Ser Glu Val Ala Pro
        1635                1640                1645

Met Thr Thr Ser Val Asp Val Leu Val Ser Ile Cys Val Ile Phe Ala
    1650                1655                1660

Met Ser Phe Val Pro Ala Ser Phe Val Val Phe Leu Ile Gln Glu Arg
1665                1670                1675                1680

Val Ser Lys Ala Lys His Leu Gln Phe Ile Ser Gly Val Lys Pro Val
                1685                1690                1695

Ile Tyr Trp Leu Ser Asn Phe Val Trp Asp Met Cys Asn Tyr Val Val
            1700                1705                1710

Pro Ala Thr Leu Val Ile Ile Ile Phe Ile Cys Phe Gln Gln Lys Ser
        1715                1720                1725

Tyr Val Ser Ser Thr Asn Leu Pro Val Leu Ala Leu Leu Leu Leu Leu
    1730                1735                1740

Tyr Gly Trp Ser Ile Thr Pro Leu Met Tyr Pro Ala Ser Phe Val Phe
1745                1750                1755                1760

Lys Ile Pro Ser Thr Ala Tyr Val Val Leu Thr Ser Val Asn Leu Phe
                1765                1770                1775

Ile Gly Ile Asn Gly Ser Val Ala Thr Phe Val Leu Glu Leu Phe Thr
            1780                1785                1790

Asp Asn Lys Leu Asn Asn Ile Asn Asp Ile Leu Lys Ser Val Phe Leu
        1795                1800                1805

Ile Phe Pro His Phe Cys Leu Gly Arg Gly Leu Ile Asp Met Val Lys
    1810                1815                1820

Asn Gln Ala Met Ala Asp Ala Leu Glu Arg Phe Gly Glu Asn Arg Phe
1825                1830                1835                1840

Val Ser Pro Leu Ser Trp Asp Leu Val Gly Arg Asn Leu Phe Ala Met
                1845                1850                1855

Ala Val Glu Gly Val Val Phe Phe Leu Ile Thr Val Leu Ile Gln Tyr
            1860                1865                1870

Arg Phe Phe Ile Arg Pro Arg Pro Val Asn Ala Lys Leu Ser Pro Leu
        1875                1880                1885

Asn Asp Glu Asp Glu Asp Val Arg Arg Glu Arg Gln Arg Ile Leu Asp
    1890                1895                1900

Gly Gly Gly Gln Asn Asp Ile Leu Glu Ile Lys Glu Leu Thr Lys Ile
1905                1910                1915                1920

Tyr Arg Arg Lys Arg Lys Pro Ala Val Asp Arg Ile Cys Val Gly Ile
                1925                1930                1935

Pro Pro Gly Glu Cys Phe Gly Leu Leu Gly Val Asn Gly Ala Gly Lys
            1940                1945                1950

Ser Ser Thr Phe Lys Met Leu Thr Gly Asp Thr Thr Val Thr Arg Gly
        1955                1960                1965

Asp Ala Phe Leu Asn Arg Asn Ser Ile Leu Ser Asn Ile His Glu Val
    1970                1975                1980

His Gln Asn Met Gly Tyr Cys Pro Gln Phe Asp Ala Ile Thr Glu Leu
1985                1990                1995                2000

Leu Thr Gly Arg Glu His Val Glu Phe Phe Ala Leu Leu Arg Gly Val
                2005                2010                2015

Pro Glu Lys Glu Val Gly Lys Val Gly Glu Trp Ala Ile Arg Lys Leu
            2020                2025                2030

Gly Leu Val Lys Tyr Gly Glu Lys Tyr Ala Gly Asn Tyr Ser Gly Gly
        2035                2040                2045

Asn Lys Arg Lys Leu Ser Thr Ala Met Ala Leu Ile Gly Gly Pro Pro
    2050                2055                2060

Val Val Phe Leu Asp Glu Pro Thr Thr Gly Met Asp Pro Lys Ala Arg
2065                2070                2075                2080

Arg Phe Leu Trp Asn Cys Ala Leu Ser Val Val Lys Glu Gly Arg Ser
                2085                2090                2095

Val Val Leu Thr Ser His Ser Met Glu Glu Cys Glu Ala Leu Cys Thr
            2100                2105                2110

Arg Met Ala Ile Met Val Asn Gly Arg Phe Arg Cys Leu Gly Ser Val
        2115                2120                2125

Gln His Leu Lys Asn Arg Phe Gly Asp Gly Tyr Thr Ile Val Val Arg
    2130                2135                2140

Ile Ala Gly Ser Asn Pro Asp Leu Lys Pro Val Gln Asp Phe Phe Gly
2145                2150                2155                2160

Leu Ala Phe Pro Gly Ser Val Pro Lys Glu Lys His Arg Asn Met Leu
                2165                2170                2175

Gln Tyr Gln Leu Pro Ser Ser Leu Ser Ser Leu Ala Arg Ile Phe Ser
            2180                2185                2190

Ile Leu Ser Gln Ser Lys Lys Arg Leu His Ile Glu Asp Tyr Ser Val
        2195                2200                2205

Ser Gln Thr Thr Leu Asp Gln Val Phe Val Asn Phe Ala Lys Asp Gln

2210                    2215                    2220

Ser Asp Asp Asp His Leu Lys Asp Leu Ser Leu His Lys Asn Gln Thr
2225                    2230                    2235                    2240

Val Val Asp Val Ala Val Leu Thr Ser Phe Leu Gln Asp Glu Lys Val
                2245                    2250                    2255

Lys Glu Ser Tyr Val
            2260

<210> 96
<211> 1144
<212> PRT
<213> Homo sapiens

<400> 96
Met Ala Cys Trp Pro Gln Leu Arg Leu Leu Leu Trp Lys Asn Leu Thr
1               5                   10                  15

Phe Arg Arg Arg Gln Thr Cys Gln Leu Leu Leu Glu Val Ala Trp Pro
            20                  25                  30

Leu Phe Ile Phe Leu Ile Leu Ile Ser Val Arg Leu Ser Tyr Pro Pro
        35                  40                  45

Tyr Glu Gln His Glu Cys His Phe Pro Asn Lys Ala Met Pro Ser Ala
    50                  55                  60

Gly Thr Leu Pro Trp Val Gln Gly Ile Ile Cys Asn Ala Asn Asn Pro
65                  70                  75                  80

Cys Phe Arg Tyr Pro Thr Pro Gly Glu Ala Pro Gly Val Val Gly Asn
                85                  90                  95

Phe Asn Lys Ser Ile Val Ala Arg Leu Phe Ser Asp Ala Arg Arg Leu
            100                 105                 110

Leu Leu Tyr Ser Gln Lys Asp Thr Ser Met Lys Asp Met Arg Lys Val
        115                 120                 125

Leu Arg Thr Leu Gln Gln Ile Lys Lys Ser Ser Ser Asn Leu Lys Leu
    130                 135                 140

Gln Asp Phe Leu Val Asp Asn Glu Thr Phe Ser Gly Phe Leu Tyr His
145                 150                 155                 160

Asn Leu Ser Leu Pro Lys Ser Thr Val Asp Lys Met Leu Arg Ala Asp
                165                 170                 175

Val Ile Leu His Lys Val Phe Leu Gln Gly Tyr Gln Leu His Leu Thr
            180                 185                 190

Ser Leu Cys Asn Gly Ser Lys Ser Glu Glu Met Ile Gln Leu Gly Asp
        195                 200                 205

Gln Glu Val Ser Glu Leu Cys Gly Leu Pro Arg Glu Lys Leu Ala Ala

210                 215                 220

Ala Glu Arg Val Leu Arg Ser Asn Met Asp Ile Leu Lys Pro Ile Leu
225                 230                 235                 240

Arg Thr Leu Asn Ser Thr Ser Pro Phe Pro Ser Lys Glu Leu Ala Glu
                245                 250                 255

Ala Thr Lys Thr Leu Leu His Ser Leu Gly Thr Leu Ala Gln Glu Leu
            260                 265                 270

Phe Ser Met Arg Ser Trp Ser Asp Met Arg Gln Glu Val Met Phe Leu
        275                 280                 285

Thr Asn Val Asn Ser Ser Ser Ser Ser Thr Gln Ile Tyr Gln Ala Val
    290                 295                 300

Ser Arg Ile Val Cys Gly His Pro Glu Gly Gly Gly Leu Lys Ile Lys
305                 310                 315                 320

Ser Leu Asn Trp Tyr Glu Asp Asn Asn Tyr Lys Ala Leu Phe Gly Gly
                325                 330                 335

Asn Gly Thr Glu Glu Asp Ala Glu Thr Phe Tyr Asp Asn Ser Thr Thr
            340                 345                 350

Pro Tyr Cys Asn Asp Leu Met Lys Asn Leu Glu Ser Ser Pro Leu Ser
        355                 360                 365

Arg Ile Ile Trp Lys Ala Leu Lys Pro Leu Leu Val Gly Lys Ile Leu
    370                 375                 380

Tyr Thr Pro Asp Thr Pro Ala Thr Arg Gln Val Met Ala Glu Val Asn
385                 390                 395                 400

Lys Thr Phe Gln Glu Leu Ala Val Phe His Asp Leu Glu Gly Met Trp
                405                 410                 415

Glu Glu Leu Ser Pro Lys Ile Trp Thr Phe Met Glu Asn Ser Gln Glu
            420                 425                 430

Met Asp Leu Val Arg Met Leu Leu Asp Ser Arg Asp Asn Asp His Phe
        435                 440                 445

Trp Glu Gln Gln Leu Asp Gly Leu Asp Trp Thr Ala Gln Asp Ile Val
    450                 455                 460

Ala Phe Leu Ala Lys His Pro Glu Asp Val Gln Ser Ser Asn Gly Ser
465                 470                 475                 480

Val Tyr Thr Trp Arg Glu Ala Phe Asn Glu Thr Asn Gln Ala Ile Arg
                485                 490                 495

Thr Ile Ser Arg Phe Met Glu Cys Val Asn Leu Asn Lys Leu Glu Pro
            500                 505                 510

Ile Ala Thr Glu Val Trp Leu Ile Asn Lys Ser Met Glu Leu Leu Asp
        515                 520                 525

Glu Arg Lys Phe Trp Ala Gly Ile Val Phe Thr Gly Ile Thr Pro Gly
    530                 535                 540

Ser Ile Glu Leu Pro His His Val Lys Tyr Lys Ile Arg Met Asp Ile
545                 550                 555                 560

Asp Asn Val Glu Arg Thr Asn Lys Ile Lys Asp Gly Tyr Trp Asp Pro
                565                 570                 575

Gly Pro Arg Ala Asp Pro Phe Glu Asp Met Arg Tyr Val Trp Gly Gly
            580                 585                 590

Phe Ala Tyr Leu Gln Asp Val Val Glu Gln Ala Ile Ile Arg Val Leu
        595                 600                 605

Thr Gly Thr Glu Lys Lys Thr Gly Val Tyr Met Gln Gln Met Pro Tyr
    610                 615                 620

Pro Cys Tyr Val Asp Asp Ile Phe Leu Arg Val Met Ser Arg Ser Met
625                 630                 635                 640

Pro Leu Phe Met Thr Leu Ala Trp Ile Tyr Ser Val Ala Val Ile Ile
                645                 650                 655

Lys Gly Ile Val Tyr Glu Lys Glu Ala Arg Leu Lys Glu Thr Met Arg
            660                 665                 670

Ile Met Gly Leu Asp Asn Ser Ile Leu Trp Phe Ser Trp Phe Ile Ser
        675                 680                 685

Ser Leu Ile Pro Leu Leu Val Ser Ala Gly Leu Leu Val Val Ile Leu
    690                 695                 700

Lys Leu Gly Asn Leu Leu Pro Tyr Ser Asp Pro Ser Val Val Phe Val
705                 710                 715                 720

Phe Leu Ser Val Phe Ala Val Val Thr Ile Leu Gln Cys Phe Leu Ile
                725                 730                 735

Ser Thr Leu Phe Ser Arg Ala Asn Leu Ala Ala Ala Cys Gly Gly Ile
            740                 745                 750

Ile Tyr Phe Thr Leu Tyr Leu Pro Tyr Val Leu Cys Val Ala Trp Gln
        755                 760                 765

Asp Tyr Val Gly Phe Thr Leu Lys Ile Phe Ala Ser Leu Leu Ser Pro
    770                 775                 780

Val Ala Phe Gly Phe Gly Cys Glu Tyr Phe Ala Leu Phe Glu Glu Gln
785                 790                 795                 800

Gly Ile Gly Val Gln Trp Asp Asn Leu Phe Glu Ser Pro Val Glu Glu
                805                 810                 815

Asp Gly Phe Asn Leu Thr Thr Ser Val Ser Met Met Leu Phe Asp Thr
            820                 825                 830

Phe Leu Tyr Gly Val Met Thr Trp Tyr Ile Glu Ala Val Phe Pro Gly
        835                 840                 845

Gln Tyr Gly Ile Pro Arg Pro Trp Tyr Phe Pro Cys Thr Lys Ser Tyr
    850                 855                 860

Trp Phe Gly Glu Glu Ser Asp Glu Lys Ser His Pro Gly Ser Asn Gln
865                 870                 875                 880

Lys Arg Ile Ser Glu Ile Cys Met Glu Glu Glu Pro Thr His Leu Lys
                885                 890                 895

Leu Gly Val Ser Ile Gln Asn Leu Val Lys Val Tyr Arg Asp Gly Met
            900                 905                 910

Lys Val Ala Val Asp Gly Leu Ala Leu Asn Phe Tyr Glu Gly Gln Ile
        915                 920                 925

Thr Ser Phe Leu Gly His Asn Gly Ala Gly Lys Thr Thr Thr Met Ser
    930                 935                 940

Ile Leu Thr Gly Leu Phe Pro Pro Thr Ser Gly Thr Ala Tyr Ile Leu
945                 950                 955                 960

Gly Lys Asp Ile Arg Ser Glu Met Ser Thr Ile Arg Gln Asn Leu Gly
                965                 970                 975

Val Cys Pro Gln His Asn Val Leu Phe Asp Met Leu Thr Val Glu Glu
            980                 985                 990

His Ile Trp Phe Tyr Ala Arg Leu Lys Gly Leu Ser Glu Lys His Val
        995                 1000                1005

Lys Ala Glu Met Glu Gln Met Ala Leu Asp Val Gly Leu Pro Ser Ser
    1010                1015                1020

Lys Leu Lys Ser Lys Thr Ser Gln Leu Ser Gly Gly Met Gln Arg Lys
1025                1030                1035                1040

Leu Ser Val Ala Leu Ala Phe Val Gly Gly Ser Lys Val Val Ile Leu
                1045                1050                1055

Asp Glu Pro Thr Ala Gly Val Asp Pro Tyr Ser Arg Arg Gly Ile Trp
            1060                1065                1070

Glu Leu Leu Leu Lys Tyr Arg Gln Gly Arg Thr Ile Ile Leu Ser Thr
        1075                1080                1085

His His Met Asp Glu Ala Asp Val Leu Gly Asp Arg Ile Ala Ile Ile
    1090                1095                1100

Ser His Gly Lys Leu Cys Cys Val Gly Ser Pro Val Ser Glu Glu Pro
1105                1110                1115                1120

Ala Gly Asn Arg Leu Leu Pro Asp Leu Gly Gln Glu Arg Cys Gly Ile
                1125                1130                1135

Leu Pro Gln Phe Leu Gln Lys Gln

            1140

<210> 97
<211> 1144
<212> PRT
<213> Homo sapiens

<400> 97
Met Ala Cys Trp Pro Gln Leu Arg Leu Leu Leu Trp Lys Asn Leu Thr
1               5                   10                  15

Phe Arg Arg Arg Gln Thr Cys Gln Leu Leu Leu Glu Val Ala Trp Pro
            20                  25                  30

Leu Phe Ile Phe Leu Ile Leu Ile Ser Val Arg Leu Ser Tyr Pro Pro
        35                  40                  45

Tyr Glu Gln His Glu Cys His Phe Pro Asn Lys Ala Met Pro Ser Ala
    50                  55                  60

Gly Thr Leu Pro Trp Val Gln Gly Ile Ile Cys Asn Ala Asn Asn Pro
65                  70                  75                  80

Cys Phe Arg Tyr Pro Thr Pro Gly Glu Ala Pro Gly Val Val Gly Asn
                85                  90                  95

Phe Asn Lys Ser Ile Val Ala Arg Leu Phe Ser Asp Ala Arg Arg Leu
            100                 105                 110

Leu Leu Tyr Ser Gln Lys Asp Thr Ser Met Lys Asp Met Arg Lys Val
        115                 120                 125

Leu Arg Thr Leu Gln Gln Ile Lys Lys Ser Ser Ser Asn Leu Lys Leu
    130                 135                 140

Gln Asp Phe Leu Val Asp Asn Glu Thr Phe Ser Gly Phe Leu Tyr His
145                 150                 155                 160

Asn Leu Ser Leu Pro Lys Ser Thr Val Asp Lys Met Leu Arg Ala Asp
                165                 170                 175

Val Ile Leu His Lys Val Phe Leu Gln Gly Tyr Gln Leu His Leu Thr
            180                 185                 190

Ser Leu Cys Asn Gly Ser Lys Ser Glu Glu Met Ile Gln Leu Gly Asp
        195                 200                 205

Gln Glu Val Ser Glu Leu Cys Gly Leu Pro Arg Glu Lys Leu Ala Ala
    210                 215                 220

Ala Glu Arg Val Leu Arg Ser Asn Met Asp Ile Leu Lys Pro Ile Leu
225                 230                 235                 240

Arg Thr Leu Asn Ser Thr Ser Pro Phe Pro Ser Lys Glu Leu Ala Glu
                245                 250                 255

Ala Thr Lys Thr Leu Leu His Ser Leu Gly Thr Leu Ala Gln Glu Leu

            260                 265                 270

Phe Ser Met Arg Ser Trp Ser Asp Met Arg Gln Glu Val Met Phe Leu
        275                 280                 285

Thr Asn Val Asn Ser Ser Ser Ser Ser Thr Gln Ile Tyr Gln Ala Val
    290                 295                 300

Ser Arg Ile Val Cys Gly His Pro Glu Gly Gly Gly Leu Lys Ile Lys
305                 310                 315                 320

Ser Leu Asn Trp Tyr Glu Asp Asn Asn Tyr Lys Ala Leu Phe Gly Gly
                325                 330                 335

Asn Gly Thr Glu Glu Asp Ala Glu Thr Phe Tyr Asp Asn Ser Thr Thr
            340                 345                 350

Pro Tyr Cys Asn Asp Leu Met Lys Asn Leu Glu Ser Ser Pro Leu Ser
        355                 360                 365

Arg Ile Ile Trp Lys Ala Leu Lys Pro Leu Leu Val Gly Lys Ile Leu
    370                 375                 380

Tyr Thr Pro Asp Thr Pro Ala Thr Arg Gln Val Met Ala Glu Val Asn
385                 390                 395                 400

Lys Thr Phe Gln Glu Leu Ala Val Phe His Asp Leu Glu Gly Met Trp
                405                 410                 415

Glu Glu Leu Ser Pro Lys Ile Trp Thr Phe Met Glu Asn Ser Gln Glu
            420                 425                 430

Met Asp Leu Val Arg Met Leu Leu Asp Ser Arg Asp Asn Asp His Phe
        435                 440                 445

Trp Glu Gln Gln Leu Asp Gly Leu Asp Trp Thr Ala Gln Asp Ile Val
    450                 455                 460

Ala Phe Leu Ala Lys His Pro Glu Asp Val Gln Ser Ser Asn Gly Ser
465                 470                 475                 480

Val Tyr Thr Trp Arg Glu Ala Phe Asn Glu Thr Asn Gln Ala Ile Arg
                485                 490                 495

Thr Ile Ser Arg Phe Met Glu Cys Val Asn Leu Asn Lys Leu Glu Pro
            500                 505                 510

Ile Ala Thr Glu Val Trp Leu Ile Asn Lys Ser Met Glu Leu Leu Asp
        515                 520                 525

Glu Arg Lys Phe Trp Ala Gly Ile Val Phe Thr Gly Ile Thr Pro Gly
    530                 535                 540

Ser Ile Glu Leu Pro His His Val Lys Tyr Lys Ile Arg Met Asp Ile
545                 550                 555                 560

Asp Asn Val Glu Arg Thr Asn Lys Ile Lys Asp Gly Tyr Trp Asp Pro
                565                 570                 575

Gly Pro Arg Ala Asp Pro Phe Glu Asp Met Arg Tyr Val Trp Gly Gly
            580                 585                 590

Phe Ala Tyr Leu Gln Asp Val Val Glu Gln Ala Ile Ile Arg Val Leu
        595                 600                 605

Thr Gly Thr Glu Lys Lys Thr Gly Val Tyr Met Gln Gln Met Pro Tyr
    610                 615                 620

Pro Cys Tyr Val Asp Asp Ile Phe Leu Arg Val Met Ser Arg Ser Met
625                 630                 635                 640

Pro Leu Phe Met Thr Leu Ala Trp Ile Tyr Ser Val Ala Val Ile Ile
                645                 650                 655

Lys Gly Ile Val Tyr Glu Lys Glu Ala Arg Leu Lys Glu Thr Met Arg
            660                 665                 670

Ile Met Gly Leu Asp Asn Ser Ile Leu Trp Phe Ser Trp Phe Ile Ser
        675                 680                 685

Ser Leu Ile Pro Leu Leu Val Ser Ala Gly Leu Leu Val Val Ile Leu
    690                 695                 700

Lys Leu Gly Asn Leu Leu Pro Tyr Ser Asp Pro Ser Val Val Phe Val
705                 710                 715                 720

Phe Leu Ser Val Phe Ala Val Val Thr Ile Leu Gln Cys Phe Leu Ile
                725                 730                 735

Ser Thr Leu Phe Ser Arg Ala Asn Leu Ala Ala Ala Cys Gly Gly Ile
            740                 745                 750

Ile Tyr Phe Thr Leu Tyr Leu Pro Tyr Val Leu Cys Val Ala Trp Gln
        755                 760                 765

Asp Tyr Val Gly Phe Thr Leu Lys Ile Phe Ala Ser Leu Leu Ser Pro
    770                 775                 780

Val Ala Phe Gly Phe Gly Cys Glu Tyr Phe Ala Leu Phe Glu Glu Gln
785                 790                 795                 800

Gly Ile Gly Val Gln Trp Asp Asn Leu Phe Glu Ser Pro Val Glu Glu
                805                 810                 815

Asp Gly Phe Asn Leu Thr Thr Ser Val Ser Met Met Leu Phe Asp Thr
            820                 825                 830

Phe Leu Tyr Gly Val Met Thr Trp Tyr Ile Glu Ala Val Phe Pro Gly
        835                 840                 845

Gln Tyr Gly Ile Pro Arg Pro Trp Tyr Phe Pro Cys Thr Lys Ser Tyr
    850                 855                 860

Trp Phe Gly Glu Glu Ser Asp Glu Lys Ser His Pro Gly Ser Asn Gln
865                 870                 875                 880

Lys Arg Ile Ser Glu Ile Cys Met Glu Glu Glu Pro Thr His Leu Lys
                885                 890                 895

Leu Gly Val Ser Ile Gln Asn Leu Val Lys Val Tyr Arg Asp Gly Met
            900                 905                 910

Lys Val Ala Val Asp Gly Leu Ala Leu Asn Phe Tyr Glu Gly Gln Ile
        915                 920                 925

Thr Ser Phe Leu Gly His Asn Gly Ala Gly Lys Thr Thr Thr Met Ser
    930                 935                 940

Ile Leu Thr Gly Leu Phe Pro Pro Thr Ser Gly Thr Ala Tyr Ile Leu
945                 950                 955                 960

Gly Lys Asp Ile Arg Ser Glu Met Ser Thr Ile Arg Gln Asn Leu Gly
                965                 970                 975

Val Cys Pro Gln His Asn Val Leu Phe Asp Met Leu Thr Val Glu Glu
            980                 985                 990

His Ile Trp Phe Tyr Ala Arg Leu Lys Gly Leu Ser Glu Lys His Val
        995                 1000                1005

Lys Ala Glu Met Glu Gln Met Ala Leu Asp Val Gly Leu Pro Ser Ser
   1010                1015                1020

Lys Leu Lys Ser Lys Thr Ser Gln Leu Ser Gly Gly Met Gln Arg Lys
1025                1030                1035                1040

Leu Ser Val Ala Leu Ala Phe Val Gly Gly Ser Lys Val Val Ile Leu
                1045                1050                1055

Asp Glu Pro Thr Ala Gly Val Asp Pro Tyr Ser Arg Arg Gly Ile Trp
            1060                1065                1070

Glu Leu Leu Leu Lys Tyr Arg Gln Gly Arg Thr Ile Ile Leu Ser Thr
        1075                1080                1085

His His Met Asp Glu Ala Asp Val Leu Gly Asp Arg Ile Ala Ile Ile
   1090                1095                1100

Ser His Gly Lys Leu Cys Cys Val Gly Ser Pro Val Ser Glu Glu Pro
1105                1110                1115                1120

Ala Gly Asn Arg Leu Leu Pro Asp Leu Gly Gln Glu Arg Cys Gly Ile
                1125                1130                1135

Leu Pro Gln Phe Leu Gln Lys Gln
            1140


<210> 98
<211> 2261
<212> PRT
<213> Homo sapiens

<400> 98

Met Ala Cys Trp Pro Gln Leu Arg Leu Leu Leu Trp Lys Asn Leu Thr
1               5                   10                  15

Phe Arg Arg Arg Gln Thr Cys Gln Leu Leu Leu Glu Val Ala Trp Pro
            20                  25                  30

Leu Phe Ile Phe Leu Ile Leu Ile Ser Val Arg Leu Ser Tyr Pro Pro
        35                  40                  45

Tyr Glu Gln His Glu Cys His Phe Pro Asn Lys Ala Met Pro Ser Ala
    50                  55                  60

Gly Thr Leu Pro Trp Val Gln Gly Ile Ile Cys Asn Ala Asn Asn Pro
65                  70                  75                  80

Cys Phe Arg Tyr Pro Thr Pro Gly Glu Ala Pro Gly Val Val Gly Asn
                85                  90                  95

Phe Asn Lys Ser Ile Val Ala Arg Leu Phe Ser Asp Ala Arg Arg Leu
            100                 105                 110

Leu Leu Tyr Ser Gln Lys Asp Thr Ser Met Lys Asp Met Arg Lys Val
        115                 120                 125

Leu Arg Thr Leu Gln Gln Ile Lys Lys Ser Ser Ser Asn Leu Lys Leu
    130                 135                 140

Gln Asp Phe Leu Val Asp Asn Glu Thr Phe Ser Gly Phe Leu Tyr His
145                 150                 155                 160

Asn Leu Ser Leu Pro Lys Ser Thr Val Asp Lys Met Leu Arg Ala Asp
                165                 170                 175

Val Ile Leu His Lys Val Phe Leu Gln Gly Tyr Gln Leu His Leu Thr
            180                 185                 190

Ser Leu Cys Asn Gly Ser Lys Ser Glu Glu Met Ile Gln Leu Gly Asp
        195                 200                 205

Gln Glu Val Ser Glu Leu Cys Gly Leu Pro Arg Glu Lys Leu Ala Ala
    210                 215                 220

Ala Glu Arg Val Leu Arg Ser Asn Met Asp Ile Leu Lys Pro Ile Leu
225                 230                 235                 240

Arg Thr Leu Asn Ser Thr Ser Pro Phe Pro Ser Lys Glu Leu Ala Glu
                245                 250                 255

Ala Thr Lys Thr Leu Leu His Ser Leu Gly Thr Leu Ala Gln Glu Leu
            260                 265                 270

Phe Ser Met Arg Ser Trp Ser Asp Met Arg Gln Glu Val Met Phe Leu
        275                 280                 285

Thr Asn Val Asn Ser Ser Ser Ser Ser Thr Gln Ile Tyr Gln Ala Val
    290                 295                 300

Ser Arg Ile Val Cys Gly His Pro Glu Gly Gly Gly Leu Lys Ile Lys

305                 310                 315                 320

Ser Leu Asn Trp Tyr Glu Asp Asn Asn Tyr Lys Ala Leu Phe Gly Gly
                325                 330                 335

Asn Gly Thr Glu Glu Asp Ala Glu Thr Phe Tyr Asp Asn Ser Thr Thr
            340                 345                 350

Pro Tyr Cys Asn Asp Leu Met Lys Asn Leu Glu Ser Ser Pro Leu Ser
        355                 360                 365

Arg Ile Ile Trp Lys Ala Leu Lys Pro Leu Leu Val Gly Lys Ile Leu
    370                 375                 380

Tyr Thr Pro Asp Thr Pro Ala Thr Arg Gln Val Met Ala Glu Val Asn
385                 390                 395                 400

Lys Thr Phe Gln Glu Leu Ala Val Phe His Asp Leu Glu Gly Met Trp
                405                 410                 415

Glu Glu Leu Ser Pro Lys Ile Trp Thr Phe Met Glu Asn Ser Gln Glu
            420                 425                 430

Met Asp Leu Val Arg Met Leu Leu Asp Ser Arg Asp Asn Asp His Phe
        435                 440                 445

Trp Glu Gln Gln Leu Asp Gly Leu Asp Trp Thr Ala Gln Asp Ile Val
    450                 455                 460

Ala Phe Leu Ala Lys His Pro Glu Asp Val Gln Ser Ser Asn Gly Ser
465                 470                 475                 480

Val Tyr Thr Trp Arg Glu Ala Phe Asn Glu Thr Asn Gln Ala Ile Arg
                485                 490                 495

Thr Ile Ser Arg Phe Met Glu Cys Val Asn Leu Asn Lys Leu Glu Pro
            500                 505                 510

Ile Ala Thr Glu Val Trp Leu Ile Asn Lys Ser Met Glu Leu Leu Asp
        515                 520                 525

Glu Arg Lys Phe Trp Ala Gly Ile Val Phe Thr Gly Ile Thr Pro Gly
    530                 535                 540

Ser Ile Glu Leu Pro His His Val Lys Tyr Lys Ile Arg Met Asp Ile
545                 550                 555                 560

Asp Asn Val Glu Arg Thr Asn Lys Ile Lys Asp Gly Tyr Trp Asp Pro
                565                 570                 575

Gly Pro Arg Ala Asp Pro Phe Glu Asp Met Arg Tyr Val Trp Gly Gly
            580                 585                 590

Phe Ala Tyr Leu Gln Asp Val Val Glu Gln Ala Ile Ile Arg Val Leu
        595                 600                 605

Thr Gly Thr Glu Lys Lys Thr Gly Val Tyr Met Gln Gln Met Pro Tyr
    610                 615                 620

Pro Cys Tyr Val Asp Asp Ile Phe Leu Arg Val Met Ser Arg Ser Met
625                 630                 635                 640

Pro Leu Phe Met Thr Leu Ala Trp Ile Tyr Ser Val Ala Val Ile Ile
                645                 650                 655

Lys Gly Ile Val Tyr Glu Lys Glu Ala Arg Leu Lys Glu Thr Met Arg
            660                 665                 670

Ile Met Gly Leu Asp Asn Ser Ile Leu Trp Phe Ser Trp Phe Ile Ser
        675                 680                 685

Ser Leu Ile Pro Leu Leu Val Ser Ala Gly Leu Leu Val Val Ile Leu
    690                 695                 700

Lys Leu Gly Asn Leu Leu Pro Tyr Ser Asp Pro Ser Val Val Phe Val
705                 710                 715                 720

Phe Leu Ser Val Phe Ala Val Val Thr Ile Leu Gln Cys Phe Leu Ile
                725                 730                 735

Ser Thr Leu Phe Ser Arg Ala Asn Leu Ala Ala Ala Cys Gly Gly Ile
            740                 745                 750

Ile Tyr Phe Thr Leu Tyr Leu Pro Tyr Val Leu Cys Val Ala Trp Gln
        755                 760                 765

Asp Tyr Val Gly Phe Thr Leu Lys Ile Phe Ala Ser Leu Leu Ser Pro
    770                 775                 780

Val Ala Phe Gly Phe Gly Cys Glu Tyr Phe Ala Leu Phe Glu Glu Gln
785                 790                 795                 800

Gly Ile Gly Val Gln Trp Asp Asn Leu Phe Glu Ser Pro Val Glu Glu
                805                 810                 815

Asp Gly Phe Asn Leu Thr Thr Ser Val Ser Met Met Leu Phe Asp Thr
            820                 825                 830

Phe Leu Tyr Gly Val Met Thr Trp Tyr Ile Glu Ala Val Phe Pro Gly
        835                 840                 845

Gln Tyr Gly Ile Pro Arg Pro Trp Tyr Phe Pro Cys Thr Lys Ser Tyr
    850                 855                 860

Trp Phe Gly Glu Glu Ser Asp Glu Lys Ser His Pro Gly Ser Asn Gln
865                 870                 875                 880

Lys Arg Ile Ser Glu Ile Cys Met Glu Glu Glu Pro Thr His Leu Lys
                885                 890                 895

Leu Gly Val Ser Ile Gln Asn Leu Val Lys Val Tyr Arg Asp Gly Met
            900                 905                 910

Lys Val Ala Val Asp Gly Leu Ala Leu Asn Phe Tyr Glu Gly Gln Ile
        915                 920                 925

Thr Ser Phe Leu Gly His Asn Gly Ala Gly Lys Thr Thr Thr Met Ser
    930                 935                 940

Ile Leu Thr Gly Leu Phe Pro Pro Thr Ser Gly Thr Ala Tyr Ile Leu
945                 950                 955                 960

Gly Lys Asp Ile Arg Ser Glu Met Ser Thr Ile Arg Gln Asn Leu Gly
                965                 970                 975

Val Cys Pro Gln His Asn Val Leu Phe Asp Met Leu Thr Val Glu Glu
            980                 985                 990

His Ile Trp Phe Tyr Ala Arg Leu Lys Gly Leu Ser Glu Lys His Val
        995                 1000                1005

Lys Ala Glu Met Glu Gln Met Ala Leu Asp Val Gly Leu Pro Ser Ser
   1010                1015                1020

Lys Leu Lys Ser Lys Thr Ser Gln Leu Ser Gly Gly Met Gln Arg Lys
1025                1030                1035                1040

Leu Ser Val Ala Leu Ala Phe Val Gly Gly Ser Lys Val Val Ile Leu
                1045                1050                1055

Asp Glu Pro Thr Ala Gly Val Asp Pro Tyr Ser Arg Arg Gly Ile Trp
            1060                1065                1070

Glu Leu Leu Leu Lys Tyr Arg Gln Gly Arg Thr Ile Ile Leu Ser Thr
        1075                1080                1085

His His Met Asp Glu Ala Asp Val Leu Gly Asp Arg Ile Ala Ile Ile
   1090                1095                1100

Ser His Gly Lys Leu Cys Cys Val Gly Ser Ser Leu Phe Leu Lys Asn
1105                1110                1115                1120

Gln Leu Gly Thr Gly Tyr Tyr Leu Thr Leu Val Lys Lys Asp Val Glu
                1125                1130                1135

Ser Ser Leu Ser Ser Cys Arg Asn Ser Ser Ser Thr Val Ser Tyr Leu
            1140                1145                1150

Lys Lys Glu Asp Ser Val Ser Gln Ser Ser Ser Asp Ala Gly Leu Gly
        1155                1160                1165

Ser Asp His Glu Ser Asp Thr Leu Thr Ile Asp Val Ser Ala Ile Ser
   1170                1175                1180

Asn Leu Ile Arg Lys His Val Ser Glu Ala Arg Leu Val Glu Asp Ile
1185                1190                1195                1200

Gly His Glu Leu Thr Tyr Val Leu Pro Tyr Glu Ala Ala Lys Glu Gly
                1205                1210                1215

Ala Phe Val Glu Leu Phe His Glu Ile Asp Asp Arg Leu Ser Asp Leu
            1220                1225                1230

Gly Ile Ser Ser Tyr Gly Ile Ser Glu Thr Thr Leu Glu Glu Ile Phe

1235                1240                1245

Leu Lys Val Ala Glu Glu Ser Gly Val Asp Ala Glu Thr Ser Asp Gly
    1250                1255                1260

Thr Leu Pro Ala Arg Arg Asn Arg Arg Ala Phe Gly Asp Lys Gln Ser
1265                1270                1275                1280

Cys Leu Arg Pro Phe Thr Glu Asp Asp Ala Ala Asp Pro Asn Asp Ser
                1285                1290                1295

Asp Ile Asp Pro Glu Ser Arg Glu Thr Asp Leu Leu Ser Gly Met Asp
            1300                1305                1310

Gly Lys Gly Ser Tyr Gln Val Lys Gly Trp Lys Leu Thr Gln Gln Gln
        1315                1320                1325

Phe Val Ala Leu Leu Trp Lys Arg Leu Leu Ile Ala Arg Trp Ser Arg
    1330                1335                1340

Lys Gly Phe Phe Ala Gln Ile Val Leu Pro Ala Val Phe Val Cys Ile
1345                1350                1355                1360

Ala Leu Val Phe Ser Leu Ile Val Pro Pro Phe Gly Lys Tyr Pro Ser
                1365                1370                1375

Leu Glu Leu Gln Pro Trp Met Tyr Asn Glu Gln Tyr Thr Phe Val Ser
            1380                1385                1390

Asn Asp Ala Pro Glu Asp Thr Gly Thr Leu Glu Leu Leu Asn Ala Leu
        1395                1400                1405

Thr Lys Asp Pro Gly Phe Gly Thr Arg Cys Met Glu Gly Asn Pro Ile
    1410                1415                1420

Pro Asp Thr Pro Cys Gln Ala Gly Glu Glu Glu Trp Thr Thr Ala Pro
1425                1430                1435                1440

Val Pro Gln Thr Ile Met Asp Leu Phe Gln Asn Gly Asn Trp Thr Met
                1445                1450                1455

Gln Asn Pro Ser Pro Ala Cys Gln Cys Ser Ser Asp Lys Ile Lys Lys
            1460                1465                1470

Met Leu Pro Val Cys Pro Pro Gly Ala Gly Gly Leu Pro Pro Pro Gln
        1475                1480                1485

Arg Lys Gln Asn Thr Ala Asp Ile Leu Gln Asp Leu Thr Gly Arg Asn
    1490                1495                1500

Ile Ser Asp Tyr Leu Val Lys Thr Tyr Val Gln Ile Ile Ala Lys Ser
1505                1510                1515                1520

Leu Lys Asn Lys Ile Trp Val Asn Glu Phe Arg Tyr Gly Gly Phe Ser
                1525                1530                1535

Leu Gly Val Ser Asn Thr Gln Ala Leu Pro Pro Ser Gln Glu Val Asn
            1540                1545                1550

Asp Ala Thr Lys Gln Met Lys Lys His Leu Lys Leu Ala Lys Asp Ser
        1555                1560                1565

Ser Ala Asp Arg Phe Leu Asn Ser Leu Gly Arg Phe Met Thr Gly Leu
    1570                1575                1580

Asp Thr Arg Asn Asn Val Lys Val Trp Phe Asn Asn Lys Gly Trp His
1585                1590                1595                1600

Ala Ile Ser Ser Phe Leu Asn Val Ile Asn Asn Ala Ile Leu Arg Ala
                1605                1610                1615

Asn Leu Gln Lys Gly Glu Asn Pro Ser His Tyr Gly Ile Thr Ala Phe
            1620                1625                1630

Asn His Pro Leu Asn Leu Thr Lys Gln Gln Leu Ser Glu Val Ala Pro
        1635                1640                1645

Met Thr Thr Ser Val Asp Val Leu Val Ser Ile Cys Val Ile Phe Ala
    1650                1655                1660

Met Ser Phe Val Pro Ala Ser Phe Val Val Phe Leu Ile Gln Glu Arg
1665                1670                1675                1680

Val Ser Lys Ala Lys His Leu Gln Phe Ile Ser Gly Val Lys Pro Val
                1685                1690                1695

Ile Tyr Trp Leu Ser Asn Phe Val Trp Asp Met Cys Asn Tyr Val Val
            1700                1705                1710

Pro Ala Thr Leu Val Ile Ile Ile Phe Ile Cys Phe Gln Gln Lys Ser
        1715                1720                1725

Tyr Val Ser Ser Thr Asn Leu Pro Val Leu Ala Leu Leu Leu Leu Leu
    1730                1735                1740

Tyr Gly Trp Ser Ile Thr Pro Leu Met Tyr Pro Ala Ser Phe Val Phe
1745                1750                1755                1760

Lys Ile Pro Ser Thr Ala Tyr Val Val Leu Thr Ser Val Asn Leu Phe
                1765                1770                1775

Ile Gly Ile Asn Gly Ser Val Ala Thr Phe Val Leu Glu Leu Phe Thr
            1780                1785                1790

Asp Asn Lys Leu Asn Asn Ile Asn Asp Ile Leu Lys Ser Val Phe Leu
        1795                1800                1805

Ile Phe Pro His Phe Cys Leu Gly Arg Gly Leu Ile Asp Met Val Lys
    1810                1815                1820

Asn Gln Ala Met Ala Asp Ala Leu Glu Arg Phe Gly Glu Asn Arg Phe
1825                1830                1835                1840

Val Ser Pro Leu Ser Trp Asp Leu Val Gly Arg Asn Leu Phe Ala Met
                1845                1850                1855

Ala Val Glu Gly Val Val Phe Phe Leu Ile Thr Val Leu Ile Gln Tyr
            1860                1865                1870

Arg Phe Phe Ile Arg Pro Arg Pro Val Asn Ala Lys Leu Ser Pro Leu
        1875                1880                1885

Asn Asp Glu Asp Glu Asp Val Arg Arg Glu Arg Gln Arg Ile Leu Asp
    1890                1895                1900

Gly Gly Gly Gln Asn Asp Ile Leu Glu Ile Lys Glu Leu Thr Lys Ile
1905                1910                1915                1920

Tyr Arg Arg Lys Arg Lys Pro Ala Val Asp Arg Ile Cys Val Gly Ile
                1925                1930                1935

Pro Pro Gly Glu Cys Phe Gly Leu Leu Gly Val Asn Gly Ala Gly Lys
            1940                1945                1950

Ser Ser Thr Phe Lys Met Leu Thr Gly Asp Thr Thr Val Thr Arg Gly
        1955                1960                1965

Asp Ala Phe Leu Asn Arg Asn Ser Ile Leu Ser Asn Ile His Glu Val
    1970                1975                1980

His Gln Asn Met Gly Tyr Cys Pro Gln Phe Asp Ala Ile Thr Glu Leu
1985                1990                1995                2000

Leu Thr Gly Arg Glu His Val Glu Phe Phe Ala Leu Leu Arg Gly Val
                2005                2010                2015

Pro Glu Lys Glu Val Gly Lys Val Gly Glu Trp Ala Ile Arg Lys Leu
            2020                2025                2030

Gly Leu Val Lys Tyr Gly Glu Lys Tyr Ala Gly Asn Tyr Ser Gly Gly
        2035                2040                2045

Asn Lys Arg Lys Leu Ser Thr Ala Met Ala Leu Ile Gly Gly Pro Pro
    2050                2055                2060

Val Val Phe Leu Asp Glu Pro Thr Thr Gly Met Asp Pro Lys Ala Arg
2065                2070                2075                2080

Arg Phe Leu Trp Asn Cys Ala Leu Ser Val Val Lys Glu Gly Arg Ser
                2085                2090                2095

Val Val Leu Thr Ser His Ser Met Glu Glu Cys Glu Ala Leu Cys Thr
            2100                2105                2110

Arg Met Ala Ile Met Val Asn Gly Arg Phe Arg Cys Leu Gly Ser Val
        2115                2120                2125

Gln His Leu Lys Asn Arg Phe Gly Asp Gly Tyr Thr Ile Val Val Arg
    2130                2135                2140

Ile Ala Gly Ser Asn Pro Asp Leu Lys Pro Val Gln Asp Phe Phe Gly
2145                2150                2155                2160

Leu Ala Phe Pro Gly Ser Val Pro Lys Glu Lys His Arg Asn Met Leu

2165                2170                2175

Gln Tyr Gln Leu Pro Ser Ser Leu Ser Ser Leu Ala Arg Ile Phe Ser
        2180                2185                2190

Ile Leu Ser Gln Ser Lys Lys Arg Leu His Ile Glu Asp Tyr Ser Val
    2195                2200                2205

Ser Gln Thr Thr Leu Asp Gln Val Phe Val Asn Phe Ala Lys Asp Gln
  2210                2215                2220

Ser Asp Asp Asp His Leu Lys Asp Leu Ser Leu His Lys Asn Gln Thr
2225                2230                2235                2240

Val Val Asp Val Ala Val Leu Thr Ser Phe Leu Gln Asp Glu Lys Val
                2245                2250                2255

Lys Glu Ser Tyr Val
        2260

<210> 99
<211> 1525
<212> PRT
<213> Homo sapiens

<400> 99
Met Ala Cys Trp Pro Gln Leu Arg Leu Leu Leu Trp Lys Asn Leu Thr
1               5                   10                  15

Phe Arg Arg Arg Gln Thr Cys Gln Leu Leu Leu Glu Val Ala Trp Pro
            20                  25                  30

Leu Phe Ile Phe Leu Ile Leu Ile Ser Val Arg Leu Ser Tyr Pro Pro
        35                  40                  45

Tyr Glu Gln His Glu Cys His Phe Pro Asn Lys Ala Met Pro Ser Ala
    50                  55                  60

Gly Thr Leu Pro Trp Val Gln Gly Ile Ile Cys Asn Ala Asn Asn Pro
65                  70                  75                  80

Cys Phe Arg Tyr Pro Thr Pro Gly Glu Ala Pro Gly Val Val Gly Asn
                85                  90                  95

Phe Asn Lys Ser Ile Val Ala Arg Leu Phe Ser Asp Ala Arg Arg Leu
            100                 105                 110

Leu Leu Tyr Ser Gln Lys Asp Thr Ser Met Lys Asp Met Arg Lys Val
        115                 120                 125

Leu Arg Thr Leu Gln Gln Ile Lys Lys Ser Ser Ser Asn Leu Lys Leu
    130                 135                 140

Gln Asp Phe Leu Val Asp Asn Glu Thr Phe Ser Gly Phe Leu Tyr His
145                 150                 155                 160

Asn Leu Ser Leu Pro Lys Ser Thr Val Asp Lys Met Leu Arg Ala Asp

165                 170                 175

Val Ile Leu His Lys Val Phe Leu Gln Gly Tyr Gln Leu His Leu Thr
            180                 185                 190

Ser Leu Cys Asn Gly Ser Lys Ser Glu Glu Met Ile Gln Leu Gly Asp
        195                 200                 205

Gln Glu Val Ser Glu Leu Cys Gly Leu Pro Arg Glu Lys Leu Ala Ala
    210                 215                 220

Ala Glu Arg Val Leu Arg Ser Asn Met Asp Ile Leu Lys Pro Ile Leu
225                 230                 235                 240

Arg Thr Leu Asn Ser Thr Ser Pro Phe Pro Ser Lys Glu Leu Ala Glu
                245                 250                 255

Ala Thr Lys Thr Leu Leu His Ser Leu Gly Thr Leu Ala Gln Glu Leu
            260                 265                 270

Phe Ser Met Arg Ser Trp Ser Asp Met Arg Gln Glu Val Met Phe Leu
        275                 280                 285

Thr Asn Val Asn Ser Ser Ser Ser Ser Thr Gln Ile Tyr Gln Ala Val
    290                 295                 300

Ser Arg Ile Val Cys Gly His Pro Glu Gly Gly Gly Leu Lys Ile Lys
305                 310                 315                 320

Ser Leu Asn Trp Tyr Glu Asp Asn Asn Tyr Lys Ala Leu Phe Gly Gly
                325                 330                 335

Asn Gly Thr Glu Glu Asp Ala Glu Thr Phe Tyr Asp Asn Ser Thr Thr
            340                 345                 350

Pro Tyr Cys Asn Asp Leu Met Lys Asn Leu Glu Ser Ser Pro Leu Ser
        355                 360                 365

Arg Ile Ile Trp Lys Ala Leu Lys Pro Leu Leu Val Gly Lys Ile Leu
    370                 375                 380

Tyr Thr Pro Asp Thr Pro Ala Thr Arg Gln Val Met Ala Glu Val Asn
385                 390                 395                 400

Lys Thr Phe Gln Glu Leu Ala Val Phe His Asp Leu Glu Gly Met Trp
                405                 410                 415

Glu Glu Leu Ser Pro Lys Ile Trp Thr Phe Met Glu Asn Ser Gln Glu
            420                 425                 430

Met Asp Leu Val Arg Met Leu Leu Asp Ser Arg Asp Asn Asp His Phe
        435                 440                 445

Trp Glu Gln Gln Leu Asp Gly Leu Asp Trp Thr Ala Gln Asp Ile Val
    450                 455                 460

Ala Phe Leu Ala Lys His Pro Glu Asp Val Gln Ser Ser Asn Gly Ser
465                 470                 475                 480

Val Tyr Thr Trp Arg Glu Ala Phe Asn Glu Thr Asn Gln Ala Ile Arg
                485                 490                 495

Thr Ile Ser Arg Phe Met Glu Cys Val Asn Leu Asn Lys Leu Glu Pro
            500                 505                 510

Ile Ala Thr Glu Val Trp Leu Ile Asn Lys Ser Met Glu Leu Leu Asp
        515                 520                 525

Glu Arg Lys Phe Trp Ala Gly Ile Val Phe Thr Gly Ile Thr Pro Gly
    530                 535                 540

Ser Ile Glu Leu Pro His His Val Lys Tyr Lys Ile Arg Met Asp Ile
545                 550                 555                 560

Asp Asn Val Glu Arg Thr Asn Lys Ile Lys Asp Gly Tyr Trp Asp Pro
                565                 570                 575

Gly Pro Arg Ala Asp Pro Phe Glu Asp Met Arg Tyr Val Trp Gly Gly
            580                 585                 590

Phe Ala Tyr Leu Gln Asp Val Val Glu Gln Ala Ile Ile Arg Val Leu
        595                 600                 605

Thr Gly Thr Glu Lys Lys Thr Gly Val Tyr Met Gln Gln Met Pro Tyr
    610                 615                 620

Pro Cys Tyr Val Asp Asp Ile Phe Leu Arg Val Met Ser Arg Ser Met
625                 630                 635                 640

Pro Leu Phe Met Thr Leu Ala Trp Ile Tyr Ser Val Ala Val Ile Ile
                645                 650                 655

Lys Gly Ile Val Tyr Glu Lys Glu Ala Arg Leu Lys Glu Thr Met Arg
            660                 665                 670

Ile Met Gly Leu Asp Asn Ser Ile Leu Trp Phe Ser Trp Phe Ile Ser
        675                 680                 685

Ser Leu Ile Pro Leu Leu Val Ser Ala Gly Leu Leu Val Val Ile Leu
    690                 695                 700

Lys Leu Gly Asn Leu Leu Pro Tyr Ser Asp Pro Ser Val Val Phe Val
705                 710                 715                 720

Phe Leu Ser Val Phe Ala Val Val Thr Ile Leu Gln Cys Phe Leu Ile
                725                 730                 735

Ser Thr Leu Phe Ser Arg Ala Asn Leu Ala Ala Ala Cys Gly Gly Ile
            740                 745                 750

Ile Tyr Phe Thr Leu Tyr Leu Pro Tyr Val Leu Cys Val Ala Trp Gln
        755                 760                 765

Asp Tyr Val Gly Phe Thr Leu Lys Ile Phe Ala Ser Leu Leu Ser Pro
    770                 775                 780

Val Ala Phe Gly Phe Gly Cys Glu Tyr Phe Ala Leu Phe Glu Glu Gln
785                 790                 795                 800

Gly Ile Gly Val Gln Trp Asp Asn Leu Phe Glu Ser Pro Val Glu Glu
                805                 810                 815

Asp Gly Phe Asn Leu Thr Thr Ser Val Ser Met Met Leu Phe Asp Thr
            820                 825                 830

Phe Leu Tyr Gly Val Met Thr Trp Tyr Ile Glu Ala Val Phe Pro Gly
        835                 840                 845

Gln Tyr Gly Ile Pro Arg Pro Trp Tyr Phe Pro Cys Thr Lys Ser Tyr
    850                 855                 860

Trp Phe Gly Glu Glu Ser Asp Glu Lys Ser His Pro Gly Ser Asn Gln
865                 870                 875                 880

Lys Arg Ile Ser Glu Ile Cys Met Glu Glu Glu Pro Thr His Leu Lys
                885                 890                 895

Leu Gly Val Ser Ile Gln Asn Leu Val Lys Val Tyr Arg Asp Gly Met
            900                 905                 910

Lys Val Ala Val Asp Gly Leu Ala Leu Asn Phe Tyr Glu Gly Gln Ile
        915                 920                 925

Thr Ser Phe Leu Gly His Asn Gly Ala Gly Lys Thr Thr Thr Met Ser
    930                 935                 940

Ile Leu Thr Gly Leu Phe Pro Pro Thr Ser Gly Thr Ala Tyr Ile Leu
945                 950                 955                 960

Gly Lys Asp Ile Arg Ser Glu Met Ser Thr Ile Arg Gln Asn Leu Gly
                965                 970                 975

Val Cys Pro Gln His Asn Val Leu Phe Asp Met Leu Thr Val Glu Glu
            980                 985                 990

His Ile Trp Phe Tyr Ala Arg Leu Lys Gly Leu Ser Glu Lys His Val
        995                 1000                1005

Lys Ala Glu Met Glu Gln Met Ala Leu Asp Val Gly Leu Pro Ser Ser
    1010                1015                1020

Lys Leu Lys Ser Lys Thr Ser Gln Leu Ser Gly Gly Met Gln Arg Lys
1025                1030                1035                1040

Leu Ser Val Ala Leu Ala Phe Val Gly Gly Ser Lys Val Val Ile Leu
                1045                1050                1055

Asp Glu Pro Thr Ala Gly Val Asp Pro Tyr Ser Arg Arg Gly Ile Trp
            1060                1065                1070

Glu Leu Leu Leu Lys Tyr Arg Gln Gly Arg Thr Ile Ile Leu Ser Thr
        1075                1080                1085

His His Met Asp Glu Ala Asp Val Leu Gly Asp Arg Ile Ala Ile Ile

1090                1095                1100

Ser His Gly Lys Leu Cys Cys Val Gly Ser Ser Leu Phe Leu Lys Asn
1105                1110                1115                1120

Gln Leu Gly Thr Gly Tyr Tyr Leu Thr Leu Val Lys Lys Asp Val Glu
                1125                1130                1135

Ser Ser Leu Ser Ser Cys Arg Asn Ser Ser Ser Thr Val Ser Tyr Leu
            1140                1145                1150

Lys Lys Glu Asp Ser Val Ser Gln Ser Ser Ser Asp Ala Gly Leu Gly
        1155                1160                1165

Ser Asp His Glu Ser Asp Thr Leu Thr Ile Asp Val Ser Ala Ile Ser
    1170                1175                1180

Asn Leu Ile Arg Lys His Val Ser Glu Ala Arg Leu Val Glu Asp Ile
1185                1190                1195                1200

Gly His Glu Leu Thr Tyr Val Leu Pro Tyr Glu Ala Ala Lys Glu Gly
                1205                1210                1215

Ala Phe Val Glu Leu Phe His Glu Ile Asp Asp Arg Leu Ser Asp Leu
            1220                1225                1230

Gly Ile Ser Ser Tyr Gly Ile Ser Glu Thr Thr Leu Glu Glu Ile Phe
        1235                1240                1245

Leu Lys Val Ala Glu Glu Ser Gly Val Asp Ala Glu Thr Ser Asp Gly
    1250                1255                1260

Thr Leu Pro Ala Arg Arg Asn Arg Arg Ala Phe Gly Asp Lys Gln Ser
1265                1270                1275                1280

Cys Leu Arg Pro Phe Thr Glu Asp Asp Ala Ala Asp Pro Asn Asp Ser
                1285                1290                1295

Asp Ile Asp Pro Glu Ser Arg Glu Thr Asp Leu Leu Ser Gly Met Asp
            1300                1305                1310

Gly Lys Gly Ser Tyr Gln Val Lys Gly Trp Lys Leu Thr Gln Gln Gln
        1315                1320                1325

Phe Val Ala Leu Leu Trp Lys Arg Leu Leu Ile Ala Arg Arg Ser Arg
    1330                1335                1340

Lys Gly Phe Phe Ala Gln Ile Val Leu Pro Ala Val Phe Val Cys Ile
1345                1350                1355                1360

Ala Leu Val Phe Ser Leu Ile Val Pro Pro Phe Gly Lys Tyr Pro Ser
                1365                1370                1375

Leu Glu Leu Gln Pro Trp Met Tyr Asn Glu Gln Tyr Thr Phe Val Ser
            1380                1385                1390

Asn Asp Ala Pro Glu Asp Thr Gly Thr Leu Glu Leu Leu Asn Ala Leu
        1395                1400                1405

Thr Lys Asp Pro Gly Phe Gly Thr Arg Cys Met Glu Gly Asn Pro Ile
    1410                1415                1420

Pro Asp Thr Pro Cys Gln Ala Gly Glu Glu Glu Trp Thr Thr Ala Pro
1425                1430                1435                1440

Val Pro Gln Thr Ile Met Asp Leu Phe Gln Asn Gly Asn Trp Thr Met
                1445                1450                1455

Gln Asn Pro Ser Pro Ala Cys Gln Cys Ser Ser Asp Lys Ile Lys Lys
            1460                1465                1470

Met Leu Pro Val Cys Pro Pro Gly Ala Gly Gly Leu Pro Pro Pro Gln
        1475                1480                1485

Arg Lys Gln Asn Thr Ala Asp Ile Leu Gln Asp Leu Thr Gly Arg Asn
    1490                1495                1500

Ile Ser Asp Tyr Leu Val Lys Thr Tyr Val Gln Ile Ile Ala Lys Ser
1505                1510                1515                1520

Leu Lys Asn Lys Ile
                1525


<210> 100
<211> 2261
<212> PRT
<213> Homo sapiens

<400> 100
Met Ala Cys Trp Pro Gln Leu Arg Leu Leu Leu Trp Lys Asn Leu Thr
  1               5                  10                  15

Phe Arg Arg Arg Gln Thr Cys Gln Leu Leu Leu Glu Val Ala Trp Pro
             20                  25                  30

Leu Phe Ile Phe Leu Ile Leu Ile Ser Val Arg Leu Ser Tyr Pro Pro
         35                  40                  45

Tyr Glu Gln His Glu Cys His Phe Pro Asn Lys Ala Met Pro Ser Ala
     50                  55                  60

Gly Thr Leu Pro Trp Val Gln Gly Ile Ile Cys Asn Ala Asn Asn Pro
 65                  70                  75                  80

Cys Phe Arg Tyr Pro Thr Pro Gly Glu Ala Pro Gly Val Val Gly Asn
                 85                  90                  95

Phe Asn Lys Ser Ile Val Ala Arg Leu Phe Ser Asp Ala Arg Arg Leu
            100                 105                 110

Leu Leu Tyr Ser Gln Lys Asp Thr Ser Met Lys Asp Met Arg Lys Val
        115                 120                 125

Leu Arg Thr Leu Gln Gln Ile Lys Lys Ser Ser Ser Asn Leu Lys Leu
    130                 135                 140

Gln Asp Phe Leu Val Asp Asn Glu Thr Phe Ser Gly Phe Leu Tyr His
145                 150                 155                 160

Asn Leu Ser Leu Pro Lys Ser Thr Val Asp Lys Met Leu Arg Ala Asp
                165                 170                 175

Val Ile Leu His Lys Val Phe Leu Gln Gly Tyr Gln Leu His Leu Thr
            180                 185                 190

Ser Leu Cys Asn Gly Ser Lys Ser Glu Glu Met Ile Gln Leu Gly Asp
        195                 200                 205

Gln Glu Val Ser Glu Leu Cys Gly Leu Pro Arg Glu Lys Leu Ala Ala
    210                 215                 220

Ala Glu Arg Val Leu Arg Ser Asn Met Asp Ile Leu Lys Pro Ile Leu
225                 230                 235                 240

Arg Thr Leu Asn Ser Thr Ser Pro Phe Pro Ser Lys Glu Leu Ala Glu
                245                 250                 255

Ala Thr Lys Thr Leu Leu His Ser Leu Gly Thr Leu Ala Gln Glu Leu
            260                 265                 270

Phe Ser Met Arg Ser Trp Ser Asp Met Arg Gln Glu Val Met Phe Leu
        275                 280                 285

Thr Asn Val Asn Ser Ser Ser Ser Ser Thr Gln Ile Tyr Gln Ala Val
    290                 295                 300

Ser Arg Ile Val Cys Gly His Pro Glu Gly Gly Gly Leu Lys Ile Lys
305                 310                 315                 320

Ser Leu Asn Trp Tyr Glu Asp Asn Asn Tyr Lys Ala Leu Phe Gly Gly
                325                 330                 335

Asn Gly Thr Glu Glu Asp Ala Glu Thr Phe Tyr Asp Asn Ser Thr Thr
            340                 345                 350

Pro Tyr Cys Asn Asp Leu Met Lys Asn Leu Glu Ser Ser Pro Leu Ser
        355                 360                 365

Arg Ile Ile Trp Lys Ala Leu Lys Pro Leu Leu Val Gly Lys Ile Leu
    370                 375                 380

Tyr Thr Pro Asp Thr Pro Ala Thr Arg Gln Val Met Ala Glu Val Asn
385                 390                 395                 400

Lys Thr Phe Gln Glu Leu Ala Val Phe His Asp Leu Glu Gly Met Trp
                405                 410                 415

Glu Glu Leu Ser Pro Lys Ile Trp Thr Phe Met Glu Asn Ser Gln Glu
            420                 425                 430

Met Asp Leu Val Arg Met Leu Leu Asp Ser Arg Asp Asn Asp His Phe
        435                 440                 445

Trp Glu Gln Gln Leu Asp Gly Leu Asp Trp Thr Ala Gln Asp Ile Val
    450                 455                 460

Ala Phe Leu Ala Lys His Pro Glu Asp Val Gln Ser Ser Asn Gly Ser
465                 470                 475                 480

Val Tyr Thr Trp Arg Glu Ala Phe Asn Glu Thr Asn Gln Ala Ile Arg
                485                 490                 495

Thr Ile Ser Arg Phe Met Glu Cys Val Asn Leu Asn Lys Leu Glu Pro
            500                 505                 510

Ile Ala Thr Glu Val Trp Leu Ile Asn Lys Ser Met Glu Leu Leu Asp
        515                 520                 525

Glu Arg Lys Phe Trp Ala Gly Ile Val Phe Thr Gly Ile Thr Pro Gly
    530                 535                 540

Ser Ile Glu Leu Pro His His Val Lys Tyr Lys Ile Arg Met Asp Ile
545                 550                 555                 560

Asp Asn Val Glu Arg Thr Asn Lys Ile Lys Asp Gly Tyr Trp Asp Pro
                565                 570                 575

Gly Pro Arg Ala Asp Pro Phe Glu Asp Met Arg Tyr Val Trp Gly Gly
            580                 585                 590

Phe Ala Tyr Leu Gln Asp Val Val Glu Gln Ala Ile Ile Arg Val Leu
        595                 600                 605

Thr Gly Thr Glu Lys Lys Thr Gly Val Tyr Met Gln Gln Met Pro Tyr
    610                 615                 620

Pro Cys Tyr Val Asp Asp Ile Phe Leu Arg Val Met Ser Arg Ser Met
625                 630                 635                 640

Pro Leu Phe Met Thr Leu Ala Trp Ile Tyr Ser Val Ala Val Ile Ile
                645                 650                 655

Lys Gly Ile Val Tyr Glu Lys Glu Ala Arg Leu Lys Glu Thr Met Arg
            660                 665                 670

Ile Met Gly Leu Asp Asn Ser Ile Leu Trp Phe Ser Trp Phe Ile Ser
        675                 680                 685

Ser Leu Ile Pro Leu Leu Val Ser Ala Gly Leu Leu Val Val Ile Leu
    690                 695                 700

Lys Leu Gly Asn Leu Leu Pro Tyr Ser Asp Pro Ser Val Val Phe Val
705                 710                 715                 720

Phe Leu Ser Val Phe Ala Val Val Thr Ile Leu Gln Cys Phe Leu Ile
                725                 730                 735

Ser Thr Leu Phe Ser Arg Ala Asn Leu Ala Ala Ala Cys Gly Gly Ile
            740                 745                 750

Ile Tyr Phe Thr Leu Tyr Leu Pro Tyr Val Leu Cys Val Ala Trp Gln

755                 760                 765

Asp Tyr Val Gly Phe Thr Leu Lys Ile Phe Ala Ser Leu Leu Ser Pro
    770                 775                 780

Val Ala Phe Gly Phe Gly Cys Glu Tyr Phe Ala Leu Phe Glu Glu Gln
785                 790                 795                 800

Gly Ile Gly Val Gln Trp Asp Asn Leu Phe Glu Ser Pro Val Glu Glu
                805                 810                 815

Asp Gly Phe Asn Leu Thr Thr Ser Val Ser Met Met Leu Phe Asp Thr
            820                 825                 830

Phe Leu Tyr Gly Val Met Thr Trp Tyr Ile Glu Ala Val Phe Pro Gly
        835                 840                 845

Gln Tyr Gly Ile Pro Arg Pro Trp Tyr Phe Pro Cys Thr Lys Ser Tyr
    850                 855                 860

Trp Phe Gly Glu Glu Ser Asp Glu Lys Ser His Pro Gly Ser Asn Gln
865                 870                 875                 880

Lys Arg Ile Ser Glu Ile Cys Met Glu Glu Glu Pro Thr His Leu Lys
                885                 890                 895

Leu Gly Val Ser Ile Gln Asn Leu Val Lys Val Tyr Arg Asp Gly Met
            900                 905                 910

Lys Val Ala Val Asp Gly Leu Ala Leu Asn Phe Tyr Glu Gly Gln Ile
        915                 920                 925

Thr Ser Phe Leu Gly His Asn Gly Ala Gly Lys Thr Thr Thr Met Ser
    930                 935                 940

Ile Leu Thr Gly Leu Phe Pro Pro Thr Ser Gly Thr Ala Tyr Ile Leu
945                 950                 955                 960

Gly Lys Asp Ile Arg Ser Glu Met Ser Thr Ile Arg Gln Asn Leu Gly
                965                 970                 975

Val Cys Pro Gln His Asn Val Leu Phe Asp Met Leu Thr Val Glu Glu
            980                 985                 990

His Ile Trp Phe Tyr Ala Arg Leu Lys Gly Leu Ser Glu Lys His Val
        995                 1000                1005

Lys Ala Glu Met Glu Gln Met Ala Leu Asp Val Gly Leu Pro Ser Ser
    1010                1015                1020

Lys Leu Lys Ser Lys Thr Ser Gln Leu Ser Gly Gly Met Gln Arg Lys
1025                1030                1035                1040

Leu Ser Val Ala Leu Ala Phe Val Gly Gly Ser Lys Val Val Ile Leu
                1045                1050                1055

Asp Glu Pro Thr Ala Gly Val Asp Pro Tyr Ser Arg Arg Gly Ile Trp
            1060                1065                1070

Glu Leu Leu Leu Lys Tyr Arg Gln Gly Arg Thr Ile Ile Leu Ser Thr
        1075                1080                1085

His His Met Asp Glu Ala Asp Val Leu Gly Asp Arg Ile Ala Ile Ile
    1090                1095                1100

Ser His Gly Lys Leu Cys Cys Val Gly Ser Ser Leu Phe Leu Lys Asn
1105                1110                1115                1120

Gln Leu Gly Thr Gly Tyr Tyr Leu Thr Leu Val Lys Lys Asp Val Glu
                1125                1130                1135

Ser Ser Leu Ser Ser Cys Arg Asn Ser Ser Ser Thr Val Ser Tyr Leu
            1140                1145                1150

Lys Lys Glu Asp Ser Val Ser Gln Ser Ser Ser Asp Ala Gly Leu Gly
        1155                1160                1165

Ser Asp His Glu Ser Asp Thr Leu Thr Ile Asp Val Ser Ala Ile Ser
    1170                1175                1180

Asn Leu Ile Arg Lys His Val Ser Glu Ala Arg Leu Val Glu Asp Ile
1185                1190                1195                1200

Gly His Glu Leu Thr Tyr Val Leu Pro Tyr Glu Ala Ala Lys Glu Gly
                1205                1210                1215

Ala Phe Val Glu Leu Phe His Glu Ile Asp Asp Arg Leu Ser Asp Leu
            1220                1225                1230

Gly Ile Ser Ser Tyr Gly Ile Ser Glu Thr Thr Leu Glu Glu Ile Phe
        1235                1240                1245

Leu Lys Val Ala Glu Glu Ser Gly Val Asp Ala Glu Thr Ser Asp Gly
    1250                1255                1260

Thr Leu Pro Ala Arg Arg Asn Arg Arg Ala Phe Gly Asp Lys Gln Ser
1265                1270                1275                1280

Cys Leu Arg Pro Phe Thr Glu Asp Asp Ala Ala Asp Pro Asn Asp Ser
                1285                1290                1295

Asp Ile Asp Pro Glu Ser Arg Glu Thr Asp Leu Leu Ser Gly Met Asp
            1300                1305                1310

Gly Lys Gly Ser Tyr Gln Val Lys Gly Trp Lys Leu Thr Gln Gln Gln
        1315                1320                1325

Phe Val Ala Leu Leu Trp Lys Arg Leu Leu Ile Ala Arg Arg Ser Arg
    1330                1335                1340

Lys Gly Phe Phe Ala Gln Ile Val Leu Pro Ala Val Phe Val Cys Ile
1345                1350                1355                1360

Ala Leu Val Phe Ser Leu Ile Val Pro Pro Phe Gly Lys Tyr Pro Ser
                1365                1370                1375

Leu Glu Leu Gln Pro Trp Met Tyr Asn Glu Gln Tyr Thr Phe Val Ser
            1380                1385                1390

Asn Asp Ala Pro Glu Asp Thr Gly Thr Leu Glu Leu Leu Asn Ala Leu
        1395                1400                1405

Thr Lys Asp Pro Gly Phe Gly Thr Arg Cys Met Glu Gly Asn Pro Ile
    1410                1415                1420

Pro Asp Thr Pro Cys Gln Ala Gly Glu Glu Glu Trp Thr Thr Ala Pro
1425                1430                1435                1440

Val Pro Gln Thr Ile Met Asp Leu Phe Gln Asn Gly Asn Trp Thr Met
                1445                1450                1455

Gln Asn Pro Ser Pro Ala Cys Gln Cys Ser Ser Asp Lys Ile Lys Lys
            1460                1465                1470

Met Leu Pro Val Cys Pro Pro Gly Ala Gly Gly Leu Pro Pro Pro Gln
        1475                1480                1485

Arg Lys Gln Asn Thr Ala Asp Ile Leu Gln Asp Leu Thr Gly Arg Asn
    1490                1495                1500

Ile Ser Asp Tyr Leu Val Lys Thr Tyr Val Gln Ile Ile Ala Lys Ser
1505                1510                1515                1520

Leu Lys Asn Lys Ile Trp Val Asn Glu Phe Arg Tyr Gly Gly Phe Ser
                1525                1530                1535

Leu Gly Val Ser Asn Thr Gln Ala Leu Pro Pro Ser Gln Glu Val Asn
            1540                1545                1550

Asp Ala Thr Lys Gln Met Lys Lys His Leu Lys Leu Ala Lys Asp Ser
        1555                1560                1565

Ser Ala Asp Arg Phe Leu Asn Ser Leu Gly Arg Phe Met Thr Gly Leu
    1570                1575                1580

Asp Thr Lys Asn Asn Val Lys Val Trp Phe Asn Asn Lys Gly Trp His
1585                1590                1595                1600

Ala Ile Ser Ser Phe Leu Asn Val Ile Asn Asn Ala Ile Leu Arg Ala
                1605                1610                1615

Asn Leu Gln Lys Gly Glu Asn Pro Ser His Tyr Gly Ile Thr Ala Phe
            1620                1625                1630

Asn His Pro Leu Asn Leu Thr Lys Gln Gln Leu Ser Glu Val Ala Pro
        1635                1640                1645

Met Thr Thr Ser Val Asp Val Leu Val Ser Ile Cys Val Ile Phe Ala
    1650                1655                1660

Met Ser Phe Val Pro Ala Ser Phe Val Val Phe Leu Ile Gln Glu Arg
1665                1670                1675                1680

Val Ser Lys Ala Lys His Leu Gln Phe Ile Ser Gly Val Lys Pro Val

1685                1690                1695

Ile Tyr Trp Leu Ser Asn Phe Val Trp Asp Met Cys Asn Tyr Val Val
        1700                1705                1710

Pro Ala Thr Leu Val Ile Ile Ile Phe Ile Cys Phe Gln Gln Lys Ser
    1715                1720                1725

Tyr Val Ser Ser Thr Asn Leu Pro Val Leu Ala Leu Leu Leu Leu Leu
  1730                1735                1740

Tyr Gly Trp Ser Ile Thr Pro Leu Met Tyr Pro Ala Ser Phe Val Phe
1745                1750                1755                1760

Lys Ile Pro Ser Thr Ala Tyr Val Val Leu Thr Ser Val Asn Leu Phe
                1765                1770                1775

Ile Gly Ile Asn Gly Ser Val Ala Thr Phe Val Leu Glu Leu Phe Thr
        1780                1785                1790

Asp Asn Lys Leu Asn Asn Ile Asn Asp Ile Leu Lys Ser Val Phe Leu
    1795                1800                1805

Ile Phe Pro His Phe Cys Leu Gly Arg Gly Leu Ile Asp Met Val Lys
  1810                1815                1820

Asn Gln Ala Met Ala Asp Ala Leu Glu Arg Phe Gly Glu Asn Arg Phe
1825                1830                1835                1840

Val Ser Pro Leu Ser Trp Asp Leu Val Gly Arg Asn Leu Phe Ala Met
                1845                1850                1855

Ala Val Glu Gly Val Val Phe Phe Leu Ile Thr Val Leu Ile Gln Tyr
        1860                1865                1870

Arg Phe Phe Ile Arg Pro Arg Pro Val Asn Ala Lys Leu Ser Pro Leu
    1875                1880                1885

Asn Asp Glu Asp Glu Asp Val Arg Arg Glu Arg Gln Arg Ile Leu Asp
  1890                1895                1900

Gly Gly Gly Gln Asn Asp Ile Leu Glu Ile Lys Glu Leu Thr Lys Ile
1905                1910                1915                1920

Tyr Arg Arg Lys Arg Lys Pro Ala Val Asp Arg Ile Cys Val Gly Ile
                1925                1930                1935

Pro Pro Gly Glu Cys Phe Gly Leu Leu Gly Val Asn Gly Ala Gly Lys
        1940                1945                1950

Ser Ser Thr Phe Lys Met Leu Thr Gly Asp Thr Thr Val Thr Arg Gly
    1955                1960                1965

Asp Ala Phe Leu Asn Arg Asn Ser Ile Leu Ser Asn Ile His Glu Val
  1970                1975                1980

His Gln Asn Met Gly Tyr Cys Pro Gln Phe Asp Ala Ile Thr Glu Leu
1985                1990                1995                2000

Leu Thr Gly Arg Glu His Val Glu Phe Phe Ala Leu Leu Arg Gly Val
                2005                2010                2015

Pro Glu Lys Glu Val Gly Lys Val Gly Glu Trp Ala Ile Arg Lys Leu
            2020                2025                2030

Gly Leu Val Lys Tyr Gly Glu Lys Tyr Ala Gly Asn Tyr Ser Gly Gly
        2035                2040                2045

Asn Lys Arg Lys Leu Ser Thr Ala Met Ala Leu Ile Gly Gly Pro Pro
    2050                2055                2060

Val Val Phe Leu Asp Glu Pro Thr Thr Gly Met Asp Pro Lys Ala Arg
2065                2070                2075                2080

Arg Phe Leu Trp Asn Cys Ala Leu Ser Val Val Lys Glu Gly Arg Ser
                2085                2090                2095

Val Val Leu Thr Ser His Ser Met Glu Glu Cys Glu Ala Leu Cys Thr
            2100                2105                2110

Arg Met Ala Ile Met Val Asn Gly Arg Phe Arg Cys Leu Gly Ser Val
        2115                2120                2125

Gln His Leu Lys Asn Arg Phe Gly Asp Gly Tyr Thr Ile Val Val Arg
    2130                2135                2140

Ile Ala Gly Ser Asn Pro Asp Leu Lys Pro Val Gln Asp Phe Phe Gly
2145                2150                2155                2160

Leu Ala Phe Pro Gly Ser Val Pro Lys Glu Lys His Arg Asn Met Leu
                2165                2170                2175

Gln Tyr Gln Leu Pro Ser Ser Leu Ser Ser Leu Ala Arg Ile Phe Ser
            2180                2185                2190

Ile Leu Ser Gln Ser Lys Lys Arg Leu His Ile Glu Asp Tyr Ser Val
        2195                2200                2205

Ser Gln Thr Thr Leu Asp Gln Val Phe Val Asn Phe Ala Lys Asp Gln
    2210                2215                2220

Ser Asp Asp Asp His Leu Lys Asp Leu Ser Leu His Lys Asn Gln Thr
2225                2230                2235                2240

Val Val Asp Val Ala Val Leu Thr Ser Phe Leu Gln Asp Glu Lys Val
                2245                2250                2255

Lys Glu Ser Tyr Val
            2260

<210> 101
<211> 2261
<212> PRT
<213> Homo sapiens

<400> 101
Met Ala Cys Trp Pro Gln Leu Arg Leu Leu Leu Trp Lys Asn Leu Thr
1               5                   10                  15

Phe Arg Arg Arg Gln Thr Cys Gln Leu Leu Leu Glu Val Ala Trp Pro
            20                  25                  30

Leu Phe Ile Phe Leu Ile Leu Ile Ser Val Arg Leu Ser Tyr Pro Pro
        35                  40                  45

Tyr Glu Gln His Glu Cys His Phe Pro Asn Lys Ala Met Pro Ser Ala
    50                  55                  60

Gly Thr Leu Pro Trp Val Gln Gly Ile Ile Cys Asn Ala Asn Asn Pro
65                  70                  75                  80

Cys Phe Arg Tyr Pro Thr Pro Gly Glu Ala Pro Gly Val Val Gly Asn
                85                  90                  95

Phe Asn Lys Ser Ile Val Ala Arg Leu Phe Ser Asp Ala Arg Arg Leu
            100                 105                 110

Leu Leu Tyr Ser Gln Lys Asp Thr Ser Met Lys Asp Met Arg Lys Val
        115                 120                 125

Leu Arg Thr Leu Gln Gln Ile Lys Lys Ser Ser Ser Asn Leu Lys Leu
    130                 135                 140

Gln Asp Phe Leu Val Asp Asn Glu Thr Phe Ser Gly Phe Leu Tyr His
145                 150                 155                 160

Asn Leu Ser Leu Pro Lys Ser Thr Val Asp Lys Met Leu Arg Ala Asp
                165                 170                 175

Val Ile Leu His Lys Val Phe Leu Gln Gly Tyr Gln Leu His Leu Thr
            180                 185                 190

Ser Leu Cys Asn Gly Ser Lys Ser Glu Glu Met Ile Gln Leu Gly Asp
        195                 200                 205

Gln Glu Val Ser Glu Leu Cys Gly Leu Pro Arg Glu Lys Leu Ala Ala
    210                 215                 220

Ala Glu Arg Val Leu Arg Ser Asn Met Asp Ile Leu Lys Pro Ile Leu
225                 230                 235                 240

Arg Thr Leu Asn Ser Thr Ser Pro Phe Pro Ser Lys Glu Leu Ala Glu
                245                 250                 255

Ala Thr Lys Thr Leu Leu His Ser Leu Gly Thr Leu Ala Gln Glu Leu
            260                 265                 270

Phe Ser Met Arg Ser Trp Ser Asp Met Arg Gln Glu Val Met Phe Leu
        275                 280                 285

Thr Asn Val Asn Ser Ser Ser Ser Ser Thr Gln Ile Tyr Gln Ala Val
    290                 295                 300

Ser Arg Ile Val Cys Gly His Pro Glu Gly Gly Gly Leu Lys Ile Lys
305                 310                 315                 320

Ser Leu Asn Trp Tyr Glu Asp Asn Asn Tyr Lys Ala Leu Phe Gly Gly
                325                 330                 335

Asn Gly Thr Glu Glu Asp Ala Glu Thr Phe Tyr Asp Asn Ser Thr Thr
            340                 345                 350

Pro Tyr Cys Asn Asp Leu Met Lys Asn Leu Glu Ser Ser Pro Leu Ser
        355                 360                 365

Arg Ile Ile Trp Lys Ala Leu Lys Pro Leu Leu Val Gly Lys Ile Leu
    370                 375                 380

Tyr Thr Pro Asp Thr Pro Ala Thr Arg Gln Val Met Ala Glu Val Asn
385                 390                 395                 400

Lys Thr Phe Gln Glu Leu Ala Val Phe His Asp Leu Glu Gly Met Trp
                405                 410                 415

Glu Glu Leu Ser Pro Lys Ile Trp Thr Phe Met Glu Asn Ser Gln Glu
            420                 425                 430

Met Asp Leu Val Arg Met Leu Leu Asp Ser Arg Asp Asn Asp His Phe
        435                 440                 445

Trp Glu Gln Gln Leu Asp Gly Leu Asp Trp Thr Ala Gln Asp Ile Val
    450                 455                 460

Ala Phe Leu Ala Lys His Pro Glu Asp Val Gln Ser Ser Asn Gly Ser
465                 470                 475                 480

Val Tyr Thr Trp Arg Glu Ala Phe Asn Glu Thr Asn Gln Ala Ile Arg
                485                 490                 495

Thr Ile Ser Arg Phe Met Glu Cys Val Asn Leu Asn Lys Leu Glu Pro
            500                 505                 510

Ile Ala Thr Glu Val Trp Leu Ile Asn Lys Ser Met Glu Leu Leu Asp
        515                 520                 525

Glu Arg Lys Phe Trp Ala Gly Ile Val Phe Thr Gly Ile Thr Pro Gly
    530                 535                 540

Ser Ile Glu Leu Pro His His Val Lys Tyr Lys Ile Arg Met Asp Ile
545                 550                 555                 560

Asp Asn Val Glu Arg Thr Asn Lys Ile Lys Asp Gly Tyr Trp Asp Pro
                565                 570                 575

Gly Pro Arg Ala Asp Pro Phe Glu Asp Met Arg Tyr Val Trp Gly Gly
            580                 585                 590

Phe Ala Tyr Leu Gln Asp Val Val Glu Gln Ala Ile Ile Arg Val Leu
        595                 600                 605

Thr Gly Thr Glu Lys Lys Thr Gly Val Tyr Met Gln Gln Met Pro Tyr

610                 615                 620

Pro Cys Tyr Val Asp Asp Ile Phe Leu Arg Val Met Ser Arg Ser Met
625                 630                 635                 640

Pro Leu Phe Met Thr Leu Ala Trp Ile Tyr Ser Val Ala Val Ile Ile
                645                 650                 655

Lys Gly Ile Val Tyr Glu Lys Glu Ala Arg Leu Lys Glu Thr Met Arg
            660                 665                 670

Ile Met Gly Leu Asp Asn Ser Ile Leu Trp Phe Ser Trp Phe Ile Ser
        675                 680                 685

Ser Leu Ile Pro Leu Leu Val Ser Ala Gly Leu Leu Val Val Ile Leu
    690                 695                 700

Lys Leu Gly Asn Leu Leu Pro Tyr Ser Asp Pro Ser Val Val Phe Val
705                 710                 715                 720

Phe Leu Ser Val Phe Ala Val Val Thr Ile Leu Gln Cys Phe Leu Ile
                725                 730                 735

Ser Thr Leu Phe Ser Arg Ala Asn Leu Ala Ala Ala Cys Gly Gly Ile
            740                 745                 750

Ile Tyr Phe Thr Leu Tyr Leu Pro Tyr Val Leu Cys Val Ala Trp Gln
        755                 760                 765

Asp Tyr Val Gly Phe Thr Leu Lys Ile Phe Ala Ser Leu Leu Ser Pro
    770                 775                 780

Val Ala Phe Gly Phe Gly Cys Glu Tyr Phe Ala Leu Phe Glu Glu Gln
785                 790                 795                 800

Gly Ile Gly Val Gln Trp Asp Asn Leu Phe Glu Ser Pro Val Glu Glu
                805                 810                 815

Asp Gly Phe Asn Leu Thr Thr Ser Val Ser Met Met Leu Phe Asp Thr
            820                 825                 830

Phe Leu Tyr Gly Val Met Thr Trp Tyr Ile Glu Ala Val Phe Pro Gly
        835                 840                 845

Gln Tyr Gly Ile Pro Arg Pro Trp Tyr Phe Pro Cys Thr Lys Ser Tyr
    850                 855                 860

Trp Phe Gly Glu Glu Ser Asp Glu Lys Ser His Pro Gly Ser Asn Gln
865                 870                 875                 880

Lys Arg Ile Ser Glu Ile Cys Met Glu Glu Glu Pro Thr His Leu Lys
                885                 890                 895

Leu Gly Val Ser Ile Gln Asn Leu Val Lys Val Tyr Arg Asp Gly Met
            900                 905                 910

Lys Val Ala Val Asp Gly Leu Ala Leu Asn Phe Tyr Glu Gly Gln Ile
        915                 920                 925

Thr Ser Phe Leu Gly His Asn Gly Ala Gly Lys Thr Thr Thr Met Ser
    930                 935                 940

Ile Leu Thr Gly Leu Phe Pro Pro Thr Ser Gly Thr Ala Tyr Ile Leu
945                 950                 955                 960

Gly Lys Asp Ile Arg Ser Glu Met Ser Thr Ile Arg Gln Asn Leu Gly
                965                 970                 975

Val Cys Pro Gln His Asn Val Leu Phe Asp Met Leu Thr Val Glu Glu
            980                 985                 990

His Ile Trp Phe Tyr Ala Arg Leu Lys Gly Leu Ser Glu Lys His Val
        995                 1000                1005

Lys Ala Glu Met Glu Gln Met Ala Leu Asp Val Gly Leu Pro Ser Ser
    1010                1015                1020

Lys Leu Lys Ser Lys Thr Ser Gln Leu Ser Gly Gly Met Gln Arg Lys
1025                1030                1035                1040

Leu Ser Val Ala Leu Ala Phe Val Gly Gly Ser Lys Val Val Ile Leu
                1045                1050                1055

Asp Glu Pro Thr Ala Gly Val Asp Pro Tyr Ser Arg Arg Gly Ile Trp
            1060                1065                1070

Glu Leu Leu Leu Lys Tyr Arg Gln Gly Arg Thr Ile Ile Leu Ser Thr
        1075                1080                1085

His His Met Asp Glu Ala Asp Val Leu Gly Asp Arg Ile Ala Ile Ile
    1090                1095                1100

Ser His Gly Lys Leu Cys Cys Val Gly Ser Ser Leu Phe Leu Lys Asn
1105                1110                1115                1120

Gln Leu Gly Thr Gly Tyr Tyr Leu Thr Leu Val Lys Lys Asp Val Glu
                1125                1130                1135

Ser Ser Leu Ser Ser Cys Arg Asn Ser Ser Ser Thr Val Ser Tyr Leu
            1140                1145                1150

Lys Lys Glu Asp Ser Val Ser Gln Ser Ser Ser Asp Ala Gly Leu Gly
        1155                1160                1165

Ser Asp His Glu Ser Asp Thr Leu Thr Ile Asp Val Ser Ala Ile Ser
    1170                1175                1180

Asn Leu Ile Arg Lys His Val Ser Glu Ala Arg Leu Val Glu Asp Ile
1185                1190                1195                1200

Gly His Glu Leu Thr Tyr Val Leu Pro Tyr Glu Ala Ala Lys Glu Gly
                1205                1210                1215

Ala Phe Val Glu Leu Phe His Glu Ile Asp Asp Arg Leu Ser Asp Leu
            1220                1225                1230

Gly Ile Ser Ser Tyr Gly Ile Ser Glu Thr Thr Leu Glu Glu Ile Phe
        1235                1240                1245

Leu Lys Val Ala Glu Glu Ser Gly Val Asp Ala Glu Thr Ser Asp Gly
    1250                1255                1260

Thr Leu Pro Ala Arg Arg Asn Arg Arg Ala Phe Gly Asp Lys Gln Ser
1265                1270                1275                1280

Cys Leu Arg Pro Phe Thr Glu Asp Asp Ala Ala Asp Pro Asn Asp Ser
                1285                1290                1295

Asp Ile Asp Pro Glu Ser Arg Glu Thr Asp Leu Leu Ser Gly Met Asp
            1300                1305                1310

Gly Lys Gly Ser Tyr Gln Val Lys Gly Trp Lys Leu Thr Gln Gln Gln
        1315                1320                1325

Phe Val Ala Leu Leu Trp Lys Arg Leu Leu Ile Ala Arg Arg Ser Arg
    1330                1335                1340

Lys Gly Phe Phe Ala Gln Ile Val Leu Pro Ala Val Phe Val Cys Ile
1345                1350                1355                1360

Ala Leu Val Phe Ser Leu Ile Val Pro Pro Phe Gly Lys Tyr Pro Ser
                1365                1370                1375

Leu Glu Leu Gln Pro Trp Met Tyr Asn Glu Gln Tyr Thr Phe Val Ser
            1380                1385                1390

Asn Asp Ala Pro Glu Asp Thr Gly Thr Leu Glu Leu Leu Asn Ala Leu
        1395                1400                1405

Thr Lys Asp Pro Gly Phe Gly Thr Arg Cys Met Glu Gly Asn Pro Ile
    1410                1415                1420

Pro Asp Thr Pro Cys Gln Ala Gly Glu Glu Glu Trp Thr Thr Ala Pro
1425                1430                1435                1440

Val Pro Gln Thr Ile Met Asp Leu Phe Gln Asn Gly Asn Trp Thr Met
                1445                1450                1455

Gln Asn Pro Ser Pro Ala Cys Gln Cys Ser Ser Asp Lys Ile Lys Lys
            1460                1465                1470

Met Leu Pro Val Cys Pro Pro Gly Ala Gly Gly Leu Pro Pro Pro Gln
        1475                1480                1485

Arg Lys Gln Asn Thr Ala Asp Ile Leu Gln Asp Leu Thr Gly Arg Asn
    1490                1495                1500

Ile Ser Asp Tyr Leu Val Lys Thr Tyr Val Gln Ile Ile Ala Lys Ser
1505                1510                1515                1520

Leu Lys Asn Lys Ile Trp Val Asn Glu Phe Arg Tyr Gly Gly Phe Ser
                1525                1530                1535

Leu Gly Val Ser Asn Thr Gln Ala Leu Pro Pro Ser Gln Glu Val Asn

1540                 1545                 1550

Asp Ala Thr Lys Gln Met Lys Lys His Leu Lys Leu Ala Lys Asp Ser
        1555                 1560                 1565

Ser Ala Asp Arg Phe Leu Asn Ser Leu Gly Arg Phe Met Thr Gly Leu
    1570                 1575                 1580

Asp Thr Arg Asn Asn Val Lys Val Trp Phe Asn Asn Lys Gly Trp His
1585                 1590                 1595                 1600

Ala Ile Ser Ser Phe Leu Asn Val Ile Asn Asn Ala Ile Leu Arg Ala
                1605                 1610                 1615

Asn Leu Gln Lys Gly Glu Asn Pro Ser His Tyr Gly Ile Thr Ala Phe
            1620                 1625                 1630

Asn His Pro Leu Asn Leu Thr Lys Gln Gln Leu Ser Glu Val Ala Pro
        1635                 1640                 1645

Met Thr Thr Ser Val Asp Val Leu Val Ser Ile Cys Val Ile Phe Ala
    1650                 1655                 1660

Met Ser Phe Val Pro Ala Ser Phe Val Val Phe Leu Ile Gln Glu Arg
1665                 1670                 1675                 1680

Val Ser Lys Ala Lys His Leu Gln Phe Ile Ser Gly Val Lys Pro Val
                1685                 1690                 1695

Ile Tyr Trp Leu Ser Asn Phe Val Trp Asp Met Cys Asn Tyr Val Val
            1700                 1705                 1710

Pro Ala Thr Leu Val Ile Ile Ile Phe Ile Cys Phe Gln Gln Lys Ser
        1715                 1720                 1725

Tyr Val Ser Ser Thr Asn Leu Pro Val Leu Ala Leu Leu Leu Leu Leu
    1730                 1735                 1740

Tyr Gly Trp Ser Ile Thr Pro Leu Met Tyr Pro Ala Ser Phe Val Phe
1745                 1750                 1755                 1760

Lys Ile Pro Ser Thr Ala Tyr Val Val Leu Thr Ser Val Asn Leu Phe
                1765                 1770                 1775

Ile Gly Ile Asn Gly Ser Val Ala Thr Phe Val Leu Glu Leu Phe Thr
            1780                 1785                 1790

Asp Asn Lys Leu Asn Asn Ile Asn Asp Ile Leu Lys Ser Val Phe Leu
        1795                 1800                 1805

Ile Phe Pro His Phe Cys Leu Gly Arg Gly Leu Ile Asp Met Val Lys
    1810                 1815                 1820

Asn Gln Ala Met Ala Asp Ala Leu Glu Arg Phe Gly Glu Asn Arg Phe
1825                 1830                 1835                 1840

Val Ser Pro Leu Ser Trp Asp Leu Val Gly Arg Asn Leu Phe Ala Met
                1845                 1850                 1855

Ala Val Glu Gly Val Val Phe Phe Leu Ile Thr Val Leu Ile Gln Tyr
            1860                1865                1870

Arg Phe Phe Ile Arg Pro Arg Pro Val Asn Ala Lys Leu Ser Pro Leu
        1875                1880                1885

Asn Asp Glu Asp Glu Asp Val Arg Arg Glu Arg Gln Arg Ile Leu Asp
    1890                1895                1900

Gly Gly Gly Gln Asn Asp Ile Leu Glu Ile Lys Glu Leu Thr Lys Ile
1905                1910                1915                1920

Tyr Arg Arg Lys Arg Lys Pro Ala Val Asp Arg Ile Cys Val Gly Ile
                1925                1930                1935

Pro Pro Gly Glu Cys Phe Gly Leu Leu Gly Val Asn Gly Ala Gly Lys
            1940                1945                1950

Ser Ser Thr Phe Lys Met Leu Thr Gly Asp Thr Thr Val Thr Arg Gly
        1955                1960                1965

Asp Ala Phe Leu Asn Arg Asn Ser Ile Leu Ser Asn Ile His Glu Val
    1970                1975                1980

His Gln Asn Met Gly Tyr Cys Pro Gln Phe Asp Ala Ile Thr Glu Leu
1985                1990                1995                2000

Leu Thr Gly Arg Glu His Val Glu Phe Phe Ala Leu Leu Arg Gly Val
                2005                2010                2015

Pro Glu Lys Glu Val Gly Lys Val Gly Glu Trp Ala Ile Arg Lys Leu
            2020                2025                2030

Gly Leu Val Lys Tyr Gly Glu Lys Tyr Ala Gly Asn Tyr Ser Gly Gly
        2035                2040                2045

Asn Lys Arg Lys Leu Ser Thr Ala Met Ala Leu Ile Gly Gly Pro Pro
    2050                2055                2060

Val Val Phe Leu Asp Glu Pro Thr Thr Gly Met Asp Pro Lys Ala Arg
2065                2070                2075                2080

Arg Phe Leu Trp Asn Cys Ala Leu Ser Val Val Lys Glu Gly Arg Ser
                2085                2090                2095

Val Val Leu Thr Ser His Ser Val Glu Glu Cys Glu Ala Leu Cys Thr
            2100                2105                2110

Arg Met Ala Ile Met Val Asn Gly Arg Phe Arg Cys Leu Gly Ser Val
        2115                2120                2125

Gln His Leu Lys Asn Arg Phe Gly Asp Gly Tyr Thr Ile Val Val Arg
    2130                2135                2140

Ile Ala Gly Ser Asn Pro Asp Leu Lys Pro Val Gln Asp Phe Phe Gly
2145                2150                2155                2160

Leu Ala Phe Pro Gly Ser Val Pro Lys Glu Lys His Arg Asn Met Leu
                2165                2170                2175

Gln Tyr Gln Leu Pro Ser Ser Leu Ser Ser Leu Ala Arg Ile Phe Ser
            2180                2185                2190

Ile Leu Ser Gln Ser Lys Lys Arg Leu His Ile Glu Asp Tyr Ser Val
        2195                2200                2205

Ser Gln Thr Thr Leu Asp Gln Val Phe Val Asn Phe Ala Lys Asp Gln
    2210                2215                2220

Ser Asp Asp Asp His Leu Lys Asp Leu Ser Leu His Lys Asn Gln Thr
2225                2230                2235                2240

Val Val Asp Val Ala Val Leu Thr Ser Phe Leu Gln Asp Glu Lys Val
                2245                2250                2255

Lys Glu Ser Tyr Val
            2260

<210> 102
<211> 2130
<212> PRT
<213> Homo sapiens

<400> 102
Met Ala Cys Trp Pro Gln Leu Arg Leu Leu Leu Trp Lys Asn Leu Thr
1               5                   10                  15

Phe Arg Arg Arg Gln Thr Cys Gln Leu Leu Leu Glu Val Ala Trp Pro
            20                  25                  30

Leu Phe Ile Phe Leu Ile Leu Ile Ser Val Arg Leu Ser Tyr Pro Pro
        35                  40                  45

Tyr Glu Gln His Glu Cys His Phe Pro Asn Lys Ala Met Pro Ser Ala
    50                  55                  60

Gly Thr Leu Pro Trp Val Gln Gly Ile Ile Cys Asn Ala Asn Asn Pro
65                  70                  75                  80

Cys Phe Arg Tyr Pro Thr Pro Gly Glu Ala Pro Gly Val Val Gly Asn
                85                  90                  95

Phe Asn Lys Ser Ile Val Ala Arg Leu Phe Ser Asp Ala Arg Arg Leu
            100                 105                 110

Leu Leu Tyr Ser Gln Lys Asp Thr Ser Met Lys Asp Met Arg Lys Val
        115                 120                 125

Leu Arg Thr Leu Gln Gln Ile Lys Lys Ser Ser Ser Asn Leu Lys Leu
    130                 135                 140

Gln Asp Phe Leu Val Asp Asn Glu Thr Phe Ser Gly Phe Leu Tyr His
145                 150                 155                 160

Asn Leu Ser Leu Pro Lys Ser Thr Val Asp Lys Met Leu Arg Ala Asp
                165                 170                 175

Val Ile Leu His Lys Val Phe Leu Gln Gly Tyr Gln Leu His Leu Thr
            180                 185                 190

Ser Leu Cys Asn Gly Ser Lys Ser Glu Glu Met Ile Gln Leu Gly Asp
        195                 200                 205

Gln Glu Val Ser Glu Leu Cys Gly Leu Pro Arg Glu Lys Leu Ala Ala
    210                 215                 220

Ala Glu Arg Val Leu Arg Ser Asn Met Asp Ile Leu Lys Pro Ile Leu
225                 230                 235                 240

Arg Thr Leu Asn Ser Thr Ser Pro Phe Pro Ser Lys Glu Leu Ala Glu
                245                 250                 255

Ala Thr Lys Thr Leu Leu His Ser Leu Gly Thr Leu Ala Gln Glu Leu
            260                 265                 270

Phe Ser Met Arg Ser Trp Ser Asp Met Arg Gln Glu Val Met Phe Leu
        275                 280                 285

Thr Asn Val Asn Ser Ser Ser Ser Ser Thr Gln Ile Tyr Gln Ala Val
    290                 295                 300

Ser Arg Ile Val Cys Gly His Pro Glu Gly Gly Gly Leu Lys Ile Lys
305                 310                 315                 320

Ser Leu Asn Trp Tyr Glu Asp Asn Asn Tyr Lys Ala Leu Phe Gly Gly
                325                 330                 335

Asn Gly Thr Glu Glu Asp Ala Glu Thr Phe Tyr Asp Asn Ser Thr Thr
            340                 345                 350

Pro Tyr Cys Asn Asp Leu Met Lys Asn Leu Glu Ser Ser Pro Leu Ser
        355                 360                 365

Arg Ile Ile Trp Lys Ala Leu Lys Pro Leu Leu Val Gly Lys Ile Leu
    370                 375                 380

Tyr Thr Pro Asp Thr Pro Ala Thr Arg Gln Val Met Ala Glu Val Asn
385                 390                 395                 400

Lys Thr Phe Gln Glu Leu Ala Val Phe His Asp Leu Glu Gly Met Trp
                405                 410                 415

Glu Glu Leu Ser Pro Lys Ile Trp Thr Phe Met Glu Asn Ser Gln Glu
            420                 425                 430

Met Asp Leu Val Arg Met Leu Leu Asp Ser Arg Asp Asn Asp His Phe
        435                 440                 445

Trp Glu Gln Gln Leu Asp Gly Leu Asp Trp Thr Ala Gln Asp Ile Val
    450                 455                 460

Ala Phe Leu Ala Lys His Pro Glu Asp Val Gln Ser Ser Asn Gly Ser

465                 470                 475                 480

Val Tyr Thr Trp Arg Glu Ala Phe Asn Glu Thr Asn Gln Ala Ile Arg
                485                 490                 495

Thr Ile Ser Arg Phe Met Glu Cys Val Asn Leu Asn Lys Leu Glu Pro
            500                 505                 510

Ile Ala Thr Glu Val Trp Leu Ile Asn Lys Ser Met Glu Leu Leu Asp
        515                 520                 525

Glu Arg Lys Phe Trp Ala Gly Ile Val Phe Thr Gly Ile Thr Pro Gly
    530                 535                 540

Ser Ile Glu Leu Pro His His Val Lys Tyr Lys Ile Arg Met Asp Ile
545                 550                 555                 560

Asp Asn Val Glu Arg Thr Asn Lys Ile Lys Asp Gly Tyr Trp Asp Pro
                565                 570                 575

Gly Pro Arg Ala Asp Pro Phe Glu Asp Met Arg Tyr Val Trp Gly Gly
            580                 585                 590

Phe Ala Tyr Leu Gln Asp Val Val Glu Gln Ala Ile Ile Arg Val Leu
        595                 600                 605

Thr Gly Thr Glu Lys Lys Thr Gly Val Tyr Met Gln Gln Met Pro Tyr
    610                 615                 620

Pro Cys Tyr Val Asp Asp Ile Phe Leu Arg Val Met Ser Arg Ser Met
625                 630                 635                 640

Pro Leu Phe Met Thr Leu Ala Trp Ile Tyr Ser Val Ala Val Ile Ile
                645                 650                 655

Lys Gly Ile Val Tyr Glu Lys Glu Ala Arg Leu Lys Glu Thr Met Arg
            660                 665                 670

Ile Met Gly Leu Asp Asn Ser Ile Leu Trp Phe Ser Trp Phe Ile Ser
        675                 680                 685

Ser Leu Ile Pro Leu Leu Val Ser Ala Gly Leu Leu Val Val Ile Leu
    690                 695                 700

Lys Leu Gly Asn Leu Leu Pro Tyr Ser Asp Pro Ser Val Val Phe Val
705                 710                 715                 720

Phe Leu Ser Val Phe Ala Val Val Thr Ile Leu Gln Cys Phe Leu Ile
                725                 730                 735

Ser Thr Leu Phe Ser Arg Ala Asn Leu Ala Ala Ala Cys Gly Gly Ile
            740                 745                 750

Ile Tyr Phe Thr Leu Tyr Leu Pro Tyr Val Leu Cys Val Ala Trp Gln
        755                 760                 765

Asp Tyr Val Gly Phe Thr Leu Lys Ile Phe Ala Ser Leu Leu Ser Pro
    770                 775                 780

Val Ala Phe Gly Phe Gly Cys Glu Tyr Phe Ala Leu Phe Glu Glu Gln
785                 790                 795                 800

Gly Ile Gly Val Gln Trp Asp Asn Leu Phe Glu Ser Pro Val Glu Glu
                805                 810                 815

Asp Gly Phe Asn Leu Thr Thr Ser Val Ser Met Met Leu Phe Asp Thr
            820                 825                 830

Phe Leu Tyr Gly Val Met Thr Trp Tyr Ile Glu Ala Val Phe Pro Gly
        835                 840                 845

Gln Tyr Gly Ile Pro Arg Pro Trp Tyr Phe Pro Cys Thr Lys Ser Tyr
    850                 855                 860

Trp Phe Gly Glu Glu Ser Asp Glu Lys Ser His Pro Gly Ser Asn Gln
865                 870                 875                 880

Lys Arg Ile Ser Glu Ile Cys Met Glu Glu Glu Pro Thr His Leu Lys
                885                 890                 895

Leu Gly Val Ser Ile Gln Asn Leu Val Lys Val Tyr Arg Asp Gly Met
            900                 905                 910

Lys Val Ala Val Asp Gly Leu Ala Leu Asn Phe Tyr Glu Gly Gln Ile
        915                 920                 925

Thr Ser Phe Leu Gly His Asn Gly Ala Gly Lys Thr Thr Thr Met Ser
    930                 935                 940

Ile Leu Thr Gly Leu Phe Pro Pro Thr Ser Gly Thr Ala Tyr Ile Leu
945                 950                 955                 960

Gly Lys Asp Ile Arg Ser Glu Met Ser Thr Ile Arg Gln Asn Leu Gly
                965                 970                 975

Val Cys Pro Gln His Asn Val Leu Phe Asp Met Leu Thr Val Glu Glu
            980                 985                 990

His Ile Trp Phe Tyr Ala Arg Leu Lys Gly Leu Ser Glu Lys His Val
        995                 1000                1005

Lys Ala Glu Met Glu Gln Met Ala Leu Asp Val Gly Leu Pro Ser Ser
    1010                1015                1020

Lys Leu Lys Ser Lys Thr Ser Gln Leu Ser Gly Gly Met Gln Arg Lys
1025                1030                1035                1040

Leu Ser Val Ala Leu Ala Phe Val Gly Gly Ser Lys Val Val Ile Leu
                1045                1050                1055

Asp Glu Pro Thr Ala Gly Val Asp Pro Tyr Ser Arg Arg Gly Ile Trp
            1060                1065                1070

Glu Leu Leu Leu Lys Tyr Arg Gln Gly Arg Thr Ile Ile Leu Ser Thr
        1075                1080                1085

His His Met Asp Glu Ala Asp Val Leu Gly Asp Arg Ile Ala Ile Ile
    1090                1095                1100

Ser His Gly Lys Leu Cys Cys Val Gly Ser Ser Leu Phe Leu Lys Asn
1105                1110                1115                1120

Gln Leu Gly Thr Gly Tyr Tyr Leu Thr Leu Val Lys Lys Asp Val Glu
                1125                1130                1135

Ser Ser Leu Ser Ser Cys Arg Asn Ser Ser Ser Thr Val Ser Tyr Leu
            1140                1145                1150

Lys Lys Glu Asp Ser Val Ser Gln Ser Ser Ser Asp Ala Gly Leu Gly
        1155                1160                1165

Ser Asp His Glu Ser Asp Thr Leu Thr Ile Asp Val Ser Ala Ile Ser
    1170                1175                1180

Asn Leu Ile Arg Lys His Val Ser Glu Ala Arg Leu Val Glu Asp Ile
1185                1190                1195                1200

Gly His Glu Leu Thr Tyr Val Leu Pro Tyr Glu Ala Ala Lys Glu Gly
                1205                1210                1215

Ala Phe Val Glu Leu Phe His Glu Ile Asp Asp Arg Leu Ser Asp Leu
            1220                1225                1230

Gly Ile Ser Ser Tyr Gly Ile Ser Glu Thr Thr Leu Glu Glu Ile Phe
        1235                1240                1245

Leu Lys Val Ala Glu Glu Ser Gly Val Asp Ala Glu Thr Ser Asp Gly
    1250                1255                1260

Thr Leu Pro Ala Arg Arg Asn Arg Arg Ala Phe Gly Asp Lys Gln Ser
1265                1270                1275                1280

Cys Leu Arg Pro Phe Thr Glu Asp Asp Ala Ala Asp Pro Asn Asp Ser
                1285                1290                1295

Asp Ile Asp Pro Glu Ser Arg Glu Thr Asp Leu Leu Ser Gly Met Asp
            1300                1305                1310

Gly Lys Gly Ser Tyr Gln Val Lys Gly Trp Lys Leu Thr Gln Gln Gln
        1315                1320                1325

Phe Val Ala Leu Leu Trp Lys Arg Leu Leu Ile Ala Arg Arg Ser Arg
    1330                1335                1340

Lys Gly Phe Phe Ala Gln Ile Val Leu Pro Ala Val Phe Val Cys Ile
1345                1350                1355                1360

Ala Leu Val Phe Ser Leu Ile Val Pro Pro Phe Gly Lys Tyr Pro Ser
                1365                1370                1375

Leu Glu Leu Gln Pro Trp Met Tyr Asn Glu Gln Tyr Thr Phe Val Ser
            1380                1385                1390

Asn Asp Ala Pro Glu Asp Thr Gly Thr Leu Glu Leu Leu Asn Ala Leu

1395                1400                1405

Thr Lys Asp Pro Gly Phe Gly Thr Arg Cys Met Glu Gly Asn Pro Ile
    1410                1415                1420

Pro Asp Thr Pro Cys Gln Ala Gly Glu Glu Glu Trp Thr Thr Ala Pro
1425                1430                1435                1440

Val Pro Gln Thr Ile Met Asp Leu Phe Gln Asn Gly Asn Trp Thr Met
                1445                1450                1455

Gln Asn Pro Ser Pro Ala Cys Gln Cys Ser Ser Asp Lys Ile Lys Lys
            1460                1465                1470

Met Leu Pro Val Cys Pro Pro Gly Ala Gly Gly Leu Pro Pro Pro Gln
        1475                1480                1485

Arg Lys Gln Asn Thr Ala Asp Ile Leu Gln Asp Leu Thr Gly Arg Asn
    1490                1495                1500

Ile Ser Asp Tyr Leu Val Lys Thr Tyr Val Gln Ile Ile Ala Lys Ser
1505                1510                1515                1520

Leu Lys Asn Lys Ile Trp Val Asn Glu Phe Arg Tyr Gly Gly Phe Ser
                1525                1530                1535

Leu Gly Val Ser Asn Thr Gln Ala Leu Pro Pro Ser Gln Glu Val Asn
            1540                1545                1550

Asp Ala Thr Lys Gln Met Lys Lys His Leu Lys Leu Ala Lys Asp Ser
        1555                1560                1565

Ser Ala Asp Arg Phe Leu Asn Ser Leu Gly Arg Phe Met Thr Gly Leu
    1570                1575                1580

Asp Thr Arg Asn Asn Val Lys Val Trp Phe Asn Asn Lys Gly Trp His
1585                1590                1595                1600

Ala Ile Ser Ser Phe Leu Asn Val Ile Asn Asn Ala Ile Leu Arg Ala
                1605                1610                1615

Asn Leu Gln Lys Gly Glu Asn Pro Ser His Tyr Gly Ile Thr Ala Phe
            1620                1625                1630

Asn His Pro Leu Asn Leu Thr Lys Gln Gln Leu Ser Glu Val Ala Pro
        1635                1640                1645

Met Thr Thr Ser Val Asp Val Leu Val Ser Ile Cys Val Ile Phe Ala
    1650                1655                1660

Met Ser Phe Val Pro Ala Ser Phe Val Val Phe Leu Ile Gln Glu Arg
1665                1670                1675                1680

Val Ser Lys Ala Lys His Leu Gln Phe Ile Ser Gly Val Lys Pro Val
                1685                1690                1695

Ile Tyr Trp Leu Ser Asn Phe Val Trp Asp Met Cys Asn Tyr Val Val
            1700                1705                1710

Pro Ala Thr Leu Val Ile Ile Ile Phe Ile Cys Phe Gln Gln Lys Ser
        1715                1720                1725

Tyr Val Ser Ser Thr Asn Leu Pro Val Leu Ala Leu Leu Leu Leu Leu
    1730                1735                1740

Tyr Gly Trp Ser Ile Thr Pro Leu Met Tyr Pro Ala Ser Phe Val Phe
1745                1750                1755                1760

Lys Ile Pro Ser Thr Ala Tyr Val Val Leu Thr Ser Val Asn Leu Phe
                1765                1770                1775

Ile Gly Ile Asn Gly Ser Val Ala Thr Phe Val Leu Glu Leu Phe Thr
            1780                1785                1790

Asp Asn Lys Leu Asn Asn Ile Asn Asp Ile Leu Lys Ser Val Phe Leu
        1795                1800                1805

Ile Phe Pro His Phe Cys Leu Gly Arg Gly Leu Ile Asp Met Val Lys
    1810                1815                1820

Asn Gln Ala Met Ala Asp Ala Leu Glu Arg Phe Gly Glu Asn Arg Phe
1825                1830                1835                1840

Val Ser Pro Leu Ser Trp Asp Leu Val Gly Arg Asn Leu Phe Ala Met
                1845                1850                1855

Ala Val Glu Gly Val Val Phe Phe Leu Ile Thr Val Leu Ile Gln Tyr
            1860                1865                1870

Arg Phe Phe Ile Arg Pro Arg Pro Val Asn Ala Lys Leu Ser Pro Leu
        1875                1880                1885

Asn Asp Glu Asp Glu Asp Val Arg Arg Glu Arg Gln Arg Ile Leu Asp
    1890                1895                1900

Gly Gly Gly Gln Asn Asp Ile Leu Glu Ile Lys Glu Leu Thr Lys Ile
1905                1910                1915                1920

Tyr Arg Arg Lys Arg Lys Pro Ala Val Asp Arg Ile Cys Val Gly Ile
                1925                1930                1935

Pro Pro Gly Glu Cys Phe Gly Leu Leu Gly Val Asn Gly Ala Gly Lys
            1940                1945                1950

Ser Ser Thr Phe Lys Met Leu Thr Gly Asp Thr Thr Val Thr Arg Gly
        1955                1960                1965

Asp Ala Phe Leu Asn Arg Asn Ser Ile Leu Ser Asn Ile His Glu Val
    1970                1975                1980

His Gln Asn Met Gly Tyr Cys Pro Gln Phe Asp Ala Ile Thr Glu Leu
1985                1990                1995                2000

Leu Thr Gly Arg Glu His Val Glu Phe Phe Ala Leu Leu Arg Gly Val
                2005                2010                2015

Pro Glu Lys Glu Val Gly Lys Val Gly Glu Trp Ala Ile Arg Lys Leu
        2020                2025                2030

Gly Leu Val Lys Tyr Gly Glu Lys Tyr Ala Gly Asn Tyr Ser Gly Gly
    2035                2040                2045

Asn Lys Arg Lys Leu Ser Thr Ala Met Ala Leu Ile Gly Gly Pro Pro
  2050                2055                2060

Val Val Phe Leu Asp Glu Pro Thr Thr Gly Met Asp Pro Lys Ala Arg
2065                2070                2075                2080

Arg Phe Leu Trp Asn Cys Ala Leu Ser Val Val Lys Glu Gly Arg Ser
                2085                2090                2095

Val Val Leu Thr Ser His Ser Met Glu Glu Cys Glu Ala Leu Cys Ile
        2100                2105                2110

Gly Trp Gln Ser Trp Ser Met Glu Gly Ser Gly Ala Leu Ala Val Ser
    2115                2120                2125

Ser Ile
  2130

<210> 103
<211> 603
<212> DNA
<213> Homo sapiens

<400> 103
   ttcattgcnt gacactttac tgagaccaag atagggaact tcacatacca gaccttttct 60
   cccaaggcgg aagagggctg ttcaatttct acactagagt tcggggagtt ttagaaatga 120
   gccagttgtc gaggatgaga gcagttcctg ataggctcaa ccacaatgag atgtagctgt 180
   tcagagaaag cattcttatc tataaactgg aagataatcc cggtgaaaca aagcccagcc 240
   ccaggggctt cactaactcc aggctgtgct tctcaaactt tagtgagcat aggaatcacc 300
   tgggcatctt gtgaagctgt agatttgaat tctgcaggtc ggcagagggg tctcagaatc 360
   cgcatttcca acaatgtctc cagtaatgct gatgctgctc gtccctggac cacagattgg 420
   gtagccaggt tctggcaagc tcatcccaag gctttgagat gacatcagac aaaatatgtt 480
   ctgggacatg gcttttgaga ggtcaagaaa ataagatgtt tctttctctt ctcatcccca 540
   acccttgcac tgcccttttc tcccttcccc taccctcctt tctgtcccca tccctgacgc 600
   cag 603

<210> 104
<211> 1667
<212> DNA
<213> Homo sapiens

<400> 104
gtaagttaag tggctgactg tcggaatata tagcaaggcc aaatgtccta aggccagacc 60
agtagcctgc attgggagca ggattatcat ggagttagtc attgagtttt taggtcatcg 120
acatctgatt aatgttggcc ccagtgagcc atttaagatg gtagtgggag atagcaggaa 180
agaagtgttt tcctctgtac cacagtacat gcctgagatt tgtgtgttga aaccagtggt 240
acctaacaca tttacatccc aaccttaaac tcctatgcac ttatttaccc tttaatgagc 300
ctctttactt aagtacagtg tgaggaacag cggcatcagg atcacttggg aacttgttag 360
aaatacagca acttgggccc agctcagacc tactgaatca gaatcaggag caattctctg 420
gtgtgactgt gtcacagcca ggtatcaact ggattctcat acataggaaa tgacaaacgt 480

ttatggatgg atagtctact tgtgccaggt gctgagattt gttttttgtt ttttgatttt 540
tttttaatca ctgtgacctc atttaattct caaaaaaaga tgaaaaaatg aacactcagg 600
aatgctgaca tgagattcag aatcaggggt ttggggcttc aaagtccatc ctctctttat 660
ccatgtaatg cctcccctta gagatacaac atcacagacc ttgaaggctg aaggggatat 720
aaaagctgtc tggccaagtg gtctccaagc ttgacagtgc agcagaatca cctggggata 780
ttattaaaaa taaacatact aaggtttggc ttcagggcct gtgaatcaga atttctggag 840
gtgaggcctt gaagtctgta tttctattgc atactttgga cacagtggtc tatagactag 900
agtttggaaa tgattgcgct cattcagatt ctcttctgat gtttgaattg ctgccatcat 960
atttctagtg ctctatttcc tcctgctcat tctgtcttgg ataacttatc atagtactag 1020
cctactcaaa gatttagagc cacagtcctg aaagaagcca cttgactcat tccctgtagg 1080
ttcagaataa atttcttctg cgcagtgtct gtcatagctt tttttaaatt tttttttatt 1140
tttgatgaga ctggagtttt gctcttattg cccaagctgg agtgcagtgg tgcgattttg 1200
gctcactgca acctccacct cccaggttca agcgattctc ctgcctcagc ctcccaagta 1260
gctgagatta caagcatgtg ctaccacgcc cagctaattt tgtattttta gtagagatgg 1320
gttttatcca tgttggtcag gctggtctcg agctccagac ctcaggtgat ctgcccgcct 1380
cggcctccca aagtgctggg attataggcc tgagccacag cgctcagcca taactttaat 1440
ttgaaaatga ttgtctagct tgatagctct caccactgag gaaatgttct ctggcaaaaa 1500
cggcttctct cccaggtaac tctgagaaag tgttattaag aaatgtggct tctactttct 1560
ctgtcttacg gggctaacat gccactcagt aatataataa tcgtggcagt ggtgactact 1620
ctcgtaatgt tggtgcttat aatgttctca tctctctcat tttccag 1667

<210> 105
<211> 1309
<212> DNA
<213> Homo sapiens

<400> 105
gtgacttttt actaaacttg gcccctgccg tattattact aattagagga attaaagacc 60
tacaaataac agactgaaac agtgggggaa atgccagatt atggcctgat tctgtctatt 120
ggaagtttag gatattatcc caaactagaa aagatgacga gagggactgt gaacattcag 180
ttgtcagctt caaggctgag gcagcctggt ctagaatgaa aatagaaatg gattcaacgt 240
caaattttgc cacttagtag caacttgacc aggtaactgg ttatcctttt aaagccttag 300
tttatctaaa ttgtgatatt aatgttgctc ttataagttt gtcatgagga ctaaattaaa 360
tggtgtacat agagtgcctt gggtactctc tgatggggga ctccatgata atttgtggtc 420
tcatggaggg agctctggga aggtttagga gcctgccttg gctctgcagc cttgggagag 480
ccttctagct tcccaggaca tggcagccta gtgttgaatg cttggctcag caaatgtttg 540
ttctcgtttc cttcccatca acttggtcag ttggggtctt tcagttagga gtatctcagt 600
gactttaaat ggcatgggca tgctggagtg atagtgacca tgagtttcta agaaagaagc 660
ataatttctc catatgtcat ccacaattga aatattattg ttaattgaaa aagcttctag 720
gccaggcacg gtggctcatg cctgtaatcc cagcacttta ggaggccaag gcgggtggat 780
cacttgaggt caggagtttg agaccagcct ggccaacatg gggaaaccct gtctctacta 840
aaaatacaaa ataagctggg cgtggtggtg cgtgcctgta atcccagcta cttgggaggc 900
tgaggcagga gaattgcttg aatctgggag gcggaggttg cagtgagctg agttcatgcc 960
attgcattcc agcctgggca acaagagcga aaccatctcc caaaagaaaa aaaaaagaaa 1020
gaaaaagctt ctagtttggt tacatcttgg tctataaggt ggtttgtaaa ttggtttaac 1080
ccaaggcctg gttctcatat aagtaatagg gtatttatga tggagagaag gctggaagag 1140
gcctgaacac aggcttcttt tctctagcac aaccctacaa ggccagctga ttctagggtt 1200
atttctgtcc gttccttata tcctcaggtg gatatttact ccttttgcat cattaggaat 1260
aggctcagtg ctttctttga actgattttt tgtttctttg tctctgcag 1309

<210> 106
<211> 1124
<212> DNA
<213> Homo sapiens

<400> 106

gtaagttgct gtctttctgg cacgtttagc tcagggggag gatggtgttg taggtgtctt 60
ggattgaaga aagccttggg gattgtttgt cactcacaca cttgtgggtg ccatctcact 120
gtgaggagga cagaagccct gtgaacatgt ggagcacaca ggggcacaga cagatttaga 180
ttaggcctgc tttatagagt ttctgcctag agcatcatgg ctcagtgccc agcagcccct 240
ccagaggcct ctgaaatatt tgatatactg atttccttga ggagaatcag aaatctcctg 300
caggtgtcta gggatttcaa gtaagtagtg ttgtgagggg aatacctact tgtactttcc 360
ccccaaacca gattcccgag gcttcttaag gactcaagga caatttctag gcatttagca 420
cgggactaaa aaggtcttag aggaaataag aagcgccaaa accatctctt tgcactgtat 480
ttcaacccat ttgtccttct gggttttgaa ggaacaggtg ggactgggga cagaagagtt 540
cttgaagcca gtttgtccat catggaaaat gagataggtg atgtggctac gtcagggggc 600
ccgaaggctc cttgttactg atttccgtct tttctctctg ccttttcccc aagggccagg 660
acccctggat ctctgggcag agcagacgca ggcccctata atagccctca tgctagaaag 720
gagccggagc ctgtgtataa ggccagcgca gcctactctg gacagtgcag ggttcccact 780
ctcccaactc cccatctgct tgcctccaga cccacattca cacacgagcc actgggttgg 840
aggagcatct gtgagatgaa acaccattct ttcctcaatg tctcagctat ctaactgtgt 900
gtgtaatcag gccaggtcct ccctgctggg cagaaaccat gggagttaag agattgccaa 960
catttattag aggaagctga cgtgtaactt ctctgaggca aaatttagcc ctcctttgaa 1020
caggaatttg actcagtgaa ccttgtacac actcgcactg agtctgctgc tgatgatact 1080
gtgcacccca ctgtctgggt tttaatgtca ggctgttctt ttag 1124

<210> 107
<211> 1124
<212> DNA
<213> Homo sapiens

<400> 107
gtaagttgct gtctttctgg cacgtttagc tcagggggag gatggtgttg taggtgtctt 60
ggattgaaga aagccttggg gattgtttgt cactcacaca cttgtgggtg ccatctcact 120
gtgaggagga cagaagccct gtgaacatgt ggagcacaca ggggcacaga cagatttaga 180
ttaggcctgc tttatagagt ttctgcctag agcatcatgg ctcagtgccc agcagcccct 240
ccagaggcct ctgaaatatt tgatatactg atttccttga ggagaatcag aaatctcctg 300
caggtgtcta gggatttcaa gtaagtagtg ttgtgagggg aatacctact tgtactttcc 360
ccccaaacca gattcccgag gcttcttaag gactcaagga caatttctag gcatttagca 420
cgggactaaa aaggtcttag aggaaataag aagcgccaaa accatctctt tgcactgtat 480
ttcaacccat ttgtccttct gggttttgaa ggaacaggtg ggactgggga cagaagagtt 540
cttgaagcca gtttgtccat catggaaaat gagataggtg atgtggctac gtcagggggc 600
ccgaaggctc cttgttactg atttccgtct tttctctctg ccttttcccc aagggccagg 660
acccctggat ctctgggcag agcagacgca ggcccctata atagccctca tgctagaaag 720
gagccggagc ctgtgtataa ggccagcgca gcctactctg gacagtgcag ggttcccact 780
ctcccaactc cccatctgct tgcctccaga cccacattca cacacgagcc actgggttgg 840
aggagcatct gtgagatgaa acaccattct ttcctcaatg tctcagctat ctaactgtgt 900
gtgtaatcag gccaggtcct ccctgctggg cagaaaccat gggagttaag agattgccaa 960
catttattag aggaagctga cgtgtaactt ctctgaggca aaatttagcc ctcctttgaa 1020
caggaatttg actcagtgaa ccttgtacac actcgcactg agtctgctgc tgatgatact 1080
gtgcacccca ctgtctgggt tttaatgtca ggctgttctt ttag 1124

<210> 108
<211> 264
<212> DNA
<213> Homo sapiens

<400> 108
gtgagctttt tcttagaacc cgtggagcac ctggttgagg gtcacagagg aggcgcacag 60
ggaaacactc accaatgggg gttgcattga actgaactca aaatatgtga taaaactgat 120
tttcctgatg tgggcatccc gcagccccct ccctgcccat cctggagact gtggcaagta 180
ggttttataa tactacgtta gagactgaat ctttgtcctg aaaaatagtt tgaaaggttc 240

attttcttg tttttcccc caag 264

<210> 109
<211> 274
<212> DNA
<213> Homo sapiens

<400> 109
gtaataaaga taatttcttt gggatangtg cctagtgaga aggcttgata tttattcttt 60
tgtngagtat ataaatggtg cctctaaaat aaagggaaat aaaactgagc aaaacagtgt 120
agtggaaaga atgagggctt tgaagtccga actgcattca aattctgtct ttaccattta 180
ctggttctgt gactcttggg caagttactt aactactgta agagttagtt tccctggaag 240
atctacctcc tagctttgtg ctatagatga aatg 274

<210> 110
<211> 274
<212> DNA
<213> Homo sapiens

<400> 110
gtaataaaga taatttcttt gggatangtg cctagtgaga aggcttgata tttattcttt 60
tgtngagtat ataaatggtg cctctaaaat aaagggaaat aaaactgagc aaaacagtgt 120
agtggaaaga atgagggctt tgaagtccga actgcattca aattctgtct ttaccattta 180
ctggttctgt gactcttggg caagttactt aactactgta agagttagtt tccctggaag 240
atctacctcc tagctttgtg ctatagatga aatg 274

<210> 111
<211> 604
<212> DNA
<213> Homo sapiens

<400> 111
ttcattgcnt gacactttac tgagaccaag atagggaact tcacatacca gaccttttct 60
cccaaggcgg aagagggctg ttcaatttct acactagagt tcggggagtt ttagaaatga 120
gccagttgtc gaggatgaga gcagttcctg ataggctcaa ccacaatgag atgtagctgt 180
tcagagaaag cattcttatc tataaactgg aagataatcc cggtgaaaca aagcccagcc 240
ccaggggctt cactaactcc aggctgtgct tctcaaactt tagtgagcat aggaatcacc 300
tgggcatctt gtgaagctgt agatttgaat tctgcaggtc ggcagagggg tctcagaatc 360
cgcatttcca acaatgtctc cagtaatgct gatgctgctc gtccctggac cacagattgg 420
gtagccaggt tctggcaagc tcatcccaag gctttgagat gacatcagac aaaatatgtt 480
ctgggacatg gcttttgaga ggtcaagaaa ataagatgtt tctttctctt ctcatcccca 540
acccttgcac tgcccttttc tcccttcccc taccctcctt tctgtcccca atccctgacg 600
ccag 604

<210> 112
<211> 1667
<212> DNA
<213> Homo sapiens

<400> 112
gtaagttaag tggctgactg tcagaatata tagcaaggcc aaatgtccta aggccagacc 60
agtagcctgc attgggagca ggattatcat ggagttagtc attgagtttt taggtcatcg 120
acatctgatt aatgttggcc ccagtgagcc atttaagatg gtagtgggag atagcaggaa 180
agaagtgttt tcctctgtac cacagtacat gcctgagatt tgtgtgttga aaccagtggt 240

acctaacaca tttacatccc aaccttaaac tcctatgcac ttatttaccc tttaatgagc 300
ctctttactt aagtacagtg tgaggaacag cggcatcagg atcacttggg aacttgttag 360
aaatacagca acttgggccc agctcagacc tactgaatca gaatcaggag caattctctg 420
gtgtgactgt gtcacagcca ggtatcaact ggattctcat acataggaaa tgacaaacgt 480
ttatggatgg atagtctact tgtgccaggt gctgagattt gttttttgtt ttttgatttt 540
tttttaatca ctgtgacctc atttaattct caaaaaaaga tgaaaaaatg aacactcagg 600
aatgctgaca tgagattcag aatcaggggt ttggggcttc aaagtccatc ctctctttat 660
ccatgtaatg cctcccctta gagatacaac atcacagacc ttgaaggctg aaggggatat 720
aaaagctgtc tggccaagtg gtctccaagc ttgacagtgc agcagaatca cctggggata 780
ttattaaaaa taaacatact aaggtttggc ttcagggcct gtgaatcaga atttctggag 840
gtgaggcctt gaagtctgta tttctattgc atactttgga cacagtggtc tatagactag 900
agtttggaaa tgattgcgct cattcagatt ctcttctgat gtttgaattg ctgccatcat 960
atttctagtg ctctatttcc tcctgctcat tctgtcttgg ataacttatc atagtactag 1020
cctactcaaa gatttagagc cacagtcctg aaagaagcca cttgactcat tccctgtagg 1080
ttcagaataa atttcttctg cgcagtgtct gtcatagctt tttttaaatt tttttttatt 1140
tttgatgaga ctggagtttt gctcttattg cccaagctgg agtgcagtgg tgcgattttg 1200
gctcactgca acctccacct cccaggttca agcgattctc ctgcctcagc ctcccaagta 1260
gctgagatta caagcatgtg ctaccacgcc cagctaattt tgtattttta gtagagatgg 1320
gttttatcca tgttggtcag gctggtctcg agctccagac ctcaggtgat ctgcccgcct 1380
cggcctccca aagtgctggg attataggcc tgagccacag cgctcagcca taactttaat 1440
ttgaaaatga ttgtctagct tgatagctct caccactgag gaaatgttct ctggcaaaaa 1500
cggcttctct cccaggtaac tctgagaaag tgttattaag aaatgtggct tctactttct 1560
ctgtcttacg gggctaacat gccactcagt aatataataa tcgtggcagt ggtgactact 1620
ctcgtaatgt tggtgcttat aatgttctca tctctctcat tttccag 1667

<210> 113
<211> 1309
<212> DNA
<213> Homo sapiens

<400> 113
gtgacttttt actaaacttg gcccctgcct tattattact aattagagga attaaagacc 60
tacaaataac agactgaaac agtgggggaa atgccagatt atggcctgat tctgtctatt 120
ggaagtttag gatattatcc caaactagaa aagatgacga gagggactgt gaacattcag 180
ttgtcagctt caaggctgag gcagcctggt ctagaatgaa aatagaaatg gattcaacgt 240
caaattttgc cacttagtag caacttgacc aggtaactgg ttatcctttt aaagccttag 300
tttatctaaa ttgtgatatt aatgttgctc ttataagttt gtcatgagga ctaaattaaa 360
tggtgtacat agagtgcctt gggtactctc tgatggggga ctccatgata atttgtggtc 420
tcatggaggg agctctggga aggtttagga gcctgccttg gctctgcagc cttgggagag 480
ccttctagct tcccaggaca tggcagccta gtgttgaatg cttggctcag caaatgtttg 540
ttctcgtttc cttcccatca acttggtcag ttggggtctt tcagttagga gtatctcagt 600
gactttaaat ggcatgggca tgctggagtg atagtgacca tgagtttcta agaaagaagc 660
ataatttctc catatgtcat ccacaattga aatattattg ttaattgaaa aagcttctag 720
gccaggcacg gtggctcatg cctgtaatcc cagcacttta ggaggccaag gcgggtggat 780
cacttgaggt caggagtttg agaccagcct ggccaacatg gggaaaccct gtctctacta 840
aaaatacaaa ataagctggg cgtggtggtg cgtgcctgta atcccagcta cttgggaggc 900
tgaggcagga gaattgcttg aatctgggag gcggaggttg cagtgagctg agttcatgcc 960
attgcattcc agcctgggca acaagagcga aaccatctcc caaaagaaaa aaaaaagaaa 1020
gaaaaagctt ctagtttggt tacatcttgg tctataaggt ggtttgtaaa ttggtttaac 1080
ccaaggcctg gttctcatat aagtaatagg gtatttatga tggagagaag gctggaagag 1140
gcctgaacac aggcttcttt tctctagcac aaccctacaa ggccagctga ttctagggtt 1200
atttctgtcc gttccttata tcctcaggtg gatatttact ccttttgcat cattaggaat 1260
aggctcagtg ctttctttga actgattttt tgtttctttg tctctgcag 1309

<210> 114
<211> 1124

<212> DNA
<213> Homo sapiens

<400> 114
gtaagttgct gtctttctgg cacgtttagc tcagggggag gatggtgttg taggtgtctt 60
ggattgaaga aagccttggg gattgtttgt cactcacaca cttgtgggtg ccatctcact 120
gtgaggagga cagaagccct gtgaacatgt ggagcacaca ggggcacaga cagatttaga 180
ttaggcctgc tttatagagt ttctgcctag agcatcatgg ctcagtgccc agcagcccct 240
ccagaggcct ctgaaatatt tgatatactg atttccttga ggagaatcag aaatctcctg 300
caggtgtcta gggatttcaa gtaagtagtg ttgtgagggg aatacctact tgtactttcc 360
ccccaaacca gattcccgag gcttcttaag gactcaagga caatttctag gcatttagca 420
cgggactaaa aaggtcttag aggaaataag aagcgccaaa accatctctt tgcactgtat 480
ttcaacccat ttgtccttct gggttttgaa ggaacaggtg ggactgggga cagaagagtt 540
cttgaagcca gtttgtccat catggaaaat gagataggtg atgtggctac gtcagggggc 600
ccgaaggctc cttgttactg atttccgtct tttctctctg ccttttcccc aagggccagg 660
acccctggat ctctgggcag agcagacgca ggcccctata atagccctca tgctagaaag 720
gagccggagc ctgtgtataa ggccagcgca gcctactctg gacagtgcag ggttcccact 780
ctcccaactc cccatctgct tgcctccaga cccacattca cacacgagcc actgggttgg 840
aggagcatct gtgagatgaa acaccattct ttcctcaatg tctcagctat ctaactgtgt 900
gtgtaatcag gccaggtcct ccctgctggg cagaaaccat gggagttaag agattgccaa 960
catttattag aggaagctga catgtaactt ctctgaggca aaatttagcc ctcctttgaa 1020
caggaatttg actcagtgaa ccttgtacac actcgcactg agtctgctgc tgatgatact 1080
gtgcacccca ctgtctggct tttaatgtca ggctgttctt ttag 1124


<210> 115
<211> 1124
<212> DNA
<213> Homo sapiens

<400> 115
gtaagttgct gtctttctgg cacgtttagc tcagggggag gatggtgttg taggtgtctt 60
ggattgaaga aagccttggg gattgtttgt cactcacaca cttgtgggtg ccatctcact 120
gtgaggagga cagaagccct gtgaacatgt ggagcacaca ggggcacaga cagatttaga 180
ttaggcctgc tttatagagt ttctgcctag agcatcatgg ctcagtgccc agcagcccct 240
ccagaggcct ctgaaatatt tgatatactg atttccttga ggagaatcag aaatctcctg 300
caggtgtcta gggatttcaa gtaagtagtg ttgtgagggg aatacctact tgtactttcc 360
ccccaaacca gattcccgag gcttcttaag gactcaagga caatttctag gcatttagca 420
cgggactaaa aaggtcttag aggaaataag aagcgccaaa accatctctt tgcactgtat 480
ttcaacccat ttgtccttct gggttttgaa ggaacaggtg ggactgggga cagaagagtt 540
cttgaagcca gtttgtccat catggaaaat gagataggtg atgtggctac gtcagggggc 600
ccgaaggctc cttgttactg atttccgtct tttctctctg ccttttcccc aagggccagg 660
acccctggat ctctgggcag agcagacgca ggcccctata atagccctca tgctagaaag 720
gagccggagc ctgtgtataa ggccagcgca gcctactctg gacagtgcag ggttcccact 780
ctcccaactc cccatctgct tgcctccaga cccacattca cacacgagcc actgggttgg 840
aggagcatct gtgagatgaa acaccattct ttcctcaatg tctcagctat ctaactgtgt 900
gtgtaatcag gccaggtcct ccctgctggg cagaaaccat gggagttaag agattgccaa 960
catttattag aggaagctga cgtgtaactt ctctgaggca aaatttagcc ctcctttgaa 1020
caggaatttg actcagtgaa ccttgtacac actcgcactg agtctgctgc tgatgatact 1080
gtgcacccca ctgtctggct tttaatgtca ggctgttctt ttag 1124


<210> 116
<211> 264
<212> DNA
<213> Homo sapiens

<400> 116

gtgagctttt tcttagaacc cgtggagcac ctggttgagg gtcacagagg aggcgcacag 60
ggaaacactc accaatgggg gttgcattga actgaactca aaatatgtga taaaactgat 120
tttcctgatg tgggcatccc gcagctccct ccctgcccat cctggagact gtggcaagta 180
ggttttataa tactacgtta gagactgaat ctttgtcctg aaaaatagtt tgaaaggttc 240
atttttcttg tttttcccc caag 264

<210> 117
<211> 274
<212> DNA
<213> Homo sapiens

<400> 117
gtaataaaga tagtttcttt gggatangtg cctagtgaga aggcttgata tttattcttt 60
tgtngagtat ataaatggtg cctctaaaat aaagggaaat aaaactgagc aaaacagtgt 120
agtggaaaga atgagggctt tgaagtccga actgcattca aattctgtct ttaccattta 180
ctggttctgt gactcttggg caagttactt aactactgta agagttagtt tccctggaag 240
atctacctcc tagctttgtg ctatagatga aatg 274

<210> 118
<211> 274
<212> DNA
<213> Homo sapiens

<400> 118
gtaataaaga taatttcttt gggatangtg cctagtgaga aggcttgata tttattcttt 60
tgtngagtat ataaatggtg cctctaacat aaagggaaat aaaactgagc aaaacagtgt 120
agtggaaaga atgagggctt tgaagtccga actgcattca aattctgtct ttaccattta 180
ctggttctgt gactcttggg caagttactt aactactgta agagttagtt tccctggaag 240
atctacctcc tagctttgtg ctatagatga aatg 274

<210> 119
<211> 22
<212> DNA
<213> Artificial Sequence

<220>
<223> Description of Artificial Sequence: PCR Primer
      Oligonucleotide

<400> 119
cagtgttcta tcttatttcc ac 22

<210> 120
<211> 21
<212> DNA
<213> Artificial Sequence

<220>
<223> Description of Artificial Sequence: PCR Primer
      Oligonucleotide

<400> 120
aatttagccc acgttaattg c 21

<210> 121
<211> 20
<212> DNA
<213> Artificial Sequence

<220>
<223> Description of Artificial Sequence: PCR Primer
      Oligonucleotide

<400> 121
gagcctcaaa atcgcttcag                                                  20


<210> 122
<211> 21
<212> DNA
<213> Artificial Sequence

<220>
<223> Description of Artificial Sequence: PCR Primer
      Oligonucleotide

<400> 122
tgatggatac cccaaatacc c                                                21


<210> 123
<211> 21
<212> DNA
<213> Artificial Sequence

<220>
<223> Description of Artificial Sequence: PCR Primer
      Oligonucleotide

<400> 123
tgggtgtgta gaccaatgga g                                                21


<210> 124
<211> 18
<212> DNA
<213> Artificial Sequence

<220>
<223> Description of Artificial Sequence: PCR Primer
      Oligonucleotide

<400> 124
aatgggaaca agccccag                                                    18


<210> 125
<211> 21
<212> DNA
<213> Artificial Sequence

<220>
<223> Description of Artificial Sequence: PCR Primer
      Oligonucleotide

<400> 125
gatgctcccc tatctcattc c                                                21


<210> 126
<211> 20
<212> DNA
<213> Artificial Sequence

<220>
<223> Description of Artificial Sequence: PCR Primer
      Oligonucleotide

<400> 126
actacctccc tccccttcac                                                  20


<210> 127
<211> 20
<212> DNA
<213> Artificial Sequence

<220>
<223> Description of Artificial Sequence: PCR Primer
      Oligonucleotide

<400> 127
ccaaaagact tcaaggaccc                                                  20


<210> 128
<211> 18
<212> DNA
<213> Artificial Sequence

<220>
<223> Description of Artificial Sequence: PCR Primer
      Oligonucleotide

<400> 128
gcctcacatt ccgaaagc                                                    18


<210> 129
<211> 22
<212> DNA
<213> Artificial Sequence

<220>
<223> Description of Artificial Sequence: PCR Primer
      Oligonucleotide

<400> 129
taaactcaag attgtgaacg ag                                               22

<210> 130
<211> 20
<212> DNA
<213> Artificial Sequence

<220>
<223> Description of Artificial Sequence: PCR Primer
      Oligonucleotide

<400> 130
agacacaact tacctcctgg                                                    20


<210> 131
<211> 22
<212> DNA
<213> Artificial Sequence

<220>
<223> Description of Artificial Sequence: PCR Primer
      Oligonucleotide

<400> 131
aggctttgag atgacatcag ac                                                 22


<210> 132
<211> 19
<212> DNA
<213> Artificial Sequence

<220>
<223> Description of Artificial Sequence: PCR Primer
      Oligonucleotide

<400> 132
gaactaaggg agggatggg                                                     19


<210> 133
<211> 22
<212> DNA
<213> Artificial Sequence

<220>
<223> Description of Artificial Sequence: PCR Primer
      Oligonucleotide

<400> 133
ttttacagag gagccaactg ag                                                 22


<210> 134
<211> 19
<212> DNA
<213> Artificial Sequence

<220>
<223> Description of Artificial Sequence: PCR Primer
      Oligonucleotide

<400> 134
tatacccacg actggggag                                                19


<210> 135
<211> 22
<212> DNA
<213> Artificial Sequence

<220>
<223> Description of Artificial Sequence: PCR Primer
      Oligonucleotide

<400> 135
cccagagtga aaacctcaag tc                                            22


<210> 136
<211> 20
<212> DNA
<213> Artificial Sequence

<220>
<223> Description of Artificial Sequence: PCR Primer
      Oligonucleotide

<400> 136
tgctcactac aatttccccc                                               20


<210> 137
<211> 274
<212> DNA
<213> Homo sapiens

<400> 137
gtaataaaga taatttcttt gggatangtg cctagtgaga aggcttgata tttattcttt 60
tgtngagtat ataaatggtg cctctaaaat aaagggaaat aaaactgagc aaaacagtgt 120
agtggaaaga atgagggctt tgaagtccga actgcattca aattctgtct ttaccattta 180
ctggttctgt gactcttggg caagttactt aactactgta agagttagtt tccctggaag 240
atctacctcc tagctttgtg ctatagatga aatg                              274


<210> 138
<211> 22
<212> DNA
<213> Artificial Sequence

<220>
<223> Description of Artificial Sequence: PCR Primer
      Oligonucleotide

<400> 138

gcaacagatt acctccatga ag                                              22

<210> 139
<211> 21
<212> DNA
<213> Artificial Sequence

<220>
<223> Description of Artificial Sequence:
      Oligonucleotide Primer

<400> 139
ccaacaactc cgggagcctc c                                               21

<210> 140
<211> 23
<212> DNA
<213> Artificial Sequence

<220>
<223> Description of Artificial Sequence:
      Oligonucleotide Primer

<400> 140
agcggataac aatttcacac agg                                             23

<210> 141
<211> 19
<212> DNA
<213> Artificial Sequence

<220>
<223> Description of Artificial Sequence: PCR Primer
      Oligonucleotide

<400> 141
tcctgcctgc ctgagaaac                                                  19

<210> 142
<211> 20
<212> DNA
<213> Artificial Sequence

<220>
<223> Description of Artificial Sequence: PCR Primer
      Oligonucleotide

<400> 142
ccctcattga catggtggtc                                                 20

<210> 143
<211> 21
<212> DNA

<213> Artificial Sequence

<220>
<223> Description of Artificial Sequence: PCR Primer
      Oligonucleotide

<400> 143
ttgaggtagt tacgtgttag g                                                21


<210> 144
<211> 22
<212> DNA
<213> Artificial Sequence

<220>
<223> Description of Artificial Sequence: PCR Primer
      Oligonucleotide

<400> 144
cttcttttct cacctatttc tg                                               22


<210> 145
<211> 21
<212> DNA
<213> Artificial Sequence

<220>
<223> Description of Artificial Sequence: PCR Primer
      Oligonucleotide

<400> 145
gcagtatctt atcaaacatc c                                                21


<210> 146
<211> 19
<212> DNA
<213> Artificial Sequence

<220>
<223> Description of Artificial Sequence: PCR Primer
      Oligonucleotide

<400> 146
gatgccaaag gagactagg                                                   19


<210> 147
<211> 18
<212> DNA
<213> Artificial Sequence

<220>
<223> Description of Artificial Sequence: PCR Primer
      Oligonucleotide

<400> 147
tcccttctcc ctgctttg                                                    18


<210> 148
<211> 19
<212> DNA
<213> Artificial Sequence

<220>
<223> Description of Artificial Sequence: PCR Primer
      Oligonucleotide

<400> 148
agcttccctc tgcttttcc                                                   19


<210> 149
<211> 21
<212> DNA
<213> Artificial Sequence

<220>
<223> Description of Artificial Sequence: PCR Primer
      Oligonucleotide

<400> 149
cagaatatgt gagtttcctg c                                                21


<210> 150
<211> 22
<212> DNA
<213> Artificial Sequence

<220>
<223> Description of Artificial Sequence: PCR Primer
      Oligonucleotide

<400> 150
aatgatcgca tattctactt gg                                               22


<210> 151
<211> 20
<212> DNA
<213> Artificial Sequence

<220>
<223> Description of Artificial Sequence: PCR Primer
      Oligonucleotide

<400> 151
acactgaaca gcatcatccc                                                  20


<210> 152
<211> 22

<212> DNA
<213> Artificial Sequence

<220>
<223> Description of Artificial Sequence: PCR Primer
      Oligonucleotide

<400> 152
aagccctcat tctttccact ac                                          22


<210> 153
<211> 23
<212> DNA
<213> Artificial Sequence

<220>
<223> Description of Artificial Sequence:
      Oligonucleotide Primer

<400> 153
cccagtcacg acgttgtaaa acg                                         23


<210> 154
<211> 20
<212> DNA
<213> Artificial Sequence

<220>
<223> Description of Artificial Sequence:
      Oligonucleotide Primer

<400> 154
taatacgact cactataggg                                             20


<210> 155
<211> 22
<212> DNA
<213> Artificial Sequence

<220>
<223> Description of Artificial Sequence:
      Oligonucleotide Primer

<400> 155
gctcagaaac ttcttggtca cc                                          22


<210> 156
<211> 25
<212> DNA
<213> Artificial Sequence

<220>
<223> Description of Artificial Sequence:
      Oligonucleotide Primer

<400> 156
cttgtggaga atgacatcag ccctc 25